# EUROPEAN PATENT SPECIFICATION

(11) **EP 4 257 588 B1**
(45) Date of publication and mention of the grant of the patent: **04.11.2026**
(21) Application number: 21900085.8
(22) Date of filing: 03.12.2021
(51) Int. Cl.: C07D 471/14, C07D 493/14, C07C 13/60, A61K 31/4353, A61K 31/438, A61K 31/352, A61P 11/06, A61P 11/08, A61P 19/02, A61P 3/10, A61P 37/00, A61P 13/12, C07D 267/10, C07D 309/14, C07D 405/12, C07D 407/12, C07D 407/14, C07D 413/12, C07D 413/14, C07D 491/052, C07D 513/04

(54) **SMALL MOLECULE INHIBITOR OF CATHEPSIN C AND MEDICINAL USE THEREOF**
KLEINMOLEKÜLIGER CATHEPSIN-C-HEMMER UND MEDIZINISCHE VERWENDUNG DAVON
INHIBITEUR À PETITES MOLÉCULES DE LA CATHEPSINE C ET SON UTILISATION MÉDICINALE

(30) Priority: 04.12.2020 CN 202011408286; 21.12.2020 CN 202011517041; 01.04.2021 CN 202110357042; 27.04.2021 CN 202110458563; 09.08.2021 CN 202110910610
(43) Date of publication of application: 11.10.2023
(60) Divisional application: 26208049.2
(73) Proprietor: Jiangsu Hengrui Pharmaceuticals Co., Ltd., Lianyungang, Jiangsu 222047 (CN)
(72) Inventor: HAO, Xin, Shanghai 201210 (CN); GONG, Shuyi, Shanghai 201210 (CN); SHEN, Defeng, Shanghai 201210 (CN); DU, Xinming, Shanghai 201210 (CN)
(74) Representative: EP&C
(86) International application number: PCT/CN2021/135202
(87) International publication number: WO 2022/117059

(56) References cited:
- EP-B1- 3 097 086
- WO-A1-2010/128324
- WO-A1-2013/041497
- WO-A1-2014/140075
- WO-A1-2016/038007
- CN-A- 103 814 028
- CN-A- 105 026 395
- CN-A- 105 980 367
- CN-A- 107 074 870
- DATABASE REGISTRY 7 July 2021 (2021-07-07), ANONYMOUS: "2-Azabicyclo[2.2.1]heptane-3-carboxamide, N-[(1R)-1-cyano-2-[5,6-dihydro-6-oxo-3-(trifluoromethyl)-8- phenanthridinyl]ethyl]-, (1S,3R,4R)-rel- (CA INDEX NAME)", XP055937159, retrieved from STN Database accession no. 2649958-05-2

## Description

### FIELD OF THE INVENTION

The present disclosure belongs to the field of medicine, and relates to a small molecule inhibitor of cathepsin C.

### BACKGROUND OF THE INVENTION

Cathepsins are a class of proteolytic enzymes widely present in lysosomes of various tissue cells. According to their structure and catalytic type, cathepsins are divided into three classes: serine proteases (cathepsins A and G), aspartic proteases (cathepsins D and E), and cysteine proteases. Cysteine proteases are the largest family of cathepsins and include 11 proteases: cathepsins B, C, F, H, K, L, O, S, W, V, and Z.

Cathepsin C is also known as dipeptidyl peptidase I or "DPP1". DPP1 is constitutively expressed in many tissues with the highest levels in the lung, kidney, liver, and spleen. Several recently published studies have described the role played by cathepsin C in certain inflammatory processes. For example, Adkison et al., J Clin Invest. 2002 Feb; 109(3):363-71; Tinh et al., Archives of Biochemistry and Biophysics. 2002 403:160-170, it can be seen from these studies that cathepsin C is co-expressed in granules with certain serine proteases, and functions to process the precursor forms of these proteases into active forms, which are then released from inflammatory cell granules recruited to sites of inflammation. Once activated, these proteases have numerous functions, including the degradation of various extracellular matrix components, which together can propagate tissue damage and chronic inflammation.

WO 2004/110988 relates to certain nitrile derivatives and the use thereof as DPP1 inhibitors.

WO 2009/074829 relates to peptidyl nitriles and the use thereof as DPP1 inhibitors.

WO 2010/128324 relates to α-aminoamide nitriles and the use thereof as DPP1 inhibitors.

WO 2012/119941 relates to peptidyl nitrile compounds and the use thereof as DPP1 inhibitors.

WO 2013/041497 relates to N-[1-cyano-2-(phenyl)ethyl]-2-azabicyclo[2.2.1]heptane-3-carboxamide and the use thereof as DPP1 inhibitor.

WO 2001/096285 and WO 2003/048123 relate to β-aminoamide nitriles having inhibitory activity against cysteine proteases.

WO 2015/110826 relates to α-aminoamide nitriles and the use thereof as DPP1 inhibitors.

WO2014140075 relates to 2-Aza-bicyclo[2.2.1]heptane-3-carboxylic acid (benzyl-cyano-methyl)- amides of formula (1) and their use as inhibitors of Cathepsin C, pharmaceutical compositions containing the same, and methods of using the same as agents for treatment and/or prevention of diseases connected with dipeptidyl peptidase I activity, e.g. respiratory diseases.

EP3097086 relates to certain (2S)-N-[(1S)-1-cyano-2-phenylethyl]-1,4-oxazepane-2-carboxamide compounds (including pharmaceutically acceptable salts thereof), that inhibit dipeptidyl peptidase 1 (DPP1) activity, to their utility in treating and/or preventing clinical conditions including respiratory diseases, such as asthma and chronic obstructive pulmonary disease (COPD), to their use in therapy, to pharmaceutical compositions containing them and to processes for preparing such compounds.

WO2010128324 relates to nitrile compounds, processes for their preparation, pharmaceutical compositions containing them and their use in therapy.

However, the amide nitrile compound of formula I of the present disclosure is not disclosed in any literature.

### SUMMARY OF THE INVENTION

The invention is defined in the appended claims. Embodiments not encompassed by the claims are provided for reference purposes. Any references in the description to methods of treatment refer to the compounds or compositions of the invention for use in a method of treatment of the human (or animal) body by therapy.

In the first aspect, the present disclosure provides a compound of formula I or a pharmaceutically acceptable salt thereof 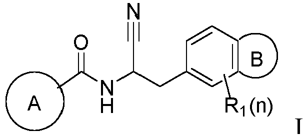 wherein:
ring A is selected from the group consisting of heterocycloalkyl, heteroaryl and aryl, and the heterocycloalkyl, heteroaryl, and aryl are each independently optionally substituted by one or more substituent(s) selected from the group consisting of deuterium, halogen, hydroxy, cyano, nitro, amino, acyl, amido, oxo, alkyl and alkoxy, and the alkyl and alkoxy are each independently optionally substituted by one or more R₃ₐ;
ring B is a cycloalkyl or heterocycloalkyl, and the cycloalkyl or heterocycloalkyl is optionally substituted by one or more substituent(s) selected from the group consisting of hydrogen, deuterium, halogen, hydroxy, cyano, amino, nitro, acyl, amido, oxo, alkoxy, alkyl, alkenyloxy, alkynyloxy, 3- to 20-membered cycloalkyl, 3-- to 20-membered heterocycloalkyl, aryl, heteroaryl, cycloalkoxy, heterocycloalkoxy, and cycloalkenyloxy, and/or the cycloalkyl or heterocycloalkyl is fused with an aryl or heteroaryl, and the alkyl, alkoxy, alkenyloxy, alkynyloxy, 3- to 20-membered cycloalkyl, 3- to 20-membered heterocycloalkyl, aryl, heteroaryl, cycloalkoxy, cycloalkenyloxy or fused ring is optionally substituted by one or more R_{3b};
R₁ is independently selected from the group consisting of halogen, hydroxy, oxo, nitro, cyano, alkyl, cycloalkyl, amino, amido, acyl, alkoxy, alkenyloxy, alkynyloxy, and cycloalkoxy;
R₃ₐ is independently selected from the group consisting of hydrogen, halogen, deuterium, hydroxy, oxo, nitro, cyano, amino, acyl, amido, C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl, and the C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl are each independently optionally substituted by one or more substituent(s) selected from the group consisting of halogen, deuterium, hydroxy, oxo, nitro and cyano;
R_{3b} is independently selected from the group consisting of hydrogen, halogen, deuterium, hydroxy, oxo, nitro, cyano, amino, acyl, amido, C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl, 3- to 6-membered heteroaryl, methanesulfonyl and 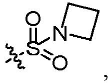 and the C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl are each independently optionally substituted by one or more substituent(s) selected from the group consisting of halogen, deuterium, hydroxy, oxo, nitro and cyano; preferably, R_{3b} is independently selected from the group consisting of hydrogen, halogen, deuterium, hydroxy, oxo, nitro, cyano, amino, acyl, amido, C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3-to 6-membered heterocycloalkoxy, C₃₋₈ cycloalkenyloxy, 5- to 6-membered aryl and 3- to 6-membered heteroaryl, and the C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl are each independently optionally substituted by one or more substituent(s) selected from the group consisting of halogen, deuterium, hydroxy, oxo, nitro and cyano;
n is an integer selected from the group consisting of 0 to 3; and preferably, n is an integer selected from the group consisting of 1 to 3.

In some embodiments, in the compound of formula I or the pharmaceutically acceptable salt thereof, ring B is a 3- to 15-membered cycloalkyl or 3- to 15-membered heterocycloalkyl comprising 1 to 3 heteroatom(s), and the 3- to 15-membered cycloalkyl or 3- to 15-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl, C₁₋₆ alkoxy, C₂₋₆ alkenyloxy, C₂₋₆ alkynyloxy, 3- to 20-membered cycloalkyl, 3- to 20-membered heterocycloalkyl, C₆₋₈ aryl, heteroaryl, cycloalkoxy, heterocycloalkoxy and cycloalkenyloxy, and/or the 3- to 15-membered cycloalkyl or 3- to 15-membered heterocycloalkyl comprising 1 to 3 heteroatom(s) is fused with an aryl or heteroaryl, and the alkyl, alkoxy, alkenyloxy, alkynyloxy, 3- to 20-membered cycloalkyl, 3- to 20-membered heterocycloalkyl, aryl, heteroaryl, cycloalkoxy, cycloalkenyloxy or fused ring is optionally substituted by one or more R_{3b}, and R_{3b} is as defined in formula I.

In other embodiments, in the compound of formula I or the pharmaceutically acceptable salt thereof, ring B is a 3- to 10-membered cycloalkyl or 3- to 10-membered heterocycloalkyl comprising 1 to 3 heteroatom(s), and the 3- to 10-membered cycloalkyl or 3- to 10-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl, C₁₋₆ alkoxy, C₂₋₆ alkenyloxy, C₂₋₆ alkynyloxy, 3- to 20-membered cycloalkyl, 3- to 20-membered heterocycloalkyl, C₆₋₈ aryl, heteroaryl, cycloalkoxy, heterocycloalkoxy and cycloalkenyloxy, and/or the 3- to 10-membered cycloalkyl or 3- to 10-membered heterocycloalkyl comprising 1 to 3 heteroatom(s) is fused with a C₆₋₈ aryl or heteroaryl, and the alkyl, alkoxy, alkenyloxy, alkynyloxy, 3- to 20-membered cycloalkyl, 3- to 20-membered heterocycloalkyl, C₆₋₈ aryl, heteroaryl, cycloalkoxy, cycloalkenyloxy or fused ring is optionally substituted by one or more R_{3b}, and R_{3b} is as defined in formula I.

In other embodiments, in the compound of formula I or the pharmaceutically acceptable salt thereof, ring B is a 3- to 10-membered cycloalkyl or 3- to 10-membered heterocycloalkyl comprising 1 to 3 heteroatom(s), and the 3- to 10-membered cycloalkyl or 3- to 10-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl, C₁₋₆ alkoxy, 3- to 10-membered cycloalkyl, 3- to 10-membered heterocycloalkyl, C₆₋₈ aryl, 5-to 8-membered heteroaryl comprising 1 to 3 heteroatom(s) and cycloalkoxy, and/or the 3- to 10-membered cycloalkyl or 3- to 10-membered heterocycloalkyl comprising 1 to 3 heteroatom(s) is fused with a C₆₋₈ aryl or 5- to 8-membered heteroaryl comprising 1 to 3 heteroatom(s), and the C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) or fused ring is optionally substituted by one or more R_{3b}, and R_{3b} is as defined in formula I.

In other embodiments, in the compound of formula I or the pharmaceutically acceptable salt thereof, ring B is a 3- to 8-membered cycloalkyl or 3- to 8-membered heterocycloalkyl comprising 1 to 3 heteroatom(s), and the 3- to 8-membered cycloalkyl or 3- to 8-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl, C₁₋₆ alkoxy, 3- to 10-membered cycloalkyl, 3- to 10-membered heterocycloalkyl, C₆₋₈ aryl, 5-to 8-membered heteroaryl comprising 1 to 3 heteroatom(s) and cycloalkoxy, and/or the 3- to 8-membered cycloalkyl or 3- to 8-membered heterocycloalkyl comprising 1 to 3 heteroatom(s) is fused with a C₆₋₈ aryl or 5- to 8-membered heteroaryl comprising 1 to 3 heteroatom(s), and the C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) or fused ring is optionally substituted by one to three R_{3b}, and R_{3b} is as defined in formula I.

In other embodiments, in the compound of formula I or the pharmaceutically acceptable salt thereof, ring B is a 5- to 8-membered cycloalkyl or 5- to 8-membered heterocycloalkyl comprising 1 to 3 heteroatom(s), and the 5- to 8-membered cycloalkyl or 5- to 8-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl, C₁₋₆ alkoxy, 3- to 10-membered cycloalkyl, 3- to 10-membered heterocycloalkyl, C₆₋₈ aryl, 5-to 8-membered heteroaryl comprising 1 to 3 heteroatom(s) and cycloalkoxy, and/or the 5- to 8-membered cycloalkyl or 5- to 8-membered heterocycloalkyl comprising 1 to 3 heteroatom(s) is fused with a C₆₋₈ aryl or 5- to 8-membered heteroaryl comprising 1 to 3 heteroatom(s), and the C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) or fused ring is optionally substituted by one to three R_{3b}, and R_{3b} is as defined in formula I.

In other embodiments, in the compound of formula I or the pharmaceutically acceptable salt thereof, ring B is a 5- to 8-membered cycloalkyl or 5- to 8-membered heterocycloalkyl comprising 1 to 3 heteroatom(s), the 5- to 8-membered cycloalkyl or 5-to 8-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl, C₁₋₆ alkoxy, 3- to 8-membered cycloalkyl, 3- to 8-membered heterocycloalkyl and C₆₋₈ aryl, and/or the 5- to 8-membered cycloalkyl or 5- to 8-membered heterocycloalkyl comprising 1 to 3 heteroatom(s) is fused with a C₆₋₈ aryl, the C₁₋₆ alkyl, C₁₋₆ alkoxy, 3- to 8-membered cycloalkyl, 3- to 8-membered heterocycloalkyl, C₆₋₈ aryl or fused ring is optionally substituted by one to three R_{3b}, and R_{3b} is as defined in formula I.

In other embodiments, in the compound of formula I or the pharmaceutically acceptable salt thereof, ring B is a 5- to 8-membered cycloalkyl or 5- to 8-membered heterocycloalkyl comprising 1 to 3 heteroatom(s), and the 5- to 8-membered cycloalkyl or 5- to 8-membered heterocycloalkyl comprising 1 to 3 heteroatom(s) is optionally substituted by one or more substituent(s) selected from the group consisting of hydrogen, deuterium, halogen, oxo, C₁₋₆ alkyl, C₁₋₆ alkoxy, 3- to 8-membered cycloalkyl and C₆₋₈ aryl, and/or the 5- to 8-membered cycloalkyl or 5- to 8-membered heterocycloalkyl comprising 1 to 3 heteroatom(s) is fused with a C₆₋₈ aryl, and the C₁₋₆ alkyl, C₁₋₆ alkoxy, 3- to 8-membered cycloalkyl, C₆₋₈ aryl or fused ring is optionally substituted by one to three R_{3b}, and R_{3b} is as defined in formula I.

In other embodiments, in the compound of formula I or the pharmaceutically acceptable salt thereof, ring B is selected from the group consisting of 5-membered cycloalkyl, 6-membered cycloalkyl, 5-membered heterocycloalkyl comprising 1 to 3 heteroatom(s) and 6-membered heterocycloalkyl comprising 1 to 3 heteroatom(s), and the 5-membered cycloalkyl, 6-membered cycloalkyl, 5-membered heterocycloalkyl comprising 1 to 3 heteroatom(s) and 6-membered heterocycloalkyl comprising 1 to 3 heteroatom(s) are each independently optionally substituted by one or more substituent(s) selected from the group consisting of hydrogen, deuterium, halogen, oxo, C₁₋₆ alkyl, C₁₋₆ alkoxy, 3- to 8-membered cycloalkyl and C₆₋₈ aryl, and/or the 5-membered cycloalkyl, 6-membered cycloalkyl, 5-membered heterocycloalkyl comprising 1 to 3 heteroatom(s) or 6-membered heterocycloalkyl comprising 1 to 3 heteroatom(s) is fused with a C₆₋₈ aryl, and the C₁₋₆ alkyl, C₁₋₆ alkoxy, 3- to 8-membered cycloalkyl, C₆₋₈ aryl or fused ring is optionally substituted by one to three R_{3b}, and R_{3b} is as defined in formula I.

In other embodiments, in the compound of formula I or the pharmaceutically acceptable salt thereof, ring B is selected from the group consisting of 5-membered cycloalkyl, 6-membered cycloalkyl, 5-membered heterocycloalkyl comprising 1 heteroatom and 6-membered heterocycloalkyl comprising 1 heteroatom, and the 5-membered cycloalkyl, 6-membered cycloalkyl, 5-membered heterocycloalkyl comprising 1 heteroatom and 6-membered heterocycloalkyl comprising 1 heteroatom are each independently optionally substituted by one or more substituent(s) selected from the group consisting of hydrogen, deuterium, halogen, oxo, C₁₋₆ alkyl, C₁₋₆ alkoxy, 3- to 6-membered cycloalkyl and C₆₋₈ aryl, and/or the 5-membered cycloalkyl, 6-membered cycloalkyl, 5-membered heterocycloalkyl comprising 1 to 3 heteroatom(s) or 6-membered heterocycloalkyl comprising 1 to 3 heteroatom(s) is fused with a C₆₋₈ aryl, and the C₁₋₆ alkyl, C₁₋₆ alkoxy, 3- to 8-membered cycloalkyl, C₆₋₈ aryl or fused ring is optionally substituted by one to three R_{3b}, and R_{3b} is as defined in formula I.

In other embodiments, in the compound of formula I or the pharmaceutically acceptable salt thereof, ring B is selected from the group consisting of 5-membered cycloalkyl, 6-membered cycloalkyl, 5-membered heterocycloalkyl comprising 1 heteroatom and 6-membered heterocycloalkyl comprising 1 heteroatom, and the 5-membered cycloalkyl, 6-membered cycloalkyl and 6-membered heterocycloalkyl comprising 1 heteroatom are each independently optionally substituted by one or more substituent(s) selected from the group consisting of hydrogen, deuterium, halogen, oxo, C₁₋₆ alkyl and C₁₋₆ alkoxy, and/or the 5-membered cycloalkyl, 6-membered cycloalkyl or 6-membered heterocycloalkyl comprising 1 to 3 heteroatom(s) is fused with a C₆₋₈ aryl;
the 5-membered heterocycloalkyl comprising 1 heteroatom is optionally substituted by one or more substituent(s) selected from the group consisting of hydrogen, deuterium, halogen, oxo, C₁₋₆ alkyl, C₁₋₆ alkoxy, 3- to 6-membered cycloalkyl and C₆₋₈ aryl;
the C₁₋₆ alkyl, C₁₋₆ alkoxy, 3- to 8-membered cycloalkyl, C₆₋₈ aryl or fused ring is optionally substituted by one to three R_{3b}, and R_{3b} is as defined in formula I.

In the second aspect, the present disclosure provides a compound of formula VII or a pharmaceutically acceptable salt thereof, 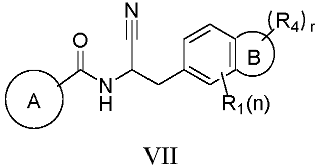 wherein
ring B is a 5- to 8-membered cycloalkyl or 5- to 8-membered heterocycloalkyl comprising 1 to 3 heteroatom(s);
each R₄ is independently selected from the group consisting of hydrogen, deuterium, halogen, hydroxy, cyano, amino, nitro, acyl, amido, oxo, C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) and cycloalkoxy, and the C₁₋₆ alkyl, C₁₋₆ alkoxy, 3- to 10-membered cycloalkyl, 3- to 10-membered heterocycloalkyl, C₆₋₈ aryl and 5- to 8-membered heteroaryl comprising 1 to 3 heteroatom(s) are each optionally substituted by one to three R_{3b};
r is an integer selected from the group consisting of 0 to 3, preferably, r is an integer selected from the group consisting of 0 to 2, and more preferably, r is an integer of 0 or 1;
R₁, n, ring A and R_{3b} are as defined in formula I.

In some embodiments, in the compound of formula VII or the pharmaceutically acceptable salt thereof, ring B is a 5- to 8-membered cycloalkyl;
each R₄ is independently selected from the group consisting of hydrogen, deuterium, halogen, hydroxy, cyano, amino, nitro, acyl, amido, oxo, C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) and cycloalkoxy, and the C₁₋₆ alkyl, C₁₋₆ alkoxy, 3- to 10-membered cycloalkyl, 3- to 10-membered heterocycloalkyl, C₆₋₈ aryl and 5- to 8-membered heteroaryl comprising 1 to 3 heteroatom(s) are each optionally substituted by one to three R_{3b};
R₁, n, ring A and R_{3b} are as defined in formula I.

In some embodiments, in the compound of formula VII or the pharmaceutically acceptable salt thereof, ring B is a 5- to 8-membered heterocycloalkyl comprising 1 to 3 heteroatom(s);
each R₄ is independently selected from the group consisting of hydrogen, deuterium, halogen, hydroxy, cyano, amino, nitro, acyl, amido, oxo, C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) and cycloalkoxy, and the C₁₋₆ alkyl, C₁₋₆ alkoxy, 3- to 10-membered cycloalkyl, 3- to 10-membered heterocycloalkyl, C₆₋₈ aryl and 5- to 8-membered heteroaryl comprising 1 to 3 heteroatom(s) are each optionally substituted by one to three R_{3b};
R₁, n, ring A and R_{3b} are as defined in formula I.

In some embodiments, in the compound of formula VII or the pharmaceutically acceptable salt thereof, each R₄ is independently selected from the group consisting of hydrogen, deuterium, oxo, C₁₋₆ alkyl, C₁₋₆ alkoxy, 3- to 10-membered cycloalkyl, 3- to 10-membered heterocycloalkyl, C₆₋₈ aryl, 5- to 8-membered heteroaryl comprising 1 to 3 heteroatom(s) and 3- to 10-membered cycloalkoxy, and the C₁₋₆ alkyl, C₁₋₆ alkoxy, 3- to 10-membered cycloalkyl, 3- to 10-membered heterocycloalkyl, C₆₋₈ aryl and 5- to 8-membered heteroaryl comprising 1 to 3 heteroatom(s) are each optionally substituted by one to three R_{3b}.

In some embodiments, in the compound of formula VII or the pharmaceutically acceptable salt thereof, each R₄ is independently selected from the group consisting of hydrogen, deuterium, oxo, C₁₋₆ alkyl and phenyl, and the C₁₋₆ alkyl and phenyl are each optionally substituted by one to three R_{3b}.

In some embodiments, in the compound of formula VII or the pharmaceutically acceptable salt thereof, each R₄ is independently selected from the group consisting of hydrogen, deuterium, oxo, methyl and phenyl, and the C₁₋₆ alkyl and phenyl are each optionally substituted by one to three R_{3b}; and preferably, R₄ is a phenyl substituted by one to three R_{3b}. In some embodiments, in the compound of formula VII or the pharmaceutically acceptable salt thereof, the compound of formula VII is selected from the group consisting of

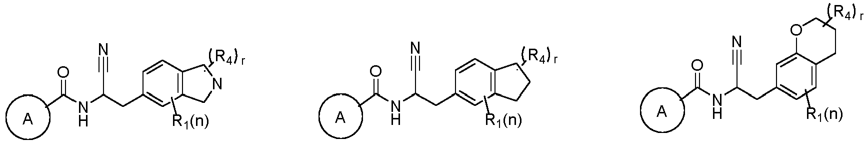

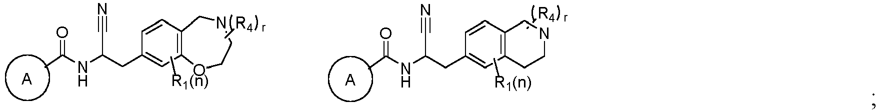

R₁, n, ring A, R₄ and R_{3b} are as defined in formula VII.

In other embodiments, in the compound of formula VII or the pharmaceutically acceptable salt thereof, the compound of formula VII is selected from the group consisting of

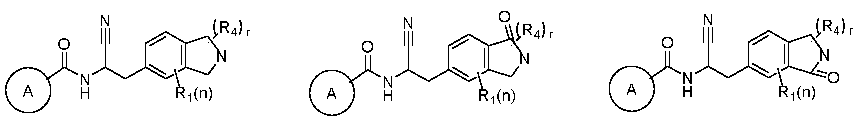

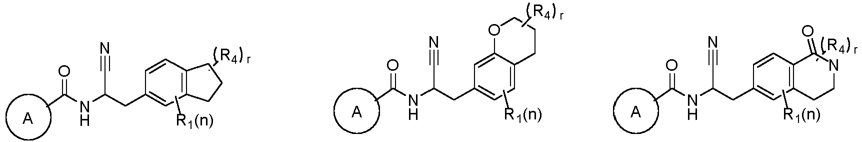

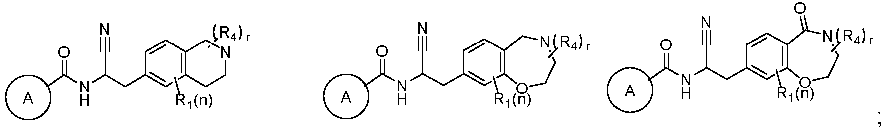

R₁, n, ring A, R₄ and R_{3b} are as defined in formula VII.

In the third aspect, the present disclosure also provides a compound of formula VI or a pharmaceutically acceptable salt thereof, 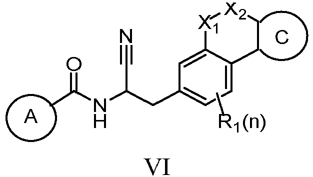
wherein, X₁ and X₂ are each independently selected from the group consisting of a single bond, -C(R_{3b})₂-O-, -C(R_{3b})₂-C(R_{3b})₂-, -O-C(R_{3b})₂-, -C(R_{3b})₂-, oxygen atom and - NR_{3b}-, wherein at least one of X₁ and X₂ is -C(R_{3b})₂-;
ring C is selected from the group consisting of phenyl, naphthyl and 5- to 8-membered heteroaryl comprising 1 to 3 heteroatom(s), wherein the phenyl, naphthyl and 5- to 8-membered heteroaryl comprising 1 to 3 heteroatom(s) are each independently optionally substituted by one to three R_{3b}; and
R₁, n, ring A and R_{3b} are as defined in formula I.

In the fourth aspect, the present disclosure also provides a compound of formula VIa, VI-b, VI-c, VI-d, VI-e, VI-f, VI-g, VI-h, VI-i or a pharmaceutically acceptable salt thereof, 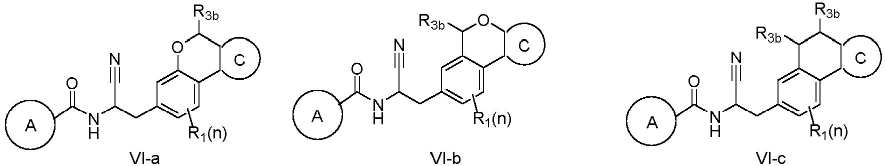 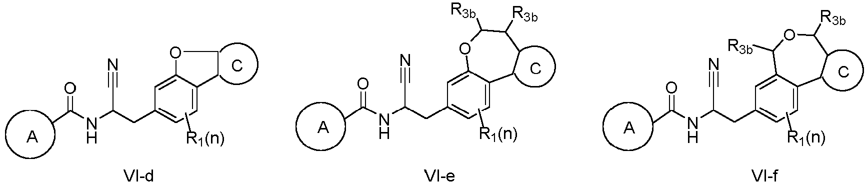 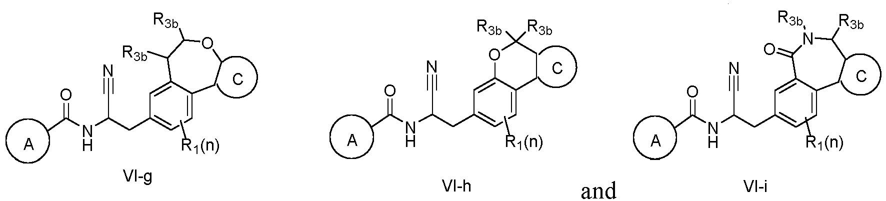 wherein,
ring C is selected from the group consisting of phenyl, naphthyl and 5- to 8-membered heteroaryl comprising 1 to 3 heteroatom(s), wherein the phenyl, naphthyl and 5- to 8-membered heteroaryl comprising 1 to 3 heteroatom(s) are each independently optionally substituted by one to three R_{3b}; and
R₁, n, ring A and R_{3b} are as defined in formula I.

In the fifth aspect, the present disclosure also provides a compound of formula II or a pharmaceutically acceptable salt thereof, 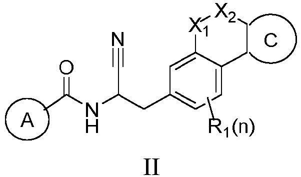 wherein,
X₁ and X₂ are each independently selected from the group consisting of -C(R_{3b})₂-, oxygen atom and -NR_{3b}-, wherein at least one of X₁ and X₂ is -C(R_{3b})₂-;
ring C is selected from the group consisting of phenyl, naphthyl and 5- to 8-membered heteroaryl comprising 1 to 3 heteroatom(s), wherein the phenyl, naphthyl and 5- to 8-membered heteroaryl comprising 1 to 3 heteroatom(s) are each optionally substituted by one to three R_{3b}; and
R₁, n, ring A and R_{3b} are as defined in formula I. In some embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is selected from the group consisting of 3- to 15-membered heterocycloalkyl, 3- to 10-membered heteroaryl and C₆₋₈ aryl, and the 3- to 15-membered heterocycloalkyl, 3- to 10-membered heteroaryl and C₆₋₈ aryl are each independently optionally substituted by one or more substituent(s) selected from the group consisting of deuterium, halogen, hydroxy, cyano, nitro, amino, acyl, amido, oxo, alkyl and alkoxy, and the alkyl and alkoxy are each independently optionally substituted by one or more R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is selected from the group consisting of 3- to 15-membered heterocycloalkyl, 3- to 10-membered heteroaryl and C₆₋₈ aryl, the 3- to 15-membered heterocycloalkyl, 3- to 10-membered heteroaryl and C₆₋₈ aryl are each independently optionally substituted by one or more substituent(s) selected from the group consisting of hydrogen, deuterium, halogen, hydroxy, cyano, amino, nitro, acyl, amido, oxo, C₁₋₆ alkyl and C₁₋₆ alkoxy, and the C₁₋₆ alkyl and C₁₋₆ alkoxy are each optionally substituted by one or more R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is selected from the group consisting of 3- to 15-membered heterocycloalkyl comprising 1 to 3 heteroatom(s), 3- to 10-membered heteroaryl comprising 1 to 3 heteroatom(s) and C₆₋₈ aryl, the 3- to 15-membered heterocycloalkyl comprising 1 to 3 heteroatom(s), 3- to 10-membered heteroaryl comprising 1 to 3 heteroatom(s) and C₆₋₈ aryl are each independently optionally substituted by one or more substituent(s) selected from the group consisting of hydrogen, deuterium, halogen, hydroxy, cyano, amino, nitro, acyl, amido, oxo, C₁₋₆ alkyl and C₁₋₆ alkoxy, and the C₁₋₆ alkyl and C₁₋₆ alkoxy are each independently optionally substituted by one or more R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is a 3- to 15-membered heterocycloalkyl comprising 1 to 3 heteroatom(s), the 3-to 15-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl and C₁₋₆ alkoxy, the C₁₋₆ alkyl and C₁₋₆ alkoxy are each independently optionally substituted by one to three R₃ₐ, R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is a 3- to 10-membered heterocycloalkyl comprising 1 to 3 heteroatom(s), the 3-to 10-membered heterocycloalkyl comprising 1 to 3 heteroatom(s) is optionally substituted by one or more substituent(s) selected from the group consisting of hydrogen, deuterium, halogen, hydroxy, cyano, amino, nitro, acyl, amido, oxo, C₁₋₆ alkyl and C₁₋₆ alkoxy, and the C₁₋₆ alkyl and C₁₋₆ alkoxy are each independently optionally substituted by one to three R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is a 3- to 10-membered heterocycloalkyl comprising 1 to 3 heteroatom(s), the 3-to 10-membered heterocycloalkyl comprising 1 to 3 heteroatom(s) is optionally substituted by one or more substituent(s) selected from the group consisting of hydrogen, deuterium, halogen, hydroxy, cyano, amino, nitro, oxo, C₁₋₆ alkyl and C₁₋₆ alkoxy, and the C₁₋₆ alkyl and C₁₋₆ alkoxy are each independently optionally substituted by one to three R₃ₐ, and R₃ₐ is as defined in formula I.

The aforementioned heteroatom in the present disclosure is selected from the group consisting of nitrogen atom, oxygen atom and sulfur atom, and preferably nitrogen atom or oxygen atom.

In some embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is 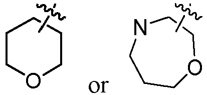 optionally substituted by one or more substituent(s) selected from the group consisting of hydrogen, deuterium, halogen, hydroxy, cyano, amino, nitro, oxo, C₁₋₆ alkyl and C₁₋₆ alkoxy;
the C₁₋₆ alkyl and C₁₋₆ alkoxy are each independently optionally substituted by one to three R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is 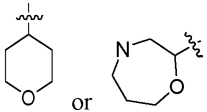 optionally substituted by one or more substituent(s) selected from the group consisting of hydrogen, deuterium, halogen, hydroxy, cyano, amino, nitro, oxo, C₁₋₆ alkyl and C₁₋₆ alkoxy;
the C₁₋₆ alkyl and C₁₋₆ alkoxy are each independently optionally substituted by one to three R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is 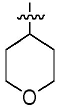 optionally substituted by one or more substituent(s) selected from the group consisting of hydrogen, deuterium, halogen, hydroxy, cyano, amino, nitro, oxo, C₁₋₆ alkyl and C₁₋₆ alkoxy;
the C₁₋₆ alkyl and C₁₋₆ alkoxy are each independently optionally substituted by one to three R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is

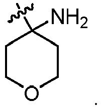

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is 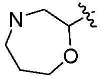 optionally substituted by one or more substituent(s) selected from the group consisting of hydrogen, deuterium, halogen, hydroxy, cyano, amino, nitro, oxo, C₁₋₆ alkyl and C₁₋₆ alkoxy;
the C₁₋₆ alkyl and C₁₋₆ alkoxy are each independently optionally substituted by one to three R₃ₐ, and R₃ₐ is as defined in formula I.

In other embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is

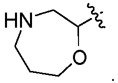

In some embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is 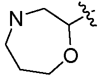 optionally substituted by one or more substituent(s) selected from the group consisting of hydrogen, deuterium, halogen, hydroxy, cyano, amino and nitro; and preferably, A is 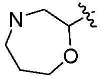 optionally substituted by one or more substituent(s) selected from the group consisting of hydrogen, deuterium and halogen.

In some embodiments, in the compound of formula I, formula II, formula VI, formula VI-a, formula VI-b, formula VI-c, formula VI-d, formula VI-e, formula VI-f, formula VI-g, VI-h, VI-i, formula VII or the pharmaceutically acceptable salt thereof, ring A is 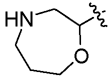 optionally substituted by one or more halogen(s).

In the sixth aspect, the present disclosure also provides a compound of formula VIa', formula VI-b', formula VI-c', formula VI-d', formula VI-e', formula VI-f', formula VI-g', formula VI-h', formula VI-i' or a pharmaceutically acceptable salt thereof, 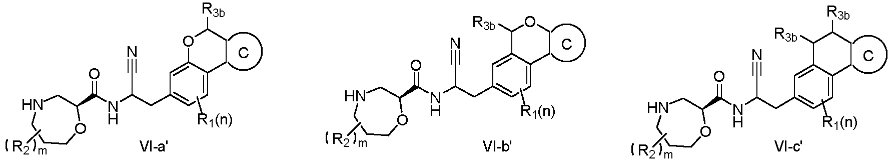 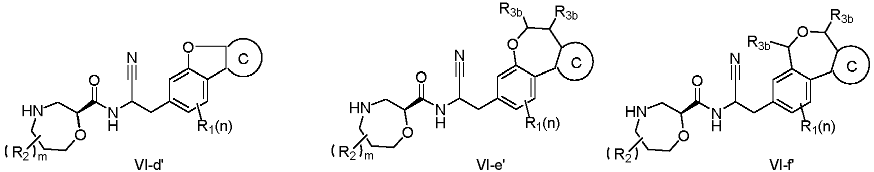 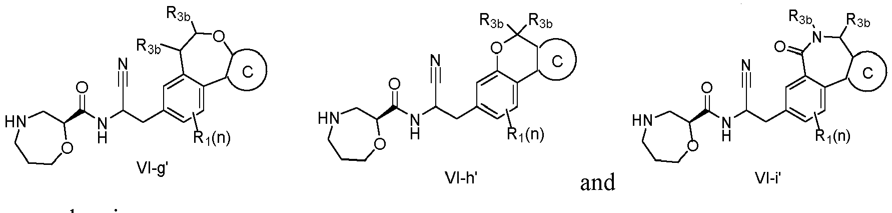 wherein,
each R₂ is independently selected from the group consisting of halogen, nitro, cyano, amino, oxo and hydroxy;
m is an integer selected from the group consisting of 0 to 3, and preferably, m is 0; and
R₁, n, ring C and R_{3b} are as defined in formula VI-a.

In the seventh aspect, the present disclosure also provides a compound of formula VI-a", formula VI-b", formula VI-c", formula VI-d", formula VI-e", formula VI-f", formula VI-g", formula VI-h", formula VI-i" or a pharmaceutically acceptable salt thereof, 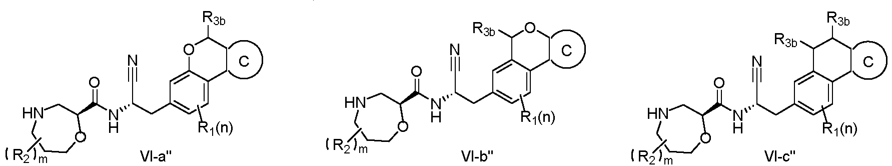 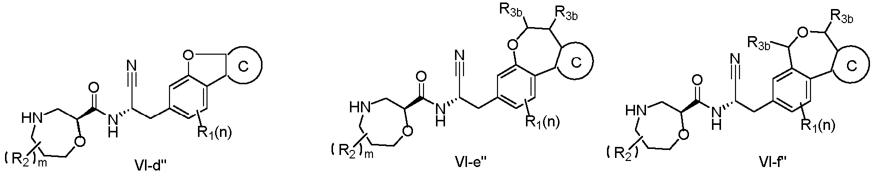 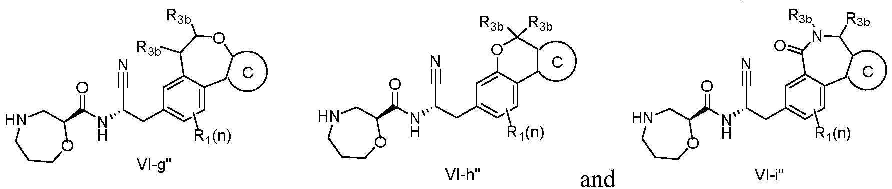 wherein,
each R₂ is independently selected from the group consisting of halogen, nitro, cyano, amino, oxo and hydroxy;
m is an integer selected from the group consisting of 0 to 3, and preferably, m is 0; and
R₁, n, ring C and R_{3b} are as defined in formula VI-a.

In the eighth aspect, the present disclosure also provides a compound of formula III or a pharmaceutically acceptable salt thereof, 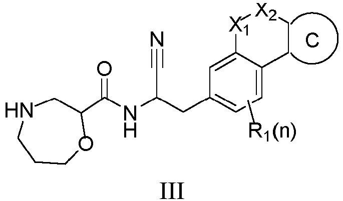 wherein, R₁, n, X₁, X₂ and ring C are as defined in formula I or formula II.

In the ninth aspect, the present disclosure also provides a compound of formula IV or a pharmaceutically acceptable salt thereof, 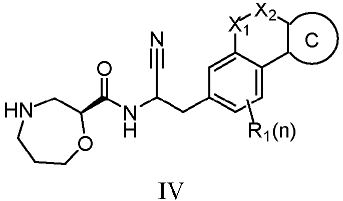 wherein, R₁, n, X₁, X₂ and ring C are as defined in formula I or formula II.

In the tenth aspect, the present disclosure also provides a compound of formula V or a pharmaceutically acceptable salt thereof, 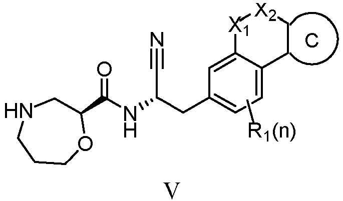 wherein, R₁, n, X₁, X₂ and ring C are as defined in formula I or formula II.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, R₁ is selected from the group consisting of hydrogen, deuterium, halogen, hydroxy, cyano, amino, nitro, acyl, amido, C₁₋₆ alkyl, C₃₋₆ cycloalkyl and C₁₋₆ alkoxy; R₁ can also be selected from the group consisting of hydrogen, deuterium, halogen, hydroxy, cyano, amino, nitro, C₁₋₆ alkyl, C₃₋₆ cycloalkyl and C₁₋₆ alkoxy; R₁ can also be selected from the group consisting of hydrogen, deuterium, halogen, hydroxy, C₁₋₆ alkyl and C₃₋₆ cycloalkyl; R₁ can also be selected from the group consisting of hydrogen, deuterium, halogen, hydroxy and C₁₋₆ alkyl; and R₁ can also be selected from the group consisting of hydrogen, deuterium and halogen.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, R₁ is selected from the group consisting of hydrogen, halogen, nitro and cyano; preferably, R₁ is halogen; and more preferably, R₁ is fluorine.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, n is an integer selected from the group consisting of 0 to 2; and referably, n is an integer selected from the group consisting of 1 to 2.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, ring C is selected from the group consisting of phenyl, thiazolyl and pyridinyl, and the phenyl, thiazolyl and pyridinyl are each independently optionally substituted by one to three R_{3b}.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i" or the pharmaceutically acceptable salt thereof, ring C is selected from the group consisting of phenyl, 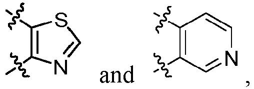 and the phenyl, 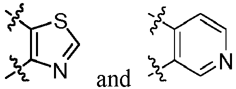 are each independently optionally substituted by one to three R_{3b}.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i" or the pharmaceutically acceptable salt thereof, ring C is phenyl, and the phenyl is optionally substituted by one to three R_{3b}.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i" or the pharmaceutically acceptable salt thereof, ring C is 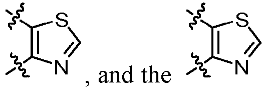 is optionally substituted by one to three R_{3b}.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i" or the pharmaceutically acceptable salt thereof, ring C is 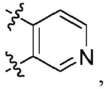 and the 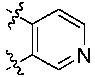 is optionally substituted by one to three R_{3b}.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R₃ₐ is independently selected from the group consisting of hydrogen, halogen, deuterium, hydroxy, oxo, nitro, cyano, amino, acyl and amido.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R₃ₐ is independently selected from the group consisting of hydrogen, halogen, deuterium, hydroxy, oxo, nitro, cyano and amino.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R₃ₐ is independently selected from the group consisting of C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, 5- to 6-membered aryl and heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, 5- to 6-membered aryl and heteroaryl are each independently optionally substituted by one or more substituent(s) selected from the group consisting of halogen, deuterium, hydroxy, oxo, nitro and cyano.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R₃ₐ is independently selected from the group consisting of C₁₋₆ alkoxy, C₃₋₆ cycloalkoxy, 3- to 6-membered heterocycloalkoxy, 5- to 6-membered aryl and heteroaryl, and the C₁₋₆ alkoxy, C₃₋₆ cycloalkoxy, 3- to 6-membered heterocycloalkoxy, 5- to 6-membered aryl and heteroaryl are each independently optionally substituted by one to three substituent(s) selected from the group consisting of fluorine, chlorine, deuterium, hydroxy, oxo, nitro and cyano.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R₃ₐ is independently selected from the group consisting of hydrogen, fluorine, chlorine, deuterium, oxo (=O), hydroxy, amino, methoxy, cyclopropoxy, cyclopropyl, cyclopentyl, pyridinyl, piperidinyl and phenyl, and preferably, R₃ₐ is hydrogen or amino.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of hydrogen, halogen, deuterium, hydroxy, oxo, nitro, cyano and amino; and preferably, R_{3b} is halogen or cyano.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, R_{3b} is a 3- to 6-membered heterocycloalkyl; the 3- to 6-membered heterocycloalkyl can be substituted by one to three substituent(s) selected from the group consisting of halogen, cyano and hydroxy.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of hydrogen, halogen, deuterium, cyano, 3- to 6-membered heterocycloalkyl, methanesulfonyl and 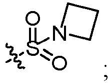 and preferably, the 3- to 6-membered heterocycloalkyl is selected from the group consisting of

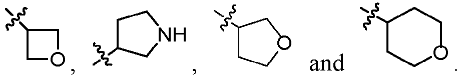

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of hydrogen, halogen, deuterium, hydroxy, oxo, nitro, cyano, amino, amido, methanesulfonyl and

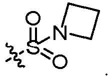

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of hydrogen, halogen, deuterium, hydroxy, oxo, nitro, cyano, amino, amido, acetyl, methanesulfonyl,

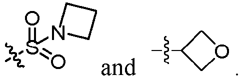

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of cyano, methanesulfonyl and 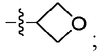 and preferably, R_{3b} is a cyano.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of hydrogen, halogen, deuterium, hydroxy, oxo, nitro, cyano, amino, acyl and amido.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of C₁₋₆ alkoxy, C₃₋₆ cycloalkoxy, 3- to 6-membered heterocycloalkoxy, 5- to 6-membered aryl and heteroaryl, the C₁₋₆ alkoxy, C₃₋₆ cycloalkoxy, 3- to 6-membered heterocycloalkoxy, 5- to 6-membered aryl and heteroaryl are each independently optionally substituted by one to three substituent(s) selected from the group consisting of fluorine, chlorine, deuterium, hydroxy, oxo, nitro and cyano.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of halogen, cyano, methanesulfonyl, 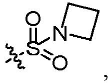 3- to 6-membered heterocycloalkyl and deuterium.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, each R_{3b} is independently selected from the group consisting of hydrogen, fluorine, chlorine, deuterium, oxo (=O), hydroxy, amino, methoxy, cyclopropoxy, cyclopropyl, cyclopentyl, pyridinyl, piperidinyl and phenyl, and preferably, R_{3b} is selected from the group consisting of hydrogen, methyl, oxo, fluorine and chlorine.

In some embodiments, in the compound of formula I, formula II, formula III, formula IV, formula V, formula VI, formula VI-a, formula VI-b, formula VI-c, formula VI-d, formula VI-e, formula VI-f, formula VI-g, VI-h, VI-i, formula VI-a', formula VI-b', formula VI-c', formula VI-d', formula VI-e', formula VI-f', formula VI-g', VI-h', VI-i', formula VI-a", formula VI-b", formula VI-c", formula VI-d", formula VI-e", formula VI-f", formula VI-g", VI-h", VI-i", formula VII or the pharmaceutically acceptable salt thereof, R_{3b} is a C₁₋₆ alkyl or C₁₋₆ alkoxy, the C₁₋₆ alkyl or C₁₋₆ alkoxy is optionally substituted by one or more substituent(s) selected from the group consisting of halogen, deuterium, hydroxy, oxo, nitro and cyano.

In some embodiments, in the compound of formula VII or the pharmaceutically acceptable salt thereof, R_{3b} is a phenyl, and the phenyl is optionally substituted by one or more substituent(s) selected from the group consisting of halogen, deuterium, hydroxy, oxo, nitro and cyano.

In the eleventh aspect, the present disclosure also provides typical compounds of formula I or pharmaceutically acceptable salts thereof, the compounds of formula I include but are not limited to:

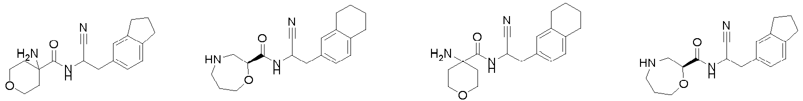

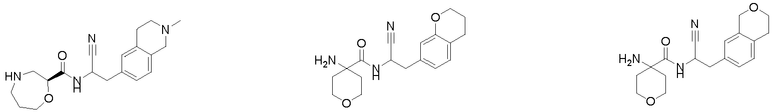

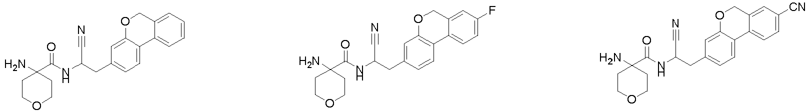

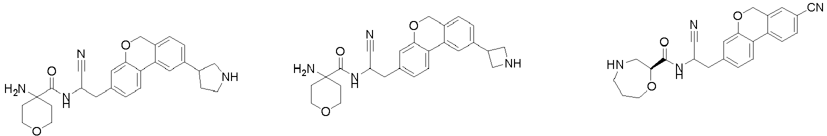

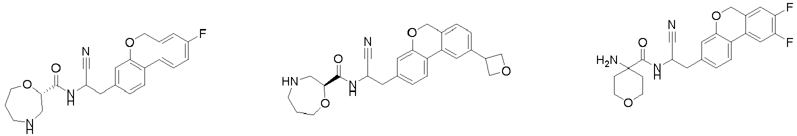

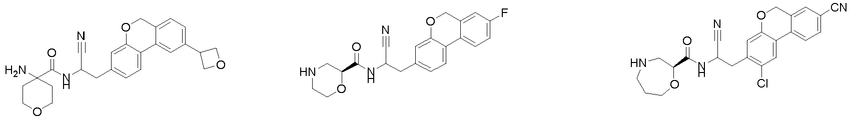

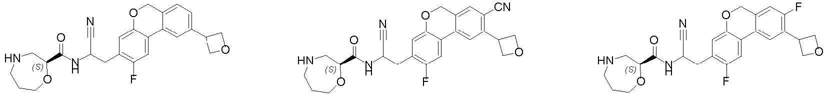

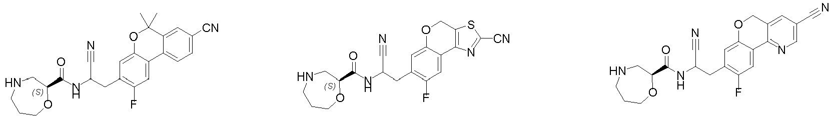

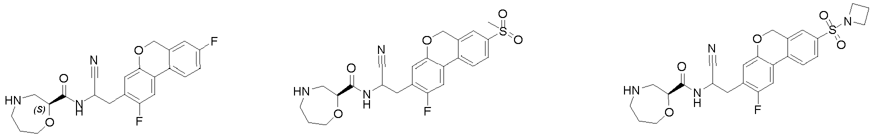

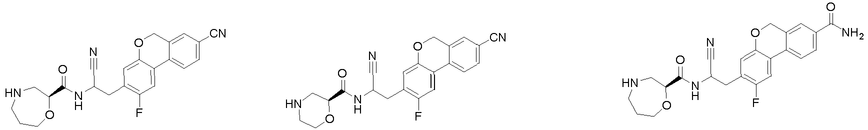

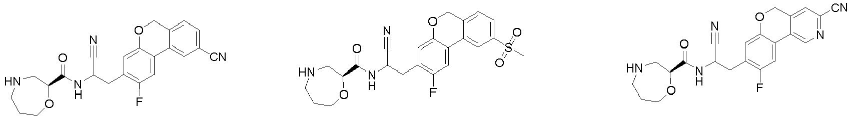

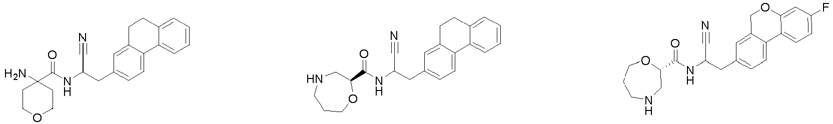

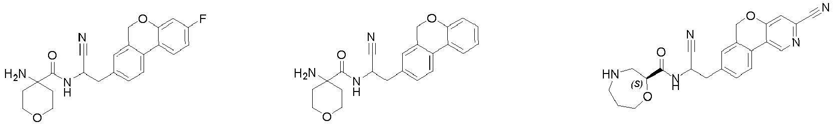

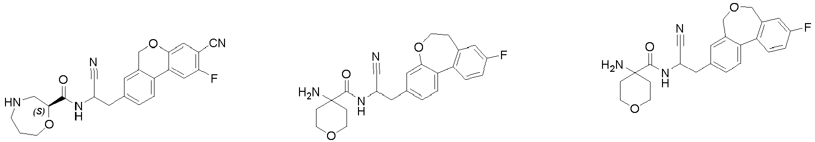

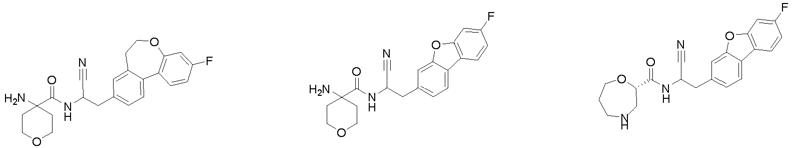

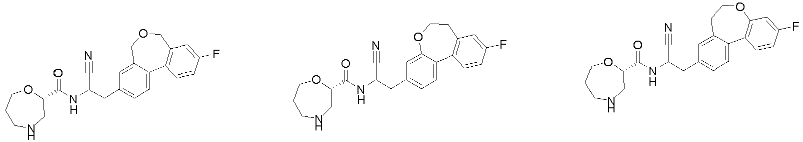

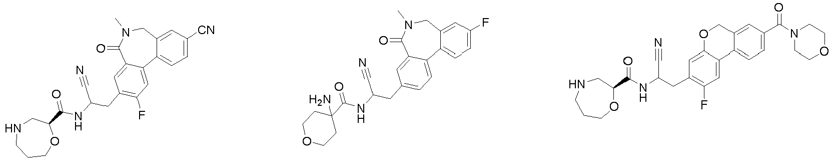

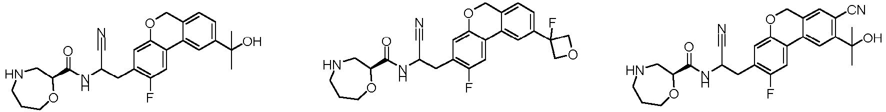

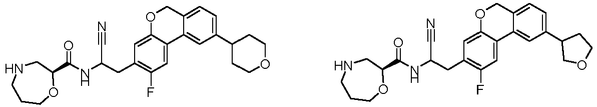

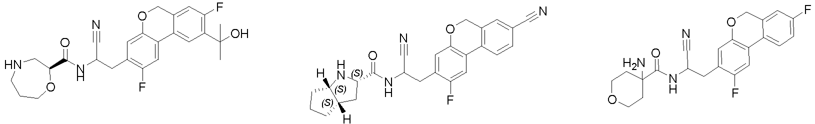

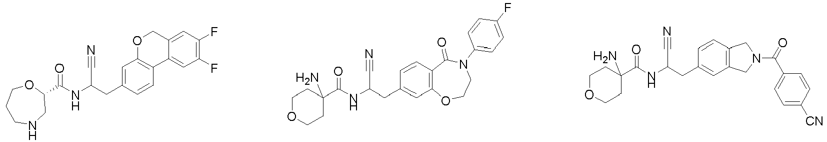

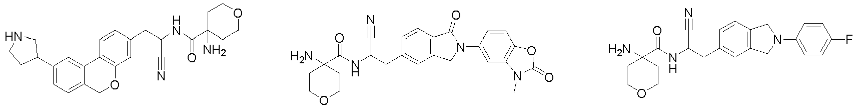

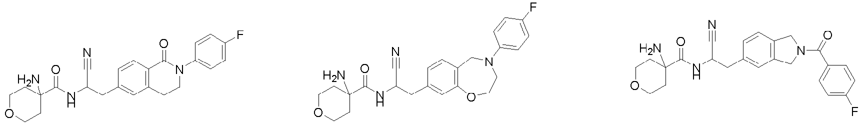

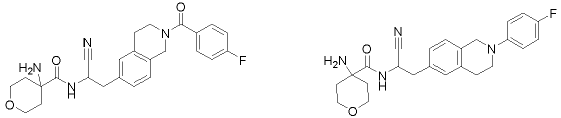

In the twelfth aspect, the present disclosure also provides typical compounds of formula I or pharmaceutically acceptable salts thereof, the compounds of formula I include but are not limited to:

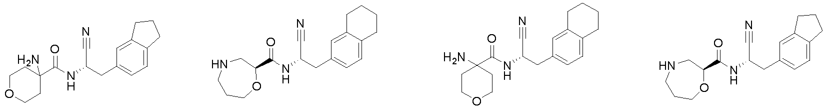

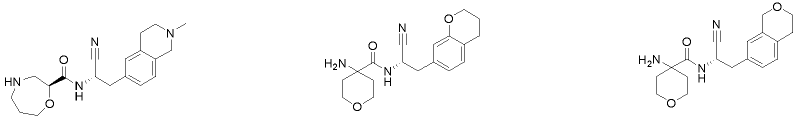

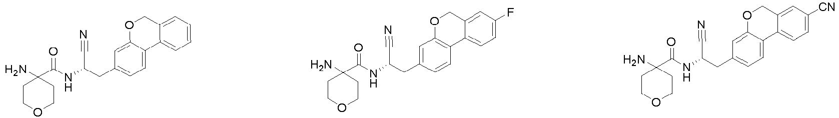

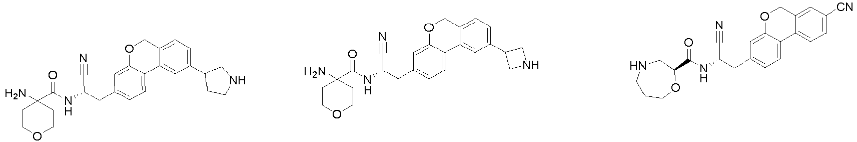

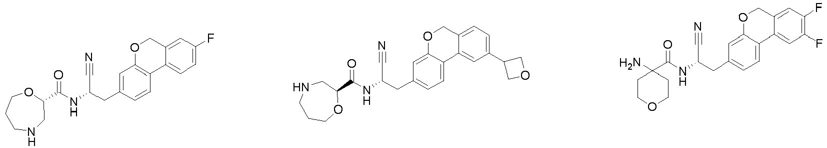

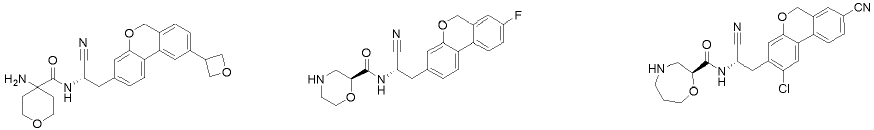

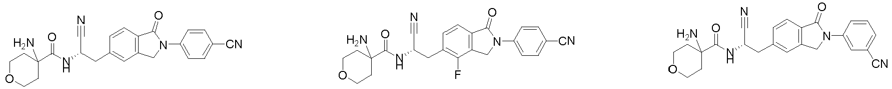

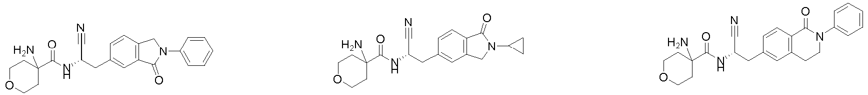

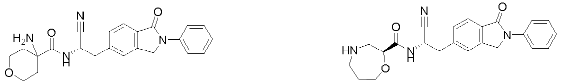

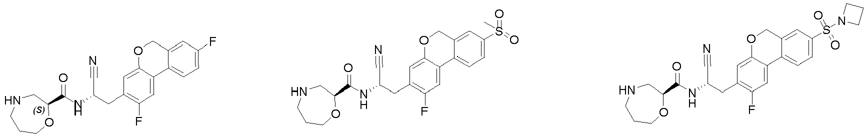

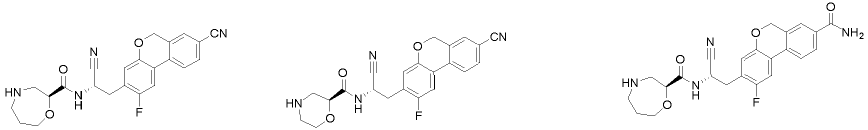

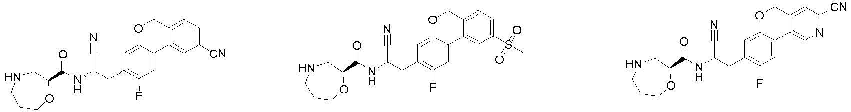

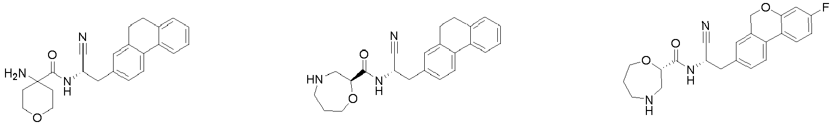

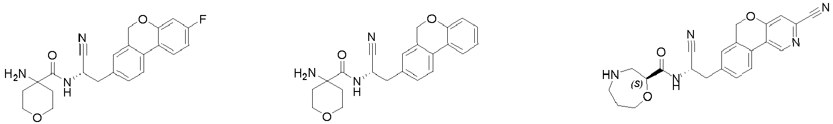

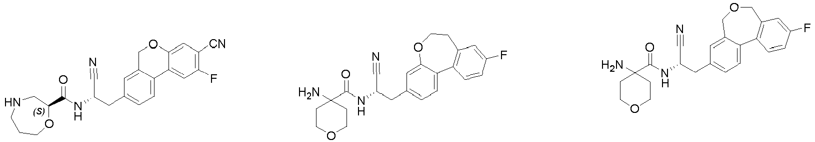

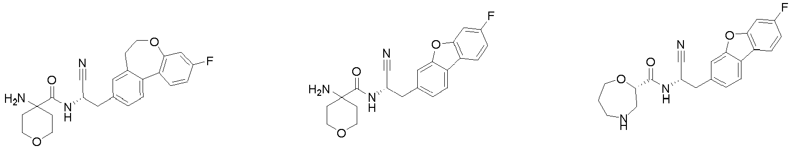

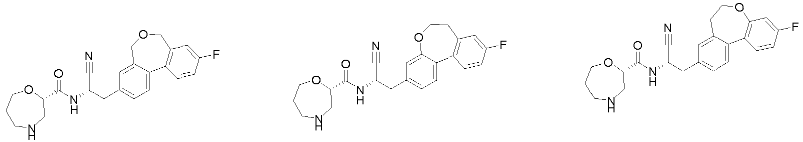

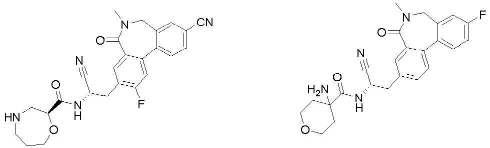

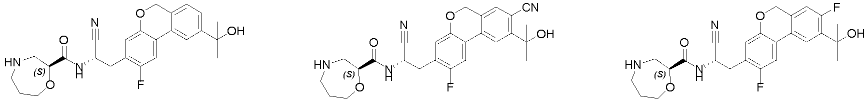

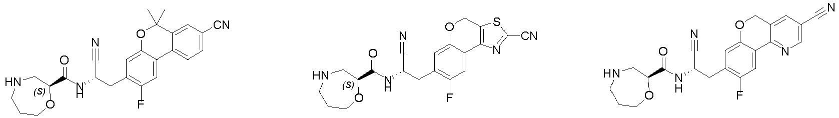

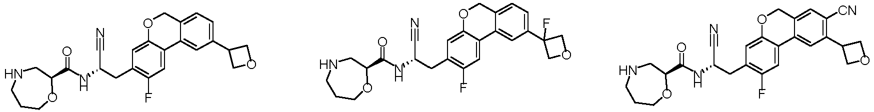

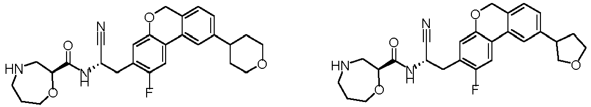

In the thirteenth aspect, the present disclosure also provides typical compounds of formula I or pharmaceutically acceptable salts thereof, the compounds of formula I include but are not limited to:

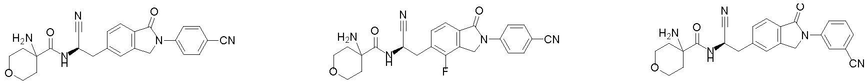

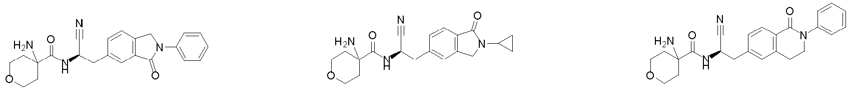

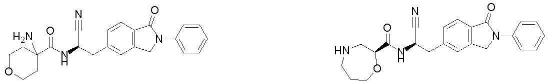

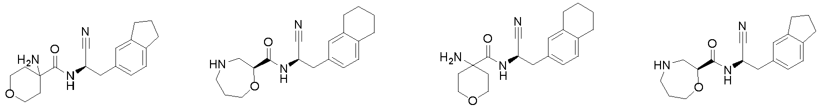

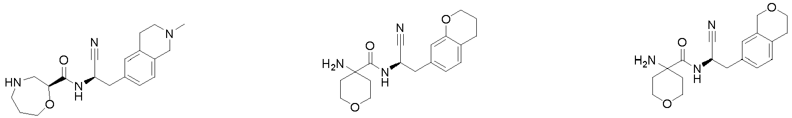

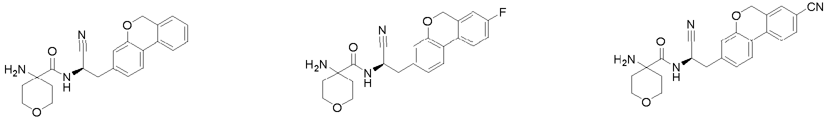

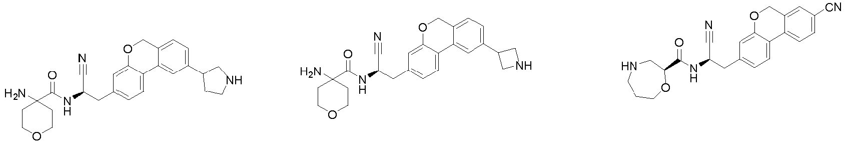

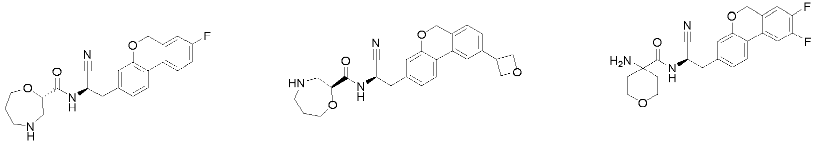

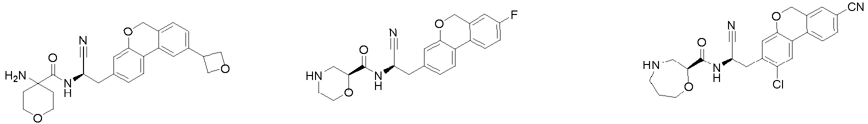

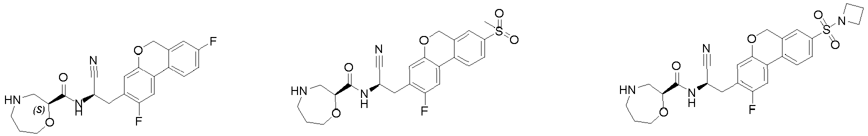

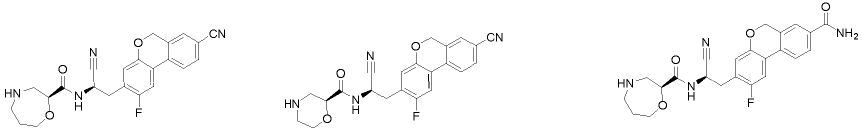

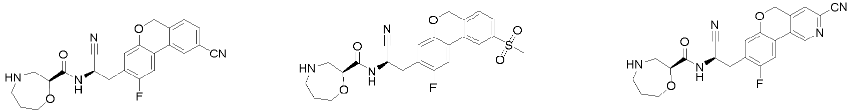

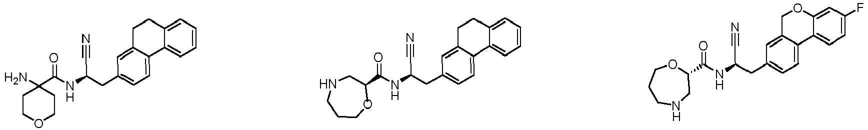

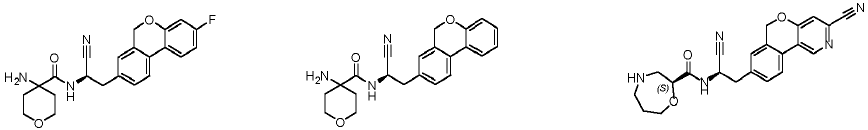

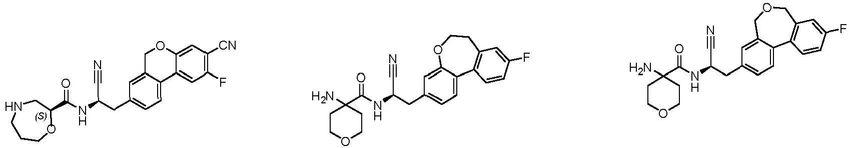

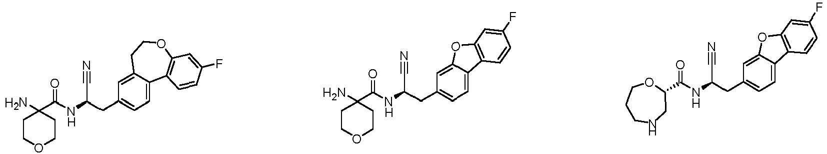

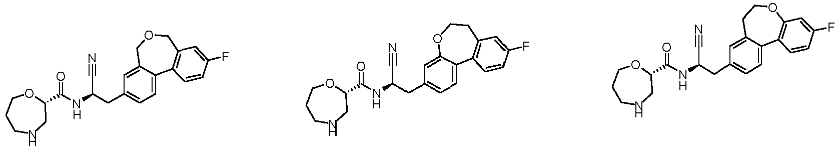

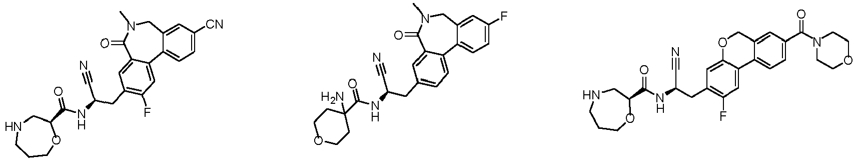

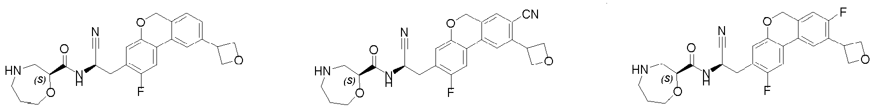

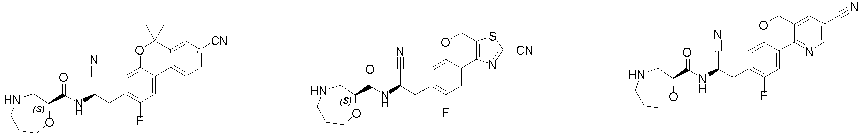

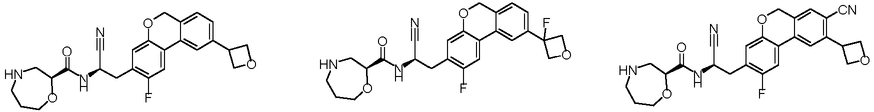

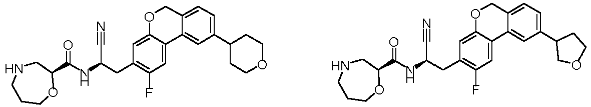

In the fourteenth aspect, the present disclosure also provides an isotope substitute of the compound or pharmaceutically acceptable salt thereof according to the first aspect to the twelfth aspect, and preferably, the isotope substitute is a deuterium atom substitute.

In the fifteenth aspect, the present disclosure also provides a pharmaceutical composition comprising a therapeutically effective amount of at least one compound or pharmaceutically acceptable salt thereof according to the first aspect to the thirteenth aspect or the isotope substitute according to the fourteenth aspect, as well as a pharmaceutically acceptable excipient.

In some embodiments, the unit dosage of the pharmaceutical composition is 0.001 mg to 1000 mg.

In some embodiments, the pharmaceutical composition comprises 0.01% to 99.99% of the aforementioned compound or the pharmaceutically acceptable salt thereof based on the total weight of the composition. In some embodiments, the pharmaceutical composition comprises 0.1% to 99.9% of the aforementioned compound or the pharmaceutically acceptable salt thereof. In some embodiments, the pharmaceutical composition comprises 0.5% to 99.5% of the aforementioned compound or the pharmaceutically acceptable salt thereof. In some embodiments, the pharmaceutical composition comprises 1% to 99% of the aforementioned compound or the pharmaceutically acceptable salt thereof. In some embodiments, the pharmaceutical composition comprises 2% to 98% of the aforementioned compound or the pharmaceutically acceptable salt thereof.

In some embodiments, the pharmaceutical composition comprises 0.01% to 99.99% of the pharmaceutically acceptable excipient based on the total weight of the composition. In some embodiments, the pharmaceutical composition comprises 0.1% to 99.9% of the pharmaceutically acceptable excipient. In some embodiments, the pharmaceutical composition comprises 0.5% to 99.5% of the pharmaceutically acceptable excipient. In some embodiments, the pharmaceutical composition comprises 1% to 99% of the pharmaceutically acceptable excipient. In some embodiments, the pharmaceutical composition comprises 2% to 98% of the pharmaceutically acceptable excipient.

The present disclosure also provides a compound or pharmaceutically acceptable salt thereof or the isotope substitute thereof or a pharacetical composition for use in preventing and/or treating cathepsin C-related disorder in a patient.

In some embodiments, the cathepsin C-related disorder includes but is not limited to respiratory diseases such as asthma, obstructive pulmonary disease, bronchiectasis and the like, as well as autoimmune diseases such as ANCA-associated vasculitis, psoriasis, alpha 1-antitrypsin deficiency, lupus nephritis, diabetes, inflammatory bowel disease or rheumatoid arthritis.

The present disclosure also provides the compound or pharmaceutically acceptable salt thereof according to the first aspect to the thirteenth aspect, or the isotope substitute according to the fourteenth aspect, or the aforementioned pharmaceutical composition for use as a medicament.

The present disclosure also provides a compound or intermediate of the following formula 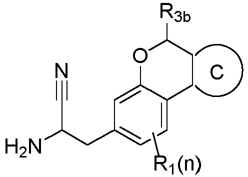 wherein R₁, n, ring C and R_{3b} are as defined in formula IV.

The present disclosure also provides a compound or intermediate of the following formula 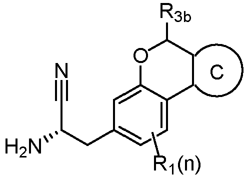 wherein R₁, n, ring C and R_{3b} are as defined in formula IV.

The pharmaceutically acceptable salt of the compound of the present disclosure can be selected from the group consisting of inorganic salt and organic salt.

The compound of the present disclosure may exist in a specific stereoisomeric form. The present disclosure contemplates all such compounds, including *cis-* and *trans-*isomers, (-)- and (+)-enantiomers, (R)- and (S)-enantiomers, diastereomers, (D)-isomers, (L)-isomers, and racemic mixtures and other mixtures thereof, such as mixtures enriched with enantiomers or diastereomers, all of which are within the scope of the present disclosure. Additional asymmetric carbon atoms may be present in substituents such as an alkyl group. All such isomers and mixtures thereof are included within the scope of the present disclosure. The compound of the present disclosure containing one or more asymmetric carbon atom(s) can be isolated in optically pure or racemic form. The optically pure form can be resolved from racemic mixture, or synthesized by using chiral starting materials or reagents.

Optically active (R)- and (S)-enantiomers, and D- and L-isomers can be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (*e.g.,* amino) or an acidic functional group (*e.g*., carboxyl), salts of diastereomers are formed with an appropriate optically active acid or base, diastereomers are then resolved by conventional methods known in the art, and then the pure enantiomers are obtained by recovery. Furthermore, the separation of enantiomers and diastereomers is typically accomplished by chromatography using a chiral stationary phase, optionally in combination with chemical derivatization (*e.g*., carbamate formed from amine).

In the chemical structure of the compound of the present disclosure, a bond " 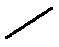 " represents an unspecified configuration, namely if chiral isomers exist in the chemical structure, the bond " 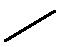 " may be " 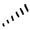 " or " 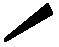 ",or contains both the configurations of " 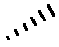 " and " 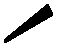 ".

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine and lactam-lactim isomerization. An example of a lactam-lactim equilibrium is present between A and B as shown below.

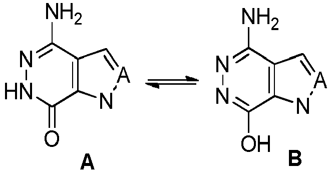

All compounds in the present disclosure can be drawn as form A or form B. All tautomeric forms are within the scope of the present disclosure. The nomenclature of the compounds does not exclude any tautomers.

The present disclosure also comprises isotopically-labeled compounds which are identical to those recited herein but have one or more atoms replaced by an atom having an atomic mass or mass number different from the atomic mass or mass number usually found in nature. Examples of isotopes that can be incorporated into the compound of the present disclosure include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

Unless otherwise stated, when a position is specifically designated as deuterium (D), the position is understood to have an abundance of deuterium that is at least 1000 times greater than the natural abundance of deuterium (which is 0.015%) (*i.e.*, at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula VIa. Each available hydrogen atom connected to a carbon atom may be independently replaced with a deuterium atom. Those skilled in the art are able to synthesize the compound of formula VI-a in deuterated form with reference to the relevant literature. Commercially available deuterated starting materials can be used in preparing the compound of formula VIa in deuterated form, or it can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Optionally" or "optional" means that the event or circumstance described subsequently can, but need not, occur, and such a description includes the situation in which the event or circumstance does or does not occur. For example, "the C₁₋₆ alkyl optionally substituted by a halogen or cyano" means that a halogen or cyano can be, but need not be, present, and such a description includes the situation of the alkyl being substituted by a halogen or cyano and the alkyl being not substituted by a halogen or cyano.

Definition of the terms:
A "pharmaceutical composition" refers to a mixture of one or more of the compounds according to the present disclosure or physiologically/pharmaceutically acceptable salts thereof with other chemical components, and other components such as physiologically/pharmaceutically acceptable carriers and excipients. The purpose of the pharmaceutical composition is to facilitate the administration of a compound to an organism, which is conducive to the absorption of the active ingredient so as to show biological activity.

A "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that has been approved by the U.S. food and drug administration for use in humans or livestock animals.

An "effective amount" or "therapeutically effective amount" as used in the present disclosure includes an amount sufficient to ameliorate or prevent a symptom or condition of a medical disorder. An effective amount also means an amount sufficient to permit or facilitate diagnosis. The effective amount for a particular patient or veterinary subject may vary depending on factors such as the disorder being treated, the general health of the patient, the method, route and dosage of administration, and the severity of side effects. The effective amount may be the maximum dose or dosing regimen that avoids significant side effects or toxic effects.

An "alkyl" refers to a saturated aliphatic hydrocarbon group, including straight and branched chain groups of 1 to 20 carbon atoms. An alkyl containing 1 to 6 carbon atoms is preferred. Non-limiting examples include methyl, ethyl, *n*-propyl, isopropyl, *n*-butyl, isobutyl, *tert-*butyl, *sec*-butyl, *n*-pentyl, 1,1-dimethylpropyl, 1,2-dimethylpropyl, 2,2-dimethylpropyl and various branched isomers thereof. The alkyl can be substituted or unsubstituted. When substituted, the substituent(s) can be substituted at any available connection point. The substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

An "alkenyl" includes branched and straight chain alkenes or alkenes containing aliphatic hydrocarbon groups having 2 to 12 carbon atoms. For example, a "C₂₋₆ alkenyl" refers to an alkenyl having 2, 3, 4, 5 or 6 carbon atoms. Examples of alkenyl include, but are not limited to, vinyl, allyl, 1-propenyl, 1-butenyl, 2-butenyl, 3-butenyl, 2-methylbut-2-enyl, 3-methylbut-1-enyl, 1-pentenyl, 3-pentenyl and 4-hexenyl. The alkenyl can be substituted or unsubstituted. When substituted, the substituent(s) can be substituted at any available connection point. The substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

An "alkynyl" includes branched and straight chain alkynyl or alkyne containing aliphatic hydrocarbon groups having 2 to 12 carbon atoms, or if the number of carbon atoms is specified, then the alkynyl refers to that specific number. Examples include ethynyl, propynyl (such as 1-propynyl, 2-propynyl), 3-butynyl, pentynyl, hexynyl and 1-methylpent-2-ynyl. The alkynyl can be substituted or unsubstituted. When substituted, the substituent(s) can be substituted at any available connection point. The substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

The term "cycloalkyl" or "carbocycle" refers to a saturated or partially unsaturated monocyclic or polycyclic hydrocarbon substituent, the cycloalkyl ring contains 3 to 20 carbon atoms, and preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl and the like. Polycyclic cycloalkyl includes a cycloalkyl having a spiro ring, fused ring or bridged ring. The cycloalkyl can be substituted or unsubstituted. When substituted, the substituent(s) can be substituted at any available connection point. The substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

The cycloalkyl ring can be fused to the ring of aryl or heteroaryl, wherein the ring bound to the parent structure is the cycloalkyl. Non-limiting examples thereof include indanyl, tetrahydronaphthyl, benzocycloheptyl and the like. The cycloalkyl can be optionally substituted or unsubstituted. When substituted, the substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

The term "cycloalkenyl" refers to a partially unsaturated monocyclic or polycyclic hydrocarbon substituent, and the cycloalkenyl ring contains 3 to 20 carbon atoms, and preferably 3 to 8 carbon atoms. Examples include, but are not limited to, cyclopentenyl, cyclohexenyl, or cyclohexadienyl. The cycloalkenyl can be optionally substituted or unsubstituted. When substituted, the substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5-to 6-membered heteroaryl are each independently optionally substituted by one or more substituent(s) selected from the group consisting of halogen, deuterium, hydroxy, oxo, nitro and cyano.

The term "heterocycloalkyl" or "heterocyclyl" refers to a 3- to 20-membered saturated or partially unsaturated monocyclic or polycyclic hydrocarbon substituent group, wherein one or more ring atoms are heteroatoms selected from the group consisting of N, O and S(O)ₘ (wherein m is an integer of 0 to 2), but excluding -O-O-, - O-S- or -S-S- in the ring, with the remaining ring atoms being carbon atoms. Preferably, the heterocycloalkyl has 3 to 15 ring atoms wherein 1 to 4 atoms are heteroatoms; more preferably, 3 to 10 ring atoms; and even more preferably 3 to 6 ring atoms. Non-limiting examples of monocyclic heterocycloalkyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like. Polycyclic heterocycloalkyl includes a heterocycloalkyl having a spiro ring, fused ring or bridged ring. Non-limiting examples of "heterocycloalkyl" include: 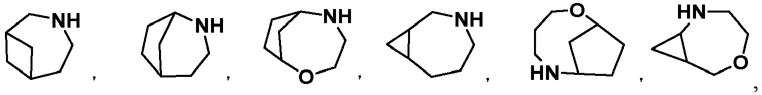 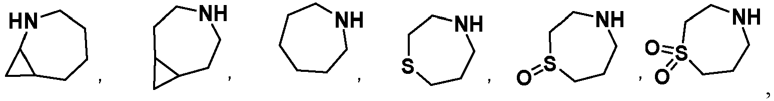 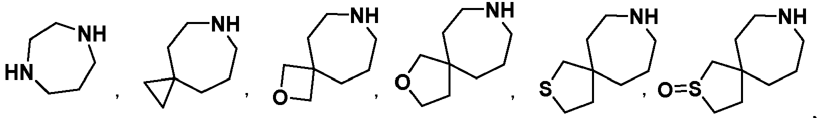 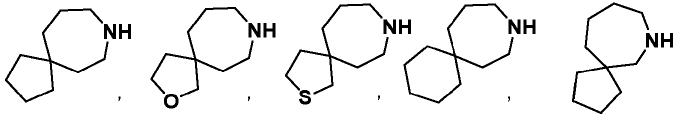 and the like.

The heterocycloalkyl ring can be fused to the ring of aryl or heteroaryl, wherein the ring bound to the parent structure is the heterocycloalkyl. Non-limiting examples thereof include: 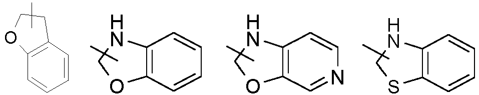 and the like.

The heterocycloalkyl can be optionally substituted or unsubstituted. When substituted, the substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

The term "aryl" refers to a 6- to 14-membered all-carbon monocyclic ring or polycyclic fused ring (*i.e.* each ring in the system shares an adjacent pair of carbon atoms with another ring in the system) having a conjugated π-electron system, a 6- to 12-membered aryl is preferred, for example, phenyl and naphthyl. The aryl ring can be fused to the ring of heteroaryl, heterocycloalkyl or cycloalkyl, wherein the ring bound to the parent structure is the aryl ring. Non-limiting examples thereof include: 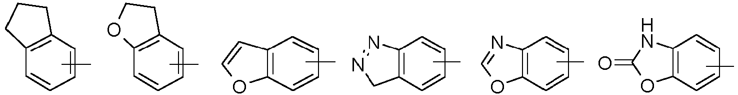 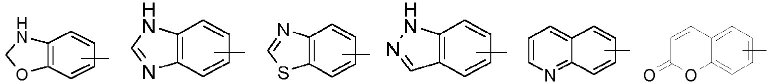 and

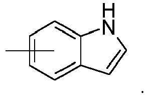

The aryl can be substituted or unsubstituted. When substituted, the substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

The term "heteroaryl" refers to a 5- to 14-membered heteroaromatic system having 1 to 4 heteroatoms selected from the group consisting of O, S and N. The heteroaryl is preferably a 3- to 10-membered heteroaryl, more preferably a 5- to 8-membered heteroaryl or 3- to 6-membered heteroaryl, and even more preferably a 5- or 6-membered heteroaryl. For example, non-limiting examples thereof include: imidazolyl, furyl, thienyl, thiazolyl, pyrazolyl, oxazolyl, pyrrolyl, tetrazolyl, pyridinyl, pyrimidinyl, thiadiazolyl, pyrazinyl, 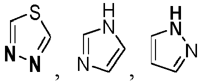 and the like.

The heteroaryl ring can be fused to the ring of aryl, heterocycloalkyl or cycloalkyl, wherein the ring bound to the parent structure is the aryl ring. Non-limiting examples thereof include:

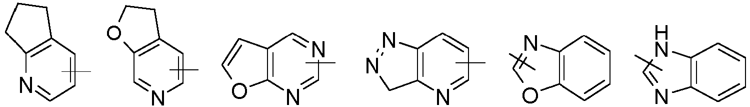

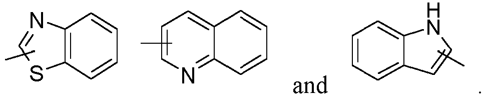

The heteroaryl can be optionally substituted or unsubstituted. When substituted, the substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

The term "alkoxy" refers to -O-(alkyl) and -O-(cycloalkyl), wherein alkyl is as defined above. Non-limiting examples of alkoxy include: methoxy, ethoxy, propoxy, butoxy, cyclopropoxy, cyclobutoxy, cyclopentyloxy, cyclohexyloxy. The alkoxy can be optionally substituted or unsubstituted. When substituted, the substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano. Similarly, the "alkynyloxy", "alkenyloxy", "cycloalkoxy", "heterocycloalkoxy", and "cycloalkenyloxy" are as defined like the above "alkoxy".

The term "hydroxy" refers to an -OH group.

The term "halogen" refers to fluorine, chlorine, bromine or iodine.

The term "cyano" refers to a -CN group.

The term "nitro" refers to a -NO₂ group.

The term "oxo" refers to a =O group.

The term "amido" refers to 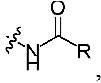 and R is C₁₋₆ alkyl, including but not limited to methyl, ethyl, propyl, and the like.

A "monovalent group" refers to a group formed by "formally" removing a monovalent atom or group from a compound. A "-ylene group" refers to a group formed by "formally" removing two monovalent or one divalent atoms or atomic groups from a compound. For example, an"alkyl" refers to the residual moiety after removing one hydrogen atom from an alkane molecule, including a straight or branched monovalent group comprising 1 to 20 carbon atoms. An "alkylene (-CH₂-)" refers to the residual moiety after removing two hydrogen atoms from an alkane molecule, including a straight or branched -ylene group comprising 1 to 20 carbon atoms. An alkylene comprising 1 to 6 carbon atoms is preferred, and non-limiting examples include methylene (-CH₂-), ethylene (such as -CH₂CH₂- or -CH(CH₃)-), propylene (such as -CH₂CH₂CH₂- or - CH(CH₂CH₃)-), butylene (such as -CH₂CH₂CH₂CH₂-). The alkylene can be substituted or unsubstituted. When substituted, the substituent(s) can be substituted at any available connection point. The substituent(s) is preferably one or more group(s) independently selected from the group consisting of halogen, deuterium, hydroxy, oxo, nitro, cyano, C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl, and the C₁₋₆ alkyl, C₁₋₆ alkoxy, C₂₋₆ alkenyloxy, C₂₋₆ alkynyloxy, C₃₋₆ cycloalkoxy, 3- to 6-membered heterocycloalkoxy, C₃₋₈ cycloalkenyloxy, phenyl and 5- to 6-membered heteroaryl are each independently optionally substituted by one or more substituent(s) selected from the group consisting of halogen, deuterium, hydroxy, oxo, nitro and cyano.

Similarly, the "alkyleneoxy", "alkenylene", "alkenyleneoxy", "cycloalkylene", and "heterocycloalkylene" are as defined like the above "alkylene".

### DESCRIPTION OF THE DRAWINGS

Figure 1 Percentage inhibition rate of compound 32, AZD7986 and vehicle control on CatC downstream neutrophil elastase in neutrophils (***P<0.0001; *P<0.05; t-test; vs vehicle control; N=3 repeated measurements of enzyme activity; percentage inhibition is shown above each column).
Figure 2 Percentage inhibition rate of compound 14, AZD7986 and vehicle control on CatC downstream neutrophil elastase in neutrophils (***P<0.0001; *P<0.05; t-test; vs vehicle control; N=5 repeated measurements of enzyme activity; percentage inhibition is shown above each column).

### DETAILED DESCRIPTION OF THE INVENTION

The present disclosure will be further described with reference to the following examples, but the examples should not be considered as limiting the scope of the present disclosure. Those of the following examples which do not fall within the scope of the appended claims are reference examples.

The experimental methods in the examples of the present disclosure that do not indicate specific conditions are usually carried out in accordance with conventional conditions, or in accordance with the conditions suggested by the raw material or product manufacturers. Reagents without specific sources indicated are conventional reagents purchased from the market.

The structures of the compounds were identified by nuclear magnetic resonance (NMR) and/or mass spectrometry (MS). NMR shifts (δ) are given in 10⁻⁶ ppm. NMR was determined by a Bruker AVANCE-400 machine. The solvents for determination were deuterated-dimethyl sulfoxide (DMSO-*d₆*), deuterated-chloroform (CDCl₃) and Methanol-*d₄*, and the internal standard was tetramethylsilane (TMS).

High performance liquid chromatography (HPLC) was determined on an Agilent 1100 high pressure liquid chromatograph equipped with a GAS15B DAD UV detector and a Water Vbridge C18 150*4.6 mm 5 µm column.

MS was determined by an Agilent 6120 triple quadrupole mass spectrometer equipped with a G1315D DAD detector and a Waters Xbridge C18 4.6*50 mm, 5 µm column, scanning in positive/negative ion mode, and the mass scanning range was 80 to 1200.

Yantai Huanghai HSGF254 was used as the thin-layer silica gel chromatography (TLC) plate. The dimension of the silica gel plates used in TLC was 0.2 mm ± 0.03 mm, and the dimension of the silica gel plates used in product purification by thin-layer chromatography was 0.4 mm to 0.5 mm.

The flash column purification system used was Combiflash Rf150 (TELEDYNE ISCO) or Isolara one (Biotage).

Yantai Huanghai 200 to 300 mesh or 300 to 400 mesh silica gel was generally used as a carrier for normal phase column chromatography, or a pre-filled ultrapure normal phase silica gel column (40-63 µm, 60 g, 24 g, 40 g, 120 g or other specifications) from Santai Technologies (Changzhou), Inc. was used.

The known starting materials of the present disclosure can be prepared by the known methods in the art, or can be purchased from Shanghai Titan Scientific Co., Ltd., ABCR GmbH & Co. KG, Acros Organnics, Aldrich Chemical Company, Accela ChemBio Inc., Bide Pharmatech Co., and the like.

Unless otherwise stated, the reactions were carried out in a nitrogen atmosphere.

"Nitrogen atmosphere" means that a reaction flask is equipped with a nitrogen balloon (about 1 L).

"Hydrogen atmosphere" means that a reaction flask is equipped with a hydrogen balloon (about 1 L).

Hydrogen was produced by a QPH-1L hydrogen generator from Shanghai Quan Pu Scientific Instruments Co., Ltd.

In nitrogen atmosphere or hydrogen atmosphere, the reaction system was generally vacuumed and filled with nitrogen or hydrogen, and the above operation was repeated three times.

Unless otherwise stated, the solution refers to an aqueous solution.

Unless otherwise stated, the reaction temperature is room temperature from 20°C to 30°C.

The reaction process in the examples was monitored by thin layer chromatography (TLC).

### Example 1

### (S)-4-Amino-N-(1-cyano-2-(1-oxo-2-phenylisoindolin-5-yl)ethyl)tetrahydro-2H-pyran-4-carboxamide

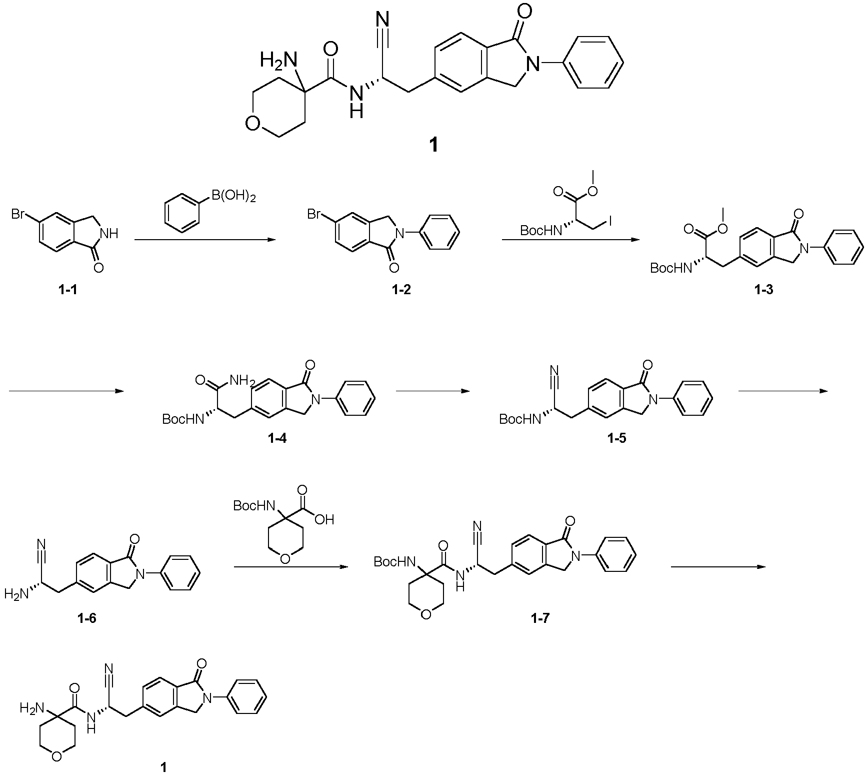

### Synthesis of compound 1-2

At room temperature, compound **1-1** (5 g, 23.70 mmol) and phenylboronic acid (5.80 g, 47.52 mmol) were dissolved in dichloromethane (100 mL), followed by the addition of triethylamine (7.20 g, 71.15 mmol) and copper acetate (8.60 g, 47.35 mmol). The reaction mixture was heated to reflux and stirred for 16 hours. The reaction mixture was cooled to room temperature and filtered, and the filtrate was concentrated under reduced pressure. The resulting residue was purified by column chromatography (SiO₂, dichloromethane/methanol = 9/1) to obtain compound **1-2.** MS-ESI: *m*/*z* 290.1 [M+1]⁺.

### Synthesis of compound 1-3

At room temperature and in a nitrogen atmosphere, activated zinc powder (900 mg, 137.76 mmol) was added to anhydrous *N*,*N*-dimethylformamide (5 mL), iodine (350 mg, 1.38 mmol) was then added, and the reaction mixture was stirred at room temperature for 30 minutes. A solution of methyl (*R*)-2-((*tert*-butoxycarbonyl)amino)-3-iodopropanoate (1.50 g, 4.55 mmol) in anhydrous *N*,*N*-dimethylformamide (1 mL) was added, and the reaction mixture was stirred at room temperature for 1 hour. Compound **1-2** (1.70 g, 5.92 mmol), tris-(dibenzylideneacetone)dipalladium (105 mg, 0.11 mmol) and 2-bicyclohexylphosphino-2',6'-dimethoxybiphenyl (94 mg, 0.23 mmol) were added, and the reaction mixture was heated to 60°C and stirred for 3 hours. After the reaction was complete, water (10 mL) and ethyl acetate (50 mL) were added, and the reaction mixture was filtered and separated into two phases, and the organic phase was washed with saturated brine (20 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **1-3.** MS-ESI: *m*/*z* 411.2 [M+1]⁺.

### Synthesis of compound 1-4

At room temperature, compound **1-3** (1.50 g, 3.66 mmol) was dissolved in a 7 M solution of ammonia in methanol (15 mL), and the reaction mixture was stirred at room temperature for 12 hours. The reaction mixture was concentrated under reduced pressure to obtain the crude compound **1-4,** which was directly used in the next step. MS-ESI: *m*/*z* 396.3 [M+1]⁺.

### Synthesis of compound 1-5

At room temperature, the crude compound **1-4** (1.00 g, 2.53 mmol) was dissolved in dichloromethane (30 ml), followed by the addition of Burgess reagent (970 mg, 4.06 mmol). The reaction mixture was stirred at room temperature for 16 hours. The reaction mixture was poured into water (100 mL), extracted with dichloromethane (250 mL×2), washed with saturated brine (100 mL), dried over anhydrous sodium sulfate and filtered. The filtrate was concentrated under reduced pressure, and the resulting residue was purified by column chromatography (SiO₂, dichloromethane/methanol=9/1) to obtain the crude compound **1-5,** which was directly used in the next step. MS-ESI: *m*/*z* 378.2 [M+1]⁺.

### Synthesis of compound 1-6

At room temperature, the crude compound **1-5** (300 mg, 0.80 mmol) was dissolved in acetonitrile (5 mL), followed by successive addition of trimethylchlorosilane (358 mg, 3.29 mmol) and sodium iodide (262 mg, 1.75 mmol). The reaction mixture was stirred at room temperature for 1 hour. After the reaction was complete, methanol (5 mL) was added, and the reaction mixture was filtered. The filtrate was concentrated under reduced pressure to obtain the crude compound **1-6,** which was directly used in the next step. MS-ESI: *m*/*z* 278.2 [M+1]⁺.

### Synthesis of compound 1-7

At room temperature, the crude compound **1-6** (180 mg, 0.65 mmol) and 4-((*tert-*butoxycarbonyl)amino)tetrahydro-2*H*-pyran-4-carboxylic acid (175 mg, 0.71 mmol) were dissolved in *N*,*N*-dimethylformamide (5 mL), followed by the addition of O-benzotriazole-*N*,*N*,*N'*,*N'*-tetramethyluronium hexafluorophosphate (370 mg, 0.98 mmol) and *N*,*N*-diisopropylethylamine (168 mg, 1.35 mmol). The reaction mixture was stirred at room temperature for 16 hours. The reaction mixture was poured into saturated aqueous sodium bicarbonate solution (20 mL), extracted with ethyl acetate (50 mL×3), washed with saturated brine (50 mL), dried over anhydrous sodium sulfate and filtered. The filtrate was concentrated under reduced pressure, and the resulting residue was purified by column chromatography (SiO₂, dichloromethane/methanol=9/1) to obtain compound **1-7.** MS-ESI: *m*/*z* 449.2 [M-56+1]⁺.

### Synthesis of compound 1

At room temperature, compound **1-7** (150 mg, 0.30 mmol) was dissolved in acetonitrile (10 mL), followed by successive addition of trimethylchlorosilane (447 mg, 4.11 mmol) and sodium iodide (324 mg, 2.16 mmol). The reaction mixture was stirred at room temperature for 1 hour. After the reaction was complete, methanol (5 mL) was added, the reaction mixture was concentrated under reduced pressure. Saturated sodium bicarbonate solution (20 mL) was added to the resulting residue, and the mixture was extracted with dichloromethane (50 mL×2), washed with saturated brine (50 mL), dried over anhydrous sodium sulfate and filtered. The filtrate was concentrated under reduced pressure, and the resulting residue was purified by preparative high performance liquid chromatography (column: Waters Xbridge C18, 250*19 mm, 10 µm; mobile phase: water (0.1% ammonium bicarbonate), acetonitrile; gradient: acetonitrile phase 35-95%; flow rate: 20 mL/min; column temperature: room temperature) to obtain compound **1.** MS-ESI: *m*/*z* 405.2 [M+1]⁺. ¹H NMR (400 MHz, CDCl₃) δ 8.30 (d, 1H), 7.91 (d, 1H), 7.86 (d, 2H), 7.50 (s, 1H), 7.47-7.43 (m, 2H), 7.37 (d, 1H), 7.20 (t, 1H), 5.18-5.13 (m, 1H), 4.88 (s, 2H), 3.94-3.87 (m, 2H), 3.64-3.56 (m, 2H), 3.24 (d, 2H), 2.34-2.26 (m, 1H), 2.24-2.16 (m, 1H), 1.44 (s, 2H), 1.30-1.26 (m, 1H), 1.21-1.16 (m, 1H).

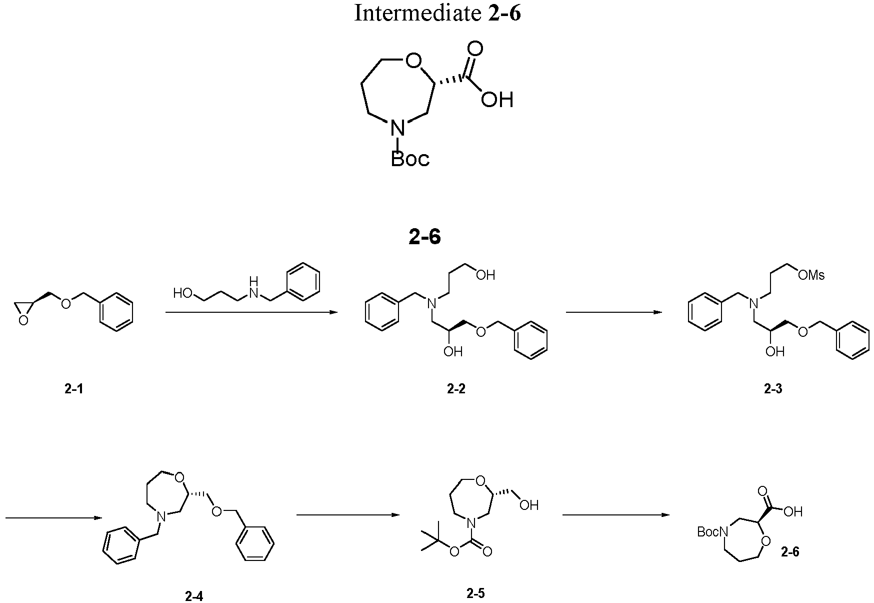

### Synthesis of compound 2-2

At room temperature, (*S*)-2-((benzyloxy)methyl)oxirane (compound **2-1**) (49.70 g, 302.90 mmol) and 3-(benzylamino)propan-1-ol (50 g, 302.81 mmol) were dissolved in ethanol (500 mL). The reaction mixture was heated to 40°C and stirred for 12 hours. The reaction mixture was cooled to room temperature and concentrated under reduced pressure. The resulting residue was purified by column chromatography (SiO₂, dichloromethane/methanol = 19/1) to obtain compound **2-2.** MS-ESI: *m*/*z* 330.2 [M+1]⁺.

### Synthesis of compound 2-3

At 0°C, compound **2-2** (52 g, 157.96 mmol) and *N*,*N*-diisopropylethylamine (30.60 g, 236.77 mmol) were dissolved in dichloromethane (1000 mL), then methanesulfonyl chloride (18.10 g, 158.01 mmol) was added dropwise. The reaction mixture was stirred at 0°C for 30 minutes. After the reaction was complete, the reaction mixture was poured into saturated aqueous sodium bicarbonate solution (1000 mL), and extracted with dichloromethane (500 mL × 3). The organic phases were combined, washed with saturated brine (200 mL), dried over anhydrous sodium sulfate and filtered. The filtrate was concentrated under reduced pressure to obtain the crude compound **2-3,** which was directly used in the next step. MS-ESI: *m*/*z* 408.2 [M+1]⁺.

### Synthesis of compound 2-4

At 0°C, the crude compound **2-3** (32 g, 78.59 mmol) was dissolved in tetrahydrofuran (500 mL), followed by the addition of sodium hydride (9.40 g, 235.90 mmol, 60%) in batches. The reaction mixture was stirred at room temperature for 16 hours. Sodium sulfate decahydrate was added, and the reaction mixture was filtered. The filtrate was concentrated under reduced pressure, and the resulting residue was purified by column chromatography (SiO₂, petroleum ether/ethyl acetate = 1/1) to obtain compound **2-4.** MS-ESI: *m*/*z* 312.1 [M+1]⁺.

### Synthesis of compound 2-5

At room temperature, compound **2-4** (28 g, 89.98 mmol) was dissolved in methanol (200 mL), followed by the addition of palladium hydroxide on carbon (2.80 g, 10%). The reaction mixture was stirred in a hydrogen atmosphere at room temperature for 48 hours. The reaction solution was filtered, and the filtrate was concentrated under reduced pressure. The resulting residue was dissolved in methanol (200 mL), followed by the addition of di-*tert*-butyl dicarbonate (27.90 g, 127.84 mmol). The reaction mixture was stirred at room temperature for 16 hours and concentrated under reduced pressure, and the resulting residue was purified by column chromatography (SiO₂, petroleum ether/ethyl acetate=1/1) to obtain compound **2-5.** MS-ESI: *m*/*z* 176.1 [M-56+1]⁺.

### Synthesis of compound 2-6

At 0°C, compound **2-5** (17 g, 73.6 mmol) was dissolved in acetone (1000 mL), followed by the addition of sodium bromide (2.30 g, 22.35 mmol), 2,2,6,6-tetramethylpiperidinooxy (1.20 g, 7.68 mmol) and saturated aqueous sodium bicarbonate solution (280 mL), and the reaction mixture was stirred at room temperature for 30 minutes. Trichloroisocyanuric acid (37.60 g, 161.78 mmol) was added, and the reaction mixture was stirred at room temperature for 16 hours. Isopropanol (50 mL) was added, and the reaction mixture was stirred for 30 minutes and filtered, and the filtrate was concentrated under reduced pressure. Water (300 mL) was added to the resulting residue, and the mixture was extracted with dichloromethane (500 mL×2). The organic phases were combined, washed with saturated brine (200 mL), dried over anhydrous sodium sulfate and filtered. The filtrate was concentrated under reduced pressure to obtain the crude compound **2-6,** which was directly used in the next step. MS-ESI: *m*/*z* 190.1 [M-56+1]⁺. ¹H NMR(400 MHz, DMSO-*d₆*): δ 12.71 (s, 1H), 4.20-4.17 (m, 1H), 3.96-3.79 (m, 2H), 3.65-3.54 (m, 2H), 3.48-3.42 (m, 1H), 3.18-3.07 (m, 1H), 1.71 (s, 2H), 1.40 (s, 9H).

### Example 2

### (S)-N-((S)-1-Cyano-2-(1-oxo-2-phenylisoindolin-5-yl)ethyl)-1,4-oxazepane-2-carboxamide

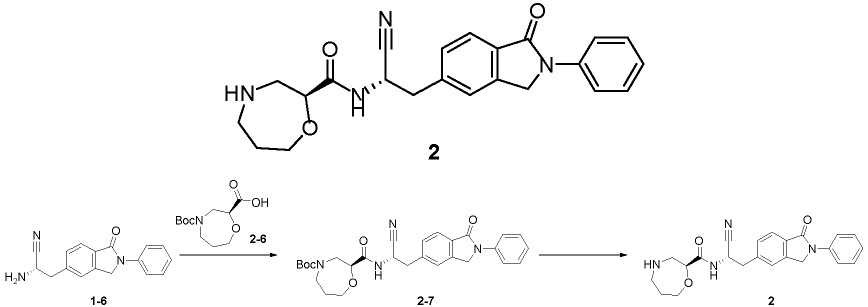

### Synthesis of compound 2-7

At room temperature, (*S*)-2-amino-3-(1-oxo-2-phenylisoindolin-5-yl)propanenitrile (compound **1-6**) (320 mg, 1.16 mmol) and compound **2-6** (311 mg, 1.27 mmol) were dissolved in dichloromethane (10 mL), followed by the addition of O-benzotriazole-*N*,*N*,*N'*,*N'*-tetramethyluronium hexafluorophosphate (659 mg, 1.74 mmol) and *N,N-*diisopropylethylamine (299 mg, 2.32 mmol). The reaction mixture was stirred at room temperature for 16 hours. The reaction solution was poured into saturated aqueous sodium bicarbonate solution (30 mL), and extracted with ethyl acetate (50 mL×3). The organic phases were combined, washed with saturated brine (20 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **2-7.** MS-ESI: *m*/*z* 449.2 [M-56+1]⁺.

### Synthesis of compound 2

At room temperature, compound **2-7** (200 mg, 0.39 mmol) was dissolved in acetonitrile (5 mL), followed by successive addition of trimethylchlorosilane (179 mg, 1.65 mmol) and sodium iodide (130 mg, 0.87 mmol). The reaction mixture was stirred at room temperature for 30 minutes. After the reaction was complete, methanol (5 mL) was added, and the reaction solution was concentrated under reduced pressure. Saturated aqueous sodium bicarbonate solution (20 mL) was added to the resulting residue, and the mixture was extracted with dichloromethane (20 mL×3). The organic phases were combined, washed with saturated brine (20 mL), dried over anhydrous sodium sulfate and filtered. The filtrate was concentrated under reduced pressure, and the resulting residue was purified by preparative high performance liquid chromatography (column: Waters sunfire C18, 250*19 mm, 10 µm; mobile phase: water (0.1% formic acid), acetonitrile; gradient: acetonitrile phase 25-95%; flow rate: 20 mL/min; column temperature: room temperature) to obtain compound **2.** MS-ESI: m/z 405.2 [M+H]⁺. ¹H NMR (400 MHz, DMSO-*d₆*) δ 8.87 (d, 1H), 7.92 (d, 2H), 7.74 (d, 1H), 7.58 (s, 1H), 7.47-7.43 (m, 3H), 7.18 (t, 1H), 5.13-5.05 (m, 1H), 5.05-4.96 (m, 2H), 4.20 (dd, 1H), 3.92-3.82 (m, 1H), 3.80-3.71 (m, 1H), 3.30-3.26 (m, 2H), 3.21-3.17 (m, 1H), 3.04-2.98 (m, 1H), 2.87-2.80 (m, 1H), 2.66 (dd, 1H), 1.86-1.85 (m, 2H).

### Example 3

### (2S)-N-(1-Cyano-2-(5,6,7,8-tetrahydronaphthalen-2-yl)ethyl)-1,4-oxazepane-2-carboxamide trifluoroacetate

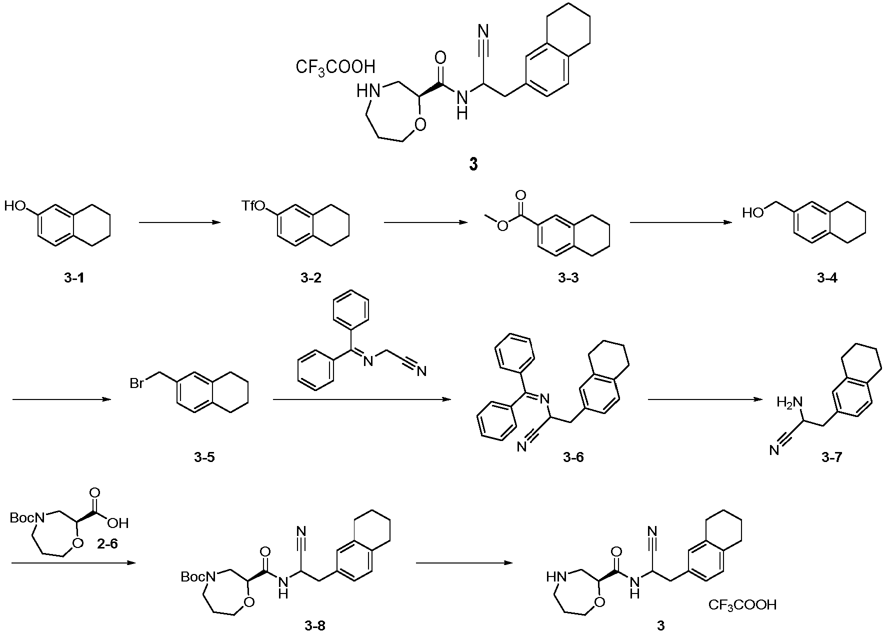

### Synthesis of compound 3-2

At room temperature, 5,6,7,8-tetrahydronaphthalen-2-ol (compound **3-1**) (10 g, 67.53 mmol) was dissolved in dichloromethane (100 mL), followed by successive addition of *N*,*N*-diisopropylethylamine (21.80 g, 168.68 mmol) and trifluoromethanesulfonic anhydride (22.85 g, 80.99 mmol) at 0°C. The reaction mixture was stirred at room temperature for 4 hours. After the reaction was complete, water (50 mL) was added, and the reaction mixture was extracted with dichloromethane (200 mL×2). The organic phases were combined, washed with saturated brine (100 mL), dried over anhydrous sodium sulfate and filtered. The filtrate was concentrated under reduced pressure to obtain the crude compound **3-2.** ¹H NMR (400 MHz, DMSO-*d₆*) δ 7.26-7.19 (m, 1H), 7.16-7.14 (m, 2H), 2.76-2.74 (m, 4H), 1.74-1.71 (m, 4H).

### Synthesis of compound 3-3

At room temperature, compound **3-2** (10 g, 35.59 mmol) was dissolved in a mixed solvent of *N*,*N*-dimethylformamide (50 mL) and methanol (150 mL), followed by the addition of *N*,*N*-diisopropylethylamine (9.23 g, 71.42 mmol) and [1,1'-bis(diphenylphosphino)ferrocene]palladium(II) dichloride (3.00 g, 4.10 mmol). The reaction mixture was stirred at reflux for 12 hours in a carbon monoxide atmosphere. The reaction mixture was cooled to room temperature, and concentrated under reduced pressure. Water (100 mL) was added to the resulting residue, and the mixture was extracted with ethyl acetate (200 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=8/1) to obtain compound **3-3.** ¹H NMR (400 MHz, DMSO-*d₆*) δ 7.69 -7.60 (m, 2H), 7.17 (d, 1H), 3.83 (s, 3H), 2.87-2.75 (m, 4H), 1.75-1.72 (m, 4H).

### Synthesis of compound 3-4

At 0°C and in a nitrogen atmosphere, compound **3-3** (3.10 g, 16.31 mmol) was dissolved in tetrahydrofuran (50 mL), followed by the addition of lithium aluminum hydride (928 mg, 24.45 mmol) in batches. The reaction mixture was stirred at 0°C for 3 hours. After the reaction was complete, water (100 mL) was added, and the reaction mixture was extracted with ethyl acetate (200 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=5/1) to obtain compound **3-4.** ¹H NMR (400 MHz, DMSO-*d₆*) δ 7.03-6.97 (m, 3H), 5.03 (s, 1H), 4.49 (s, 2H), 2.68-2.67 (m, 4H), 1.73-1.70 (m, 4H).

### Synthesis of compound 3-5

At 0°C, compound **3-4** (1.60 g, 9.87 mmol) was dissolved in toluene (20 mL), then phosphorus tribromide (4 g, 14.78 mmol) was added dropwise. The reaction mixture was stirred at room temperature for 12 hours. Water (50 mL) was added, and the reaction mixture was extracted with ethyl acetate (100 mL×2), washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=49/1) to obtain compound **3-5.** ¹H NMR (400 MHz, DMSO-*d₆*) δ 7.15-7.10 (m, 2H), 7.02 (d, 1H), 4.63 (s, 2H), 2.72-2.62 (m, 4H), 1.77-1.66 (m, 4H).

### Synthesis of compound 3-6

At 0°C and in a nitrogen atmosphere, 2-((diphenylmethylene)amino)acetonitrile (1.40 g, 6.26 mmol) was dissolved in *N*,*N*-dimethylformamide (20 mL), sodium hydride (500 mg, 12.50 mmol, 60%) was added, and the reaction mixture was stirred at 0°C for 0.5 hour. A solution of compound **3-5** (1.70 g, 7.58 mmol) in *N*,*N*-dimethylformamide (5 mL) was added, and the reaction mixture was stirred at 0°C for 2 hours. After the reaction was complete, saturated aqueous ammonium chloride solution (50 mL) was added, and the reaction mixture was extracted with ethyl acetate (100 mL×2), washed with saturated brine (100 mL), dried over anhydrous sodium sulfate and filtered. The filtrate was concentrated under reduced pressure, and the resulting residue was purified by column chromatography (SiO₂, petroleum ether/ethyl acetate) to obtain compound **3-6.** MS-ESI: *m*/*z* 365.2 [M+1]⁺.

### Synthesis of compound 3-7

At 0°C, compound **3-6** (1.80 g, 4.94 mmol) was dissolved in dioxane (10 mL), followed by the addition of 1 M hydrochloric acid (10 mL). The reaction mixture was stirred at 0°C for 4 hours. After the reaction was complete, water (20 mL) was added, and the reaction mixture was extracted with ethyl acetate (50 mL). The mixture was adjusted to pH=8 with saturated aqueous sodium bicarbonate solution, and extracted with dichloromethane (50 mL×2). The organic phases were combined, washed with saturated brine (100 mL), dried over anhydrous sodium sulfate and filtered. The filtrate was concentrated under reduced pressure to obtain the crude compound **3-7,** which was directly used in the next step. MS-ESI: *m*/*z* 184.1 [M-17+1]⁺.

### Synthesis of compound 3-8

At room temperature, the crude compound **3-7** (156 mg, 0.78 mmol) and compound **2-6** (160 mg, 0.65 mmol) were dissolved in N,N-dimethylformamide (5 mL), followed by the addition of O-benzotriazole-*N*,*N*,*N'*,*N'*-tetramethyluronium hexafluorophosphate (370 mg, 0.98 mmol) and *N*,*N*-diisopropylethylamine (170 mg, 1.32 mmol). The reaction mixture was stirred at room temperature for 16 hours. The reaction mixture was poured into saturated aqueous sodium bicarbonate solution (10 mL), and extracted with ethyl acetate (20 mL×3). The organic phases were combined, washed with saturated brine (20 mL×3), dried over anhydrous sodium sulfate and filtered. The filtrate was concentrated under reduced pressure to obtain the crude compound **3-8,** which was directly used in the next step. MS-ESI: *m*/*z* 372.1 [M-56+1]⁺.

### Synthesis of compound 3

At room temperature, the crude compound **3-8** (230 mg, 0.54 mmol) was dissolved in formic acid (5 ml), and the reaction mixture was heated to 50°C and stirred for 1 hour. After the reaction was complete, the reaction mixture was cooled to room temperature and concentrated under reduced pressure. The resulting residue was purified by preparative high performance liquid chromatography (trifluoroacetic acid/acetonitrile/water system) to obtain the trifluoroacetate salt of compound **3.** MS-ESI: *m*/*z* 328.2 [M+1]⁺. ¹H NMR (400 MHz, DMSO-*d₆*) δ 9.12-8.85 (m, 3H), 7.02-6.89 (m, 3H), 4.94-4.85 (m, 1H), 4.47-4.33 (m, 1H), 3.99-3.87 (m, 1H), 3.83-3.68 (m, 1H), 3.63-3.49 (m, 1H), 3.37-3.29 (m, 1H), 3.19-2.93 (m, 4H), 2.68 (s, 4H), 2.06-1.93 (m, 2H), 1.74 (s, 4H).

### Example 4

### 4-Amino-N-(1-cyano-2-(5,6,7,8-tetrahydronaphthalen-2-yl)ethyl)tetrahydro-2H-pyran-4-carboxamide

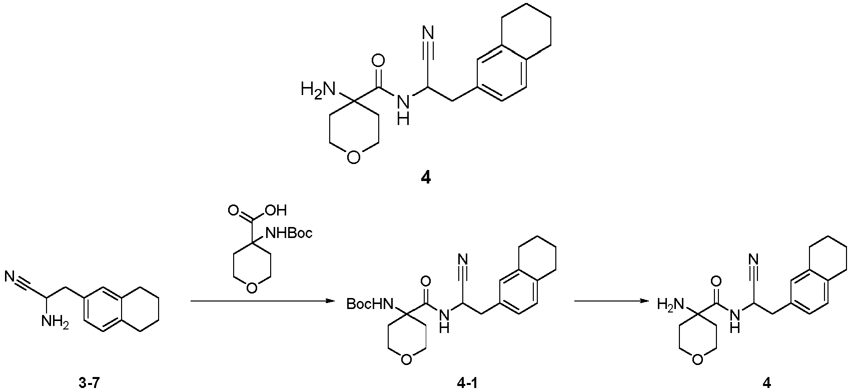

### Synthesis of compound 4-1

At room temperature, the crude 2-amino-3-(5,6,7,8-tetrahydronaphthalen-2-yl)propanenitrile (compound **3-7**) (150 mg, 0.75 mmol) and 4-((*tert-*butoxycarbonyl)amino)tetrahydro-2*H*-pyran-4-carboxylic acid (164 mg, 0.67 mmol) were dissolved in dichloromethane (5 mL), followed by the addition of O-benzotriazole-*N*,*N*,*N*',*N*'-tetramethyluronium hexafluorophosphate (345 mg, 0.91 mmol) and *N,N-*diisopropylethylamine (155 mg, 1.20 mmol). The reaction mixture was stirred at room temperature for 16 hours. The reaction mixture was poured into saturated aqueous sodium bicarbonate solution (20 mL), and extracted with ethyl acetate (20 mL×3). The organic phases were combined, washed with saturated brine (20 mL×3), dried over anhydrous sodium sulfate and filtered. The filtrate was concentrated under reduced pressure, and the resulting residue was purified by column chromatography (SiO₂, dichloromethane/methanol=10/1) to obtain compound **4-1.** MS-ESI: *m*/*z* 328.2 [M-100+1]⁺.

### Synthesis of compound 4

At room temperature, compound **4-1** (100 mg, 0.23 mmol) was dissolved in acetonitrile (5 mL), followed by successive addition of trimethylchlorosilane (76 mg, 0.70 mmol) and sodium iodide (105 mg, 0.70 mmol). The reaction mixture was stirred at room temperature for 1 hour. After the reaction was complete, methanol was added, and the reaction mixture was concentrated under reduced pressure. The resulting residue was poured into saturated aqueous sodium bicarbonate solution (20 mL), and extracted with dichloromethane (20 mL×3). The organic phases were combined, washed with saturated brine (20 mL×3), dried over anhydrous sodium sulfate and filtered. The filtrate was concentrated under reduced pressure, and the resulting crude residue was purified by preparative liquid chromatography (ammonium bicarbonate/acetonitrile/water system) to obtain compound **4.** MS-ESI: *m*/*z* 328.5 [M+1]⁺. ¹H NMR (400 MHz, DMSO-*d₆*) δ 7.00-6.93 (m, 3H), 4.88 (t, 1H), 3.67-3.55 (m, 3H), 3.52-3.48 (m, 1H), 3.09-2.95 (m, 2H), 2.67 (brs, 4H), 1.95-1.84 (m, 1H), 1.82-1.74 (m, 1H), 1.70 (brs, 4H), 1.27-1.10 (m, 2H).

### Example 5

### (2S)-N-(1-Cyano-2-(2,3-dihydro-1H-inden-5-yl)ethyl)-1,4-oxazepane-2-carboxamide trifluoroacetate

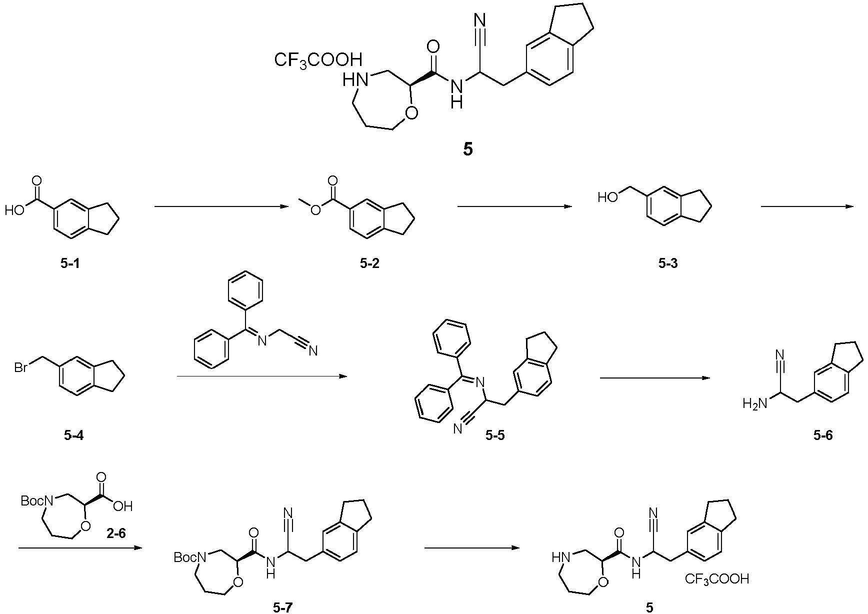

### Synthesis of compound 5-2

At room temperature, 2,3-dihydro-1*H*-indene-5-carboxylic acid (compound **5-1**) (2.00 g, 12.35 mmol) and iodomethane (1.2 mL, 19.27 mmol) were dissolved in *N,N-*dimethylformamide (10 mL), followed by the addition of potassium carbonate (3.41 g, 24.67 mmol). The reaction mixture was stirred at room temperature for 16 hours. The reaction mixture was poured into water (100 mL), and extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=3/2) to obtain compound **5-2.** ¹H NMR (400 MHz, DMSO-*d₆*) δ 7.80 (s, 1H), 7.75 (d, 1H), 7.35 (d, 1H), 3.83 (s, 3H), 2.92-2.88 (m, 4H), 2.12-1.98 (m, 2H).

### Synthesis of compound 5-3

At 0°C and in a nitrogen atmosphere, compound **5-2** (2.13 g, 12.10 mmol) was dissolved in tetrahydrofuran (20 mL), and then a solution of lithium aluminum hydride in tetrahydrofuran (11 ml, 33 mmol, 3.0 mol/L) was added dropwise. The reaction mixture was stirred at room temperature for 3 hours. After the reaction was complete, sodium sulfate decahydrate was added, and the reaction mixture was stirred for 0.5 hour and filtered. The filtrate was concentrated under reduced pressure to obtain compound **5-3.** ¹H NMR (400 MHz, DMSO-*d₆*) δ 7.16-7.14 (m, 2H), 7.05-7.03 (m, 1H), 5.06 (t, 1H), 4.43 (d, 2H), 2.87-2.78 (m, 4H), 2.08-1.93 (m, 2H).

### Synthesis of compound 5-4

At 0°C, compound **5-3** (1.60 g, 10.80 mmol) was dissolved in toluene (10 mL), then phosphorus tribromide (1.54 mL, 16.38 mmol) was added dropwise. The reaction mixture was stirred at room temperature for 12 hours. Water (50 mL) was added, and the reaction mixture was extracted with ethyl acetate (50 mL×2). The organic phases were combined, washed with water (20 mL) and saturated aqueous sodium chloride solution (20 mL) successively, dried over anhydrous sodium sulfate and filtered. The filtrate was concentrated under reduced pressure, and the resulting residue was purified by column chromatography (SiO₂, petroleum ether/ethyl acetate=9/1) to obtain compound **5-4.** ¹H NMR (400 MHz, DMSO-*d₆*) δ 7.35-7.10 (m, 3H), 4.67 (s, 2H), 2.82 (t, 4H), 2.08-1.96 (m, 2H).

### Synthesis of compound 5-5

At 0°C, 2-((diphenylmethylene)amino)acetonitrile (2.95 g, 13.40 mmol) was dissolved in *N*,*N*-dimethylformamide (10 mL), sodium hydride (1.07 g, 26.75 mmol, 60%) was added, and the reaction mixture was stirred at 0°C for 30 minutes. Compound **5-4** (1.87 g, 8.90 mmol) was added, and the reaction mixture was stirred at 0°C for 2 hours. After the reaction was complete, saturated aqueous sodium bicarbonate solution (50 mL) was added, and the reaction mixture was extracted with ethyl acetate (50 mL×2), washed with water (20 mL) and saturated brine (20 mL) successively, dried over anhydrous sodium sulfate and filtered. The filtrate was concentrated under reduced pressure, and the resulting residue was purified by column chromatography (SiO₂, petroleum ether/ethyl acetate=9/1) to obtain compound **5-5.** MS-ESI: *m*/*z* 351.2 [M+1]⁺.

### Synthesis of compound 5-6

At 0°C, compound **5-5** (1.05 g, 3.00 mmol) was dissolved in dioxane (10 mL), followed by the addition of 1 M hydrochloric acid (10 mL). The reaction mixture was stirred at 0°C for 4 hours. After the reaction was complete, saturated aqueous sodium bicarbonate solution (20 mL) was added, and the reaction mixture was extracted with ethyl acetate (50 mL×2). The organic phases were combined, washed with water (20 mL) and saturated brine (20 mL) successively, dried over anhydrous sodium sulfate and filtered. The filtrate was concentrated under reduced pressure, and the resulting residue was purified by column chromatography (SiO₂, petroleum ether/ethyl acetate=1/1) to obtain compound **5-6.** MS-ESI: *m*/*z* 187.2 [M+1]⁺.

### Synthesis of compound 5-7

At room temperature, compound **5-6** (100 mg, 0.54 mmol) and compound **2-6** (158 mg, 0.64 mmol) were dissolved in a mixed solvent of *N*,*N*-dimethylformamide (1 mL) and dichloromethane (5 mL), followed by the addition of O-benzotriazole-*N*,*N*,*N'*,*N'-*tetramethyluronium hexafluorophosphate (306 mg, 0.81 mmol) and *N,N-*diisopropylethylamine (139 mg, 1.08 mmol). The reaction mixture was stirred at room temperature for 16 hours. The reaction mixture was poured into saturated aqueous sodium bicarbonate solution (20 mL), and extracted with ethyl acetate (20 mL×3). The organic phases were combined, washed with saturated brine (20 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **5-7.** MS-ESI: *m*/*z* 358.2 [M-56+1]⁺.

### Synthesis of compound 5

At room temperature, compound **5-7** (100 mg, 0.24 mmol) was dissolved in acetonitrile (10 mL), followed by successive addition of trimethylchlorosilane (79 mg, 0.72 mmol) and sodium iodide (109 mg, 0.72 mmol). The reaction mixture was stirred at room temperature for 3 hours. After the reaction was complete, methanol (10 mL) was added, and the reaction mixture was concentrated under reduced pressure. Saturated aqueous sodium bicarbonate solution (20 mL) was added to the resulting residue, and the mixture was extracted with dichloromethane (20 mL×3). The organic phases were combined, washed with saturated brine (20 mL), dried over anhydrous sodium sulfate and filtered. The filtrate was concentrated under reduced pressure, and the resulting residue was purified by preparative high performance liquid chromatography (column: SunFire Prep C18, 250*19 mm, 10 µm; mobile phase: water (0.1% trifluoroacetic acid), acetonitrile; gradient: acetonitrile phase 28-95%; flow rate: 20 mL/min; column temperature: room temperature) to obtain the trifluoroacetate salt of compound **5.** MS-ESI: *m*/*z* 314.5 [M+1]⁺. ¹H NMR (400 MHz, DMSO-*d₆*) δ 9.20-8.70 (m, 3H), 7.19-7.11 (m, 2H), 7.02 (d, 1H), 5.00-4.82 (m, 1H), 4.46-4.32 (m, 1H), 4.01-3.85 (m, 1H), 3.84-3.70 (m, 1H), 3.61-3.46 (m, 1H), 3.22-2.90 (m, 5H), 2.83-2.80 (m, 4H), 2.03-1.88 (m, 4H).

### Example 6

### 4-Amino-N-(1-cyano-2-(2,3-dihydro-1H-inden-5-yl)ethyl)tetrahydro-2H-pyran-4-carboxamide

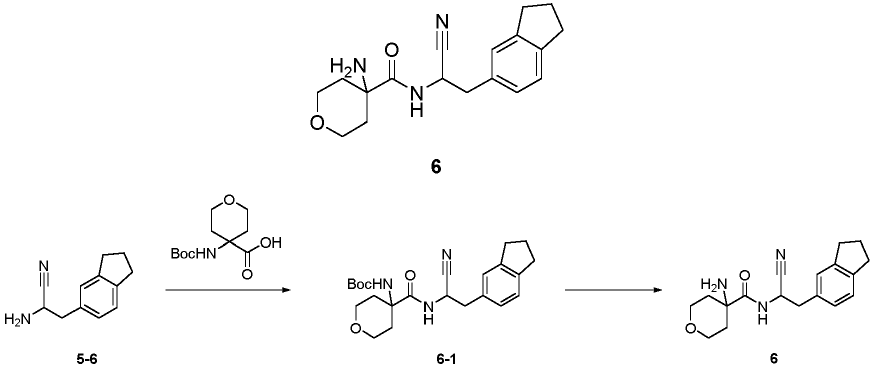

### Synthesis of compound 6-1

At room temperature, 2-amino-3-(2,3-dihydro-1*H*-inden-5-yl)propanenitrile (compound **5-6**) (150 mg, 0.81 mmol) and 4-((tert-butoxycarbonyl)amino)tetrahydro-2*H-*pyran-4-carboxylic acid (237 mg, 0.97 mmol) were dissolved in a mixed solvent of *N,N-*dimethylformamide (1 mL) and dichloromethane (5 mL), followed by the addition of O-benzotriazole-*N*,*N*,*N*',*N*'-tetramethyluronium hexafluorophosphate (458 mg, 1.21 mmol) and *N*,*N*-diisopropylethylamine (208 mg, 1.61 mmol). The reaction mixture was stirred at room temperature for 16 hours. The reaction mixture was poured into saturated aqueous sodium bicarbonate solution (20 mL), extracted with ethyl acetate (20 mL×3), washed with saturated brine (20 mL×3), dried over anhydrous sodium sulfate and filtered. The filtrate was concentrated under reduced pressure, and the resulting residue was purified by column chromatography (SiO₂, petroleum ether/ethyl acetate=1/1) to obtain compound **6-1.** MS-ESI: *m*/*z* 414.2 [M+1]⁺.

### Synthesis of compound 6

At room temperature, compound **6-1** (180 mg, 0.44 mmol) was dissolved in acetonitrile (5 mL), followed by successive addition of trimethylchlorosilane (0.2 mL, 1.58 mmol) and sodium iodide (195 mg, 1.31 mmol). The reaction mixture was stirred at room temperature for 2 hours. After the reaction was complete, methanol (10 mL) was added dropwise, and the reaction mixture was concentrated under reduced pressure. Saturated aqueous sodium bicarbonate solution (20 mL) was added to the resulting residue, and the mixture was extracted with dichloromethane (20 mL×3). The organic phases were combined, washed with saturated brine (20 mL×3), dried over anhydrous sodium sulfate and filtered. The filtrate was concentrated under reduced pressure, and the resulting residue was purified by preparative high performance liquid chromatography (ammonium bicarbonate/acetonitrile/water system) to obtain compound **6.** MS-ESI: *m*/*z* 314.5 [M+1]⁺. ¹H NMR (400 MHz, DMSO-*d₆*) δ 7.19-7.07 (m, 2H), 7.05-6.99 (m, 1H), 4.89 (t, 1H), 3.70-3.54 (m, 3H), 3.51-3.47 (m, 1H), 3.16-2.98 (m, 2H), 2.84-2.76 (m, 4H), 2.06-1.84 (m, 3H), 1.85-1.68 (m, 1H), 1.23-1.15 (m, 2H).

### Example 7

### 4-Amino-N-(1-cyano-2-(9,10-dihydrophenanthren-2-yl)ethyl)tetrahydro-2H-pyran-4-carboxamide

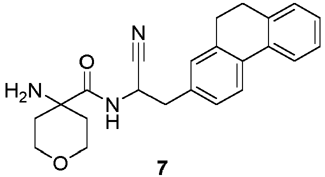

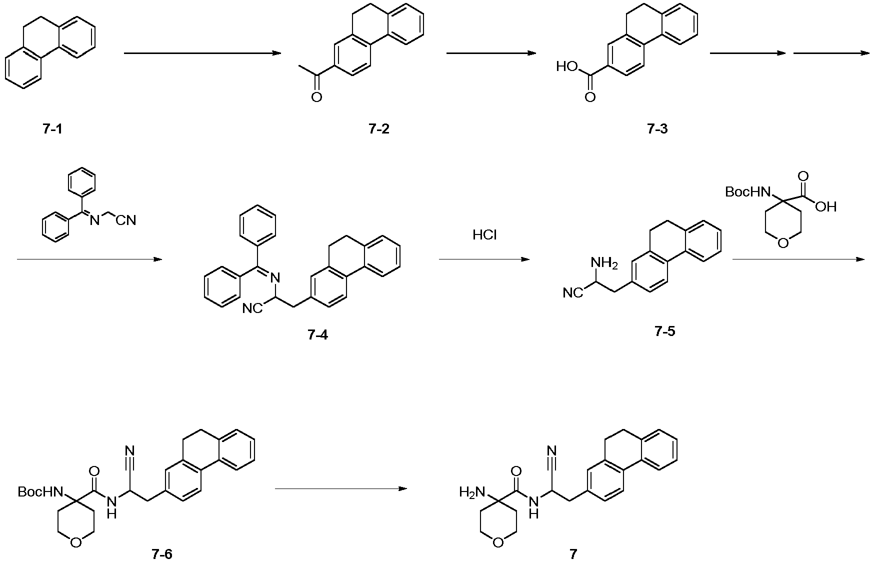

### Synthesis of compound 7-2

At room temperature, 9,10-dihydrophenanthrene (compound **7-1**) (2.00 g, 11.11 mmol) was dissolved in anhydrous dichloromethane (60 mL), followed by the addition of acetyl chloride (1.20 g, 15.28 mmol). Aluminum trichloride (2.00 g, 15.00 mmol) was added in batches at 0°C, and the reaction mixture was stirred at room temperature for 12 hours. The reaction mixture was poured into ice water (50 mL), extracted with dichloromethane (50 mL×2), washed with saturated brine (50 mL), dried over anhydrous sodium sulfate and filtered. The filtrate was concentrated under reduced pressure to obtain the crude compound **7-2.** MS-ESI: *m*/*z* 223.0 [M+1]⁺.

### Synthesis of compound 7-3

At 0°C, potassium hydroxide (3.30 g, 58.81 mmol) was dissolved in water (20 mL), followed by successive addition of liquid bromine (0.9 mL, 17.56 mmol) and a solution of compound **7-2** (1.00 g, 4.50 mmol) in 1,4-dioxane (10 mL). The reaction mixture was heated to 55°C and stirred for 16 hours. The reaction mixture was cooled to room temperature, and saturated aqueous sodium bisulfite solution (20 mL) was added. The mixture was adjusted to pH=1 with concentrated hydrochloric acid, and extracted with dichloromethane (50 mL×2). The organic phases were combined, dried over anhydrous magnesium sulfate and filtered. The filtrate was concentrated under reduced pressure, and the resulting residue was purified by column chromatography to obtain compound **7-3.** MS-ESI: *m*/*z* 225.2 [M+1]⁺.

### Synthesis of compound 7-4

At 0°C, compound **7-3** (930 mg, 4.15 mmol) was dissolved in tetrahydrofuran (10 ml), then carbonyldiimidazole (874 mg, 5.39 mmol) was added in batches, and the reaction mixture was stirred at room temperature for 1 hour. Sodium borohydride (470 mg, 12.42 mmol) was added, and the reaction mixture was stirred at room temperature for 16 hours. The reaction mixture was poured into ice water (50 mL), and extracted with dichloromethane (50 mL×2). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=3/1) to obtain the intermediate which was used in the next step.

At 0°C, the intermediate obtained in the above step (600 mg, 2.86 mmol) was dissolved in dichloromethane (10 ml), followed by successive addition of *N,N-*diisopropylethylamine (738 mg, 5.72 mmol) and methanesulfonyl chloride (493 mg, 4.29 mmol). The reaction mixture was stirred at 0°C for 2 hours. After the reaction was complete, the reaction mixture was poured into ice water (50 mL), and extracted with dichloromethane (50 mL×2). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain the intermediate which was used in the next step.

At 0°C, 2-((diphenylmethylene)amino)acetonitrile (462 mg, 2.10 mmol) was dissolved in *N*,*N*-dimethylformamide (3 mL), then sodium hydride (140 mg, 3.50 mmol, 60%) was added, and the reaction mixture was stirred at room temperature for 0.5 hours. The intermediate obtained in the above step (400 mg, 1.75 mmol) was added, and the reaction mixture was stirred at 0°C for 2 hours. After the reaction was complete, saturated aqueous ammonium chloride solution (50 mL) was added, and the reaction mixture was extracted with dichloromethane (50 mL×2). The organic phases were combined, washed with saturated brine (20 mL), dried over anhydrous sodium sulfate and filtered. The filtrate was concentrated under reduced pressure to obtain the crude compound **7-4,** which was directly used in the next step. MS-ESI: *m*/*z* 413.1 [M+1]⁺.

### Synthesis of compound 7-5

At 0°C, the crude compound **7-4** (720 mg, 1.75 mmol) was dissolved in dioxane (2 mL), followed by the addition of 4 M hydrochloric acid (1 mL). The reaction mixture was stirred at 0°C for 1 hour. After the reaction was complete, saturated aqueous sodium bicarbonate solution (20 mL) was added, and the reaction mixture was extracted with dichloromethane (50 mL×2). The organic phases were combined, washed with saturated brine (20 mL), dried over anhydrous sodium sulfate and filtered. The filtrate was concentrated under reduced pressure, and the resulting residue was purified by column chromatography (SiO₂, dichloromethane/methanol=19/1) to obtain compound **7-5** (150 mg). MS-ESI: *m*/*z* 232.2 [M-17+1]⁺.

### Synthesis of compound 7-6

At room temperature, compound **7-6** (150 mg, 0.60 mmol) and 4-((*tert-*butoxycarbonyl)amino)tetrahydro-2*H*-pyran-4-carboxylic acid (162 mg, 0.66 mmol) were dissolved in *N*,*N*-dimethylformamide (5 mL), followed by the addition of O-benzotriazole-*N*,*N*,*N*',*N*'-tetramethyluronium hexafluorophosphate (341 mg, 0.9 mmol) and N,N-diisopropylethylamine (155 mg, 1.20 mmol). The reaction mixture was stirred at room temperature for 16 hours. The reaction mixture was poured into saturated aqueous sodium bicarbonate solution (20 mL), and extracted with dichloromethane (20 mL×3). The organic phases were combined, washed with saturated brine (20 mL), dried over anhydrous sodium sulfate and filtered. The filtrate was concentrated under reduced pressure, and the resulting residue was purified by column chromatography (SiO₂, dichloromethane/methanol=19/1) to obtain compound **7-6.** MS-ESI: *m*/*z* 420.5 [M-56+1]⁺.

### Synthesis of compound 7

At room temperature, compound **7-6** (75 mg, 0.16 mmol) was dissolved in acetonitrile (5 mL), followed by successive addition of trimethylchlorosilane (72 mg, 0.66 mmol) and sodium iodide (52 mg, 0.35 mmol). The reaction mixture was stirred at room temperature for 30 minutes. After the reaction was complete, methanol (5 mL) was added, and the reaction mixture was concentrated under reduced pressure. Saturated aqueous sodium bicarbonate solution (10 mL) was added to the resulting residue, and the mixture was extracted with dichloromethane (20 mL×3).The organic phases were combined, washed with saturated brine (20 mL), dried over anhydrous sodium sulfate and filtered. The filtrate was concentrated under reduced pressure, and the resulting residue was purified by preparative high performance liquid chromatography (column: Waters Xbridge C18, 250*19 mm, 10 µm; mobile phase: water (0.1% ammonium bicarbonate), acetonitrile; gradient: acetonitrile phase 52-95%; flow rate: 20 mL/min; column temperature: room temperature) to obtain compound **7.** MS-ESI: *m*/*z* 376.7 [M+1]⁺. ¹H NMR (400 MHz, DMSO-*d₆*) δ 7.80-7.76 (m, 2H), 7.32-7.22 (m, 4H), 7.18-7.17 (m, 1H), 4.99-4.96 (m, 1H), 3.64-3.53 (m, 3H), 3.48-3.45 (m, 1H), 3.19-3.04 (m, 2H), 2.80-2.76 (m, 4H), 1.93-1.86 (m, 1H), 1.80-1.71 (m, 1H), 1.22-1.13 (m, 2H).

### Example 8

### (S)-N-((S)-1-Cyano-2-(9,10-dihydrophenanthren-2-yl)ethyl)-1,4-oxazepane-2-carboxamide

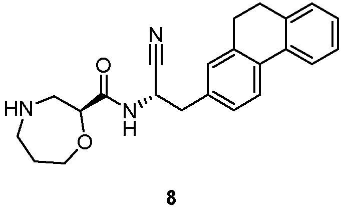

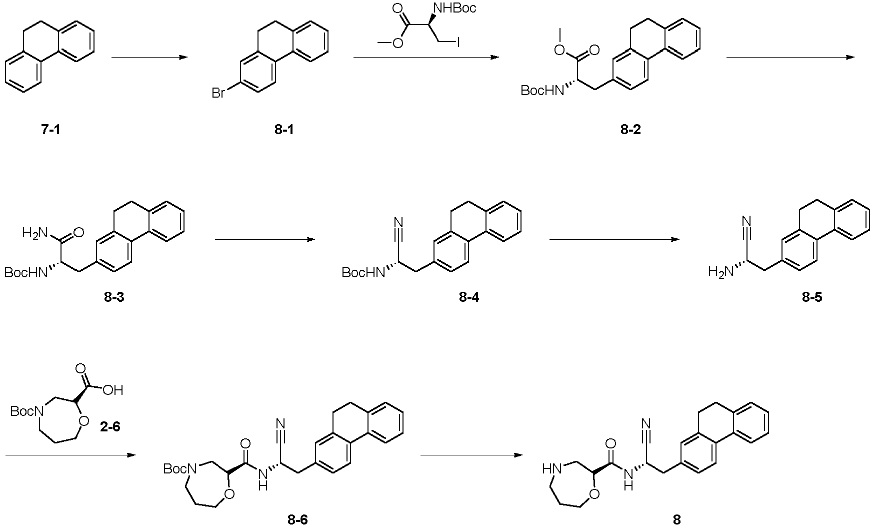

### Synthesis of compound 8-1

At room temperature, compound **7-1** (2.50 g, 13.89 mol) was dissolved in acetonitrile (20 mL), followed by the addition of *N*-bromosuccinimide (2.70 g, 15.17 mmol) and *p*-toluenesulfonic acid (67 mg, 0.39 mmol). The reaction mixture was heated to 50°C and stirred for 16 hours. The reaction mixture was cooled to room temperature, followed by the addition of saturated aqueous sodium bicarbonate solution (50 mL), and extracted with ethyl acetate (50 mL×3).The organic phases were combined, washed with saturated brine (20 mL), dried over anhydrous sodium sulfate and filtered. The filtrate was concentrated under reduced pressure, and the resulting residue was purified by column chromatography (SiO₂, petroleum ether) to obtain compound **8-1.** ¹H NMR (400 MHz, CDCl₃) δ 7.70 (d, 1H), 7.60 (d, 1H), 7.43-7.40 (m, 1H), 7.38 (d, 1H), 7.27-7.19 (m, 3H), 2.87-2.83 (m, 4H).

### Synthesis of compound 8-2

It was prepared with reference to the synthesis of compound **1-3.** MS-ESI: *m*/*z* 282.2 [M-100+1]⁺.

### Synthesis of compound 8-3

It was prepared with reference to the synthesis of compound **1-4.** MS-ESI: *m*/*z* 267.2 [M-100+1]⁺.

### Synthesis of compound 8-4

It was prepared with reference to the synthesis of compound **1-5.** MS-ESI: *m*/*z 349.2* [M+1]⁺.

### Synthesis of compound 8-5

It was prepared with reference to the synthesis of compound **1-6.** MS-ESI: *m*/*z* 232.2 [M-17+1]⁺.

### Synthesis of compound 8-6

It was prepared with reference to the synthesis of compound **1-7** with similar conditions except that *N*,*N*-dimethylformamide was replaced with dichloromethane. MS-ESI: *m*/*z* 420.1 [M-56+1]⁺.

### Synthesis of compound 8

It was prepared with reference to the synthesis of compound 1. MS-ESI: *m*/*z* 376.2 [M+1]⁺. ¹H NMR (400 MHz, CDCl₃) δ 7.74-7.72 (m, 2H), 7.33-7.28 (m, 1H), 7.25-7.23 (m, 2H), 7.19-7.17 (m, 2H), 5.21-5.15 (m, 1H), 4.10-4.07 (m, 1H), 3.99-3.94 (m, 1H), 3.77-3.69 (m, 1H), 3.33-3.28 (m, 1H), 3.10 (d, 1H), 3.04-.99 (m, 1H), 2.95-2.83 (m, 6H), 1.87-1.76 (m, 2H).

### Example 9

### (S)-N-((S)-1-Cyano-2-(2-methyl-1,2,3,4-tetrahydroisoquinolin-6-yl)ethyl)-1,4-oxazepane-2-carboxamide

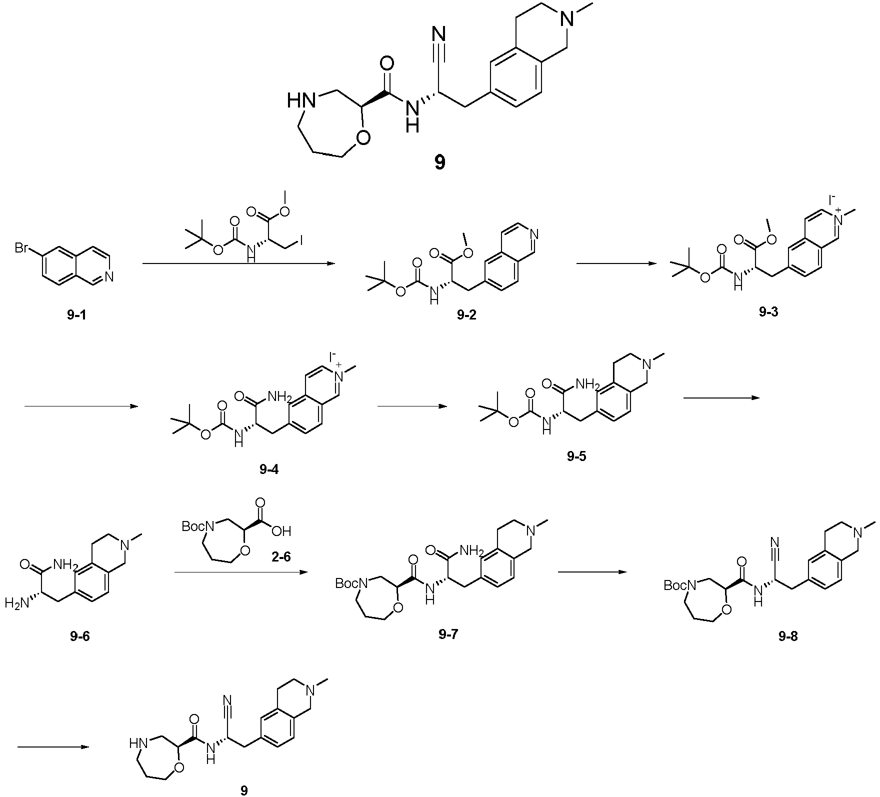

### Synthesis of compound 9-2

Compound **9-2** was prepared with reference to the synthesis of compound **1-3.** MS-ESI: *m*/*z* 331.2 [M+1]⁺.

### Synthesis of compound 9-3

At room temperature, compound **9-2** (1.50 g, 4.44 mmol) was dissolved in tetrahydrofuran (20 mL), followed by the addition of iodomethane (2.97 g, 20.92 mmol). The reaction mixture was stirred at room temperature for 12 hours, and concentrated under reduced pressure to obtain the crude compound **9-3,** which was directly used in the next step. MS-ESI: *m*/*z* 345.4 [M-127]⁺.

### Synthesis of compound 9-4

At room temperature, the crude compound **9-3** (1.70 g, 3.60 mmol) was dissolved in a 7 M solution of ammonia in methanol (30 mL), and the reaction mixture was stirred at room temperature for 12 hours, and concentrated under reduced pressure to obtain the crude compound **9-4,** which was directly used in the next step. MS-ESI: *m*/*z* 330.1 [M-127]⁺.

### Synthesis of compound 9-5

At room temperature, the crude compound **9-4** (1.20 g, 2.62 mmol) was dissolved in methanol (10 mL), followed by the addition of a small amount of platinum dioxide on carbon. The reaction mixture was stirred in a hydrogen atmosphere at room temperature for 12 hours and filtered. The filtrate was concentrated under reduced pressure, and the resulting residue was purified by column chromatography (SiO₂, dichloromethane/methanol=7/3) to obtain compound **9-5.** MS-ESI: *m*/*z* 334.2 [M+1]⁺.

### Synthesis of compound 9-6

At room temperature, compound **9-5** (530 mg, 1.54 mmol) was dissolved in a solution (10 mL) of hydrogen chloride in dioxane, and the reaction mixture was stirred at room temperature for 3 hours. After the reaction was complete, the reaction mixture was concentrated under reduced pressure to obtain the crude compound **9-6,** which was directly used in the next step. MS-ESI: *m*/*z* 234.2 [M+1]⁺.

### Synthesis of compound 9-7

It was prepared with reference to the synthesis of compound **1-7** with similar conditions except that *N*,*N*-dimethylformamide was replaced with a mixed solvent of *N*,*N-*dimethylformamide and dichloromethane (1/3). MS-ESI: *m*/*z* 461.3 [M+1]⁺.

### Synthesis of compound 9-8

It was prepared with reference to the synthesis of compound **1-5.** MS-ESI: *m*/*z 443.6* [M+1]⁺.

### Synthesis of compound 9

It was prepared with reference to the synthesis of compound **1.** MS-ESI: *m*/*z 343.5* [M+1]⁺. ¹H NMR (400 MHz, DMSO-*d₆*) δ 8.55 (d, 1H), 7.08-6.92 (m, 3H), 4.93-4.88 (m,1H), 3.98 (dd, 1H), 3.85-3.80 (m, 1H), 3.76-3.68 (m, 1H), 3.43 (s, 2H), 3.10-2.99 (m, 3H), 2.81-2.76 (m, 3H), 2.59-2.50 (m, 4H), 2.30 (s, 3H), 1.81-1.67 (m, 2H).

### Example 10

### (S)-4-Amino-N-(2-(chroman-7-yl)-1-cyanoethyl)tetrahydro-2H-pyran-4-carboxamide

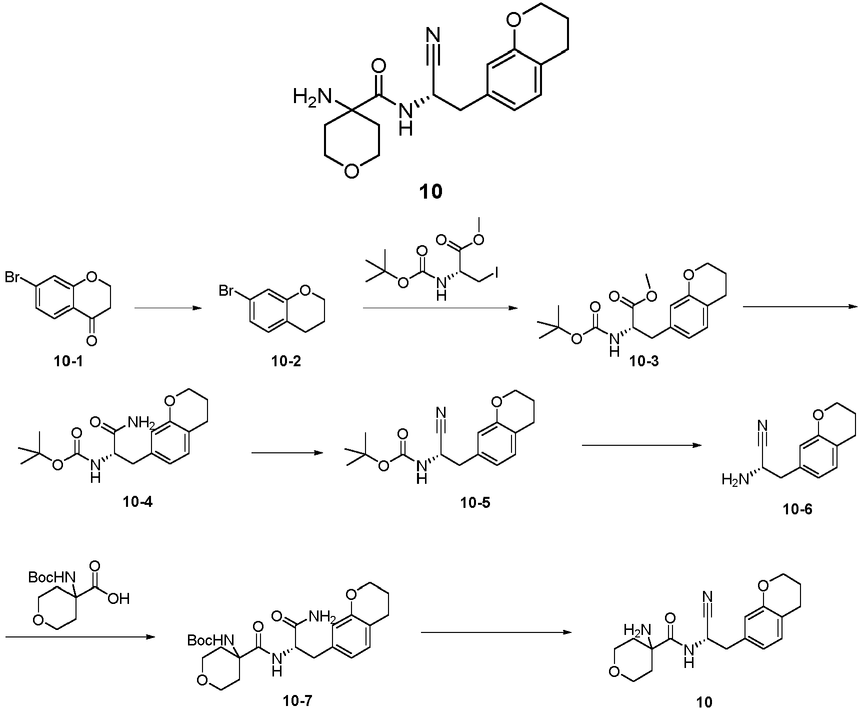

### Synthesis of compound 10-2

At room temperature and in a nitrogen atmosphere, 7-bromochroman-4-one (compound **10-1)** (3.00 g, 13.21 mmol) was dissolved in trifluoroacetic acid (50 mL), followed by the addition of triethylsilane (4.61 g, 39.64 mmol). The reaction mixture was stirred at room temperature for 16 hours and concentrated under reduced pressure, and the resulting residue was purified by column chromatography (SiO₂, petroleum ether/ethyl acetate=5/1) to obtain compound **10-2.**

### Synthesis of compound 10-3

It was prepared with reference to the synthesis of compound **1-3.** MS-ESI: *m*/*z 236.1* [M-100+1]⁺.

### Synthesis of compound 10-4

It was prepared with reference to the synthesis of compound **1-4.** MS-ESI: *m*/*z 321.2* [M+1]⁺.

### Synthesis of compound 10-5

It was prepared with reference to the synthesis of compound **1-5.** MS-ESI: *m*/*z 247.4* [M-56+1]⁺.

### Synthesis of compound 10-6

At room temperature, compound **10-5** (200 mg, 0.66 mmol) was dissolved in a solution (5 mL) of hydrogen chloride in dioxane, and the reaction mixture was stirred at room temperature for 2 hours. After the reaction was complete, the reaction mixture was concentrated under reduced pressure to obtain the crude compound **10-6,** which was directly used in the next step. MS-ESI: *m*/*z* 203.5 [M+1]⁺.

### Synthesis of compound 10-7

It was prepared with reference to the synthesis of compound **1-7** with similar conditions except that *N*,*N*-dimethylformamide was replaced with a mixed solvent of *N*,*N*-dimethylformamide and dichloromethane (1/2). MS-ESI: *m*/*z* 448.3 [M+1]⁺.

### Synthesis of compound 10

It was prepared with reference to the synthesis of compound 1. MS-ESI: *m*/*z 330.4* [M+1]⁺. ¹H NMR (400 MHz, DMSO-*d₆*) δ 8.18 (s, 1H), 6.97 (d, 1H), 6.71 (d, 1H), 6.66 (s, 1H), 4.88 (t, 1H), 4.16-4.06 (m, 2H), 3.65-3.56 (m, 3H), 3.52-3.44 (m, 1H), 3.07-2.94 (m, 2H), 2.69 (t, 2H), 1.94-1.82 (m, 3H), 1.80-1.73 (m, 1H), 1.22-1.14 (m, 2H).

### Example 11

### (2S)-N-(1-Cyano-2-(8-fluoro-6H-benzo[c]chromen-3-yl)ethyl)-1,4-oxazepane-2-carboxamide formate

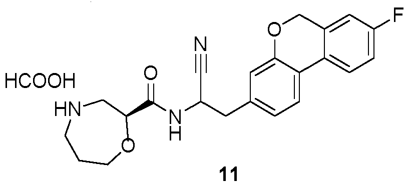

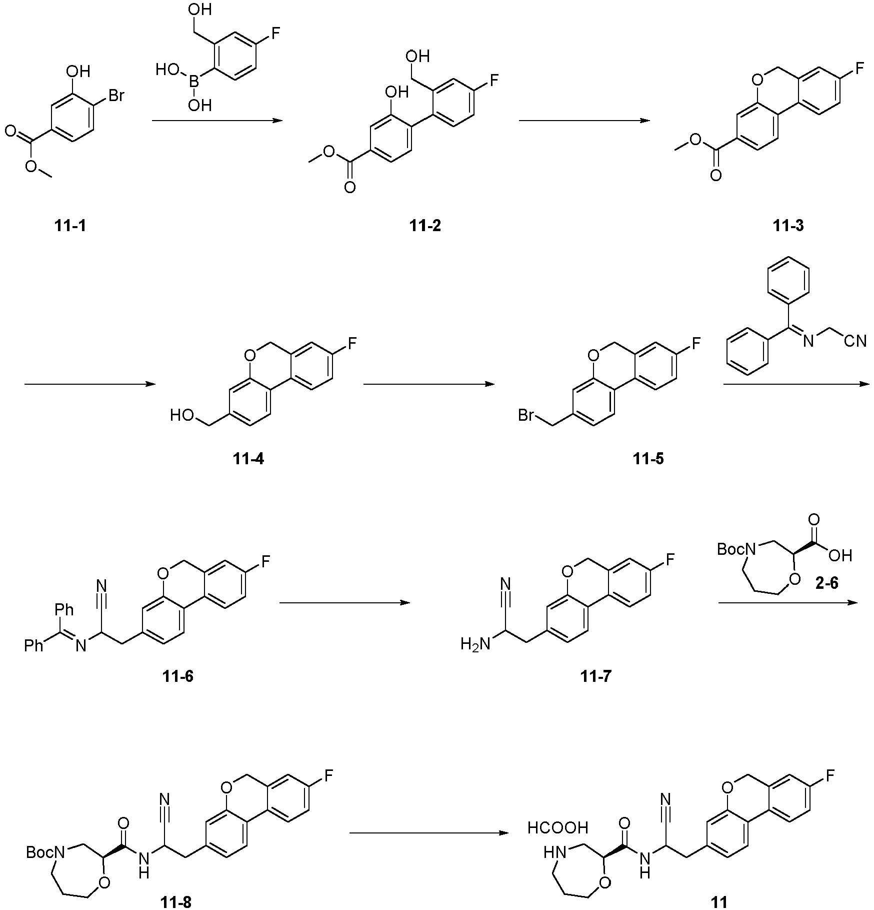

### Synthesis of compound 11-2

At room temperature and in a nitrogen atmosphere, methyl 4-bromo-3-hydroxybenzoate (compound **11-1)** (3.00 g, 13.05 mmol), (4-fluoro-2-(hydroxymethyl)phenyl)boronic acid (2.64 g, 15.52 mmol), sodium bicarbonate (2.18 g, 25.95 mmol) and [1,1'-bis(diphenylphosphino)ferrocene]palladium(II) dichloride (537 mg, 0.73 mmol) were dissolved in a mixed solvent of dioxane (28 mL) and water (7 mL). The reaction mixture was heated to 100°C and stirred for 12 hours. The reaction mixture was cooled to room temperature and filtered, and the filtrate was concentrated under reduced pressure. The resulting residue was purified by column chromatography (SiO₂, petroleum ether/ethyl acetate=3/2) to obtain compound **11-2.** MS-ESI: *m*/*z* 259.1 [M-18+1]⁺.

### Synthesis of compound 11-3

At room temperature, compound **11-2** (800 mg, 2.90 mmol) and triphenylphosphine (0.900 g, 3.43 mmol) were dissolved in toluene (10 mL), then diethyl azodicarboxylate (1.52 g, 8.73 mmol) was added dropwise. The reaction mixture was heated to 100°C and stirred for 3 hours. After the reaction was complete, the reaction mixture was cooled to room temperature and concentrated under reduced pressure. The resulting residue was purified by column chromatography (SiO₂, petroleum ether/ethyl acetate=5/1) to obtain compound **11-3.** MS-ESI: *m*/*z* 259.2 [M-18+1]⁺.

### Synthesis of compound 11-4

At 0°C, compound **11-3** (560 mg, 2.17 mmol) was dissolved in dichloromethane (10 mL), followed by the addition of diisobutylaluminum hydride in toluene (3.62 mL, 5.43 mmol, 1.5 mol/L). The reaction mixture was stirred at room temperature for 3 hours. After the reaction was complete, sodium sulfate decahydrate (10 g) was added, and the reaction mixture was stirred for 30 minutes and filtered. The filtrate was concentrated under reduced pressure, and the resulting residue was purified by column chromatography (SiO₂, petroleum ether/ethyl acetate=7/3) to obtain compound **11-4.** ¹H NMR (400 MHz, DMSO-*d₆*) δ 7.85 (dd, 1H), 7.80 (d, 1H), 7.27-7.13 (m, 2H), 7.02 (dd, 1H), 6.93 (s, 1H), 5.23 (t, 1H), 5.11 (s, 2H), 4.48 (d, 2H).

### Synthesis of compound 11-5

It was prepared with reference to the synthesis of compound **3-5.** ¹H NMR (400 MHz, CDCl₃) δ 7.64-7.62 (m, 2H), 7.10-7.05 (m, 2H), 7.05-7.02 (m, 1H), 6.87 (dd, 1H), 5.09 (s, 2H), 4.47 (s, 2H).

### Synthesis of compound 11-6

It was prepared with reference to the synthesis of compound 3-6. MS-ESI: *m*/*z 433.1* [M+1]⁺.

### Synthesis of compound 11-7

It was prepared with reference to the synthesis of compound **3-7.** MS-ESI: *m*/*z 252.1* [M-17+1]⁺.

### Synthesis of compound 11-8

It was prepared with reference to the synthesis of compound **3-8** with similar conditions except that *N*,*N*-dimethylformamide was replaced with a mixed solvent of *N*,*N*-dimethylformamide and dichloromethane (1/10). MS-ESI: *m*/*z* 396.3 [M-100+1]⁺.

### Synthesis of compound 11

Compound **11** was prepared with reference to the synthesis of compound **1.** MS-ESI: *m*/*z* 396.1 [M+1]⁺. ¹H NMR (400 MHz, DMSO-*d₆*) δ 8.65-8.63 (m, 1H), 8.20 (s, 1H), 7.88-7.84 (m, 1H), 7.79 (dd, 1H), 7.25-7.17 (m, 2H), 6.99 (d, 1H), 6.92 (s, 1H), 5.10-5.06 (m, 2H), 5.06-4.91 (m, 1H), 4.04-3.95 (m, 1H), 3.92-3.82 (m, 1H), 3.76-3.67 (m, 1H), 3.18-3.04 (m, 3H), 2.92-2.57 (m, 3H), 1.75-1.73 (m, 2H).

### Example 12

### (2S)-N-(1-Cyano-2-(2,8-difluoro-6H-benzo[c]chromen-3-yl)ethyl)-1,4-oxazepane-2-carboxamide

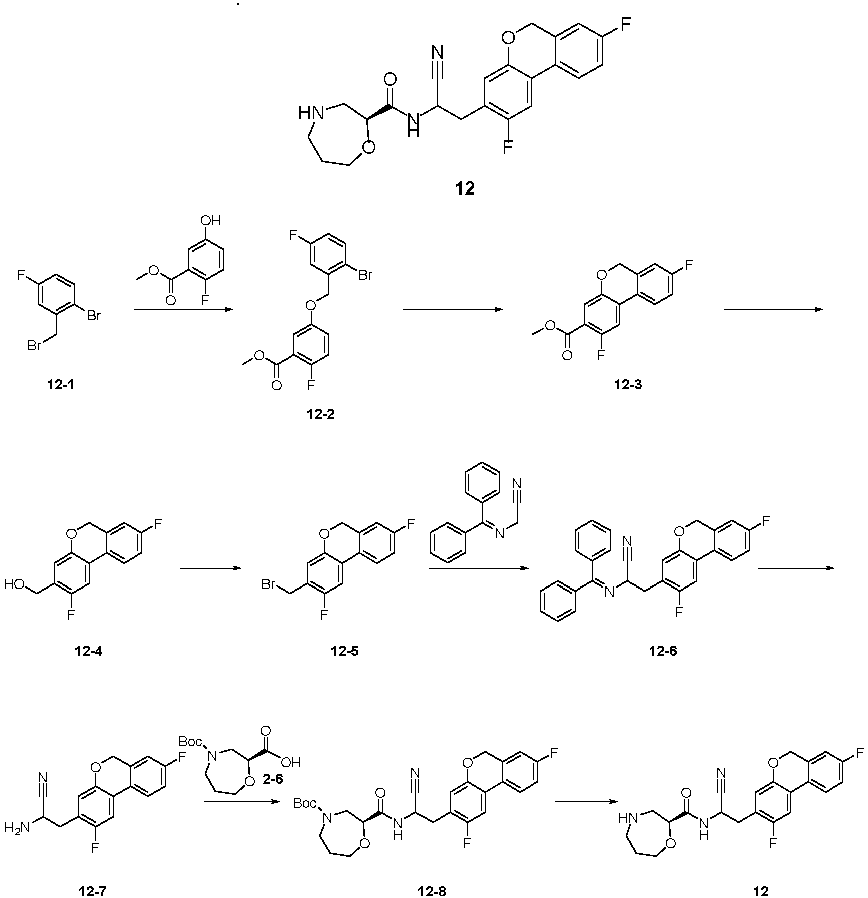

### Synthesis of compound 12-2

At room temperature, methyl 2-fluoro-5-hydroxybenzoate (4.80 g, 28.23 mmol), 1-bromo-2-(bromomethyl)-4-fluorobenzene (compound **12-1)** (9.24 g, 34.75 mmol) and potassium carbonate (5.50 g, 39.79 mmol) were dissolved in *N*,*N*-dimethylformamide (50 mL). The reaction mixture was heated to 40°C and stirred for 12 hours. Water (300 mL) was added, and the mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **12-2.** ¹H NMR (400 MHz, CDCl₃) δ 7.57-7.50 (m, 2H), 7.33-7.27 (m, 1H), 7.16-7.06 (m, 2H), 6.97-6.90 (m, 1H), 5.08 (s, 2H), 3.94 (s, 3H).

### Synthesis of compound 12-3

At room temperature and in a nitrogen atmosphere, compound **12-2** (5.00 g, 12.60 mmol), palladium acetate (0.28 g, 1.26 mmol), potassium carbonate (3.48 g, 25.18 mmol) and tricyclohexylphosphine tetrafluoroborate (0.46 g, 1.26 mmol) were dissolved in *N,N-*dimethylformamide (100 mL). The reaction mixture was heated to 130°C and stirred for 3 hours. After the reaction was complete, the reaction mixture was cooled to room temperature, followed by the addition of water (200 mL), and extracted with ethyl acetate (200 mL×2). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **12-3.** ¹H NMR (400 MHz, CDCl₃) δ 7.66-7.61 (m, 1H), 7.53 (d, 1H), 7.39 (d, 1H), 7.14-7.07 (m, 1H), 6.94-6.88 (m, 1H), 5.10 (s, 2H), 3.93 (s, 3H).

### Synthesis of compound 12-4

At room temperature, compound **12-3** (2.40 g, 8.25 mmol) was dissolved in tetrahydrofuran (50 mL), followed by the addition of lithium aluminum hydride (0.31 g, 8.25 mmol) at 0°C. The reaction mixture was stirred at 0°C for 20 minutes. After the reaction was complete, water (50 mL) was added, and the mixture was extracted with ethyl acetate (50 mL×2), washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **12-4.** ¹H NMR (400 MHz, CDCl₃) δ 7.56 (dd, 1H), 7.32 (d, 1H), 7.11-7.03 (m, 2H), 6.88 (dd, 1H), 5.06 (s, 2H), 4.73 (d, 2H).

### Synthesis of compound 12-5

At room temperature, compound **12-4** (1.60 g, 6.12 mmol) was dissolved in dichloromethane (20 mL), then phosphorus tribromide (1.66 g, 6.12 mmol) was added dropwise. The reaction mixture was stirred at room temperature for 20 minutes. After the reaction was complete, water (100 mL) was added, and the mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **12-5.** ¹H NMR (300 MHz, CDCl₃) δ 7.65-7.57 (m, 1H), 7.38 (d, 1H), 7.17-7.07 (m, 1H), 7.04 (d, 1H), 6.93 (d, 1H), 5.11 (s, 2H), 4.52 (s, 2H).

### Synthesis of compound 12-6

At room temperature, *N*-(diphenylmethylene)aminoacetonitrile (900 mg, 4.09 mmol), compound **12-5** (470 mg, 1.47 mmol), benzyltrimethylammonium chloride (80 mg, 0.41 mmol) and sodium hydroxide (210 mg, 5.31 mmol) were dissolved in a mixed solvent of dichloromethane (9 mL) and water (1 mL). The reaction mixture was heated to 35°C and stirred for 24 hours. Water (30 mL) was added, and the mixture was extracted with dichloromethane (30 mL×2). The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **12-6.** MS-ESI: *m*/*z* 451.1 [M+1]⁺.

### Synthesis of compound 12-7

At room temperature, compound **12-6** (1.50 mg, 2.97 mmol) was dissolved in tetrahydrofuran (10 mL), then 1 M aqueous hydrochloric acid solution (4 mL) was added dropwise. The reaction mixture was stirred at room temperature for 1 hour. After the reaction was complete, saturated aqueous sodium bicarbonate solution (30 mL) was added, and the mixture was extracted with ethyl acetate (30 mL×3).The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=2/1) to obtain compound **12-7.** MS-ESI: *m*/*z* 287.1 [M+1]⁺.

### Synthesis of compound 12-8

At room temperature, compound **12-7** (150 mg, 0.52 mmol), compound **2-6** (127 mg, 0.52 mmol), *N*,*N*,*N*',*N*'-tetramethyl-O-(7-azabenzotriazol-1-yl)uronium hexafluorophosphate (256 mg, 0.68 mmol) and *N*,*N*-diisopropylethylamine (200 mg, 1.56 mmol) were dissolved in *N*,*N*-dimethylformamide (5 mL). The reaction mixture was stirred at room temperature for 3 hours. After the reaction was complete, the reaction mixture was purified by preparative liquid chromatography (C18, acetonitrile/water system) to obtain compound **12-8.** MS-ESI: *m*/*z* 458.1 [M-56+1]⁺.

### Synthesis of compound 12

At room temperature, compound **12-8** (70 mg, 0.16 mmol) was dissolved in formic acid (2 ml), and the reaction mixture was heated to 30°C and stirred for 1 hour. After the reaction was complete, the reaction mixture was purified by preparative liquid chromatography (C18, ammonium bicarbonate/acetonitrile/water system) to obtain compound **12.** MS-ESI: *m*/*z* 414.1 [M+1]⁺. ¹H NMR (400 MHz, CDCl₃) δ 7.57 (dd, 1H), 7.36 (d, 1H), 7.11-7.06 (m, 1H), 6.96-6.87 (m, 2H), 5.20-5.09 (m, 1H), 5.07 (s, 2H), 4.09-4.00 (m, 2H), 3.81-3.73 (m, 1H), 3.40-3.26 (m, 1H), 3.20-3.11 (m, 2H), 3.07-2.95 (m, 1H), 2.93-2.84 (m, 2H), 1.96-1.80 (m, 2H).

### Example 13

### (2S)-N-(1-Cyano-2-(8-cyano-6H-benzo[c]chromen-3-yl)ethyl)-1,4-oxazepane-2-carboxamide

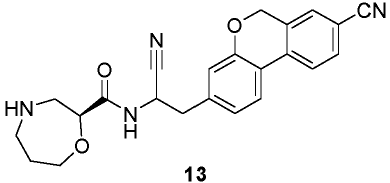

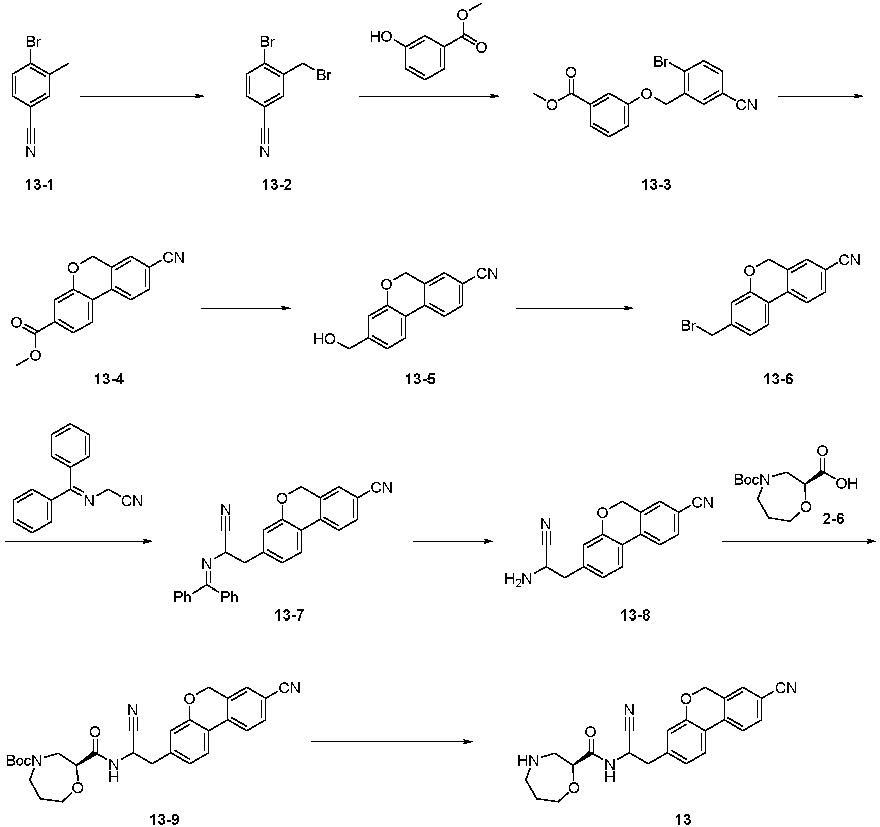

### Synthesis of compound 13-2

At room temperature, 4-bromo-3-methylbenzonitrile (compound **13-1)** (10 g, 51.29 mmol), *N*-bromosuccinimide (22.70 g, 127.54 mmol) and dibenzoyl peroxide (1.24 g, 5.12 mmol) were dissolved in 1,2-dichloroethane (50 mL). The reaction mixture was heated to 100°C and stirred for 12 hours. The reaction mixture was cooled to room temperature, followed by the addition of saturated aqueous sodium bicarbonate solution (50 mL), and extracted with ethyl acetate (50 mL×2). The organic phase was washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **13-2.** ¹H NMR (400 MHz, CDCl₃) δ 7.77-7.69 (m, 2H), 7.47-7.41 (m, 1H), 4.57 (s, 2H).

### Synthesis of compound 13-3

At room temperature, compound **13-2** (140 mg, 0.44 mmol) and methyl 3-hydroxybenzoate (0.09 mL, 0.65 mmol) were dissolved in acetonitrile (10 mL), followed by the addition of potassium carbonate (120 mg, 0.87 mmol). The reaction mixture was heated to 100°C and stirred for 3 hours. After the reaction was complete, the reaction mixture was cooled to room temperature, followed by the addition of water (50 mL), and extracted with ethyl acetate (50 mL×2). The organic phase was washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=5/1) to obtain compound **13-3.**

### Synthesis of compound 13-4

It was prepared with reference to the synthesis of compound **12-3.** ¹H NMR (400 MHz, DMSO-*d₆*) δ 8.11 (d, 1H), 8.09 (d, 1H), 7.90 (dd, 1H), 7.82 (d, 1H), 7.66 (dd, 1H), 7.49 (d, 1H), 5.24 (s, 2H), 3.87 (s, 3H).

### Synthesis of compound 13-5

It was prepared with reference to the synthesis of compound **11-4.** MS-ESI: *m*/*z* 220.2 [M-18+1]⁺.

### Synthesis of compound 13-6

It was prepared with reference to the synthesis of compound **3-5.** ¹H NMR (400 MHz, DMSO-*d₆*) δ 8.03 (d, 1H), 7.97 (d, 1H), 7.87 (d, 1H), 7.80 (s, 1H), 7.20 (d, 1H), 7.12 (s, 1H), 5.21 (d, 2H), 4.71 (s, 2H).

### Synthesis of compound 13-7

It was prepared with reference to the synthesis of compound **3-6.** MS-ESI: *m*/*z* 440.3 [M+1]⁺.

### Synthesis of compound 13-8

It was prepared with reference to the synthesis of compound **3-7.** MS-ESI: *m*/*z* 259.2 [M-17+1]⁺.

### Synthesis of compound 13-9

It was prepared with reference to the synthesis of compound **1-7** with similar conditions except that *N*,*N*-dimethylformamide was replaced with a mixed solvent of N,N-dimethylformamide and dichloromethane (10/1). MS-ESI: *m*/*z* 447.3 [M-56+1]⁺.

### Synthesis of compound 13

It was prepared with reference to the synthesis of compound **1.** MS-ESI: *m*/*z* 403.3 [M+1]⁺. ¹H NMR (400 MHz, CDCl₃) δ 7.77-7.71 (m, 2H), 7.66 (d, 1H), 7.45 (s, 1H), 7.39-7.29 (m, 1H), 7.05-7.01 (m, 1H), 6.98-6.94 (m, 1H), 5.12-5.08 (m, 3H), 4.35-4.31 (m, 1H), 4.09-4.03 (m, 1H), 3.85-3.78 (m, 2H), 3.65-3.54 (m, 1H), 3.16-3.07 (m, 5H), 2.09-2.05 (m, 2H).

### Example 14

### (S)-N-((S)-1-Cyano-2-(8-cyano-2-fluoro-6H-benzo[c]chromen-3-yl)ethyl)-1,4-oxazepane-2-carboxamide

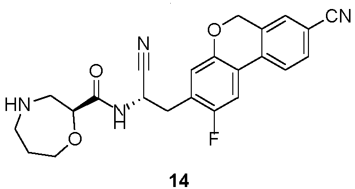

### Synthetic route A:

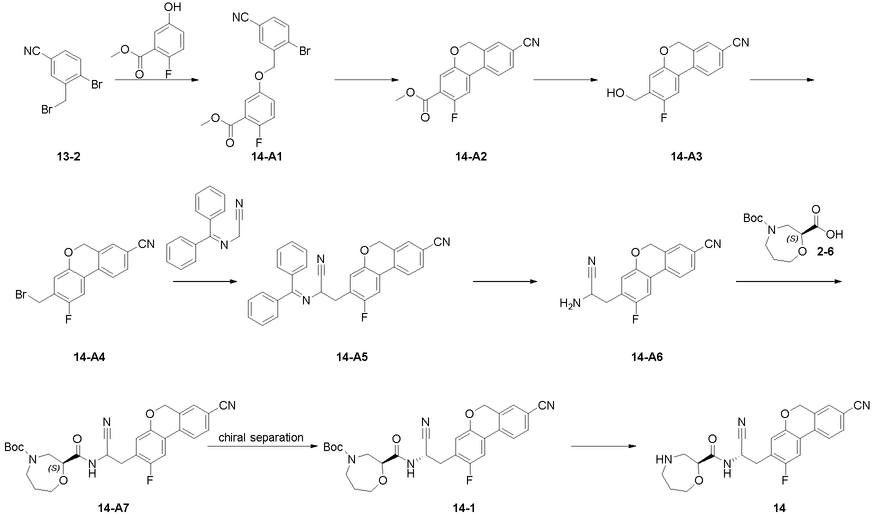

### Synthesis of compound 14-A1

At room temperature, methyl 2-fluoro-5-hydroxybenzoate (4.30 g, 25.29 mmol), 4-bromo-3-(bromomethyl)benzonitrile (compound **13-2)** (7.72 g, 25.30 mmol) and potassium carbonate (6.99 g, 50.58 mmol) were dissolved in *N*,*N*-dimethylformamide (50 mL). The reaction mixture was heated to 40°C and stirred for 12 hours. Water (300 mL) was added, and the mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **14-A1.** ¹H NMR (400 MHz, CDCl₃) δ 7.87 (d, 1H), 7.73 (d, 1H), 7.55-7.48 (m, 2H), 7.18-7.09 (m, 2H), 5.11 (s, 2H), 3.95 (s, 3H).

### Synthesis of compound 14-A2

At room temperature and in a nitrogen atmosphere, compound **14-A1** (2.90 g, 6.37 mmol), palladium acetate (0.14 g, 0.64 mmol), potassium carbonate (1.76 g, 12.73 mmol) and tricyclohexylphosphine tetrafluoroborate (0.23 g, 0.64 mmol) were dissolved in *N,N-*dimethylformamide (30 mL). The reaction mixture was heated to 120°C and stirred for 1.5 hours. After the reaction was complete, the reaction mixture was cooled to room temperature, followed by the addition of water (200 mL), and extracted with ethyl acetate (200 mL×2). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **14-A2.** ¹H NMR (400 MHz, CDCl₃) δ 7.80-7.72 (m, 2H), 7.60 (d, 1H), 7.52-7.49 (m, 2H), 5.18 (s, 2H), 3.98 (s, 3H).

### Synthesis of compound 14-A3

At room temperature, compound **14-A2** (1.20 g, 3.81 mmol) and lithium borohydride (0.25 g, 11.47 mmol) were dissolved in tetrahydrofuran (25 mL), and the reaction mixture was heated to 55°C and stirred for 2 hours. After the reaction was complete, the reaction mixture was cooled to room temperature, followed by the addition of water (200 mL), and extracted with ethyl acetate (200 mL×2). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **14-A3.** ¹H NMR (400 MHz, CDCl₃) δ 7.67 (s, 2H), 7.47 (s, 1H), 7.38 (d, 1H), 7.12 (d, 1H), 5.11 (s, 2H), 4.77 (s, 2H).

### Synthesis of compound 14-A4

At room temperature, compound **14-A3** (450 mg, 1.59 mmol) was dissolved in dichloromethane (15 mL), then phosphorus tribromide (520 mg, 1.92 mmol) was added dropwise. The reaction mixture was stirred at room temperature for 20 minutes. After the reaction was complete, water (100 mL) was added, and the mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **14-A4.** ¹H NMR (300 MHz, CDCl₃) δ 7.73 (s, 2H), 7.52 (s, 1H), 7.45 (d, 1H), 7.10 (d, 1H), 5.17 (s, 2H), 4.53 (s, 2H).

### Synthesis of compound 14-A5

At room temperature, *N*-(diphenylmethylene)aminoacetonitrile (250 mg, 1.14 mmol), compound **14-A4** (470 mg, 1.26 mmol), benzyltrimethylammonium chloride (22 mg, 0.12 mmol) and sodium hydroxide (91 mg, 2.3 mmol) were dissolved in a mixed solvent of dichloromethane (6 mL) and water (6 mL). The reaction mixture was heated to 35°C and stirred for 24 hours. Water (30 mL) was added, and the mixture was extracted with dichloromethane (30 mL×2). The organic phases were combined, washed with saturated brine (20 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **14-A5.** MS-ESI: *m*/*z* 458.4 [M+1]⁺.

### Synthesis of compound 14-A6

At room temperature, compound **14-A5** (520 mg, 0.90 mmol) was dissolved in tetrahydrofuran (10 mL), then 1 M aqueous hydrochloric acid solution (4 mL) was added dropwise. The reaction mixture was stirred at room temperature for 1 hour. After the reaction was complete, saturated aqueous sodium bicarbonate solution (30 mL) was added, and the mixture was extracted with ethyl acetate (30 mL×3).The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=2/1) to obtain compound **14-A6.** MS-ESI: *m*/*z* 293.9 [M+1]⁺.

### Synthesis of compound 14-A7

At room temperature, compound **14-A6** (220 mg, 0.68 mmol), compound **2-6** (170 mg, 0.69 mmol), *N*,*N*,*N*',*N*'-tetramethyl-O-(7-azabenzotriazol-1-yl)uronium hexafluorophosphate (360 mg, 0.95 mmol) and *N*,*N*-diisopropylethylamine (250 mg, 1.93 mmol) were dissolved in *N*,*N*-dimethylformamide (5 mL). The reaction mixture was stirred at room temperature for 3 hours. After the reaction was complete, the reaction mixture was purified by preparative liquid chromatography (C18, acetonitrile/water system) to obtain compound **14-A7.** MS-ESI: *m*/*z* 465.1 [M-56+1]⁺.

### Synthesis of compound 14-1

Compound **14-A7** (280 mg, 0.51 mmol) was subjected to chiral resolution (column: chiralpak IE, 250*25 mm, 5 µm; mobile phase: *n*-hexane, ethanol; gradient: *n*-hexane phase 30%; flow rate: 15 mL/min, column temperature: 30°C) to obtain compound **14-1** (two diastereomer peaks in total, compound **14-1** being the first elution peak). ¹H NMR (300 MHz, DMSO-*d₆*) δ 8.89 (d, 1H), 8.08 (d, 1H), 7.94-7.89 (m, 2H), 7.83 (s, 1H), 7.07 (d, 1H), 5.24-5.14 (m, 2H), 5.09-5.07 (m, 1H), 4.15-4.12 (m, 1H), 3.99-3.87 (m, 2H), 3.63-3.56 (m, 2H), 3.29-3.02 (m, 4H), 1.89-1.79 (m, 2H), 1.41-1.36 (m, 9H).

### Synthesis of compound 14

At room temperature, compound **14-1** (85 mg, 0.16 mmol) was dissolved in formic acid (1 ml), and the reaction mixture was heated to 40°C and stirred for 1 hour. After the reaction was complete, the reaction mixture was purified by preparative liquid chromatography (C18, ammonium bicarbonate/acetonitrile/water system) to obtain compound **14.** MS-ESI: *m*/*z* 421.2 [M+1]⁺. ¹H NMR (400 MHz, DMSO-*d₆*) δ 8.69 (d, 1H), 8.05 (d, 1H), 7.89-7.86 (m, 2H), 7.80 (s, 1H), 7.03 (d, 1H), 5.20-5.12 (m, 2H), 5.09-5.02 (m, 1H), 4.00-3.97 (m, 1H), 3.88-3.83 (m, 1H), 3.77-3.68 (m, 1H), 3.25-3.14 (m, 2H), 3.05-2.98 (m, 1H), 2.82-2.72 (m, 1H), 2.62-2.53 (m, 2H) , 1.80-1.66 (m, 2H).

### Synthetic route B:

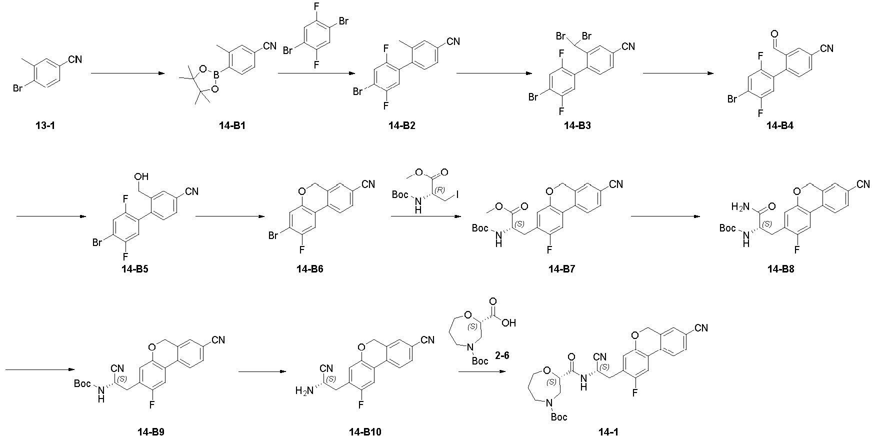

### Synthesis of compound 14-B1

At room temperature and in a nitrogen atmosphere, compound **13-1** (10.00 g, 51.01 mmol), bis(pinacolato)diboron (15.54 g, 61.21 mmol), potassium acetate (10.01 g, 102.02 mmol) and [1,1'-bis(diphenylphosphino)ferrocene]palladium(II) dichloride (0.75 g, 1.02 mmol) were dissolved in *N*,*N*-dimethylformamide (100 mL). The reaction mixture was heated to 100°C and stirred for 18 hours. The reaction mixture was cooled to room temperature, followed by the addition of water (1.0 L), and extracted with ethyl acetate (500 mL×2). The organic phases were combined, washed with saturated brine (500 mL×2), dried over anhydrous sodium sulfate and filtered. The filtrate was concentrated under reduced pressure, and the resulting residue was purified by column chromatography (SiO₂, petroleum ether/ethyl acetate=15/1) to obtain compound **14-B1.** ¹H NMR (300 MHz, CDCl₃): δ 7.82 (d, 1H), 7.46-7.39 (m, 2H), 2.55 (s, 3H), 1.35 (s, 12H).

### Synthesis of compound 14-B2

At room temperature and in a nitrogen atmosphere, compound **14-B1** (12.00 g, 43.19 mmol), 1,4-dibromo-2,5-difluorobenzene (23.49 g, 86.38 mmol), potassium carbonate (11.94 g, 86.38 mmol) and [1,1'-bis(diphenylphosphino)ferrocene]palladium(II) dichloride (1.26 g, 1.73 mmol) were dissolved in a mixed solvent of 1,4-dioxane (120 mL) and water (20 mL). The reaction mixture was heated to 100°C and stirred for 16 hours. The reaction mixture was cooled to room temperature, followed by the addition of water (1.0 L), and extracted with ethyl acetate (500 mL×2). The organic phases were combined, washed with saturated brine (500×2), dried over anhydrous sodium sulfate and filtered. The filtrate was concentrated under reduced pressure, and the resulting residue was purified by column chromatography (SiO₂, petroleum ether/ethyl acetate=9/1) to obtain compound **14-B2.** ¹H NMR (300 MHz, CDCl₃) δ 7.62-7.52 (m, 2H), 7.45-7.36 (m, 1H), 7.32-7.24 (m, 1H), 7.06-6.97 (m, 1H), 2.24 (s, 3H).

### Synthesis of compound 14-B3

At room temperature and in a nitrogen atmosphere, compound **14-B2** (7.40 g, 24.02 mmol), N-bromosuccinimide (10.26 g, 57.64 mmol) and dibenzoyl peroxide (0.35 g, 1.44 mmol) were dissolved in carbon tetrachloride (80 mL). The reaction mixture was heated to 90°C and stirred for 18 hours. The reaction mixture was cooled to room temperature, followed by the addition of water (200 mL), and extracted with dichloromethane (100 mL×2). The organic phases were combined, washed with saturated brine (500 mL×2), dried over anhydrous sodium sulfate and filtered. The filtrate was concentrated under reduced pressure, and the resulting residue was purified by column chromatography (SiO₂, petroleum ether/ethyl acetate=6/1) to obtain compound **14-B3.** ¹H NMR (300 MHz, CDCl₃): δ 8.41 (s, 1H), 7.72-7.62 (m, 1H), 7.54-7.46 (m, 1H), 7.31-7.26 (m, 1H), 7.15-7.06 (m, 1H), 6.41 (s, 1H).

### Synthesis of compound 14-B4

At room temperature, compound **14-B3** (1.00 g, 2.15 mmol) was dissolved in acetonitrile (10 mL), followed by the addition of a solution of silver nitrate (0.80 g, 4.72 mmol) in water (2 mL). The reaction mixture was heated to 90°C and stirred for 18 hours. The reaction mixture was cooled to room temperature and filtered. Water (20 mL) was added to the filtrate, and the mixture was extracted with ethyl acetate (25 mL×2 mL). The organic phases were combined, washed with saturated brine (25 mL×2), dried over anhydrous sodium sulfate and filtered. The filtrate was concentrated under reduced pressure to obtain the crude compound **14-B4,** which was directly used in the next step. ¹H NMR (300 MHz, CDCl₃): δ 9.92 (d, 1H), 8.32 (s, 1H), 7.96 (d, 1H), 7.57-7.43 (m, 2H), 7.17-7.10 (m, 1H).

### Synthesis of compound 14-B5

At room temperature, the crude compound **14-B4** (7.20 g, 22.35 mmol) was dissolved in a mixed solvent of tetrahydrofuran (40 mL) and methanol (40 mL), followed by the addition of sodium borohydride (1.01 g, 26.82 mmol) in batches in an ice-water bath. The reaction mixture was stirred at room temperature for 30 minutes. After the reaction was complete, the reaction mixture was concentrated under reduced pressure to one-third of the original volume, slowly poured into 0.5 M hydrochloric acid (50 mL) under stirring, and extracted with ethyl acetate (50 mL×3).The organic phases were combined, washed with saturated brine (100 mL×2), dried over anhydrous sodium sulfate and filtered. The filtrate was concentrated under reduced pressure, and the resulting residue was purified by column chromatography (SiO₂, petroleum ether/ethyl acetate=7/3) to obtain compound **14-B5.** ¹H NMR (300 MHz, CDCl₃): δ 7.97 (s, 1H), 7.65 (d, 1H), 7.46-7.37 (m, 1H), 7.36-7.30 (m, 1H), 7.12-7.03 (m, 1H), 4.58 (s, 2H).

### Synthesis of compound 14-B6

At room temperature, compound **14-B5** (6.50 g, 20.05 mmol) was dissolved in *N,N-*dimethylformamide (65 mL), followed by the addition of sodium hydride (0.48 g, 20.05 mmol, 60%) in batches at 0°C. The reaction mixture was stirred at room temperature for 30 minutes. After the reaction was complete, the reaction mixture was poured into 0.25 M hydrochloric acid (500 mL), stirred for 10 minutes, and filtered. The filter cake was washed with water (50 mL×5), and then washed with a mixed solvent of petroleum ether and ethyl acetate (9/1, 50 mL×3).The filter cake was dried under vacuum to constant weight to obtain the crude compound **14-B6,** which was directly used in the next step. ¹H NMR (300 MHz, DMSO-*d₆*): δ 8.12-8.06 (m, 2H), 7.94-7.86 (m, 1H), 7.82 (s, 1H), 7.46-7.40 (m, 1H), 5.21 (s, 2H).

### Synthesis of compound 14-B7

At room temperature and in a nitrogen atmosphere, zinc powder (40.00 g, 611.53 mmol) and iodine (0.5 g, 1.13 mmol) were added to *N*,*N*-dimethylformamide (50 mL), followed by the addition of methyl (*R*)-2-((*tert*-butoxycarbonyl)amino)-3-iodopropanoate (60.00 g, 182.30 mmol) in *N*,*N*-dimethylformamide (200 mL). The reaction mixture was heated to 50°C and stirred for 1 hour, and the supernatant was the zinc reagent to be used.

At room temperature and in a nitrogen atmosphere, the crude compound **14-B6** (20.00 g, 65.76 mmol) and bis(triphenylphosphine)palladium(II) dichloride (4.00 g, 0.13 mmol) were dissolved in *N*,*N*-dimethylformamide (100 mL) in another reaction flask. The above zinc reagent was added, and the reaction mixture was heated to 70°C and stirred for 6 hours. After the reaction was complete, the reaction mixture was cooled to room temperature, followed by the addition of water (800 mL), and extracted with ethyl acetate (250 mL×3).The organic phases were combined, washed with water (100 mL×3), dried over anhydrous magnesium sulfate and filtered. The filtrate was concentrated under reduced pressure, and the resulting residue was purified by column chromatography (SiO₂, petroleum ether/ethyl acetate=1/1) and then triturated in ethanol (50 mL) to obtain compound **14-B7.** ¹H NMR (400 MHz, CDCl₃) δ 8.04-8.02 (m, 1H), 7.88-7.85 (m, 2H), 7.82-7.79 (m, 1H), 7.37-7.35 (m, 1H), 6.99-6.98 (m, 1H), 5.15 (s, 2H), 4.27-4.20 (m, 1H), 3.64 (s, 3H), 3.14-3.09 (m, 1H), 2.87-2.81 (m, 1H),1.31 (s, 9H).

### Synthesis of compound 14-B8

At room temperature, compound **14-B7** (10.00 g, 23.45 mmol) was dissolved in tetrahydrofuran (100 mL), followed by the addition of a 8 M solution of ammonia in methanol (200 mL). The reaction mixture was sealed, heated to 40°C and stirred for 60 hours. The reaction mixture was cooled to room temperature, and concentrated under reduced pressure to obtain the crude compound **14-B8,** which was directly used in the next step. MS-ESI: *m*/*z* 356.0 [M-56+1]⁺.

### Synthesis of compound 14-B9

At room temperature, the crude compound **14-B8** (9.50 g, 23.09 mmol) and triethylamine (7.01 g, 69.27 mmol) were dissolved in tetrahydrofuran (100 mL). Trifluoroacetic anhydride (7.27 g, 34.64 mmol) was added dropwise at 0°C, and the reaction mixture was naturally warmed up to room temperature and stirred for 2 hours. After the reaction was complete, the reaction mixture was concentrated under reduced pressure. Water (100 mL) was added to the resulting residue, and the mixture was extracted with dichloromethane (100 mL×3).The organic phases were combined, washed with saturated brine (25 mL), dried over anhydrous sodium sulfate and filtered. The filtrate was concentrated under reduced pressure, and the resulting residue was purified by column chromatography (SiO₂, petroleum ether/ethyl acetate=10/1) and then triturated in ethanol (20 mL) to obtain compound **14-B9.** MS-ESI: *m*/*z* 392.0 [M-1]⁻.

### Synthesis of compound 14-B10

At room temperature, compound **14-B9** (7.80 g, 19.83 mmol) was dissolved in dichloromethane (20 mL), followed by the addition of formic acid (50 mL). The reaction mixture was warmed up to 35°C and stirred for 1 hour. After the reaction was complete, the reaction mixture was concentrated under reduced pressure. The resulting residue was diluted with dichloromethane (100 mL), slowly poured into 10% aqueous sodium carbonate solution (300 mL), stirred for 5 minutes, and extracted with dichloromethane (100 mL×3).The organic phases were combined, washed with saturated brine (25 mL), dried over anhydrous sodium sulfate and filtered. The filtrate was concentrated under reduced pressure to obtain the crude compound **14-B10,** which was directly used in the next step. MS-ESI: *m*/*z* 392.0 [M+1]⁺.

### Synthesis of compound 14-1

At room temperature, the crude compound **14-B10** (7.00 g, 23.87 mmol) and compound 2-6 (6.44 g, 26.25 mmol) were dissolved in *N*,*N*-dimethylformamide (100 mL). *N*,*N*,*N*',*N*'-Tetramethyl-O-(7-azabenzotriazol-1-yl)uronium hexafluorophosphate (10.89 g, 28.64 mmol) was added at 0°C, and the reaction mixture was stirred for 5 minutes. *N*,*N*-Diisopropylethylamine (4.63 g, 35.80 mmol) was added dropwise at 0°C, and the reaction mixture was stirred at 0°C for 30 minutes. After the reaction was complete, the reaction mixture was poured into water (300 mL), and extracted with ethyl acetate (100 mL×2). The organic phases were combined, washed with water (50 mL×2) and saturated brine (50 mL) successively, dried over anhydrous sodium sulfate and filtered. The filtrate was concentrated under reduced pressure, and the resulting residue was purified by column chromatography (SiO₂, petroleum ether/ethyl acetate=1/1) to obtain compound **14-1.** MS-ESI: *m*/*z* 521.1 [M+1]⁺.

### Synthesis of compound 14

At room temperature, compound **14-1** (10.50 g, 20.17 mmol) was dissolved in tetrahydrofuran (105 mL), followed by the addition of *p*-toluenesulfonic acid monohydrate (11.51 g, 60.51 mmol). The reaction mixture was heated to 35°C and stirred for 18 hours, then heated to 45°C and stirred for 5 hours. The reaction mixture was cooled to room temperature, poured into a solution of sodium carbonate (8.6 g, 4 eq) in water (200 mL), and extracted with a mixed solvent of dichloromethane and methanol (10/1, 100 mL×2). The organic phases were combined, washed with water (50 mL) and filtered. The filtrate was concentrated under reduced pressure, and the resulting residue was triturated in methanol (80 mL), and dried under reduced pressure to obtain compound **14.** MS-ESI: *m*/*z* 421.2 [M+1]⁺.

¹H NMR (400 MHz, CDCl₃) δ 8.68 (d, 2H), 7.47-7.42 (m, 2H), 7.27-7.25 (m, 1H), 7.00 (d, 1H), 5.22-5.16 (m, 1H), 5.11 (s, 2H), 4.10-4.07 (m, 1H), 4.04-3.99 (m, 1H), 3.80-3.73 (m, 1H), 3.29-3.25 (m, 1H), 3.22-3.12 (m, 2H), 3.01-2.93 (m, 2H), 2.89-2.83 (m, 1H), 1.93-1.77 (m, 2H).

¹H NMR (400 MHz, DMSO-d6) δ 8.68 (d, 1H), 8.04 (d, 1H), 7.89-7.86 (m, 2H), 7.80 (s, 1H), 7.03 (d, 1H), 5.20-5.12 (m, 2H), 5.09-5.03 (m, 1H), 4.00-3.97 (m, 1H), 3.88-3.82 (m, 1H), 3.75-3.69 (m, 1H), 3.28-3.15 (m, 2H), 3.04-3.00 (m, 1H), 2.79-2.73 (m, 1H), 2.63-2.54 (m, 2H) , 1.78-1.64 (m, 2H).

### Example 15

### (2S)-N-(1-Cyano-2-(3-fluoro-6H-benzo[c]chromen-8-yl)ethyl)-1,4-oxazepane-2-carboxamide

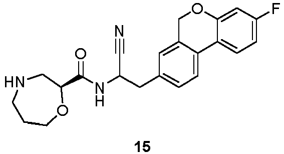

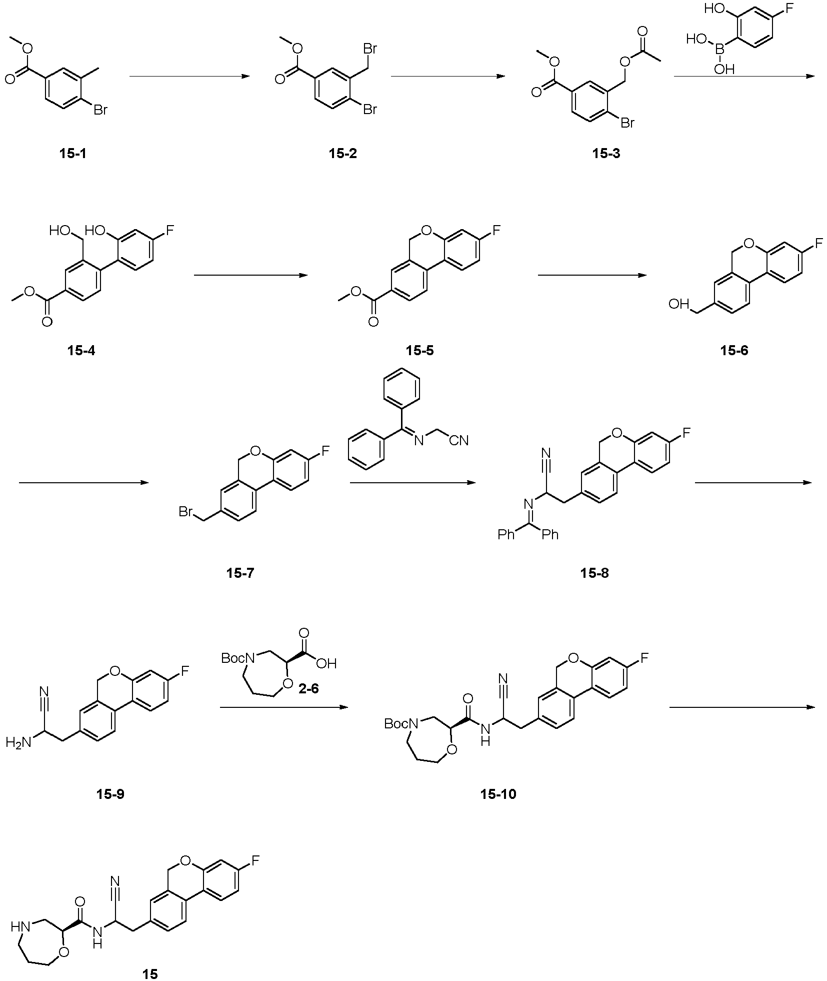

### Synthesis of compound 15-2

At room temperature, methyl 4-bromo-3-methylbenzoate (compound **15-1)** (11.30 g, 49.57 mmol) was dissolved in carbon tetrachloride (150 mL), followed by the addition of *N*-bromosuccinimide (13.20 g, 74.17 mmol) and azobisisobutyronitrile (4.10 g, 24.97 mmol). The reaction mixture was heated to 80°C and stirred for 12 hours. The reaction mixture was cooled to room temperature, followed by the addition of saturated aqueous sodium bicarbonate solution (150 mL), and extracted with dichloromethane (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **15-2** (12 g). ¹H NMR (400 MHz, DMSO-*d₆*) δ 8.21 (d, 1H), 7.85-7.79 (m, 2H), 4.83 (s, 2H), 3.87 (s, 3H).

### Synthesis of compound 15-3

At room temperature, compound **15-2** (24.0 g, 79.70 mmol) and potassium acetate (15.60 g, 158.96 mmol) were dissolved in acetic acid (200 mL). The reaction mixture was heated to 100°C and stirred for 12 hours. The reaction mixture was cooled to room temperature and concentrated under reduced pressure. The resulting residue was purified by column chromatography (SiO₂, petroleum ether/ethyl acetate=3/2) to obtain compound **15-3.** 1H NMR (400 MHz, CDCl₃) δ 8.06 (d, 1H), 7.85 (dd, 1H), 7.66 (d, 1H), 5.22 (s, 2H), 3.93 (s, 3H), 2.17 (s, 3H).

### Synthesis of compound 15-4

It was prepared with reference to the synthesis of compound **11-2.** MS-ESI: *m*/*z* 275.1 [M-1]⁻.

### Synthesis of compound 15-5

It was prepared with reference to the synthesis of compound **11-3.** MS-ESI: *m*/*z* 259.1 [M+1]⁺.

### Synthesis of compound 15-6

It was prepared with reference to the synthesis of compound **11-4.** MS-ESI: *m*/*z* 213.4 [M-18+1]⁺.

### Synthesis of compound 15-7

It was prepared with reference to the synthesis of compound **3-5.** ¹H NMR (400 MHz, CDCl₃) δ 7.68-7.64 (m, 1H), 7.60 (d, 1H), 7.39 (dd, 1H), 7.19 (s, 1H), 6.80-6.75 (m, 1H), 6.73-6.70 (m, 1H), 5.11 (s, 2H), 4.51 (s, 2H).

### Synthesis of compound 15-8

It was prepared with reference to the synthesis of compound **3-6.** MS-ESI: *m*/*z 433.1* [M+1]⁺.

### Synthesis of compound 15-9

It was prepared with reference to the synthesis of compound **3-7.** MS-ESI: *m*/*z 252.1* [M-17+1]⁺.

### Synthesis of compound 15-10

It was prepared with reference to the synthesis of compound **3-8** with similar conditions except that *N*,*N*-dimethylformamide was replaced with a mixed solvent of N,N-dimethylformamide and dichloromethane (1/10). MS-ESI: *m*/*z* 440.1 [M-56+1]⁺.

### Synthesis of compound 15

It was prepared with reference to the synthesis of compound **1.** MS-ESI: *m*/*z 396.1* [M+1]⁺. ¹H NMR (400 MHz, CDCl₃) δ 7.69-7.61 (m, 2H), 7.33-7.26 (m, 1H), 7.19-7.16 (m, 1H), 7.09 (d, 1H), 6.78 (td, 1H), 6.72 (dd, 1H), 5.19-5.09 (m, 3H), 4.10-4.07 (m, 1H), 4.03-3.96 (m, 1H), 3.80-3.72 (m, 1H), 3.38-3.28 (m, 1H), 3.17-3.01 (m, 3H), 2.96-2.87 (m, 2H), 1.88-1.78 (m, 2H).

### Example 16

### (S)-N-((S)-1-Cyano-2-(8,9-difluoro-6H-benzo[c]chromen-3-yl)ethyl)-1,4-oxazepane-2-carboxamide

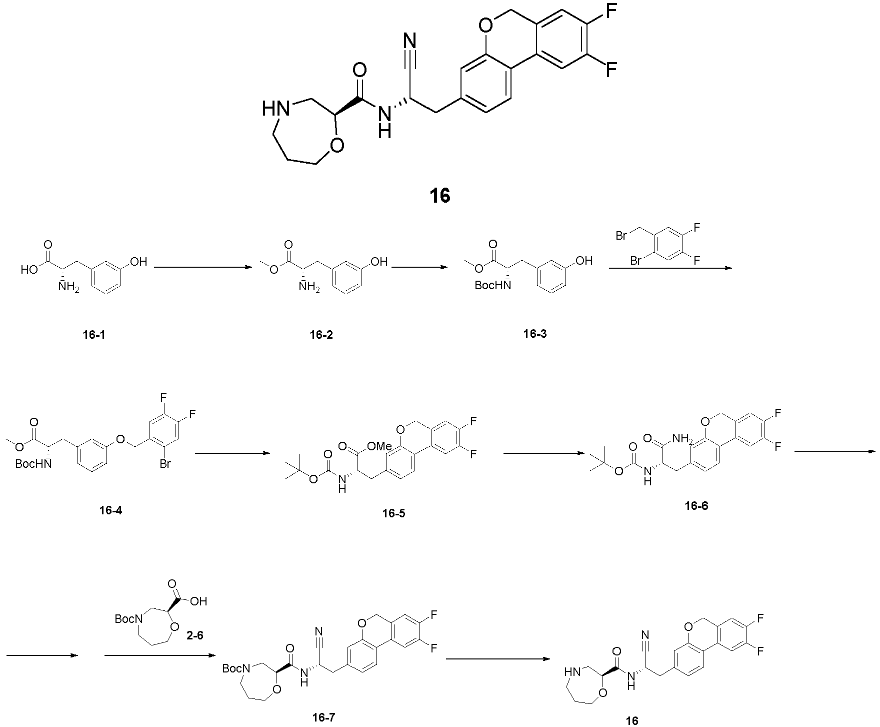

### Synthesis of compound 16-2

At 0°C, (S)-2-amino-3-(3-hydroxyphenyl)propanoic acid (compound **16-1)** (4.50 g, 24.84 mmol) was dissolved in methanol (50 mL), then thionyl chloride (3.84 g, 32.29 mmol) was slowly added dropwise. The reaction mixture was heated to 75°C and stirred for 12 hours. The reaction mixture was cooled to room temperature, and concentrated under reduced pressure to obtain the crude compound **16-2,** which was directly used in the next step. MS-ESI: *m*/*z* 196.2 [M+1]⁺.

### Synthesis of compound 16-3

At 0°C, compound **16-2** (6.00 g, 30.74 mmol) and sodium bicarbonate (7.75 g, 92.20 mmol) were dissolved in a mixed solvent of tetrahydrofuran (40 mL) and water (10 mL), followed by the addition of di-*tert*-butyl dicarbonate (7.38 g, 33.81 mmol). The reaction mixture was stirred at room temperature for 30 minutes. After the reaction was complete, water (50 mL) was added, and the reaction mixture was extracted with ethyl acetate (50 mL×2). The organic phases were combined, washed with saturated brine (50 mL×1), dried over anhydrous sodium sulfate and filtered. The filtrate was concentrated under reduced pressure, and the resulting residue was purified by column chromatography (SiO₂, petroleum ether/ethyl acetate=5/1) to obtain compound **16-3.** MS-ESI: *m*/*z* 196.2 [M-100+1]⁺.

### Synthesis of compound 16-4

At room temperature, compound **16-3** (3.38 g, 11.45 mmol) was dissolved in *N,N-*dimethylformamide (50 mL), followed by successive addition of potassium carbonate (2.37 g, 17.17 mmol), potassium iodide (1.90 g, 11.45 mmol) and 1-bromo-2-(bromomethyl)-4,5-difluorobenzene (2.40 g, 1.73 mmol). The reaction mixture was heated to 60°C and stirred for 12 hours. The reaction mixture was cooled to room temperature, followed by the addition of saturated aqueous sodium bicarbonate solution (50 mL), and extracted with ethyl acetate (50 mL×2). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **16-4.** MS-ESI: *m*/*z* 402.2 [M-100+1]⁺.

### Synthesis of compound 16-5

At room temperature, compound **16-4** (1.00 g, 2.00 mmol) was dissolved in *N,N-*dimethylacetamide (50 mL), followed by the addition of sodium acetate (0.33 g, 4.00 mmol) and bis(triphenylphosphine)palladium(II) dichloride (0.78 g, 1.00 mmol). The reaction mixture was heated to 130°C by microwave and stirred for 2 hours. After the reaction was complete, the reaction mixture was cooled to room temperature, followed by the addition of saturated aqueous sodium bicarbonate solution (50 mL), and extracted with ethyl acetate (50 mL×2). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **16-5.** MS-ESI: *m*/*z* 320.3 [M-100+1]⁺.

### Synthesis of compound 16-6

It was prepared with reference to the synthesis of compound **1-4.** MS-ESI: *m*/*z 305.3* [M-100+1]⁺.

### Synthesis of compound 16-7

It was prepared with reference to the synthesis of compounds **1-5, 1-6** and **3-8.** MS-ESI: *m*/*z* 414.2 [M-100+1]⁺.

### Synthesis of compound 16

It was prepared with reference to the synthesis of compound **1.** MS-ESI: *m*/*z 414.2* [M+1]⁺. ¹H NMR (400 MHz, CD₃OD) δ 7.72-7.64 (m, 2H), 7.18 (dd, 1H), 7.01 (d, 1H), 6.92 (s, 1H), 5.12-4.99 (m, 3H), 4.49-4.34 (m, 1H), 4.15-4.02 (m, 1H), 3.86-3.81 (m, 1H), 3.73-3.57 (m, 1H), 3.44-3.35 (m, 2H), 3.26-3.02 (m, 3H), 2.21-2.01 (m, 2H).

### Example 17

### (S)-4-Amino-N-(1-cyano-2-(8,9-difluoro-6H-benzo[c]chromen-3-yl)ethyl)tetrahydro-2H-pyran-4-carboxamide

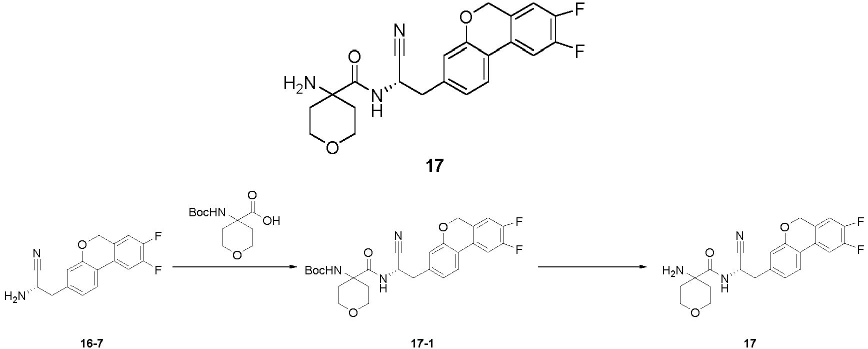

### Synthesis of compound 17-1

It was prepared with reference to the synthesis of compound **1-7.** MS-ESI: *m*/*z 414.2* [M-100+1]⁺.

### Synthesis of compound 17

It was prepared with reference to the synthesis of compound **1.** MS-ESI: *m*/*z 414.2* [M+1]⁺. ¹H NMR (400 MHz, CD₃OD) δ 7.73-7.64 (m, 2H), 7.21-7.15 (m, 1H), 7.02 (dd, 1H), 6.92 (d, 1H), 5.22-5.16 (m, 1H), 5.04 (s, 2H), 3.86-3.80 (m, 1H), 3.74-3.57 (m, 3H), 3.30-3.25 (m, 1H), 3.19-3.12 (m, 1H), 2.32-2.23 (m, 1H), 2.18-2.09 (m, 1H), 1.80-1.75 (m, 1H), 1.62-1.56 (m, 1H).

### Example 18

### (2S)-N-(1-Cyano-2-(3-cyano-9-fluoro-5H-chromeno[4,3-c]pyridin-8-yl)ethyl)-1,4-oxazepane-2-carboxamide

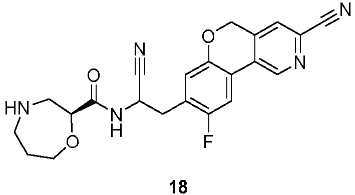

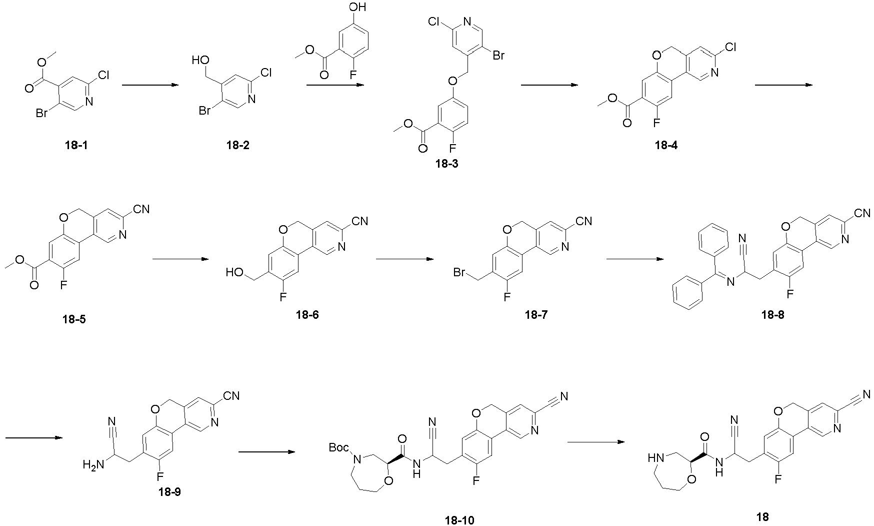

### Synthesis of compound 18-2

At room temperature, compound **18-1** (4.50 g, 17.97 mmol) was dissolved in a mixed solvent of tetrahydrofuran (40 mL) and methanol (5 mL), followed by the addition of lithium borohydride (0.78 g, 35.93 mmol) at 0°C. The reaction mixture was stirred at room temperature for 4 hours. After the reaction was complete, water (100 mL) was added, and the mixture was extracted with ethyl acetate (100 mL×2). The organic phases were combined, washed with saturated brine (50 mL), dried over anhydrous sodium sulfate and filtered. The filtrate was concentrated under reduced pressure to obtain compound **18-2.** MS-ESI: *m*/*z* 221.9 [M+1]⁺.

### Synthesis of compound 18-3

At room temperature, compound **18-2** (4.20 g, 17.56 mmol), methyl 2-fluoro-5-hydroxybenzoate (4.18 g, 24.58 mmol) and triphenylphosphine (6.91 g, 26.34 mmol) were dissolved in tetrahydrofuran (50 mL). Diisopropyl azodicarboxylate (5.33 g, 26.34 mmol) was added dropwise at 0°C, and the reaction mixture was stirred at room temperature for 12 hours. The reaction mixture was concentrated under reduced pressure, and the resulting residue was purified by column chromatography (SiO₂, petroleum ether/ethyl acetate=9/1) to obtain compound **18-3.** MS-ESI: *m*/*z* 373.9 [M+1]⁺.

### Synthesis of compound 18-4

At room temperature and in a nitrogen atmosphere, compound **18-3** (2.30 g, 5.96 mmol), palladium acetate (0.13 g, 0.60 mmol), potassium carbonate (1.65 g, 11.91 mmol) and tricyclohexylphosphine tetrafluoroborate (0.22 g, 0.60 mmol) were dissolved in *N,N-*dimethylacetamide (25 mL). The reaction mixture was heated to 110°C and stirred for 3 hours. After the reaction was complete, the reaction mixture was cooled to room temperature, followed by the addition of water (100 mL), and extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=2/1) to obtain compound **18-4.** MS-ESI: *m*/*z* 293.9 [M+1]⁺.

### Synthesis of compound 18-5

At room temperature and in a nitrogen atmosphere, compound **18-4** (1.10 g, 3.63 mmol), zinc cyanide (0.64 g, 5.45 mmol) and tetrakis(triphenylphosphine)palladium (0.63 g, 0.55 mmol) were dissolved in *N*,*N*-dimethylformamide (15 mL). The reaction mixture was heated to 90°C and stirred for 4 hours. After the reaction was complete, the reaction mixture was cooled to room temperature, followed by the addition of water (100 mL), and extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=2/1) to obtain compound **18-5.** MS-ESI: *m*/*z* 284.9 [M+1]⁺.

### Synthesis of compound 18-6

It was prepared with reference to the synthesis of compound **17-5.** ¹H NMR (400 MHz, CDCl₃) δ 8.85 (s, 1H), 7.43 (s, 1H), 7.39 (d, 1H), 7.09 (d, 1H), 5.07 (s, 2H), 4.72 (s, 2H).

### Synthesis of compound 18-7

At room temperature, compound **18-6** (120 mg, 0.40 mmol), carbon tetrabromide (145 mg, 0.44 mmol) and triphenylphosphine (115 mg, 0.44 mmol) were dissolved in dichloromethane (1 mL), and the reaction mixture was stirred at room temperature for 1 hour. After the reaction was complete, water (20 mL) was added, and the mixture was extracted with ethyl acetate (20 mL×2). The organic phases were combined, washed with saturated brine (20 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **18-7.** ¹H NMR (400 MHz, CDCl₃) δ 8.93 (s, 1H), 7.52-7.47 (m, 2H), 7.08 (d, 1H), 5.15 (s, 2H), 4.48 (s, 2H).

### Synthesis of compound 18-8

It was prepared with reference to the synthesis of compound **14-A5.** MS-ESI: *m*/*z* 459.2 [M+1]⁺.

### Synthesis of compound 18-9

It was prepared with reference to the synthesis of compound **14-A6.** MS-ESI: *m*/*z* 295.0 [M+1]⁺.

### Synthesis of compound 18-10

It was prepared with reference to the synthesis of compound **14-A7.** MS-ESI: *m*/*z* 422.1 [M-100+1]⁺.

### Synthesis of compound 18

It was prepared with reference to the synthesis of compound **14.** MS-ESI: *m*/*z 422.1* [M+1]⁺. ¹H NMR (400 MHz, CDCl₃) δ 9.24 (s, 1H), 8.71-8.65 (m, 1H), 8.05-8.00 (m, 1H), 7.98 (s, 1H), 7.08-7.05 (m, 1H), 5.28-5.22 (m, 2H), 5.10-4.95 (m, 1H), 4.01-3.82 (m, 2H), 3.76-3.66 (m, 1H), 3.25-3.00 (m, 3H), 2.84-2.51 (m, 3H), 1.80-1.69 (m, 2H).

### Example 19

### (S)-N-((S)-1-Cyano-2-(3-cyano-9-fluoro-5H-chromeno[4,3-c]pyridin-8-yl)ethyl)-1,4-oxazepane-2-carboxamide

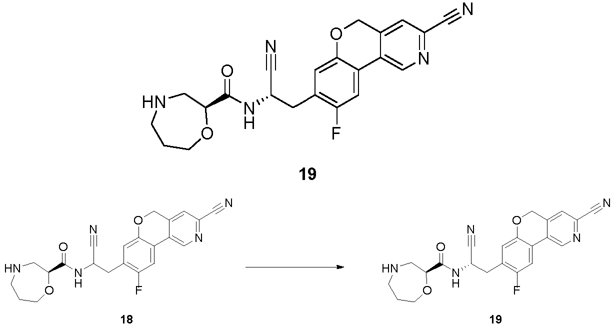

### Synthesis of compound 19

Compound **18** (50 mg, 0.312 mmol) was subjected to chiral resolution (column: chiralpak IC, 250*30 mm, 5 µm; mobile phase: acetonitrile, isopropanol (diethylamine 0.2%); gradient: acetonitrile phase 60%; flow rate: 15 mL/min, column temperature: 30°C) to obtain compound **19** (two diastereomer peaks in total, compound **19** being the second elution peak). MS-ESI: *m*/*z* 422.2 [M+1]⁺. ¹H NMR (400 MHz, DMSO-*d₆*) δ 9.24 (s, 1H), 8.69 (d, 1H), 8.02 (d, 1H), 7.98 (s, 1H), 7.06 (d, 1H), 5.29-5.21 (m, 2H), 5.11-5.01 (m, 1H), 4.01-3.95 (m, 1H), 3.89-3.81 (m, 1H), 3.77-3.68 (m, 1H), 3.30-3.20 (m, 2H), 3.02 (dd, 1H), 2.80-2.72 (m, 1H), 2.62-2.50 (m, 2H), 1.81-1.62 (m, 2H).

### Example 20

### (S)-N-((S)-1-Cyano-2-(2,8-difluoro-6H-benzo[c]chromen-3-yl)ethyl)-1,4-oxazepane-2-carboxamide

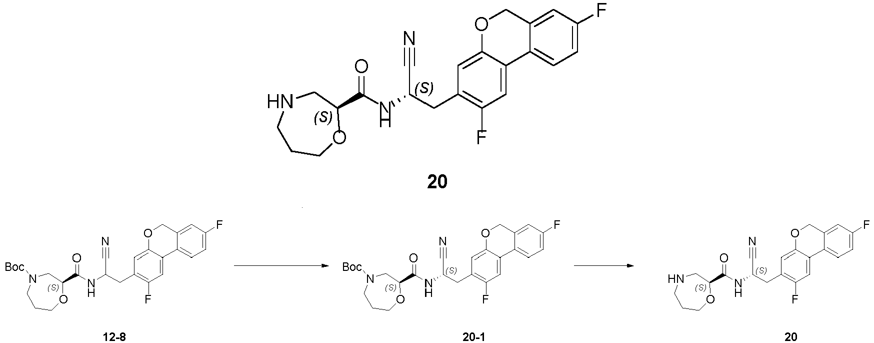

### Synthesis of compound 20-1

*Tert-butyl* (2*S*)-2-((1-cyano-2-(2,8-difluoro-6*H*-benzo[c]chromen-3-yl)ethyl)carbamoyl)-1,4-oxazepane-4-carboxylate (compound **12-8)** (160 mg, 0.31 mmol) was subjected to chiral resolution (column: chiralpak IC, 250*30 mm, 5 µm; mobile phase: *n*-hexane, ethanol; gradient: *n*-hexane phase 50%; flow rate: 15 mL/min, column temperature: 30°C) to obtain compound **20-1** (two diastereomer peaks in total, compound **20-1** being the first elution peak).

### Synthesis of compound 20

It was prepared with reference to the synthesis of compound **14.** MS-ESI: *m*/*z 414.2* [M+1]⁺. ¹H NMR (400 MHz, DMSO-*d₆*) δ 8.67 (d, 1H), 7.93-7.88 (m, 1H), 7.74 (d, 1H), 7.28-7.17 (m, 2H), 6.98 (d, 1H), 5.15-5.08 (m, 2H), 5.07-4.99 (m, 1H), 4.02-3.96 (m, 1H), 3.89-3.82 (m, 1H), 3.77-3.69 (m, 1H), 3.26-3.11 (m, 2H), 3.07-2.99 (m, 1H), 2.81-2.73 (m, 1H), 2.64-2.52 (m, 2H), 1.80-1.65 (m, 2H).

### Example 21

### (S)-N-((S)-2-(2-Chloro-8-cyano-6H-benzo[c]chromen-3-yl)-1-cyanoethyl)-1,4-oxazepane-2-carboxamide

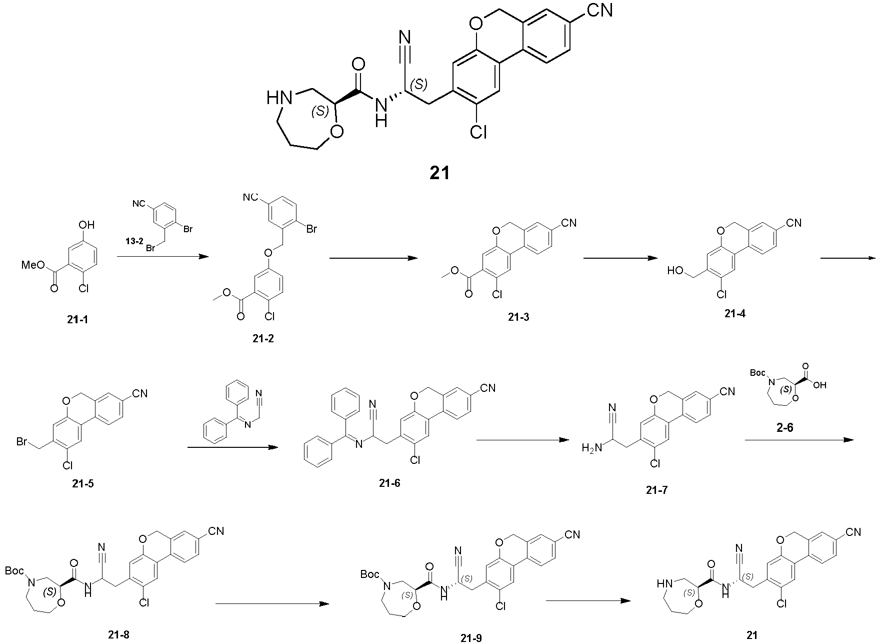

### Synthesis of compound 21-2

Compound **21-2** was prepared with reference to the synthesis of compound **14-A1** with similar conditions except that potassium carbonate was replaced with cesium carbonate. ¹H NMR (400 MHz, DMSO-*d₆*) δ 8.10 (d, 1H), 7.93 (d, 1H), 7.80 (dd, 1H), 7.52 (d, 1H), 7.49 (d, 1H), 7.30 (dd, 1H), 5.19 (s, 2H), 3.87 (s, 3H).

### Synthesis of compound 21-3

At room temperature and in a nitrogen atmosphere, compound **21-2** (13.30 g, 34.90 mmol) was dissolved in anhydrous *N*,*N*-dimethylacetamide (150 mL), followed by the addition of bis(triphenylphosphine) palladium(II) dichloride (2.45 g, 3.50 mmol) and anhydrous potassium acetate (10.30 g, 104.95 mmol). The reaction mixture was heated to 100°C and stirred for 16 hours. The reaction mixture was cooled to room temperature, diluted with ethyl acetate (100 mL), and washed with saturated brine (100 mL×3).The aqueous phase was extracted with ethyl acetate (50 mL×2). The organic phases were combined, dried over anhydrous sodium sulfate and filtered. The filtrate was concentrated under reduced pressure, and the resulting residue was triturated in ethyl acetate (50 mL) to obtain compound **21-3.** ¹H NMR (400 MHz, DMSO-*d₆*) δ 8.22 (s, 1 H), 8.19 (d, 1H), 7.91 (dd, 1H), 7.84 (s, 1H), 7.42 (s, 1H), 5.26 (s, 2H), 3.86 (s, 3H).

### Synthesis of compound 21-4

It was prepared with reference to the synthesis of compound **14-A3.** ¹H NMR (400 MHz, DMSO-*d₆*) δ 8.08 (d, 1H), 8.02 (s, 1H), 7.85 (dd, 1H), 7.79 (s, 1H), 7.16 (s, 1H), 5.50 (t, 1H), 5.20 (s, 2H), 4.54 (d, 2H).

### Synthesis of compound 21-5

It was prepared with reference to the synthesis of compound **14-A4.** ¹H NMR (400 MHz, DMSO-*d₆*) δ 8.16-8.11 (m, 2H), 7.88 (dd, 1H), 7.81 (s, 1H), 7.32 (s, 1H), 5.22 (s, 2H), 4.71 (s, 2H).

### Synthesis of compound 21-6

At room temperature and in a nitrogen atmosphere, sodium hydride (167 mg, 4.18 mmol, 60%) was added to anhydrous tetrahydrofuran (10 mL). A solution (10 mL) of *N-*(diphenylmethylene)aminoacetonitrile (1.84 g, 8.37 mmol) in tetrahydrofuran was added at -10°C, and the reaction mixture was stirred at -10°C for 10 minutes. A solution of compound **21-5** (700 mg, 2.09 mmol) in tetrahydrofuran (8 mL) was added, and the reaction mixture was stirred at -10°C for 20 minutes. A solution of the crude compound **21-6** in tetrahydrofuran was obtained, which was directly used in the next step. MS-ESI: *m*/*z* 474.1 [M+1]⁺.

### Synthesis of compound 21-7

At room temperature, a solution of the crude compound **21-6** in tetrahydrofuran (1.27 g, 2.69 mmol) was added to anhydrous tetrahydrofuran (10 mL). The reaction mixture was adjusted to pH=5-6 with 1M hydrochloric acid (10 mL), and stirred at room temperature for 16 hours. Water (30 mL) was added, and the reaction mixture was washed with ethyl acetate (50 mL×2). The aqueous phase was adjusted to pH=7-8 with saturated aqueous sodium bicarbonate solution, and extracted with ethyl acetate (30 mL×2). The organic phases were combined, dried over anhydrous sodium sulfate and filtered. The filtrate was concentrated under reduced pressure to obtain the crude compound **21-7,** which was directly used in the next step. MS-ESI: *m*/*z* 310.1 [M+1]⁺.

### Synthesis of compound 21-8

At room temperature, the crude compound **21-7** (500 mg, 1.26 mmol) and compound **2-6** (309 mg, 1.26 mmol) were dissolved in a mixed solvent of anhydrous *N,N-*dimethylformamide (3 mL) and anhydrous dichloromethane (6 mL), followed by the addition of O-benzotriazole-*N*,*N*,*N*',*N*'-tetramethyluronium hexafluorophosphate (955 mg, 2.52 mmol) and *N*,*N*-diisopropylethylamine (407 mg, 3.15 mmol). The reaction mixture was stirred at room temperature for 16 hours. Saturated aqueous sodium bicarbonate solution (30 mL) was added, and the reaction mixture was extracted with dichloromethane (30 mL×3).The organic phases were combined, washed with saturated brine (30 mL×3), dried over anhydrous sodium sulfate and filtered. The filtrate was concentrated under reduced pressure, and the resulting residue was purified by column chromatography (SiO₂, ethyl acetate), and then purified by preparative liquid chromatography (column: Phenomenex luna C18, 150*40 mm, 15 µm; mobile phase: water (0.225% formic acid), acetonitrile; gradient: acetonitrile phase 52-82%; flow rate: 60 mL/min; column temperature: room temperature) to obtain compound **21-8.** MS-ESI: *m*/*z* 481.1 [M-56+1]⁺.

### Synthesis of compound 21-9

Compound **21-8** (390 mg, 0.73 mmol) was resolved by preparative SFC (column: DAICEL CHIRALCEL OJ, 250*30 mm, 10 µm; mobile phase: supercritical carbon dioxide, methanol (0.1% ammonia monohydrate); gradient: carbon dioxide phase 55%; flow rate: 70 mL/min; column temperature: room temperature) to obtain compound **21-9** (two diastereomer peaks in total, compound **21-9** being the second elution peak).

### Synthesis of compound 21

It was prepared with reference to the synthesis of compound **14.** MS-ESI: *m*/*z 437.2* [M+1]⁺. ¹H NMR (400 MHz, DMSO-*d₆*) δ 8.70 (d, 1H), 8.14-8.09 (m, 2H), 7.87 (dd, 1H), 7.80 (d, 1H), 7.07 (s, 1H), 5.25-5.15 (m, 2H), 5.07-4.99 (m, 1H), 3.94-3.85 (m, 2H), 3.74-3.66 (m, 1H), 3.32-3.20 (m, 2H), 3.12 (dd, 1H), 2.84-2.76 (m, 1H), 2.74-2.62 (m, 2H), 1.84-1.64 (m, 2H).

### Example 22

### 4-Amino-N-(1-cyano-2-(8-fluoro-6H-benzo[c]chromen-3-yl)ethyl)tetrahydro-2H-pyran-4-carboxamide formate

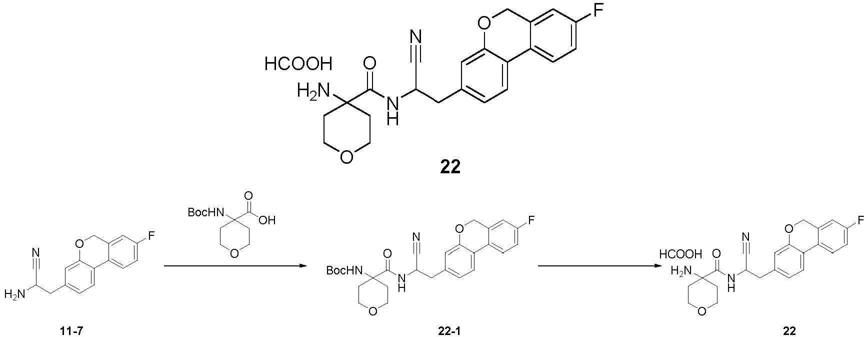

### Synthesis of compound 22-1

It was prepared with reference to the synthesis of compound **1-7** with similar conditions except that *N*,*N*-dimethylformamide was replaced with a mixed solvent of *N*,*N*-dimethylformamide and dichloromethane (10/1). MS-ESI: *m*/*z* 396.1 [M-100+1]⁺.

### Synthesis of compound 22

It was prepared with reference to the synthesis of compound **1.** MS-ESI: *m*/*z 396.1* [M+H]⁺. ¹H NMR (400 MHz, CDCl₃) δ: 8.25 (d, 1H), 8.00, (s, 1H), 7.66-7.63 (m, 2H), 7.07 (td, 1H), 6.99-6.96 (m, 1H), 6.89-6.87 (m, 2H), 5.13-5.08 (m, 3H), 3.93-3.83 (m, 2H), 3.65-3.56 (m, 2H), 3.08 (d, 2H), 2.33-2.16 (m, 2H), 1.32-1.22 (m, 2H).

### Example 23

### 4-Amino-N-(1-cyano-2-(3-fluoro-6H-benzo[c]chromen-8-yl)ethyl)tetrahydro-2H-pyran-4-carboxamide

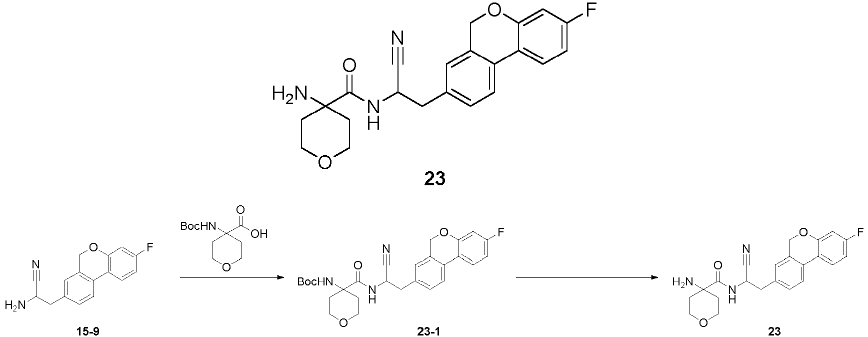

### Synthesis of compound 23-1

It was prepared with reference to the synthesis of compound **1-7** with similar conditions except that *N*,*N*-dimethylformamide was replaced with a mixed solvent of *N*,*N*-dimethylformamide and dichloromethane (10/1). MS-ESI: *m*/*z* 396.1 [M-100+1]⁺.

### Synthesis of compound 23

It was prepared with reference to the synthesis of compound **1.** MS-ESI: *m*/*z 396.1* [M+H]⁺. ¹H NMR (400 MHz, DMSO-*d₆*) δ: 7.93-7.88 (m, 1H), 7.76 (d, 1H), 7.33 (d, 1H), 7.18 (s, 1H), 6.95-6.84 (m, 2H), 6.06 (br s, 2H), 5.13 (s, 2H), 5.00-4.96 (m, 1H), 3.63-3.50 (m, 3H), 3.49-3.38 (m, 1H), 3.18-3.14 (m, 2H), 1.93-1.85 (m, 1H), 1.78-1.71 (m, 1H), 1.23-1.11 (m, 2H).

### Example 24

### 4-Amino-N-(1-cyano-2-(2,8-difluoro-6H-benzo[c]chromen-3-yl)ethyl)tetrahydro-2H-pyran-4-carboxamide

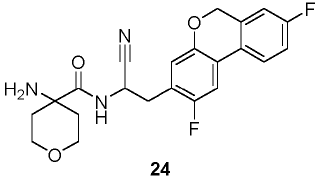

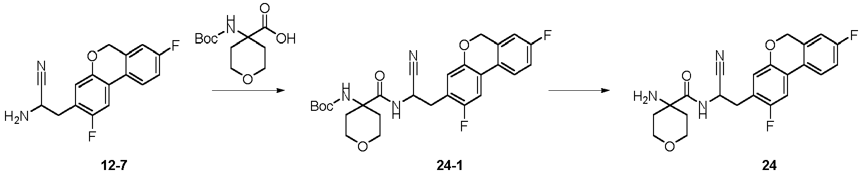

### Synthesis of compound 24-1

It was prepared with reference to the synthesis of compound **1-7.** MS-ESI: *m*/*z 458.1* [M-56+1]⁺.

### Synthesis of compound 24

It was prepared with reference to the synthesis of compound **14** with similar conditions except that the reaction temperature was changed from 40°C to 30°C. MS-ESI: *m*/*z* 414.2 [M+1]⁺. ¹H NMR (400 MHz, CDCl₃) δ 8.29 (d, 1H), 7.59-7.54 (m, 1H), 7.35 (d, 1H), 7.12-7.05 (m, 1H), 6.91-6.86 (m, 1H), 5.16-5.08 (m, 1H), 5.06 (s, 2H), 3.94-3.81 (m, 2H), 3.65-3.55 (m, 2H), 3.24-3.17 (m, 1H), 3.13-3.06 (m, 1H), 2.32-2.22 (m, 1H), 2.21-2.11 (m, 1H), 1.32-1.26 (m, 1H), 1.23-1.17 (m, 1H).

### Example 25

### 4-Amino-N-(1-cyano-2-(8-cyano-6H-benzo[c]chromen-3-yl)ethyl)tetrahydro-2H-pyran-4-carboxamide

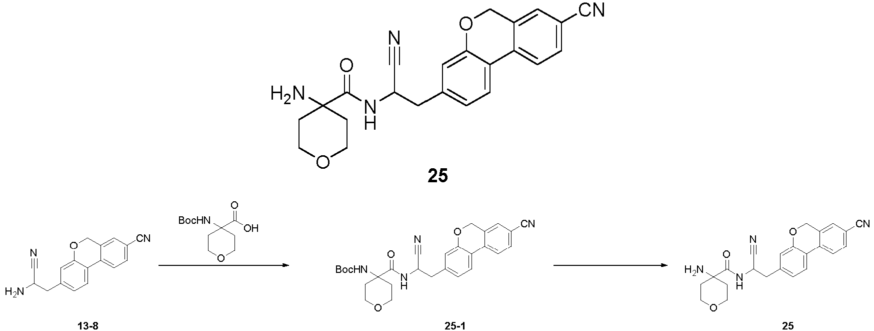

### Synthesis of compound 25-1

It was prepared with reference to the synthesis of compound **21-8.** MS-ESI: *m*/*z* 403.3 [M-100+1]⁺.

### Synthesis of compound 25

It was prepared with reference to the synthesis of compound **1.** MS-ESI: *m*/*z* 403.3 [M+1]⁺. ¹H NMR (400 MHz, CDCl₃) δ 8.28 (d, 1H), 7.77-7.71 (m, 2H), 7.67 (d, 1H), 7.46 (s, 1H), 7.02 (d, 1H), 6.91 (d, 1H), 5.15-5.07 (m, 3H), 3.94-3.84 (m, 2H), 3.65-3.56 (m, 2H), 3.10 (d, 2H), 2.34-2.16 (m, 2H), 1.31-1.21 (m, 2H).

### Example 26

### (2S,3aS,6aS)-N-((S)-1-Cyano-2-(8-cyano-2-fluoro-6H-benzo[c]chromen-3-yl)ethyl)octahydrocyclopenta[b]pyrrole-2-carboxamide

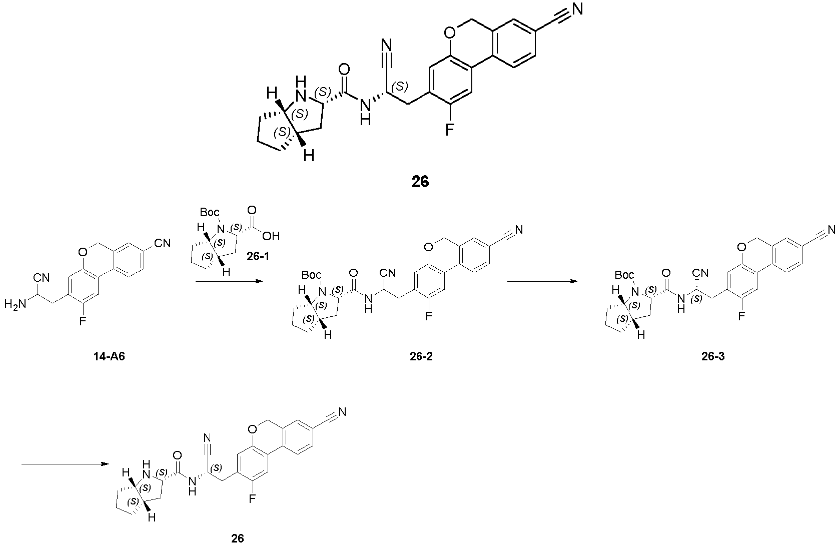

### Synthesis of compound 26-2

At room temperature, (2*S*,3a*S*,6a*S*)-1-(*tert-*butoxycarbonyl)octahydrocyclopenta[*b*]pyrrole-2-carboxylic acid (compound **26-1)** (174 mg, 0.68 mmol) and *N*,*N*,*N*',*N*'-tetramethyl-O-(7-azabenzotriazol-1-yl)uronium hexafluorophosphate (259 mg, 0.68 mmol) were dissolved in *N*,*N*-dimethylformamide (5 mL) and stirred for 10 minutes. 3-(2-Amino-2-cyanoethyl)-2-fluoro-6*H-*benzo[c]chromene-8-carbonitrile (compound **14-A6)** (200 mg, 0.68 mmol) and triethylamine (0.19 mL, 1.36 mmol) were added, and the reaction mixture was stirred at room temperature for 2 hours. After the reaction was complete, (10 mL) was added, and the reaction mixture was extracted with ethyl acetate (5 mL×3).The organic phases were combined, washed with saturated brine (10 mL), dried over anhydrous sodium sulfate and filtered. The filtrate was concentrated under reduced pressure, and the resulting residue was purified by preparative thin layer chromatography (SiO₂, petroleum ether/ethyl acetate=5/1) to obtain compound **26-2.** MS-ESI: *m*/*z* 529.1 [M-1]⁻.

### Synthesis of compound 26-3

Compound **26-2** (300 mg, 0.57 mmol) was subjected to chiral resolution (column: chiralpak IE, 250*30 mm, 5 µm; mobile phase: *n*-hexane, ethanol; gradient: *n*-hexane phase 50%; flow rate: 25 mL/min, column temperature: 30°C) to obtain compound **26-3** (two diastereomer peaks in total, compound **26-3** being the second elution peak).

### Synthesis of compound 26

It was prepared with reference to the synthesis of compound **14** with similar conditions except that the temperature was changed to 50°C. MS-ESI: *m*/*z* 431.2 [M+H]⁺. ¹H NMR (400 MHz, DMSO-*d*6) δ 8.53 (d, 1H), 8.04 (d, 1H), 7.91-7.83 (m, 2H), 7.79 (s, 1H), 7.03 (d, 2H), 5.20-5.11 (m, 2H), 5.10-5.04 (m, 1H), 3.57 (t, 1H), 3.42-3.36 (m, 1H), 3.26-3.21 (m, 2H), 2.43-2.37 (m, 1H), 2.09-2.00 (m, 1H), 1.62-1.49 (m, 2H), 1.45-1.35 (m, 3H), 1.24-1.14 (m, 1H), 1.02-0.93 (m, 1H).

### Example 27

### (S)-N-((S)-1-Cyano-2-(8-cyano-2-fluoro-6,6-dimethyl-6H-benzo[c]chromen-3-yl)ethyl)-1,4-oxazepane-2-carboxamide

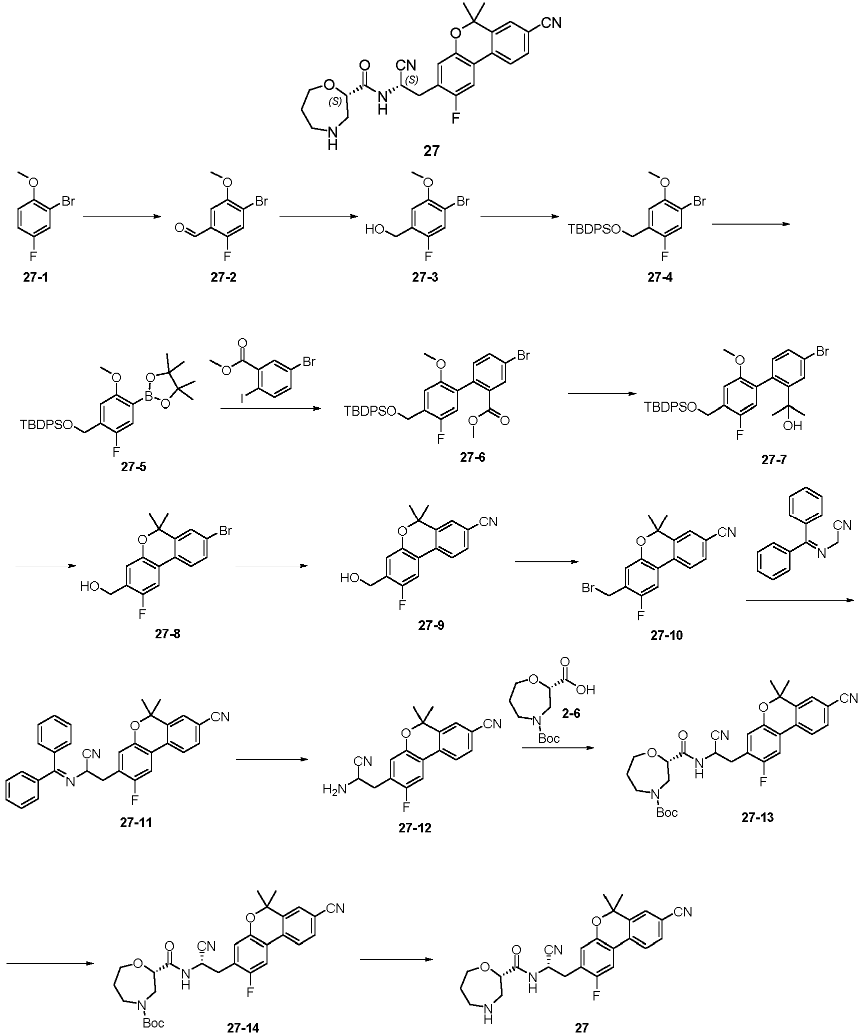

### Synthesis of compound 27-2

At room temperature and in a nitrogen atmosphere, 2-bromo-4-fluoro-1-methoxybenzene (compound **27-1)** (9.24 g, 45.29 mmol) was dissolved in titanium tetrachloride (16.8 mL, 153.23 mmol), followed by the addition of dichloromethyl methyl ether (13.9 mL, 153.68 mmol) at 0°C. The reaction mixture was heated to 30°C and stirred for 1.5 hours. After the reaction was complete, the reaction mixture was diluted with water (20 mL), and extracted with ethyl acetate (25 mL×3).The organic phases were combined, washed with saturated brine (25 mL), dried over anhydrous sodium sulfate and filtered. The filtrate was concentrated under reduced pressure, and the resulting residue was purified by column chromatography (SiO₂, petroleum ether/ethyl acetate=30/1) to obtain compound **27-2.** ¹H NMR (300 MHz, CDCl₃) δ 10.31 (s, 1H), 7.45 (d, 1H), 7.31 (d, 1H), 3.93 (s, 3H).

### Synthesis of compound 27-3

At room temperature, compound **27-2** (7.00 g, 30.18 mmol) was dissolved in methanol (60 mL), followed by the addition of sodium borohydride (1.14 g, 30.14 mmol) at 0°C. The reaction mixture was slowly warmed up to room temperature and stirred for 1 hour. After the reaction was complete, the reaction mixture was poured into water (30 mL), and extracted with ethyl acetate (25 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=5/1) to obtain compound **27-3.** ¹H NMR (400 MHz, CDCl₃) δ 7.25 (d, 1H), 6.98 (d, 1H), 4.71 (s, 2H), 3.88 (s, 3H).

### Synthesis of compound 27-4

At room temperature, compound **27-3** (4.20 g, 17.86 mmol) was dissolved in dichloromethane (60 mL), followed by the addition of imidazole (1.46 g, 21.44 mmol) and tert-butyldiphenylchlorosilane (5.57 mL, 21.44 mmol). The reaction mixture was stirred at room temperature for 1 hour. After the reaction was complete, water (30 mL) was added, and the mixture was extracted with dichloromethane (25 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=70/1) to obtain compound **27-4.** ¹H NMR (400 MHz, CDCl₃) δ 7.70-7.64 (m, 4H), 7.46-7.34 (m, 6H), 7.25-7.15 (m, 2H), 4.79 (s, 2H), 3.86 (s, 3H), 1.12 (s, 9H).

### Synthesis of compound 27-5

At room temperature and in a nitrogen atmosphere, compound **27-4** (5.00 g, 10.88 mmol), bis(pinacolato)diboron (3.59 g, 14.14 mmol), 1,1'-bis(diphenylphosphino)ferrocene palladium(II) dichloride (0.80 g, 1.08 mmol) and potassium acetate (3.20 g, 32.64 mmol) were dissolved in 1,4-dioxane (80 mL). The reaction mixture was heated to 100°C and stirred for 16 hours. The reaction mixture was cooled to room temperature, followed by the addition of water (50 mL), and extracted with ethyl acetate (25 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=70/1) to obtain compound **27-5.** ¹H NMR (400 MHz, CDCl₃) δ 7.71-7.64 (m, 4H), 7.44-7.33 (m, 6H), 7.26 (d, 1H), 7.16 (d, 1H), 4.84 (s, 2H), 3.81 (s, 3H), 1.35 (s, 12H), 1.10 (s, 9H).

### Synthesis of compound 27-6

At room temperature and in a nitrogen atmosphere, compound **27-5** (6.00 g, 11.53 mmol), methyl 5-bromo-2-iodobenzoate (4.72 g, 13.83 mmol), 1,1'-bis(diphenylphosphino)ferrocene palladium(II) dichloride (0.85 g, 1.15 mmol) and potassium carbonate (4.78 g, 34.58 mmol) were dissolved in a mixed solvent of 1,4-dioxane (80 mL) and water (16 mL). The reaction mixture was heated to 50°C and stirred for 4 hours. After the reaction was complete, the reaction mixture was cooled to room temperature, followed by the addition of water (30 mL), and extracted with ethyl acetate (25 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=30/1) to obtain compound **27-6.** ¹H NMR (400 MHz, CDCl₃) δ 8.01 (d, 1H), 7.74-7.67 (m, 4H), 7.66 (dd, 1H), 7.46-7.35 (m, 6H), 7.16 (d, 2H), 6.87 (d, 1H), 4.88 (s, 2H), 3.69 (s, 3H), 3.67 (s, 3H), 1.12 (s, 9H).

### Synthesis of compound 27-7

At room temperature and in a nitrogen atmosphere, compound **27-6** (4.20 g, 6.91 mmol) was dissolved in tetrahydrofuran (80 mL). Methyllithium (9.9 mL, 19.80 mmol, 2.0 mol/L) was added dropwise at 0°C, and the reaction mixture was slowly warmed up to room temperature and stirred for 2 hours. After the reaction was complete, water (20 mL) was added, and the reaction mixture was extracted with dichloromethane (25 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=10/1) to obtain compound **27-7.** ¹H NMR (300 MHz, CDCl₃) δ 7.79 (s, 1H), 7.71 (d, 4H), 7.43-7.34 (m, 7H), 7.25-7.16 (m, 1H), 6.87 (d, 1H), 6.76 (d, 1H), 4.89 (s, 2H), 3.71 (s, 3H), 1.49 (s, 3H), 1.35 (s, 3H), 1.13 (s, 9H).

### Synthesis of compound 27-8

At room temperature, compound **27-7** (2.00 g, 3.29 mmol) was dissolved in acetonitrile (30 mL). Hydroiodic acid (7.5 mL, 55% aqueous solution) was added dropwise, and the reaction mixture was stirred at room temperature for 1.5 hours. After the reaction was complete, saturated aqueous sodium thiosulfate solution (250 mL) was added, and the mixture was extracted with ethyl acetate (50 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=10/1) to obtain compound **27-8.** ¹H NMR (400 MHz, DMSO-*d₆*) δ 7.82 (d, 1H), 7.71 (d, 1H), 7.59-7.53 (m, 2H), 6.98 (d, 1H), 5.30 (t, 1H), 4.52 (d, 2H), 1.56 (s, 6H).

### Synthesis of compound 27-9

At room temperature and in a nitrogen atmosphere, compound **27-8** (100 mg, 0.29 mmol) and zinc cyanide (42 mg, 0.35 mmol) were dissolved in *N*,*N-*dimethylformamide (3 mL), followed by the addition of tetrakis(triphenylphosphine)palladium (34.3 mg, 0.031 mmol). The reaction mixture was sealed, heated to 140°C and stirred for 16 hours. The reaction mixture was cooled to room temperature, followed by the addition of saturated aqueous sodium bicarbonate solution (20 mL), and extracted with ethyl acetate (10 mL×3).The organic phases were combined, washed with saturated brine (25 mL), dried over anhydrous sodium sulfate and filtered. The filtrate was concentrated under reduced pressure, and the resulting residue was purified by preparative thin layer chromatography (petroleum ether/ethyl acetate=5/1) to obtain compound **27-9.** ¹H NMR (400 MHz, DMSO-*d₆*) δ 8.05 (d, 1H), 7.90-7.78 (m, 3H), 7.02 (d, 1H), 5.36 (t, 1H), 4.53 (d, 2H), 1.59 (s, 6H).

### Synthesis of compound 27-10

It was prepared with reference to the synthesis of compound **18-7.** ¹H NMR (400 MHz, DMSO-*d₆*) δ 8.08 (d, 1H), 7.96-7.83 (m, 3H), 7.15 (d, 1H), 4.68 (s, 2H), 1.60 (s, 6H).

### Synthesis of compound 27-11

It was prepared with reference to the synthesis of compound **14-A5** with similar conditions except that benzyltrimethylammonium chloride was replaced with benzyltriethylammonium chloride. ¹H NMR (300 MHz, CDCl₃) δ 7.67-7.57 (m, 4H), 7.53-7.40 (m, 5H), 7.40-7.30 (m, 3H), 7.05-6.94 (m, 2H), 6.90 (d, 1H), 4.51 (t, 1H), 3.38-3.22 (m, 2H), 1.66 (s, 3H), 1.56 (s, 3H).

### Synthesis of compound 27-12

It was prepared with reference to the synthesis of compound **14-A6.** ¹H NMR (300 MHz, CDCl₃) δ 7.71-7.60 (m, 2H), 7.52 (s, 1H), 7.42 (d, 1H), 6.91 (d, 1H), 4.01 (s, 1H), 3.10-3.05 (m, 2H), 1.71 (s, 3H), 1.64 (s, 3H).

### Synthesis of compound 27-13

It was prepared with reference to the synthesis of compound **14-A7** with similar conditions except that *N*,*N*-diisopropylethylamine was replaced with triethylamine. MS-ESI: *m*/*z* 547.2 [M-1]⁻.

### Synthesis of compound 27-14

Compound **27-13** (230 mg, 0.42 mmol) was subjected to chiral resolution (column: chiralpak IC, 250*30 mm, 5 µm; mobile phase: n-hexane, ethanol; gradient: *n*-hexane phase 50%; flow rate: 25 mL/min, column temperature: 30°C) to obtain the crude compound **27-14** (three diastereomer peaks in total, compound **27-14** being the first elution peak). The crude compound was further subjected to chiral resolution (column: chiralpak IG, 250*30 mm, 5 µm; mobile phase: *n*-hexane, ethanol; gradient: *n*-hexane phase 60%; flow rate: 25 mL/min, column temperature: 30°C) to obtain compound **27-14** (two diastereomer peaks in total, compound **27-14** being the first elution peak).

### Synthesis of compound 27

It was prepared with reference to the synthesis of compound **14.** MS-ESI: *m*/*z 449.2* [M+1]⁺. ¹H NMR (400 MHz, DMSO-*d₆*) δ 8.67 (d, 1H), 8.06 (d, 1H), 7.90-81 (m, 3H), 6.95 (d, 1H), 5.11-5.02 (m, 1H), 4.01-3.94 (m, 1H), 3.88-3.80 (m, 1H), 3.76-3.67 (m, 1H), 3.24-3.16 (m, 2H), 3.00 (dd, 1H), 2.80-2.71 (m, 1H), 2.62-2.52 (m, 2H), 1.77-1.67 (m, 2H), 1.58 (s, 6H).

### Example 28

### (2S)-N-(1-Cyano-2-(9-(oxetan-3-yl)-6H-benzo[c]chromen-3-yl)ethyl)-1,4-oxazepane-2-carboxamide

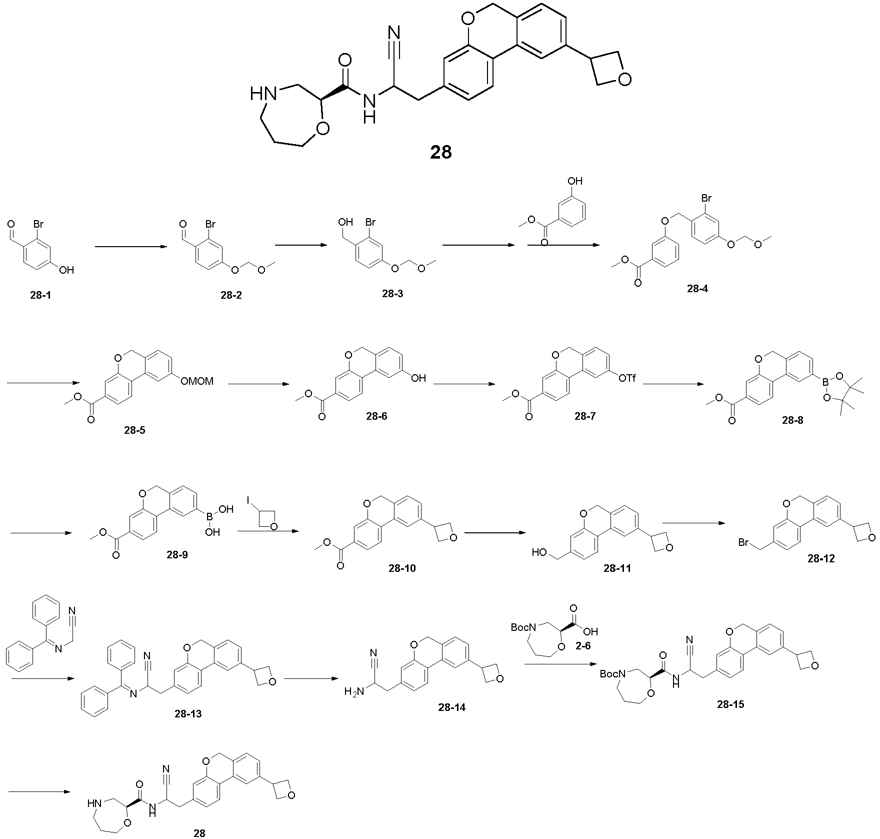

### Synthesis of compound 28-2

At room temperature, 2-bromo-4-hydroxybenzaldehyde (compound **28-1)** (4 g, 19.90 mmol) was dissolved in tetrahydrofuran (50 mL). Sodium hydride (1.19 g, 29.85 mmol, 60%) was added in batches at 0°C, and the reaction mixture was stirred at 0°C for 5 minutes. Bromomethyl methyl ether (2.98 g, 23.88 mmol) was added dropwise, and the reaction mixture was stirred at 0°C for 30 minutes. After the reaction was complete, the reaction mixture was poured into water (50 mL), and extracted with ethyl acetate (50 mL×2). The organic phases were combined, washed with saturated brine (25 mL), dried over anhydrous sodium sulfate and filtered. The filtrate was concentrated under reduced pressure, and the resulting residue was purified by column chromatography (SiO₂, petroleum ether/ethyl acetate=10/1) to obtain compound **28-2.** ¹H NMR (400 MHz, CDCl₃) δ 10.24 (d, 1H), 7.88 (d, 1H), 7.30 (d, 1H), 7.10-7.03 (m., 1H), 5.23 (s, 2H), 3.49 (s, 3H).

### Synthesis of compound 28-3

At room temperature, compound **28-2** (4.40 g, 17.95 mmol) was dissolved in methanol (50 mL), followed by the addition of sodium borohydride (1.36 g, 35.91 mmol) in batches at 0°C. The reaction mixture was stirred at 0°C for 30 minutes. After the reaction was complete, the reaction mixture was poured into water (50 mL), and extracted with ethyl acetate (25 mL×3).The organic phases were combined, washed with saturated brine (25 mL), dried over anhydrous sodium sulfate and filtered. The filtrate was concentrated under reduced pressure to obtain the crude compound **28-3.** ¹H NMR (300 MHz, CDCl₃) δ 7.39-7.33 (m, 1H), 7.30-7.24 (m, 1H), 7.03-6.96 (m, 1H), 5.15 (s, 2H), 4.69 (s, 2H), 3.47 (s, 3H).

### Synthesis of compound 28-4

At room temperature, the crude compound **28-3** (5.00 g, 20.24 mmol) and triethylamine (6.14 g, 60.71 mmol) were dissolved in dichloromethane (50 mL). Methanesulfonyl chloride (3.48 g, 30.35 mmol) was added dropwise, and the reaction mixture was stirred at room temperature for 20 minutes. After the reaction was complete, the reaction mixture was poured into water (50 mL), and extracted with dichloromethane (50 mL×2). The organic phases were combined, washed with saturated brine (25 mL), dried over anhydrous sodium sulfate and filtered. The filtrate was concentrated under reduced pressure to obtain the crude intermediate.

At room temperature, *m*-hydroxybenzoic acid (2.34 g, 15.38 mmol) was dissolved in *N*,*N*-dimethylformamide (50 mL). Sodium hydride (0.80 g, 19.99 mmol, 60%) was added at 0°C, and the reaction mixture was stirred at 0°C for 5 minutes. A solution of the above crude intermediate (5 g, 15.38 mmol) in *N*,*N*-dimethylformamide (10 mL) was added, and the reaction mixture was stirred at room temperature for 5 hours. After the reaction was complete, the reaction mixture was poured into water (150 mL), and extracted with ethyl acetate (50 mL×2). The organic phases were combined, washed with water (25 mL) and saturated brine (10 mL) successively, dried over anhydrous sodium sulfate and filtered. The filtrate was concentrated under reduced pressure, and the resulting residue was purified by column chromatography (SiO₂, petroleum ether/ethyl acetate=10/1) to obtain compound **28-4.** ¹H NMR (300 MHz, CDCl₃) δ 7.69-7.64 (m, 2H), 7.47-7.30 (m, 3H), 7.20-7.14 (m, 1H), 7.05-6.97 (m, 1H), 5.30 (s, 2H), 5.17 (s, 2H), 3.92 (s, 3H), 3.48 (s, 3H).

### Synthesis of compound 28-5

At room temperature and in a nitrogen atmosphere, compound **28-4** (4.90 g, 12.85 mmol), potassium carbonate (3.55 g, 25.71 mmol), tricyclohexylphosphine tetrafluoroborate (0.47 g, 1.28 mmol) and palladium acetate (0.29 g, 1.28 mmol) were dissolved in *N*,*N*-dimethylformamide (50 mL). The reaction mixture was heated to 120°C and stirred for 3 hours. After the reaction was complete, the reaction mixture was cooled to room temperature, poured into water (200 mL), and extracted with ethyl acetate (50 mL×2). The organic phases were combined, washed with water (25 mL×2) and saturated brine (25 mL) successively, dried over anhydrous sodium sulfate and filtered. The filtrate was concentrated under reduced pressure, and the resulting residue was purified by column chromatography (SiO₂, petroleum ether/ethyl acetate=10/1) to obtain compound **28-5.** ¹H NMR (300 MHz, CDCl₃) δ 7.77-7.70 (m, 2H), 7.69-7.62 (m, 1H), 7.43-7.38 (m, 1H), 7.13-7.07 (m, 1H), 7.06-6.98 (m, 1H), 5.23 (s, 2H), 5.10 (s, 2H), 3.92 (s, 3H), 3.51 (s, 3H).

### Synthesis of compound 28-6

At room temperature, compound **28-5** (4.00 g, 13.33 mmol) was dissolved in tetrahydrofuran (40 mL), followed by the addition of concentrated hydrochloric acid (4 mL). The reaction mixture was stirred at room temperature for 2 hours. After the reaction was complete, the reaction solution was poured into water (30 mL), and extracted with ethyl acetate (50 mL×3).The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **28-6.** ¹H NMR (400 MHz, DMSO-*d₆*) δ 9.67 (s, 1H), 7.87 (d, 1H), 7.67-7.61 (m, 1H), 7.45 (s, 1H), 7.23 (d, 1H), 7.13 (d, 1H), 6.85-6.78 (m, 1H), 5.09 (s, 2H), 3.86 (s, 3H).

### Synthesis of compound 28-7

At room temperature, compound **28-6** (1.20 g, 4.68 mmol) and triethylamine (1.40 g, 13.84 mmol) were dissolved in dichloromethane (30 mL), followed by the addition of *N*-phenylbis(trifluoromethanesulfonimide) (2.00 g, 5.60 mmol). The reaction mixture was stirred at room temperature for 2 hours. After the reaction was complete, the reaction mixture was poured into water (30 mL), and extracted with dichloromethane (30 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **28-7.** ¹H NMR (400 MHz, CDCl₃) δ 7.79-7.73 (m, 1H), 7.73-7.69 (m, 1H), 7.67 (d, 1H), 7.60 (d, 1H), 7.29-7.22 (m, 2H), 5.16 (s, 2H), 3.93 (s, 3H).

### Synthesis of compound 28-8

At room temperature and in a nitrogen atmosphere, compound **28-7** (1.60 g, 4.12 mmol) and bis(pinacolato)diboron (1.20 g, 4.73 mmol) were dissolved in 1,4-dioxane (40 mL), followed by the addition of potassium acetate (1.20 g, 12.23 mmol) and [1,1'-bis(diphenylphosphino)ferrocene]palladium(II) dichloride (300 mg, 0.410 mmol). The reaction mixture was heated to 85°C and stirred for 3 hours. After the reaction was complete, the reaction mixture was cooled to room temperature, poured into water (100 mL), extracted with ethyl acetate (80 mL×3), washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **28-8.** ¹H NMR (400 MHz, CDCl₃) δ 8.18 (s, 1H), 7.90 (d, 1H), 7.78 (d, 1H), 7.72 (d, 1H), 7.63 (s, 1H), 7.18 (d, 1H), 5.16 (s, 2H), 3.92 (s, 3H),1.37 (s, 12H).

### Synthesis of compound 28-9

At room temperature, compound **28-8** (1.50 g, 4.10 mmol) was dissolved in tetrahydrofuran (20 mL), followed by the addition of sodium periodate (2.65 g, 12.27 mmol), ammonium acetate (0.95 g, 12.32 mmol) and water (10 mL). The reaction mixture was stirred at room temperature for 2 hours. After the reaction was complete, the reaction mixture was poured into water (30 mL), and extracted with ethyl acetate (50 mL×3).The organic phases were combined, washed with saturated brine (100 mL), dried over anhydrous sodium sulfate and filtered. The filtrate was concentrated under reduced pressure, and the resulting residue was triturated in a mixed solvent (50 mL) of ethyl acetate and petroleum ether to obtain compound **28-9.** ¹H NMR (400 MHz, DMSO-*d₆*) δ 8.38 (s, 1H), 8.23 (s, 2H), 8.03 (d, 1H), 7.81 (d, 1H), 7.72-7.65 (m, 1H), 7.47 (d, 1H), 7.28 (d, 1H), 5.21 (s, 2H), 3.86 (s, 3H).

### Synthesis of compound 28-10

At room temperature and in a nitrogen atmosphere, compound **28-9** (1.10 g, 3.87 mmol) and 3-iodooxetane (800 mg, 4.35 mmol) were dissolved in isopropanol (30 mL), followed by the addition of nickel iodide (300 mg, 0.96 mmol), 2-aminocyclohexanol hydrochloride (100 mg, 0.87 mmol) and a solution (8.0 mL, 8.00 mmol, 1.0 mol/L) of potassium bis(trimethylsilyl)amide in tetrahydrofuran. The reaction mixture was heated to 100°C and stirred for 1 hour. After the reaction was complete, the reaction mixture was cooled to room temperature, poured into water (50 mL), and extracted with ethyl acetate (50 mL×3).The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=3/1) to obtain compound **28-10.**

### Synthesis of compound 28-11

It was prepared with reference to the synthesis of compound **12-4.** MS-ESI: *m*/*z* 251.2 [M-18+1]⁺.

### Synthesis of compound 28-12

It was prepared with reference to the synthesis of compound **18-7** with similar conditions except that dichloromethane was replaced with tetrahydrofuran. ¹H NMR (400 MHz, CDCl₃) δ 7.76-7.70 (m, 2H), 7.34 (d, 1H), 7.16 (d, 1H), 7.13-7.07 (m, 1H), 7.03 (s, 1H), 5.16-5.08 (m, 4H), 4.80 (t, 2H), 4.47 (s, 2H), 4.32-4.21 (m, 1H).

### Synthesis of compound 28-13

At room temperature, *N*-(diphenylmethylene)aminoacetonitrile (135 mg, 0.61 mmol) was dissolved in tetrahydrofuran (5 mL). Sodium hydroxide (75 mg, 1.88 mmol) and water (3 mL) were added, and the reaction mixture was stirred at room temperature for 10 minutes. Compound **28-12** (200 mg, 0.604 mmol) was added, and the reaction mixture was stirred at room temperature for 18 hours. The reaction mixture was poured into water (10 mL), and extracted with ethyl acetate (10 mL×3). The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=3/1) to obtain compound **28-13.** MS-ESI: *m*/*z* 471.5 [M+1]⁺.

### Synthesis of compound 28-14

It was prepared with reference to the synthesis of compound **14-A6.** MS-ESI: *m*/*z* 329.1 [M+1]⁺.

### Synthesis of compound 28-15

It was prepared with reference to the synthesis of compound **14-A7** with similar conditions except that *N*,*N*-diisopropylethylamine was replaced with triethylamine. MS-ESI: *m*/*z* 434.5 [M-100+1]⁺.

### Synthesis of compound 28

It was prepared with reference to the synthesis of compound **14.** MS-ESI: *m*/*z 434.2* [M+1]⁺. ¹H NMR (400 MHz, CD₃OD) δ 7.83 (d, 1H), 7.79 (s, 1H), 7.41-7.38 (m, 1H), 7.24 (d, 1H), 7.04-7.01 (m, 1H), 6.93 (d, 1H), 5.18-5.01 (m, 5H), 4.85-4.78 (m, 2H), 4.41-4.31 (m, 1H), 4.15-3.94 (m, 2H), 3.83-3.75 (m, 1H), 3.31-3.10 (m, 3H), 2.99-2.63 (m, 3H), 1.97-1.79 (m, 2H).

### Example 29

### (2S)-N-(1-Cyano-2-(2-fluoro-8-(methylsulfonyl)-6H-benzo[c]chromen-3-yl)ethyl)-1,4-oxazepane-2-carboxamide

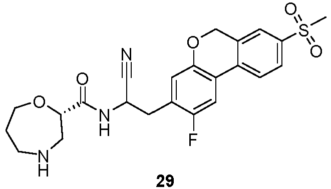

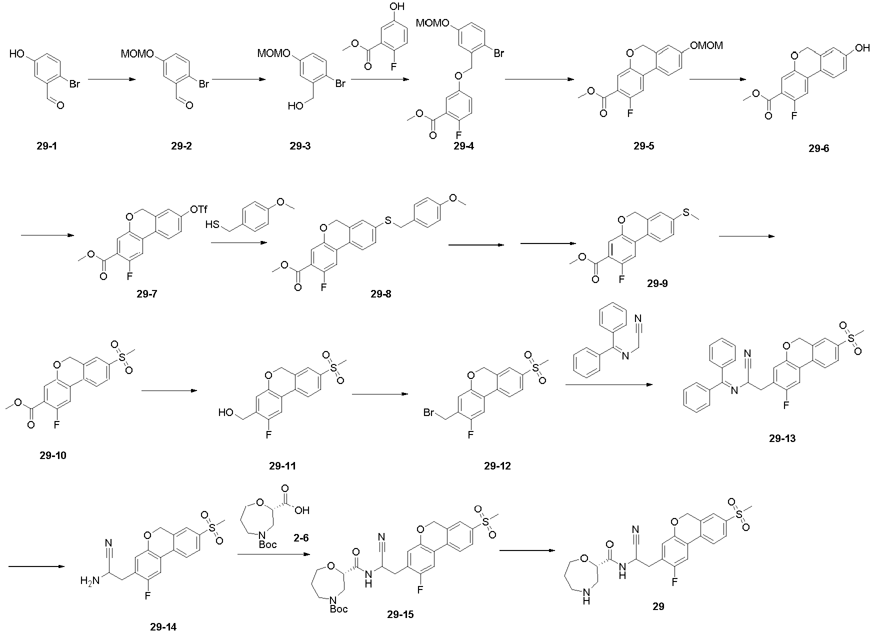

### Synthesis of compound 29-2

At room temperature and in a nitrogen atmosphere, 2-bromo-5-hydroxybenzaldehyde (compound **29-1)** (13 g, 64.67 mmol) was dissolved in *N,N-*dimethylformamide (50 mL). At 0°C, sodium hydride (1.63 g, 67.90 mmol, 60%) was added in batches and stirred at 0°C for 10 minutes. Bromomethyl methyl ether (8.49 g, 67.90 mmol) was added dropwise, and the reaction mixture was stirred at room temperature for 30 minutes. After the reaction was complete, water (100 mL) was added, and the reaction mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **29-2.** ¹H NMR (300 MHz, CDCl₃) δ 10.31 (s, 1H), 7.60-7.51 (m, 2H), 7.15 (dd, 1H), 5.20 (s, 2H), 3.47 (s, 3H).

### Synthesis of compound 29-3

At room temperature, compound **29-2** (9.60 g, 39.17 mmol) was dissolved in a mixed solvent of water (10 mL) and methanol (100 mL). At 0°C, sodium borohydride (2.22 g, 58.76 mmol) was added slowly, and the reaction mixture was stirred at room temperature for 1 hour. After the reaction was complete, ethyl acetate (20 mL) was added, and the reaction mixture was concentrated under reduced pressure. Water (30 mL) was added to the resulting residue, and the mixture was extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound 29-3. ¹H NMR (300 MHz, CDCl₃) δ 7.43 (d, 1H), 7.19(d, 1H), 6.86 (dd, 1H), 5.17 (s, 2H), 4.71 (s, 2H), 3.47 (s, 3H).

### Synthesis of compound 29-4

It was prepared with reference to the synthesis of compound **18-3.** ¹H NMR (300 MHz, CDCl₃) δ 7.54-7.50 (m, 1H), 7.47(d, 1H), 7.23 (d, 1H), 7.18-7.01 (m, 2H), 6.86 (dd, 1H), 5.15 (s, 2H), 5.07 (s, 2H), 3.94 (s, 3H), 3.46 (s, 3H).

### Synthesis of compound 29-5

It was prepared with reference to the synthesis of compound **18-4.** MS-ESI: *m*/*z* 319.2 [M+1]⁺.

### Synthesis of compound 29-6

At room temperature, compound **29-5** (3.50 g, 11.00 mmol) was dissolved in methanol (20 mL), followed by the addition of 6 M hydrochloric acid (20 mL). The reaction mixture was stirred at room temperature for 4 hours. After the reaction was complete, water (20 mL) was added, and the reaction mixture was extracted with ethyl acetate (100 mL×3).The organic phases were combined, washed with saturated brine (50 mL), dried over anhydrous sodium sulfate and filtered. The filtrate was concentrated under reduced pressure, and the resulting residue was triturated in ethyl acetate (30 mL) to obtain compound **29-6.** MS-ESI: *m*/*z* 275.1 [M+1]⁺.

### Synthesis of compound 29-7

At room temperature, compound **29-6** (2.40 g, 8.75 mmol) was dissolved in *N,N-*dimethylformamide (25 mL), followed by the addition of triethylamine (1.33 g, 13.13 mmol) and *N*-phenylbis(trifluoromethanesulfonimide) (3.75 g, 10.50 mmol). The reaction mixture was stirred at room temperature for 1.5 hours. After the reaction was complete, water (100 mL) was added, and the reaction mixture was extracted with ethyl acetate (100 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **29-7.** ¹H NMR (300 MHz, CDCl₃) δ 7.73 (d, 1H), 7.57 (d, 1H), 7.44 (d, 1H), 7.35-7.26 (m, 1H), 7.14 (d, 1H), 5.14 (s, 2H), 3.94 (s, 3H).

### Synthesis of compound 29-8

At room temperature and in a nitrogen atmosphere, compound **29-7** (1.00 g, 2.46 mmol), tris(dibenzylideneacetone)dipalladium (0.20 g, 0.25 mmol), potassium carbonate (0.68 g, 4.92 mmol) and 4,5-bis(diphenylphosphino)-9,9-dimethylxanthene (0.28 g, 0.49 mmol) were dissolved in 1,4-dioxane (10 mL), followed by the addition of 4-methoxybenzylthiol (0.42 g, 2.72 mmol). The reaction mixture was heated to 70°C and stirred for 1.5 hours. After the reaction was complete, the reaction mixture was cooled to room temperature, followed by the addition of water (30 mL), and extracted with ethyl acetate (50 mL×3).The organic phase was washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **29-8.** MS-ESI: *m*/*z* 411.3 [M+1]⁺.

### Synthesis of compound 29-9

At room temperature, compound **29-8** (860 mg, 1.86 mmol) was dissolved in trifluoroacetic acid (9 mL), and the reaction mixture was heated to 70°C and stirred for 3 hours. After the reaction was complete, the reaction mixture was concentrated under reduced pressure. Water (20 mL) was added to the resulting residue, and the mixture was extracted with ethyl acetate (25 mL×3).The organic phases were combined, washed with saturated brine (25 mL), dried over anhydrous sodium sulfate and filtered. The filtrate was concentrated under reduced pressure to obtain the crude intermediate, which was dissolved in N,N-dimethylformamide (6 mL). At 0°C, sodium hydride (32.23 mg, 1.34 mmol) was added in batches, and the mixture was stirred at room temperature for 10 minutes. Iodomethane (190 mg, 1.34 mmol) was added dropwise, and the reaction mixture was stirred at room temperature for 30 minutes. After the reaction was complete, water (50 mL) was added, and the mixture was extracted with ethyl acetate (50 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=2/1) to obtain compound **29-9.** ¹H NMR (300 MHz, CDCl₃) δ 7.98 (d, 1H), 7.83 (d, 1H), 7.79 (s, 1H), 7.58 (d, 1H), 7.52 (d, 1H), 5.20 (s, 2H), 3.95 (s, 3H), 3.10 (s, 3H).

### Synthesis of compound 29-10

At room temperature, compound **29-9** (400 mg, 1.32 mmol) and *m-*chloroperoxybenzoic acid (22 mg, 0.13 mmol, 85%) were dissolved in dichloromethane (5 mL), and the reaction mixture was stirred at room temperature for 2 hours. After the reaction was complete, water (20 mL) was added, and the reaction mixture was extracted with dichloromethane (25 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **29-10.** ¹H NMR (300 MHz, CDCl₃) δ 7.98 (d, 1H), 7.83 (d, 1H), 7.79 (s, 1H), 7.58 (d, 1H), 7.52 (d, 1H), 5.20 (s, 2H), 3.95 (s, 3H), 3.10 (s, 3H).

### Synthesis of compound 29-11

It was prepared with reference to the synthesis of compound **14-A3.** ¹H NMR (400 MHz, CDCl₃) δ 8.10 (d, 1H), 7.91 (dd, 1H), 7.89 (s, 1H), 7.83 (d, 1H), 7.09 (d, 1H), 5.37 (t, 1H), 5.24 (s, 2H), 4.55 (d, 2H), 3.24 (s, 3H).

### Synthesis of compound 29-12

It was prepared with reference to the synthesis of compound **14-A4.** ¹H NMR (400 MHz, CDCl₃) δ 7.96 (dd, 1H), 7.82-7.62 (d, 2H), 7.45 (d, 1H), 7.06 (d, 1H), 5.17 (s, 2H), 4.49 (s, 2H), 3.09 (s, 3H).

### Synthesis of compound 29-13

It was prepared with reference to the synthesis of compound **14-A5.** ¹H NMR (300 MHz, CDCl₃) δ 7.93 (dd, 1H), 7.76-7.71 (m, 2H), 7.65-7.59 (m, 2H), 7.50-7.42 (m, 4H), 7.38-7.32 (m, 3H), 7.09-6.99 (m, 2H), 6.91 (d, 1H), 5.12 (s, 2H), 4.54 (t, 1H), 3.36-3.15 (m, 2H), 3.08 (s, 3H).

### Synthesis of compound 29-14

It was prepared with reference to the synthesis of compound **14-A6.**

### Synthesis of compound 29-15

It was prepared with reference to the synthesis of compound **14-A7.**

### Synthesis of compound 29

It was prepared with reference to the synthesis of compound **14.** MS-ESI: *m*/*z 474.2* [M+1]⁺. ¹H NMR (400 MHz, CD₃OD) δ 8.50 (s, 1H), 7.99-7.89 (m, 2H), 7.82 (s, 1H), 7.66 (d, 1H), 6.97 (d, 1H), 5.17 (s, 2H), 5.16-5.03 (m, 1H), 4.23-4.10 (m, 1H), 4.07-3.94 (m, 1H), 3.81-3.71 (m, 1H), 3.45-3.38 (m, 0.5H), 3.32-3.28 (m, 0.5H), 3.27-3.14 (m, 2H), 3.13 (s, 3H), 3.09-2.95 (m, 2H), 2.95-2.87 (m, 0.5H), 2.80-2.73 (m, 0.5H), 2.03-1.83 (m, 2H).

### Example 30

### (S)-N-((S)-1-Cyano-2-(2-fluoro-8-(methylsulfonyl)-6H-benzo[c]chromen-3-yl)ethyl)-1,4-oxazepane-2-carboxamide

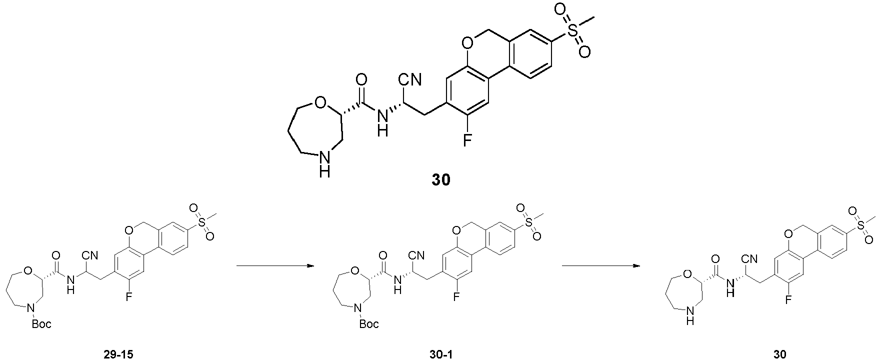

### Synthesis of compound 30-1

*Tert*-butyl (2*S*)-2-((1-cyano-2-(2-fluoro-8-(methylsulfonyl)-6*H*-benzo[*c*]chromen-3-yl)ethyl)carbamoyl)-1,4-oxazepane-4-carboxylate (compound **29-15)** (100 mg, 0.17 mmol) was subjected to chiral resolution (column: chiralpak IA, 250*25 mm, 5 µm; mobile phase: supercritical carbon dioxide, isopropanol; gradient: carbon dioxide phase 70%; flow rate: 60 mL/min, column temperature: 30°C) to obtain compound **30-1** (two diastereomer peaks in total, compound **30-1** being the first elution peak).

### Synthesis of compound 30

It was prepared with reference to the synthesis of compound **14.** MS-ESI: *m*/*z 474.1* [M+1]⁺. ¹H NMR (400 MHz, DMSO-*d₆*) δ 8.70 (d, 1H), 8.15-8.09 (m, 1H), 7.96-7.85 (m, 3H), 7.04 (d, 1H), 5.29-5.17 (m, 2H), 5.12-5.02 (m, 1H), 4.03-3.96 (m, 1H), 3.90-3.81 (m, 1H), 3.78-3.68 (m, 1H), 3.25 (s, 3H), 3.23-3.13 (m, 2H), 3.07-2.98 (m, 1H), 2.82-2.72 (m, 1H), 2.65-2.53 (m, 2H), 1.82-1.64 (m, 2H).

### Example 31

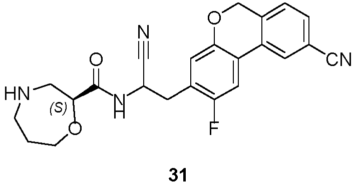

### (2S)-N-(1-Cyano-2-(9-cyano-2-fluoro-6H-benzo[c]chromen-3-yl)ethyl)-1,4-oxazepane-2-carboxamide

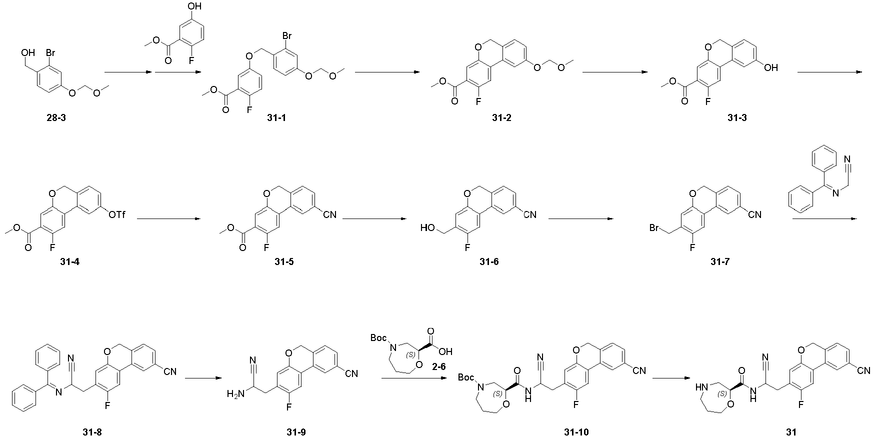

### Synthesis of compound 31-1

At room temperature, the crude (2-bromo-4-(methoxymethoxy)phenyl)methanol (compound **28-3)** (3.00 g, 12.14 mmol) and triethylamine (3.00 g, 29.65 mmol) were dissolved in dichloromethane (60 mL). Methanesulfonyl chloride (2.00 g, 1.35 mL, 17.46 mmol) was added dropwise, and the reaction mixture was stirred at room temperature for 2 hours. After the reaction was complete, the reaction mixture was washed with water (10 mL×3).The organic phases were combined, dried over anhydrous sodium sulfate and filtered. The filtrate was concentrated under reduced pressure to obtain the crude intermediate.

At room temperature, methyl 1-fluoro-4-hydroxybenzoate (3 g, 17.63 mmol) was dissolved in dichloromethane (100 mL), followed by the addition of triethylamine (3 g, 29.65 mmol). After stirring at room temperature for 10 minutes, the crude intermediate (5 g, 15.38 mmol) obtained by the above method was added, and the reaction mixture was stirred at room temperature for 2 hours. After the reaction was complete, the reaction mixture was diluted with saturated aqueous ammonium chloride solution (100 mL), and extracted with ethyl acetate (200 mL×3).The organic phases were combined, washed with water (100 mL×3), dried over anhydrous magnesium sulfate and filtered. The filtrate was concentrated under reduced pressure, and the resulting residue was purified by column chromatography (SiO₂, petroleum ether/ethyl acetate=8/1) to obtain compound **31-1.** ¹H NMR (400 MHz, CDCl₃) δ 7.59 (d, 1H), 7.49 (d, 1H), 7.38 (s, 1H), 7.22-7.07 (m, 3H), 5.29 (s, 2H), 5.14 (s, 2H), 4.00 (s, 3H), 3.57 (s, 3H).

### Synthesis of compound 31-2

At room temperature and in a nitrogen atmosphere, compound **31-1** (1 g, 2.51 mmol) and palladium acetate (0.1 g, 0.445 mmol) were dissolved in *N*,*N*-dimethylformamide (30 mL), followed by the addition of potassium carbonate (0.50 g, 3.62 mmol) and tricyclohexylphosphine tetrafluoroborate (0.30 g, 0.63 mmol). The reaction mixture was heated to 100°C and stirred for 4 hours. After the reaction was complete, the reaction mixture was cooled to room temperature, diluted with saturated aqueous ammonium chloride solution (60 mL), and extracted with ethyl acetate (20 mL×3).The organic phases were combined, washed with water (20 mL×3), dried over anhydrous magnesium sulfate and filtered. The filtrate was concentrated under reduced pressure, and the resulting residue was purified by column chromatography (SiO₂, petroleum ether/ethyl acetate=5/1) to obtain compound **31-2.** ¹H NMR (400 MHz, CDCl₃) δ 7.56 (d, 1H), 7.46 (d, 1H), 7.36 (s, 1H), 7.18-7.08 (m, 2H), 5.27 (s, 2H), 5.12 (s, 2H), 3.97 (s, 3H), 3.55(s, 3H).

### Synthesis of compound 31-3

At room temperature, compound **31-2** (5 g, 15.71 mmol) was dissolved in tetrahydrofuran (30 mL), followed by the addition of 2 M hydrochloric acid (5 mL). The reaction mixture was stirred at room temperature for 20 minutes. After the reaction was complete, dichloromethane (50 mL) was added, and the reaction mixture was separated into two phases. The organic phase was washed with saturated aqueous sodium bicarbonate solution (30 mL×3), dried over anhydrous sodium sulfate and filtered. The filtrate was concentrated under reduced pressure, and the resulting residue was triturated in ethyl acetate (20 mL) to obtain compound **31-3.** ¹H NMR (400 MHz, DMSO-*d₆*) δ 7.74 (d, 1H), 7.36 (d, 1H), 7.25 (s, 1H), 7.14 (d, 1H), 6.85 (dd, 1H), 5.07 (s, 2H), 3.85 (s, 3H).

### Synthesis of compound 31-4

Compound **31-3** (1.20 g, 3.94 mmol) and triethylamine (0.80 g, 7.88 mmol) were dissolved in *N*,*N*-dimethylformamide (10 mL), followed by the addition of *N-*phenylbis(trifluoromethanesulfonimide) (1.69 g, 4.73 mmol). The reaction mixture was stirred at room temperature for 1 hour. After the reaction was complete, water (30 mL) was added, and the reaction mixture was extracted with ethyl acetate (30 mL×3).The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **31-4.** ¹H NMR (300 MHz, DMSO-*d₆*) δ 8.26-8.21 (m, 1H), 8.12 (d, 1H), 7.64-7.52 (m, 2H), 7.48-7.42 (m, 1H), 5.28 (s, 2H), 3.90 (s, 3H).

### Synthesis of compound 31-5

At room temperature and in a nitrogen atmosphere, compound **31-4** (1.80 g, 3.54 mmol), zinc cyanide (0.62 g, 5.32 mmol) and tetrakis(triphenylphosphine)palladium (0.61 g, 0.53 mmol) were dissolved in *N,N*-dimethylformamide (20 mL). The reaction mixture was heated to 90°C and stirred for 3 hours. After the reaction was complete, the reaction mixture was cooled to room temperature, followed by the addition of water (100 mL), and extracted with ethyl acetate (100 mL×3). The organic phases were combined, washed with saturated brine (50 mL×1), dried over anhydrous sodium sulfate and filtered. The filtrate was concentrated under reduced pressure, and the resulting residue was purified by column chromatography (SiO₂, petroleum ether/ethyl acetate=2/1) to obtain compound **31-5.** ¹H NMR (300 MHz, DMSO-*d*₆) ¹H NMR (300 MHz, DMSO-*d*₆) δ 8.50 (s, 1H), 8.09-8.05 (m, 1H), 7.91-7.89 (m, 1H), 7.56-7.53 (m, 1H), 7.42-7.40 (m, 1H), 5.28 (s, 2H), 3.86 (s, 3H).

### Synthesis of compound 31-6

It was prepared with reference to the synthesis of compound **14-A3** with similar conditions except that tetrahydrofuran was replaced with a mixed solvent of tetrahydrofuran and methanol (volume ratio 10/1). ¹H NMR (300 MHz, DMSO-*d*₆) δ 8.40 (s, 1H), 7.92-7.82 (m, 2H), 7.55-7.50 (m, 1H), 7.12-7.07 (m, 1H), 5.39 (s, 2H), 4.59-4.54 (m, 2H).

### Synthesis of compound 31-7

It was prepared with reference to the synthesis of compound **18-7.** ¹H NMR (300 MHz, DMSO-*d*₆) δ 8.42 (s, 1H), 8.01-7.96 (m, 1H), 7.90-7.85 (m, 1H), 7.57-7.52 (m, 1H), 7.27-7.21 (m, 1H), 5.26 (s, 2H), 4.71 (s, 2H).

### Synthesis of compound 31-8

It was prepared with reference to the synthesis of compound **14-A5.** MS-ESI: *m*/*z* 458.1 [M+1]⁺.

### Synthesis of compound 31-9

It was prepared with reference to the synthesis of compound **14-A6.** MS-ESI: *m*/*z* 294.0 [M+1]⁺.

### Synthesis of compound 31-10

It was prepared with reference to the synthesis of compound **14-A7.** MS-ESI: *m*/*z* 538.2 [M+18]⁺.

### Synthesis of compound 31

It was prepared with reference to the synthesis of compound **14.** MS-ESI: *m*/*z* 421.1 [M+1]⁺. ¹H NMR (400 MHz, DMSO-*d*₆) δ 8.72-8.63 (m, 1H), 8.39 (s, 1H), 7.94-7.87 (m, 1H), 7.85-7.79 (m, 1H), 7.53-7.47 (m, 1H), 7.05-7.00 (m, 1H), 5.26-5.15 (m, 2H), 5.10-4.93 (m, 1H), 4.02-3.81 (m, 2H), 3.77-3.65 (m, 1H), 3.27-2.98 (m, 3H), 2.84-2.52 (m, 3H) , 1.82-1.64 (m, 2H).

### Example 32

### (S)-N-((S)-1-Cyano-2-(9-cyano-2-fluoro-6H-benzo[c]chromen-3-yl)ethyl)-1,4-oxazepane-2-carboxamide

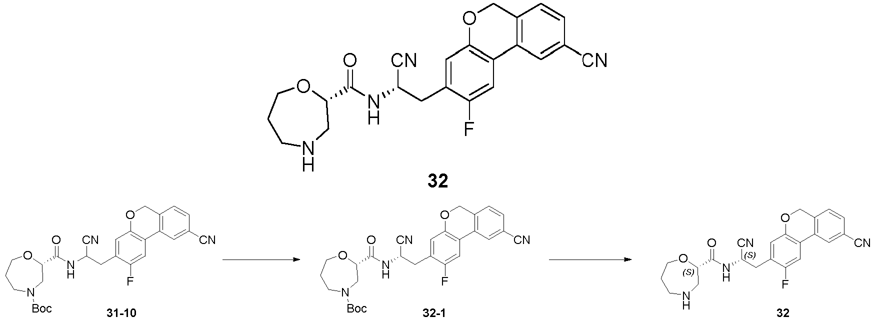

### Synthesis of compound 32-1

*Tert-*butyl (2*S*)-2-((1-cyano-2-(9-cyano-2-fluoro-6*H*-benzo[*c*]chromen-3-yl)ethyl)carbamoyl)-1,4-oxazepane-4-carboxylate (compound **31-10)** (350 mg, 0.67 mmol) was subjected to chiral resolution (column: chiralpak IC, 250*30 mm, 5 µm; mobile phase: *n*-hexane, ethanol; gradient: *n*-hexane phase 30%; flow rate: 25 mL/min, column temperature: 30°C) to obtain compound **32-1** (two diastereomer peaks in total, compound **32-1** being the second elution peak).

### Synthesis of compound 32

It was prepared with reference to the synthesis of compound **14.** MS-ESI: *m*/*z* 421.2 [M+1]⁺. ¹H NMR (400 MHz, DMSO-*d*₆) δ 8.70 (d, 1H), 8.39 (s, 1H), 7.91 (d, 1H), 7.82 (d, 1H), 7.50 (d, 1H), 7.02 (d, 1H), 5.26-5.14 (m, 2H), 5.10-5.00 (m, 1H), 4.02-3.95 (m, 1H), 3.89-3.82 (m, 1H), 3.77-3.69 (m, 1H), 3.28-3.12 (m, 2H), 3.05-2.98 (m, 1H), 2.81-2.72 (m, 1H), 2.63-2.51 (m, 2H), 1.81-1.64 (m, 2H).

### Example 33

### (2S)-N-(1-Cyano-2-(3-cyano-9-fluoro-5H-chromeno[4,3-b]pyridin-8-yl)ethyl)- ,4-oxazepane-2-carboxamide

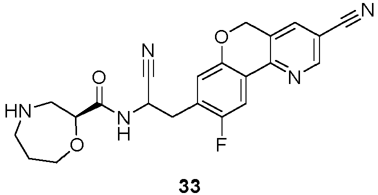

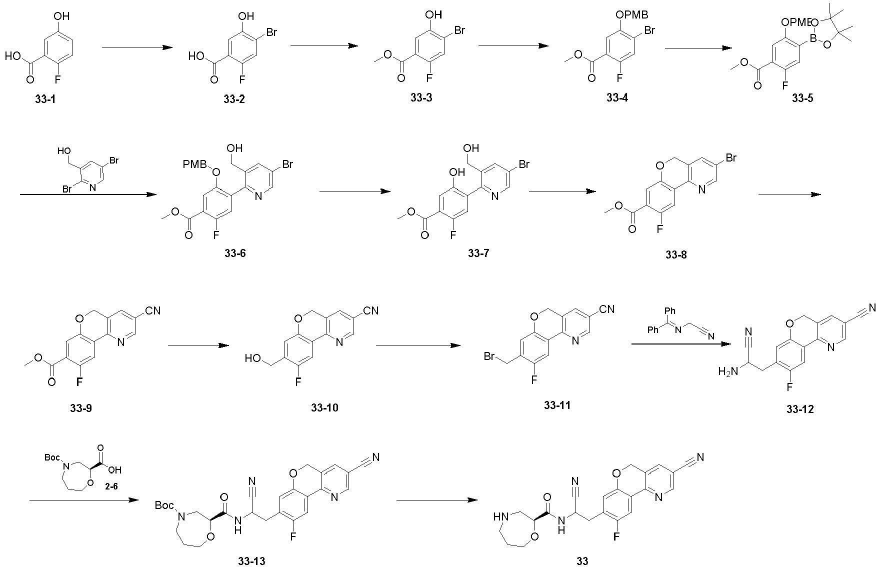

### Synthesis of compound 33-2

At room temperature, 2-fluoro-5-hydroxybenzoic acid (compound **33-1)** (25.00 g, 146.93 mmol) was dissolved in chloroform (300 mL). At 0°C, a solution of bromine (76.70 g, 480.00 mmol) in acetic acid (300 mL) was slowly added dropwise, and the reaction mixture was stirred at room temperature for 16 hours. At 0°C, saturated aqueous sodium thiosulfate solution (300 mL) was slowly added, and the reaction mixture was stirred for 15 minutes and separated into two phases. The aqueous phase was extracted with ethyl acetate (300 mL×3). The organic phases were combined, dried over anhydrous sodium sulfate and filtered. The filtrate was concentrated under reduced pressure, and the resulting residue was triturated in dichloromethane (50 mL) to obtain compound **33-2.** ¹H NMR (400 MHz, DMSO-*d*₆) δ 10.59 (s, 1H), 7.56 (d, 1H), 7.40 (d, 1H).

### Synthesis of compound 33-3

At room temperature, compound **33-2** (22.60 g, 84.60 mmol) was dissolved in methanol (100 mL). Thionyl chloride (20.10 g, 177.36 mmol) was slowly added dropwise, and the reaction mixture was heated to 70°C and stirred for 2 hours. After the reaction was complete, the reaction mixture was cooled to room temperature and concentrated under reduced pressure. The resulting residue was diluted with ethyl acetate (150 mL), washed successively with water (50 mL) and saturated aqueous sodium chloride solution (50 mL), dried over anhydrous sodium sulfate and filtered. The filtrate was concentrated under reduced pressure to obtain the crude compound **33-3.** ¹H NMR (400 MHz, CDCl₃) δ 7.58 (d, 1H), 7.32 (d, 1H), 3.94 (s, 3H).

### Synthesis of compound 33-4

At room temperature, compound **33-3** (20.70 g, 69.80 mmol), potassium carbonate (19.30 g, 139.64 mmol) and *p*-methoxybenzyl chloride (12.00 g, 76.62 mmol) were dissolved in acetonitrile (100 mL). The reaction mixture was heated to 80°C and stirred for 3 hours. After the reaction was complete, the reaction mixture was cooled to room temperature, diluted with water (300 mL), and extracted with ethyl acetate (150 mL×4). The organic phases were combined, washed with water (200 mL) and saturated aqueous sodium chloride solution (200 mL) successively, dried over anhydrous sodium sulfate and filtered. The filtrate was concentrated under reduced pressure, and the resulting residue was purified by column chromatography (SiO₂, petroleum ether/ethyl acetate=1/1) to obtain compound **33-4.** ¹H NMR (400 MHz, CDCl₃) δ 7.50 (d, 1H), 7.42-7.37 (m, 3H), 6.96-6.92 (m, 2H), 5.09 (s, 2H), 3.94 (s, 3H), 3.83 (s, 3H).

### Synthesis of compound 33-5

At room temperature and in a nitrogen atmosphere, compound **33-4** (24.30 g, 45.40 mmol), bis(pinacolato)diboron (13.80 g, 54.34 mmol), potassium acetate (8.91 g, 90.78 mmol) and 1,1'-bis(diphenylphosphino)ferrocene palladium(II) dichloride (3.32 g, 4.54 mmol) were dissolved in dioxane (100 mL). The reaction mixture was heated to 100°C and stirred for 16 hours. The reaction mixture was cooled to room temperature, diluted with water (100 mL), and extracted with ethyl acetate (200 mL×3). The organic phases were combined, washed with water (100 mL) and saturated aqueous sodium chloride solution (80 mL) successively, dried over anhydrous sodium sulfate and filtered. The filtrate was concentrated under reduced pressure, and the resulting residue was purified by column chromatography (SiO₂, petroleum ether/ethyl acetate=5/1) to obtain compound **33-5.** MS-ESI: *m*/*z* 439.2 [M+23]⁺. ¹H NMR (400 MHz, CDCl₃) δ 7.53-7.40 (m, 4H), 6.91 (d, 2H), 5.06 (s, 2H), 3.94 (s, 3H), 3.83 (s, 3H), 1.36 (s, 12H).

### Synthesis of compound 33-6

At room temperature and in a nitrogen atmosphere, compound 33-5 (16.50 g, 31.70 mmol), 2,5-dibromopyridin-3-ylmethanol (10.80 g, 38.00 mmol), 1,1'-bis(diphenylphosphino)ferrocene palladium(II) dichloride (2.32 g, 3.17 mmol) and sodium carbonate (6.72 g, 63.4 mmol) were dissolved in a mixed solvent of dioxane (150 mL) and water (5 mL). The reaction mixture was heated to 80°C and stirred for 16 hours. The reaction mixture was cooled to room temperature, diluted with water (300 mL), and extracted with ethyl acetate (150 mL×3). The organic phases were combined, washed with water (200 mL) and saturated aqueous sodium chloride solution (200 mL) successively, dried over anhydrous sodium sulfate and filtered. The filtrate was concentrated under reduced pressure, and the resulting residue was purified by column chromatography (SiO₂, petroleum ether/ethyl acetate=1/1) to obtain compound **33-6.** MS-ESI: *m*/*z* 476.1 [M+1]⁺.

### Synthesis of compound 33-7

At room temperature, compound **33-6** (6.80 g, 9.56 mmol) was dissolved in dichloromethane (10 mL), followed by the addition of trifluoroacetic acid (30 mL). The reaction mixture was stirred at room temperature for 1 hour. After the reaction was complete, the reaction mixture was concentrated under reduced pressure, and the resulting residue was purified by column chromatography (SiO₂, petroleum ether/ethyl acetate=1/1) to obtain compound **33-7.** MS-ESI: *m*/*z* 355.9 [M+1]⁺. ¹H NMR (400 MHz, CDCl₃) δ 8.63 (d, 1H), 8.31 (d, 1H), 7.58 (d, 1H), 7.33 (d, 1H), 4.83 (s, 2H), 3.96 (s, 3H).

### Synthesis of compound 33-8

At room temperature and in a nitrogen atmosphere, compound **33-7** (3.70 g, 10.10 mmol) was dissolved in tetrahydrofuran (100 mL). Triphenylphosphine (3.45 g, 13.15 mmol) was added, and the mixture was stirred at 0°C for 30 minutes. Diisopropyl azodicarboxylate (2.66 g, 13.15 mmol) was slowly added dropwise, and the reaction mixture was stirred at room temperature for 16 hours. The reaction mixture was diluted with water (30 mL), and extracted with ethyl acetate (50 mL×3). The organic phases were combined, washed with water (80 mL) and saturated aqueous sodium chloride solution (50 mL) successively, dried over anhydrous sodium sulfate and filtered. The filtrate was concentrated under reduced pressure, and the resulting residue was purified by column chromatography (SiO₂, petroleum ether/ethyl acetate=5/1) to obtain compound **33-8.** MS-ESI: *m*/*z* 338.0 [M+1]⁺. ¹H NMR (400 MHz, CDCl₃) δ 8.66 (s, 1H), 7.93 (d, 1H), 7.63 (s, 1H), 7.51 (d, 1H), 5.22 (s, 2H), 3.94 (s, 3H).

### Synthesis of compound 33-9

At room temperature, compound **33-8** (2.60 g, 7.68 mmol) and cuprous cyanide (2.06 g, 23.00 mmol) were dissolved in *N*-methylpyrrolidone (20 mL), and the reaction mixture was heated to 135°C and stirred for 16 hours. The reaction mixture was cooled to room temperature, diluted with water (50 mL), and extracted with ethyl acetate (40 mL×3). The organic phases were combined, washed with water (30 mL) and saturated aqueous sodium chloride solution (30 mL) successively, dried over anhydrous sodium sulfate and filtered. The filtrate was concentrated under reduced pressure, and the resulting residue was purified by column chromatography (SiO₂, dichloromethane) to obtain compound **33-9.** MS-ESI: *m*/*z* 285.1 [M+1]⁺. ¹H NMR (400 MHz, DMSO-*d*₆) δ 9.06 (d, 1H), 8.31-8.27 (m, 1H), 7.95 (d, 1H), 7.45 (d, 1H), 5.40 (s, 2H), 3.87 (s, 3H).

### Synthesis of compound 33-10

It was prepared with reference to the synthesis of compound **14-A3** with similar conditions except that the temperature was changed to room temperature. MS-ESI: *m*/*z* 257.1 [M+1]⁺. ¹H NMR (400 MHz, DMSO-*d₆*) δ 8.99 (d, 1H), 8.22 (d, 1H), 7.77 (d, 1H), 7.11 (d, 1H), 5.45 (t, 1H), 5.33 (s, 2H), 4.57 (d, 2H).

### Synthesis of compound 33-11

It was prepared with reference to the synthesis of compound **18-7** with similar conditions except that the temperature was changed to 0°C. MS-ESI: *m*/*z* 319.0 [M+1]⁺.

### Synthesis of compound 33-12

At room temperature, *N*-(diphenylmethylene)aminoacetonitrile (221 mg, 1.00 mmol) was dissolved in tetrahydrofuran (3 mL), followed by slowly addition of sodium hydride (20 mg, 0.50 mmol, 60%) at 0°C. After stirring at 0°C for 30 minutes, compound **33-11** (90 mg, 0.25 mmol) was added, and the reaction mixture was stirred at room temperature for 1 hour. 1M hydrochloric acid was added to adjust the pH to 2, and the reaction mixture was stirred at room temperature for 15 hours. The reaction mixture was diluted with 1M hydrochloric acid (10 mL), and washed with ethyl acetate (20 mL×3). The aqueous phase was adjusted to pH=8 with saturated aqueous sodium bicarbonate, and extracted with ethyl acetate (30 mL×3). The organic phases were combined, washed with water (20 mL) and saturated aqueous sodium chloride (20 mL) successively, dried over anhydrous sodium sulfate and filtered. The filtrate was concentrated under reduced pressure to obtain the crude compound **33-12.** MS-ESI: *m*/*z* 295.0 [M+1]⁺.

### Synthesis of compound 33-13

It was prepared with reference to the synthesis of compound **14-A7** with similar conditions except that *N*,*N*,*N'*,*N'*-tetramethyl-O-(7-azabenzotriazol-1-yl)uronium hexafluorophosphate was replaced with O-benzotriazole-*N,N,N',N'*-tetramethyluronium hexafluorophosphate. MS-ESI: *m*/*z* 422.1 [M-100+1]⁺.

### Synthesis of compound 33

It was prepared with reference to the synthesis of compound **14.** MS-ESI: *m*/*z* 422.1 [M+1]⁺. ¹H NMR (400 MHz, DMSO-*d*₆) δ 9.00 (d, 1H), 8.67 (dd, 1H), 8.22 (s, 1H), 7.80 (dd, 1H), 7.07 (d, 1H), 5.39-5.27 (m, 2H), 5.12-4.96 (m, 1H), 4.00-3.90 (m, 1H), 3.89-3.81 (m, 1H), 3.77-3.66 (m, 1H), 3.27-3.17 (m, 2H), 3.14-2.98 (m, 1H), 2.84-2.66 (m, 2H), 2.64-2.54 (m, 1H), 1.82-1.63 (m, 2H).

### Example 34

### 4-Amino-N-(1-cyano-2-(9-(pyrrolidin-3-yl)-6H-benzo[c]chromen-3-yl)ethyl)tetrahydro-2H-pyran-4-carboxamide

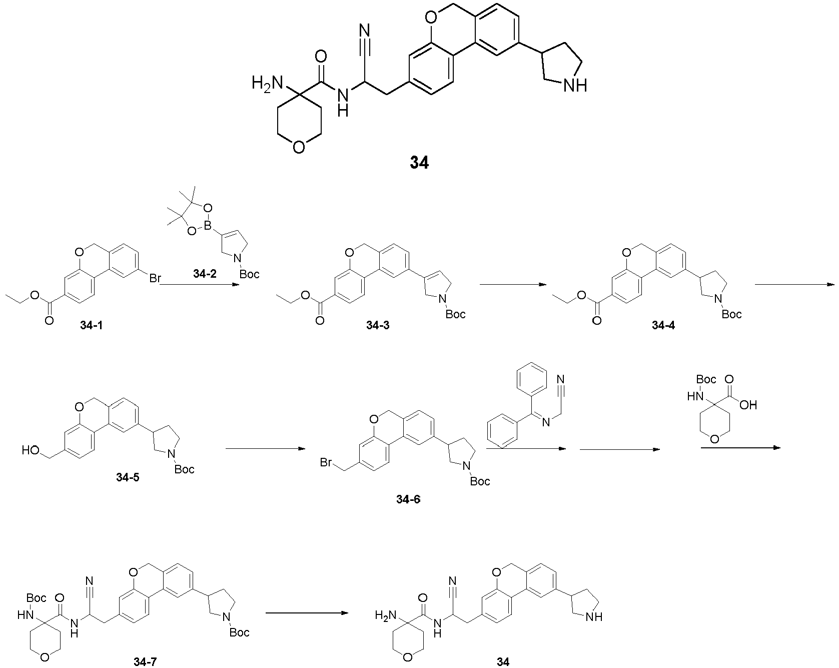

### Synthesis of compound 34-3

At room temperature and in a nitrogen atmosphere, compound **34-1** (900 mg, 3.05 mmol) and compound **34-2** (957 mg, 3.24 mmol) were dissolved in a mixed solvent of toluene (50 mL) and water (5 mL), followed by the addition of [1,1'-bis(diphenylphosphino)ferrocene]palladium(II) dichloride dichloromethane complex (100 mg, 0.12 mmol) and potassium acetate (1.00 g, 10.19 mmol). The reaction mixture was heated to 100°C and stirred for 12 hours. The reaction mixture was cooled to room temperature, diluted with saturated ammonium chloride solution (40 mL), and extracted with ethyl acetate (25 mL×3). The organic phases were combined, washed with water (10 mL×3), dried over anhydrous magnesium sulfate and filtered. The filtrate was concentrated under reduced pressure, and the resulting residue was purified by column chromatography (SiO₂, petroleum ether/ethyl acetate=10/1) to obtain compound **34-3.** ¹H NMR (300 MHz, CDCl₃) δ 7.86-7.69 (m, 4H),7.43-7.37 (m, 1H), 7.21 (d, 1H), 6.27 (d, 1H), 5.20 (s, 2H), 4.66-4.52 (m, 2H), 4.50-4.32 (m, 4H), 1.59-1.50 (m, 9H), 1.45 (t, 3H).

### Synthesis of compound 34-4

At room temperature, compound **34-3** (350 mg, 0.830 mmol) and palladium on carbon (100 mg, 10%) were dissolved in methanol (20 mL), and the reaction mixture was heated to 50°C in a hydrogen atmosphere (balloon) and stirred for 4 hours. After the reaction was complete, the reaction mixture was cooled to room temperature and filtered. The filtrate was concentrated under reduced pressure to obtain the crude compound **34-4,** which was directly used in the next step. ¹H NMR (300 MHz, CDCl₃) δ 7.85-7.74 (m, 2H), 7.66 (d, 2H), 7.27-7.16 (m, 2H), 5.16 (s, 2H), 4.41 (q, 2H), 3.97-3.80 (m, 1H), 3.76-3.58 (m,1H), 3.57-3.25 (m, 3H), 2.41-2.25 (m, 1H), 2.14-1.98 (m, 1H), 1.52 (s, 9H), 1.44(t, 3H).

### Synthesis of compound 34-5

It was prepared with reference to the synthesis of compound **14-A3** with similar conditions except that the temperature was changed from 55°C to room temperature. ¹H NMR (300 MHz, CDCl₃) δ 7.76-7.69 (m, 1H),7.58 (s, 1H), 7.24-7.01 (m, 4H), 5.18 (s, 2H), 4.52 (s, 2H), 4.00-3.78 (m, 1H), 3.75-3.55 (m,1H), 3.53-3.28 (m, 3H), 2.41-2.25 (m, 1H), 2.17-2.00 (m, 1H), 1.45 (s, 9H).

### Synthesis of compound 34-6

It was prepared with reference to the synthesis of compound **18-7.** ¹H NMR (400 MHz, CDCl₃) δ 7.91-7.83 (m, 1H), 7.82-7.74 (m, 1H), 7.67-7.51 (m, 2H), 7.23-7.10 (m, 1H), 7.08-6.96 (m, 1H), 5.15 (s, 2H), 3.97-3.81 (m, 1H), 3.79-3.59 (m, 1H), 3.56-3.31 (m, 3H), 3.07 (d, 2H), 2.42-2.26 (m, 1H), 2.14-2.01 (m, 1H), 1.54 (s, 9H).

### Synthesis of compound 34-7

It was prepared with reference to the synthesis of compounds **14-A5, 14-A6, 14-A7.** MS-ESI: *m*/*z* 491.2 [M-100-56+1]⁺.

### Synthesis of compound 34

It was prepared with reference to the synthesis of compounds **14.** MS-ESI: *m*/*z* 447.3 [M+1]⁺. ¹H NMR (400 MHz, DMSO-*d6*) δ 7.85 (d, 1H),7.73 (s, 1H), 7.25-7.18 (m, 2H), 6.99 (d, 1H), 6.91 (s, 1H), 5.07 (s, 2H), 4.98 (t, 1H), 3.78-3.69 (m, 1H), 3.64-3.55 (m, 3H), 3.54-3.40 (m, 3H), 3.30-3.20 (m, 2H), 3.15-3.10 (m, 2H), 2.27-2.17 (m, 1H), 2.08-1.97 (m, 1H), 1.94-1.83 (m, 1H), 1.78-1.68 (m, 1H), 1.25-1.08 (m, 2H).

### Example 35

### (2S)-N-(2-(8-(Azetidin-1-ylsulfonyl)-2-fluoro-6H-benzo[c]chromen-3-yl)-1-cyanoethyl)-1,4-oxazepane-2-carboxamide

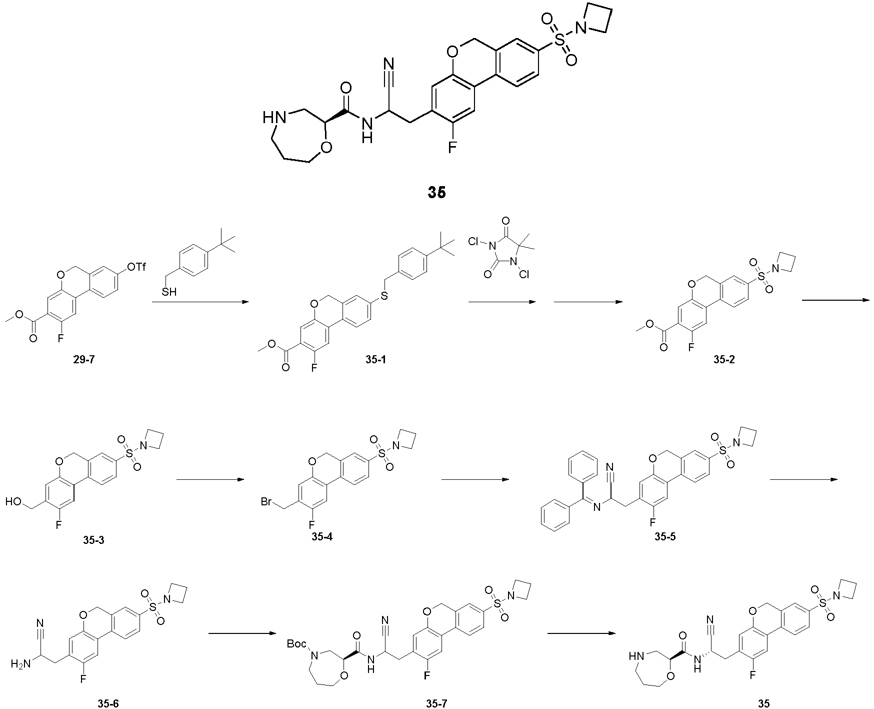

### Synthesis of compound 35-1

It was prepared with reference to the synthesis of compound **29-8** with similar conditions except that the reaction temperature was changed from 70°C to 80°C. MS-ESI: *m*/*z* 437.1 [M+1]⁺.

### Synthesis of compound 35-2

At room temperature, compound **35-1** (300 mg, 0.69 mmol) and acetic acid (0.5 mL) were dissolved in tetrahydrofuran (5 mL), followed by the addition of water (0.5 mL) and 1,3-dichloro-5,5-dimethylhydantoin (300 mg, 1.52 mmol) at 0°C. The reaction mixture was stirred at room temperature for 5 minutes. After the reaction was complete, the reaction mixture was poured into water (10 mL), and extracted with ethyl acetate (10 mL×3). The organic phases were combined, washed with saturated brine (30 mL), dried over anhydrous sodium sulfate and filtered. The filtrate was concentrated under reduced pressure to obtain the crude intermediate, which was dissolved in dichloromethane (5 mL). Azetidine (50 mg, 0.88 mmol) and triethylamine (170 mg, 1.68 mmol) were added, and the reaction mixture was stirred at room temperature for 2 hours. After the reaction was complete, the reaction solution was poured into water (10 mL), and extracted with dichloromethane (10 mL×3). The organic phases were combined, washed with saturated brine (30 mL), dried over anhydrous sodium sulfate and filtered. The filtrate was concentrated under reduced pressure, and the resulting residue was purified by column chromatography (SiO₂, dichloromethane/methanol=24/1) to obtain compound **35-2.** MS-ESI: *m*/*z* 378.2 [M+1]⁺.

### Synthesis of compound 35-3

It was prepared with reference to the synthesis of compound **12-4** with similar conditions except that the temperature was changed from 0°C to room temperature. MS-ESI: *m*/*z* 350.2 [M+1]⁺.

### Synthesis of compound 35-4

It was prepared with reference to the synthesis of compound **14-A4.** ¹H NMR (400 MHz, CDCl₃) δ 7.88 (d, 1H), 7.76-7.67 (m, 2H), 7.43 (d, 1H), 7.06 (d, 1H), 5.15 (s, 2H), 4.49 (s, 2H), 3.48-3.41 (m, 2H), 3.23-3.14 (m, 2H), 2.12-2.05 (m, 2H).

### Synthesis of compound 35-5

*N*-(diphenylmethylene)aminoacetonitrile (90 mg, 0.49 mmol) was dissolved in tetrahydrofuran (5 mL). Sodium hydroxide (45 mg, 1.13 mmol) and water (2 mL) were added, and the reaction mixture was stirred at room temperature for 10 minutes. Compound **35-4** (150 mg, 0.36 mmol) was added, and the reaction mixture was stirred at room temperature for 18 hours. The reaction mixture was poured into water (10 mL), and extracted with ethyl acetate (10 mL×3). The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=3/1) to obtain compound **35-5.** MS-ESI: *m*/*z* 552.4 [M+1]⁺.

### Synthesis of compound 35-6

It was prepared with reference to the synthesis of compound **14-A6.**

### Synthesis of compound 35-7

It was prepared with reference to the synthesis of compound **14-A7** with similar conditions except that *N,N*-diisopropylethylamine was replaced with triethylamine. MS-ESI: *m*/*z* 515.4 [M-100+1]⁺.

### Synthesis of compound 35

It was prepared with reference to the synthesis of compound **14.** MS-ESI: *m*/*z* 515.2 [M+1]⁺. ¹H NMR (400 MHz, DMSO-*d*₆) δ 8.72-8.65 (m, 1H), 8.17-8.11 (m, 1H), 7.93-7.86 (m, 1H), 7.81-7.76 (m, 2H), 7.05 (d, 1H), 5.33-5.20 (m, 2H), 5.12-4.94 (m, 1H), 4.03-3.81 (m, 2H), 3.78-3.67 (m, 5H), 3.30-3.00 (m, 3H), 2.86-2.51 (m, 3H), 2.06-1.96 (m, 2H), 1.83-1.65 (m, 2H).

### Example 36

### 4-Amino-N-(1-cyano-2-(9-fluoro-6,7-dihydrodibenzo[b,d]oxepin-3-yl)ethyl)tetrahydro-2H-pyran-4-carboxamide

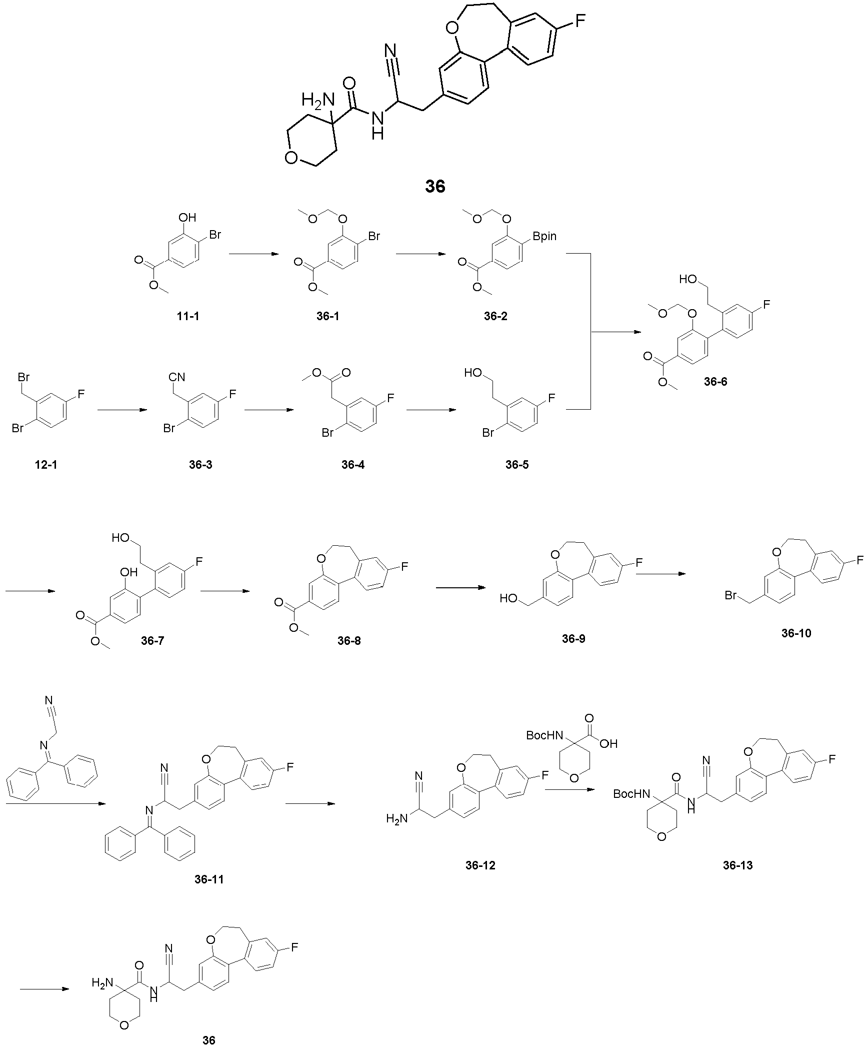

### Synthesis of compound 36-1

At room temperature, compound **11-1** (3.00 g, 12.99 mmol) was dissolved in tetrahydrofuran (25 mL). Sodium hydride (1.04 g, 25.97 mmol, 60%) was added at 0°C, and the reaction mixture was stirred at 0°C for 30 minutes. Bromomethyl methyl ether (3.25 g, 25.97 mmol) was added dropwise, and the reaction mixture was stirred at room temperature for 12 hours. After the reaction was complete, water (100 mL) was added to quench the reaction, and the resulting solution was extracted with ethyl acetate (100 mL×2). The organic phases were combined, washed with saturated brine (100 mL), dried over anhydrous sodium sulfate and filtered. The filtrate was concentrated under reduced pressure to remove the solvent, and the resulting residue was purified by column chromatography (SiO₂, petroleum ether/ethyl acetate=30/1) to obtain compound **36-1.** MS-ESI: *m*/*z* 274.9 [M+1]⁺.

### Synthesis of compound 36-2

At room temperature and in a nitrogen atmosphere, compound **36-1** (5.40 g, 19.63 mmol), bis(pinacolato)diboron (6.98 g, 27.48 mmol), potassium acetate (3.85 g, 39.26 mmol) and [1,1'-bis(diphenylphosphino)ferrocene]palladium(II) dichloride (1.44 g, 1.96 mmol) were dissolved in dioxane (50 mL). The reaction mixture was heated to 90°C and stirred for 3 hours. After the reaction was complete, the reaction mixture was cooled to room temperature, followed by the addition of water (100 mL), and extracted with ethyl acetate (100 mL×2). The organic phases were combined, washed with saturated brine (100 mL), dried over anhydrous sodium sulfate and filtered. The filtrate was concentrated under reduced pressure to remove the solvent, and the resulting residue was purified by column chromatography (SiO₂, petroleum ether/ethyl acetate=10/1) to obtain compound **36-2.** MS-ESI: *m*/*z* 323.1 [M+1]⁺.

### Synthesis of compound 36-3

At room temperature, compound **12-1** (5.00 g, 18.66 mmol) and sodium cyanide (1.30 g, 27.99 mmol) were dissolved in dimethylsulfoxide (50 mL). The reaction mixture was heated to 35°C and stirred for 3 hours. After the reaction was complete, water (100 mL) was added, and the reaction mixture was extracted with ethyl acetate (100 mL×2). The organic phases were combined, washed with saturated brine (100 mL), dried over anhydrous sodium sulfate and filtered. The filtrate was concentrated under reduced pressure to remove the solvent, and the resulting residue was purified by column chromatography (SiO₂, petroleum ether/ethyl acetate=30/1) to obtain compound **36-3.** ¹H NMR (400 MHz, CDCl₃): *δ* 7.59-7.55 (m, 1H), 7.32-7.26 (m, 1H), 7.00-6.95 (m, 1H), 3.83 (s, 2H).

### Synthesis of compound 36-4

At room temperature, compound **36-3** (3.60 g, 15.98 mmol) was dissolved in a 8 M solution (20 mL) of hydrogen chloride in methanol, and the reaction mixture was heated to 80°C and stirred for 3 hours. After the reaction was complete, the reaction mixture was cooled to room temperature, and concentrated under reduced pressure to remove the solvent. The resulting residue was adjusted to pH=7 with saturated aqueous sodium bicarbonate (50 mL), and extracted with ethyl acetate (50 mL×2). The organic phases were combined, washed with saturated brine (30 mL), dried over anhydrous sodium sulfate and filtered. The filtrate was concentrated under reduced pressure to remove the solvent and obtain the crude compound **36-4.** ¹H NMR (400 MHz, CDCl₃): *δ* 7.54-7.50 (m, 1H), 7.06-7.03 (m, 1H), 6.91-6.86 (m, 1H), 3.77 (s, 2H), 3.73 (s, 3H).

### Synthesis of compound 36-5

At room temperature, compound **36-4** (3 g, 10.93 mmol) was dissolved in tetrahydrofuran (30 mL). Lithium aluminum hydride (0.41 g, 10.93 mmol) was added at 0°C. The reaction solution was stirred at 0°C for 20 minutes. After the reaction was complete, water (100 mL) was added, and the reaction mixture was extracted with ethyl acetate (100 mL×2). The organic phases were combined, washed with saturated brine (100 mL), dried over anhydrous sodium sulfate and filtered. The filtrate was concentrated under reduced pressure to remove the solvent, and the resulting residue was purified by column chromatography (SiO₂, petroleum ether/ethyl acetate=10/1) to obtain compound **36-5.** ¹H NMR (400 MHz, CDCl₃): *δ* 7.57-7.52 (m, 1H), 7.09-7.05 (m, 1H), 6.91-6.85 (m, 1H), 3.93 (t, 2H), 3.04 (t, 2H).

### Synthesis of compound 36-6

At room temperature and in a nitrogen atmosphere, compound **36-5** (2.40 g, 10.41 mmol), compound **36-2** (4.01 g, 11.45 mmol), 1,1-bis(diphenylphosphino)ferrocene palladium(II) dichloride (0.76 g, 1.04 mmol) and potassium carbonate (2.88 g, 20.82 mmol) were dissolved in a mixed solvent of dioxane (40 mL) and water (7 mL). The reaction mixture was heated to 80°C and stirred for 3 hours. After the reaction was complete, the reaction mixture was cooled to room temperature, followed by the addition of water (100 mL), and extracted with ethyl acetate (100 mL×2). The organic phases were combined, washed with saturated brine (100 mL), dried over anhydrous sodium sulfate and filtered. The filtrate was concentrated under reduced pressure to remove the solvent, and the resulting residue was purified by column chromatography (SiO₂, petroleum ether/ethyl acetate=10/1) to obtain **36-6.** ¹H NMR (300 MHz, CDCl₃): *δ* 7.88 (s, 1H), 7.82-7.78 (m, 1H), 7.25 (d, 1H), 7.20-7.10 (m, 2H), 7.08-6.97 (m, 1H), 5.21-5.13 (m, 2H), 3.99 (s, 3H), 3.75-3.67 (m, 2H), 3.38 (s, 3H), 2.79-2.74 (m, 2H).

### Synthesis of compound 36-7

At room temperature, compound **36-6** (3.20 g, 8.61 mmol) was dissolved in a 8 M solution (30 mL) of hydrogen chloride in methanol, and the reaction mixture was stirred at room temperature for 1.5 hours. After the reaction was complete, the reaction mixture was concentrated under reduced pressure. The resulting residue was adjusted to pH=7 with saturated aqueous sodium bicarbonate (50 mL), and extracted with ethyl acetate (50 mL×2). The organic phases were combined, washed with saturated brine, dried over anhydrous sodium sulfate and filtered. The filtrate was concentrated under reduced pressure to obtain the crude compound **36-7,** which was directly used in the next step. MS-ESI: *m*/*z* 289.0 [M-1]⁻.

### Synthesis of compound 36-8

At room temperature, compound **36-7** (2.60 mg, 8.15 mmol) and triphenylphosphine (2.99 g, 11.41 mmol) were dissolved in tetrahydrofuran (150 mL). Diisopropyl azodicarboxylate (2.05 g, 9.781 mmol) was added dropwise, and the reaction mixture was stirred at room temperature for 12 hours. Water (200 mL) was added, and the reaction mixture was extracted with ethyl acetate (200 mL×2), washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=20/1) to obtain compound **36-8.** MS-ESI: *m*/*z* 273.1 [M+1]⁺.

### Synthesis of compound 36-9

It was prepared with reference to the synthesis of compound **12-4.** ¹H NMR (300 MHz, CDCl₃) δ7.44-7.32 (m, 2H), 7.25-7.16 (m, 2H), 7.12-6.98 (m, 2H), 4.73 (s,2H), 4.57 (t, 2H), 2.79 (t, 2H).

### Synthesis of compound 36-10

It was prepared with reference to the synthesis of compound **14-A4.** ¹H NMR (300 MHz, CDCl₃) δ7.47-7.36 (m, 2H), 7.35-7.28 (m, 1H), 7.25-7.20 (m, 1H), 7.17-7.03 (m, 2H), 4.62 (t, 2H), 4.56 (s, 2H), 2.85 (t, 2H).

### Synthesis of compound 36-11

It was prepared with reference to the synthesis of compound **14-A5.** MS-ESI: *m*/*z* 447.1 [M+1]⁺.

### Synthesis of compound 36-12

It was prepared with reference to the synthesis of compound **14-A6.** MS-ESI: *m*/*z* 283.1 [M+1]⁺.

### Synthesis of compound 36-13

It was prepared with reference to the synthesis of compound **14-A7** with similar conditions except that (*S*)-4-(*tert*-butoxycarbonyl)-1,4-oxazepane-2-carboxylic acid was replaced with 4-((tert-butoxycarbonyl)amino)tetrahydro-2*H*-pyran-4-carboxylic acid. MS-ESI: *m*/*z* 410.1 [M-100+1]⁺.

### Synthesis of compound 36

It was prepared with reference to the synthesis of compound **14.** MS-ESI: *m*/*z* 410.3 [M+1]⁺. ¹H NMR (400 MHz, CDCl₃) δ 8.23 (d, 1H), 7.42-7.33 (m, 2H), 7.20-7.12 (m, 1H), 7.11-6.98 (m, 3H), 5.17-5.08 (m, 1H), 4.55 (t, 2H), 3.95-3.82 (m, 2H), 3.65-3.52 (m, 2H), 3.12 (d, 2H), 2.78 (t, 2H), 2.36-2.12 (m, 2H), 1.32-1.15 (m, 2H).

### Example 37

### 4-Amino-N-(1-cyano-2-(9-fluoro-5,7-dihydrodibenzo[c,e]oxepin-3-yl)ethyl)tetrahydro-2H-pyran-4-carboxamide

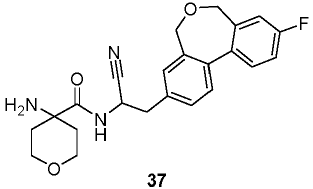

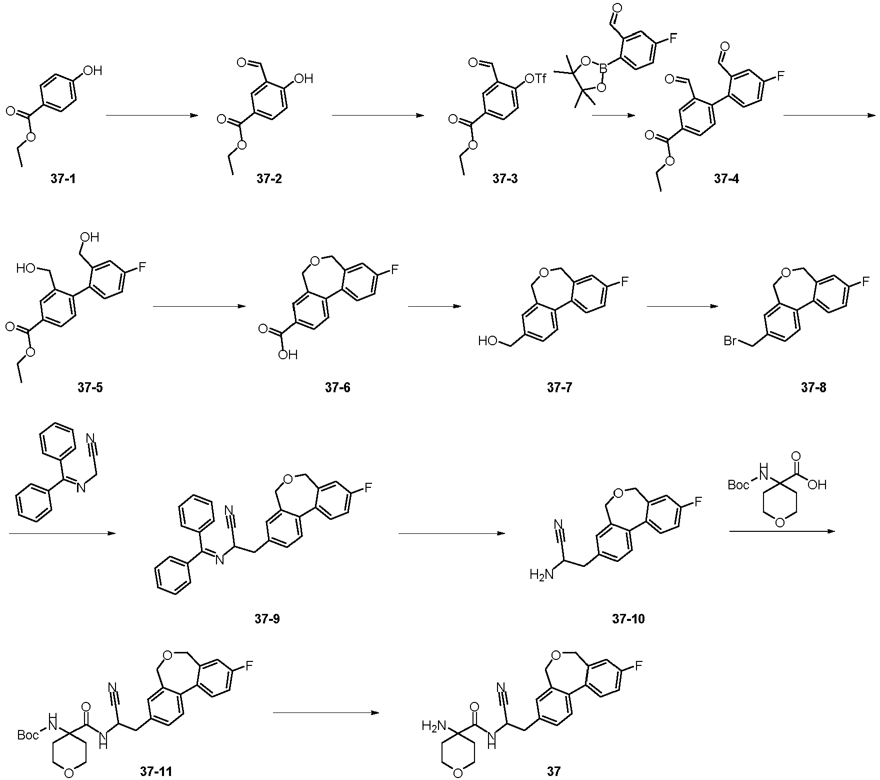

### Synthesis of compound 37-2

At room temperature, ethyl 4-hydroxybenzoate (compound **37-1)** (3.00 g, 18.05 mmol) and triethylamine (15.1 mL, 108.32 mmol) were dissolved in 1,2-dichloroethane (100 mL). Anhydrous magnesium chloride (8.59 g, 90.27 mmol) was added, and the reaction mixture was heated to 40°C and stirred for 1 hour. Paraformaldehyde (6.2 mL, 180.53 mmol) was added, and the reaction mixture was heated to 80°C and stirred for 15 hours. The reaction mixture was cooled to room temperature, followed by the addition of water (100 mL) and concentrated hydrochloric acid (5 mL), and extracted with dichloromethane (50 mL×3). The organic phases were combined, washed with saturated brine (10 mL), dried over anhydrous sodium sulfate and filtered. The filtrate was concentrated under reduced pressure, and the resulting residue was purified by column chromatography (SiO₂, petroleum ether/ethyl acetate=10/1) to obtain the product **37-2** as a white solid. ¹H NMR (300 MHz, CDCl₃) δ 11.39 (s, 1H), 9.96 (s, 1H), 8.35-8.30 (m, 1H), 8.24-8.17 (m, 1H), 7.04 (q, 1H), 4.39 (q, 2H), 1.41 (t, 3H).

### Synthesis of compound 37-3

At room temperature, compound **37-2** (2.00 g, 10.30 mmol) and triethylamine (4.3 mL, 30.9 mmol) were dissolved in dichloromethane (30 mL), followed by the addition of N-phenylbis(trifluoromethanesulfonimide) (4.42 g, 12.36 mmol). The reaction mixture was stirred at room temperature for 3 hours. After the reaction was complete, water (30 mL) was added, and the mixture was extracted with dichloromethane (25 mL×3). The organic phases were combined, washed with saturated brine (10 mL), dried over anhydrous sodium sulfate and filtered. The filtrate was concentrated under reduced pressure, and the resulting residue was purified by column chromatography (SiO₂, petroleum ether/ethyl acetate=5/1) to obtain compound **37-3.** ¹H NMR (300 MHz, CDCl₃) δ 10.29 (s, 1H), 8.68-8.63 (d, 1H), 8.43-8.35 (m, 1H), 7.54-7.47 (m, 1H), 4.44 (q, 2H), 1.43 (t, 3H).

### Synthesis of compound 37-4

It was prepared with reference to the synthesis of compound **36-6.** ¹H NMR (400 MHz, CDCl₃) δ 9.88 (s, 1H), 9.75 (d, 1H), 8.68 (d, 1H), 8.36-8.30 (m, 1H), 7.78-7.71 (m, 1H), 7.45-7.37 (m, 2H), 7.35-7.30 (m, 1H), 4.46 (q, 2H), 1.45 (t, 3H).

### Synthesis of compound 37-5

At room temperature, compound **37-4** (2.20 g, 7.33 mmol) was dissolved in a mixed solvent of tetrahydrofuran (20 mL) and ethanol (10 mL), followed by the addition of sodium borohydride (0.28 g, 7.33 mmol) in batches at 0°C. The reaction mixture was stirred at 0°C for 20 minutes. After the reaction was complete, the reaction mixture was poured into water (20 mL), adjusted to pH=6 with 2 N hydrochloric acid, and extracted with ethyl acetate (25 mL×3). The organic phases were combined, washed with saturated brine (10 mL), dried over anhydrous sodium sulfate and filtered. The filtrate was concentrated under reduced pressure, and the resulting residue was purified by column chromatography (SiO₂, petroleum ether/ethyl acetate=8/1) to obtain compound **37-5.** ¹H NMR (400 MHz, CDCl₃) δ 8.18 (d, 1H), 8.05-7.98 (m, 1H), 7.30-7.26 (m, 1H), 7.25-7.20 (m, 1H), 7.10-7.01 (m, 2H), 4.46-4.37 (m, 4H), 4.33-4.28 (m, 2H), 1.42 (t, 3H).

### Synthesis of compound 37-6

At room temperature, compound **37-5** (2.15 g, 7.07 mmol) was dissolved in phosphoric acid (30 mL), and the reaction mixture was heated to 150°C and stirred for 1 hour. After the reaction was complete, the reaction mixture was cooled to room temperature, followed by the addition of water (100 mL), and extracted with ethyl acetate (25 mL×3). The organic phases were combined, washed with water (10 mL×2) and saturated brine (5 mL), dried over anhydrous sodium sulfate and filtered. The filtrate was concentrated under reduced pressure to obtain the crude compound **37-6.** ¹H NMR (400 MHz, DMSO-*d*₆) δ 13.08 (s, 1H), 8.12-8.06 (m, 2H), 7.76-7.68 (m, 2H), 7.51-7.40 (m, 1H), 4.33 (s, 2H), 4.26 (s, 2H).

### Synthesis of compound 37-7

At 0°C, compound **37-6** (1.60 g, 6.20 mmol) was dissolved in tetrahydrofuran (40 mL). A solution of borane in tetrahydrofuran (9.29 mL, 9.29 mmol, 1.0 mol/L) was added dropwise, and the reaction mixture was heated to 50°C and stirred for 3 hours. After the reaction was complete, the reaction mixture was concentrated under reduced pressure. Water (30 mL) was added to the resulting residue, and the mixture was extracted with ethyl acetate (25 mL×3). The organic phases were combined, washed with saturated brine (10 mL), dried over anhydrous sodium sulfate and filtered. The filtrate was concentrated under reduced pressure, and the resulting residue was purified by column chromatography (SiO₂, petroleum ether/ethyl acetate=3/1) to obtain compound **37-7.** MS-ESI: *m*/*z* 226.9 [M-18+1]⁺.

### Synthesis of compound 37-8

At room temperature, compound **37-7** (1.50 g, 6.14 mmol) was dissolved in chloroform (10 mL). Phosphorus tribromide (0.7 mL, 7.37 mmol) was added dropwise, and the reaction mixture was heated to 50°C and stirred for 30 minutes. After the reaction was complete, the reaction mixture was cooled to room temperature and concentrated under reduced pressure. Water (20 mL) and ethyl acetate (20 mL) were added to the resulting residue, and the mixture was adjusted to pH=7 with saturated aqueous sodium carbonate solution, and extracted with a mixed solvent of petroleum ether and ethyl acetate (5/1, 25 mL×3). The organic phases were combined, washed with saturated brine (5 mL), dried over anhydrous sodium sulfate and filtered. The filtrate was concentrated under reduced pressure to obtain the crude compound **37-8,** which was directly used in the next step. ¹H NMR (300 MHz, CDCl₃) δ 7.57-7.44 (m, 4H), 7.25-7.12 (m, 2H), 4.57 (s, 2H), 4.35 (s, 2H), 4.32 (s, 2H).

### Synthesis of compound 37-9

It was prepared with reference to the synthesis of compound **14-A5** with similar conditions except that the temperature was changed from 35°C to room temperature. MS-ESI: *m*/*z* 447.3 [M+1]⁺.

### Synthesis of compound 37-10

It was prepared with reference to the synthesis of compound **14-A6.**

### Synthesis of compound 37-11

It was prepared with reference to the synthesis of compound **14-A7** with similar conditions except that (*S*)-4-(*tert*-butoxycarbonyl)-1,4-oxazepane-2-carboxylic acid was replaced with 4-((*tert*-butoxycarbonyl)amino)tetrahydro-2H-pyran-4-carboxylic acid. ¹H NMR (300 MHz, CDCl₃) δ 7.54-7.46 (m, 2H), 7.46-7.41 (m, 1H), 7.35-7.27 (m, 1H), 7.24-7.13 (m, 2H), 5.20-5.09 (m, 1H), 4.37-4.29 (m, 4H), 3.84-3.61 (m, 4H), 3.17-3.15 (m, 2H), 2.28-2.17 (m, 2H), 2.05-1.80 (m, 2H), 1.44 (s, 9H).

### Synthesis of compound 37

It was prepared with reference to the synthesis of compound **14.** MS-ESI: *m*/*z* 410.1 [M+1]⁺. ¹H NMR (400 MHz, DMSO-*d*₆) δ 7.65-7.60 (m, 1H), 7.56-7.52 (m, 1H), 7.49-7.44 (m, 1H), 7.44-7.34 (m, 3H), 5.08-5.01 (m, 1H), 4.27-4.16 (m, 4H), 3.66-3.52 (m, 3H), 3.49-3.40 (m, 1H), 3.26-3.20 (m, 2H), 1.93-1.83 (m, 1H), 1.76-1.66 (m, 1H), 1.22-1.08 (m, 2H).

### Example 38A and Example 38B

### 38A: (S)-N-((S)-1-Cyano-2-(2,8-difluoro-9-(oxetan-3-yl)-6H-benzo[c]chromen-3-yl)ethyl)-1,4-oxazepane-2-carboxamide

### 38B: (S)-N-((R)-1-Cyano-2-(2,8-difluoro-9-(oxetan-3-yl)-6H-benzo[c]chromen-3-yl)ethyl)-1,4-oxazepane-2-carboxamide

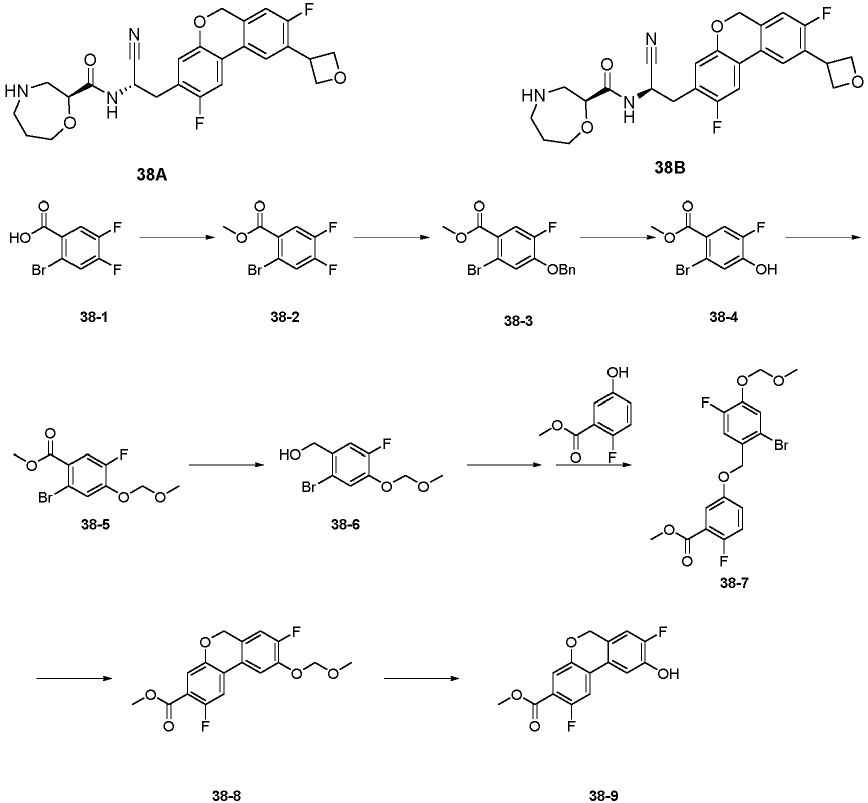

### Synthesis of compound 38-2

At room temperature, 2-bromo-4,5-difluorobenzoic acid (compound **38-1**) (15 g, 63.29 mmol) was dissolved in methanol (150 mL), followed by the addition of concentrated sulfuric acid (1.24 g, 12.66 mmol). The reaction mixture was heated to 80°C and stirred for 12 hours. The reaction mixture was cooled to room temperature and concentrated under reduced pressure. Water (100 mL) was added to the resulting residue, and the mixture was extracted with ethyl acetate (100 mL×2). The organic phases were combined, washed with saturated brine (100 mL), dried over anhydrous sodium sulfate and filtered. The filtrate was concentrated under reduced pressure to obtain compound **38-2.** ¹H NMR (300 MHz, CDCl₃) δ 7.81-7.74 (m, 1H), 7.58-7.52 (m, 1H), 3.97 (s, 3H).

### Synthesis of compound 38-3

At room temperature, benzyl alcohol (6.82 mL, 63.10 mmol) was dissolved in tetrahydrofuran (100 mL), followed by the addition of sodium hydride (2.52 g, 63.10 mmol, 60%) in batches. The reaction mixture was stirred at room temperature for 10 minutes and then at 80°C for 2 hours. At 0°C, a solution of compound **38-2** (16 g, 57.36 mmol) in tetrahydrofuran (100 mL) was added, and the reaction mixture was stirred at room temperature for 3 hours. After the reaction was complete, the reaction mixture was poured into water (300 mL), and extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, ethyl acetate/petroleum ether=9/1) to obtain compound **38-3.** ¹H NMR (300 MHz, CDCl₃) δ 7.77-7.70 (m, 1H), 7.49-7.38 (m, 5H), 7.36-7.30 (m, 1H), 5.22 (s, 2H), 3.95 (s, 3H).

### Synthesis of compound 38-4

At room temperature, compound **38-3** (17 g, 45.11 mmol) was dissolved in dichloromethane (50 mL). At 0°C, a solution of boron tribromide in dichloromethane (49.62 mL, 49.62 mmol, 1.0 M) was added dropwise, and the reaction mixture was stirred at room temperature for 1 hour. After the reaction was complete, the reaction mixture was poured into water (200 mL), and extracted with dichloromethane (200 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, ethyl acetate/petroleum ether=5/1) to obtain compound **38-4.** MS-ESI: *m*/*z* 246.9 [M-1]⁻.

### Synthesis of compound 38-5

At room temperature, compound **38-4** (19 g, 68.67 mmol) was dissolved in tetrahydrofuran (200 mL). Sodium hydride (3.57 g, 89.27 mmol, 60%) was added in batches, and the reaction mixture was stirred at room temperature for 1 hour. At 0°C, chloromethyl methyl ether (11.16 g, 89.27 mmol) was added dropwise, and the reaction mixture was stirred at room temperature for 1 hour. After the reaction was complete, water (300 mL) was added, and the reaction mixture was extracted with ethyl acetate (100 mL×2), washed with saturated brine (200 mL), dried over anhydrous sodium sulfate and filtered. The filtrate was concentrated under reduced pressure, and the resulting residue was purified by column chromatography (SiO₂, ethyl acetate/petroleum ether=10/1) to obtain compound **38-5.** ¹H NMR (300 MHz, CDCl₃) δ 7.72 (d, 1H), 7.54 (d, 1H), 5.31 (s, 2H), 3.95 (s, 3H), 3.57 (s, 3H).

### Synthesis of compound 38-6

At room temperature, compound **38-5** (17 g, 52.20 mmol) was dissolved in a mixed solvent of tetrahydrofuran (180 mL) and methanol (45 mL), followed by the addition of lithium borohydride (4.55 g, 208.81 mmol) in batches. The reaction mixture was heated to 55°C and stirred for 3 hours. After the reaction was complete, the reaction mixture was cooled to room temperature, followed by the addition of water (400 mL), and extracted with ethyl acetate (200 mL×2). The organic phases were combined, washed with saturated brine (200 mL), dried over anhydrous sodium sulfate and filtered. The filtrate was concentrated under reduced pressure, and the resulting residue was purified by column chromatography (SiO₂, ethyl acetate/petroleum ether=3/1) to obtain compound **38-6.** ¹H NMR (300 MHz, DMSO-*d*₆) δ 7.50 (d, 1H), 7.36 (d, 1H), 5.55-5.51 (m, 1H), 5.29 (s, 2H), 4.48-4.43 (m, 2H), 3.44 (s, 3H).

### Synthesis of compound 38-7

At room temperature, compound **6** (13 g, 49.04 mmol) was dissolved in dichloromethane (130 mL), followed by the addition of triethylamine (10.2 mL, 73.56 mmol). At 0°C, methanesulfonyl chloride (6.74 g, 58.85 mmol) was added dropwise, and the reaction mixture was stirred at room temperature for 1 hour. After the reaction was complete, water (100 mL) was added, and the reaction mixture was extracted with dichloromethane (100 mL×3). The organic phases were combined, washed with saturated brine (250 mL×2), dried over anhydrous sodium sulfate and filtered. The filtrate was concentrated under reduced pressure to obtain the crude intermediate, which was directly used in the next step.

At room temperature, methyl 2-fluoro-5-hydroxybenzoate (14.72 g, 86.55 mmol) was dissolved in *N,N*-dimethylformamide (165 mL). At 0°C, sodium hydride (1.50 g, 62.51 mmol, 60%) was slowly added, and the reaction mixture was stirred at room temperature for half an hour. A solution of the above crude intermediate (16.5 g, 48.08 mmol) in *N,N-*dimethylformamide (30 mL) was added dropwise, and the reaction mixture was stirred at room temperature for 1.5 hours. After the reaction was complete, water (500 mL) was added, and the mixture was extracted with ethyl acetate (250 mL×3). The organic phases were combined, washed with saturated brine (250 mL×2), dried over anhydrous sodium sulfate and filtered. The filtrate was concentrated under reduced pressure, and the resulting residue was triturated in a mixed solvent of petroleum ether and ethyl acetate (10/1) to obtain compound **38-7.** ¹H NMR (400 MHz, CDCl₃): δ 7.51-7.43 (m, 2H), 7.29 (d, 1H), 7.15-7.07 (m, 2H), 5.21 (s, 2H), 5.03 (s, 2H), 3.94 (s, 3H), 3.53 (s, 3H).

### Synthesis of compound 38-8

At room temperature and in a nitrogen atmosphere, compound **38-7** (15.50 g, 37.15 mmol), potassium carbonate (10.27 g, 74.31 mmol), palladium acetate (0.83 g, 3.70 mmol) and tricyclohexylphosphine tetrafluoroborate (1.25 g, 3.72 mmol) were dissolved in *N,N-*dimethylformamide (160 mL). The reaction mixture was heated to 115°C and stirred for 2 hours. After the reaction was complete, water (600 mL) was added, and the reaction mixture was extracted with ethyl acetate (100 mL×3). The organic phases were combined, washed with saturated brine (500 mL), dried over anhydrous sodium sulfate and filtered. The filtrate was concentrated under reduced pressure, and the resulting residue was purified by column chromatography (SiO₂, ethyl acetate/petroleum ether=5/1) to obtain compound **38-8.** ¹H NMR (300 MHz, CDCl₃): δ 7.56-7.44 (m, 2H), 7.37 (d, 1H), 6.94 (d, 1H), 5.28 (s, 2H), 5.04 (s, 2H), 3.93 (s, 3H), 3.58 (s, 3H).

### Synthesis of compound 38-9

At room temperature, compound **38-8** (7.50 g, 22.30 mmol) and hydrochloric acid (20 mL) were dissolved in methanol (70 mL), and the reaction mixture was stirred at room temperature for 2 hours. After the reaction was complete, the reaction mixture was concentrated under reduced pressure, and the resulting residue was triturated in a mixed solvent of petroleum ether and ethyl acetate (2/1) to obtain compound **38-9.** MS-ESI: *m*/*z* 293.2 [M+1]⁺. ¹H NMR (400 MHz, DMSO-*d6*): δ 10.13 (s, 1H), 7.70 (dd, 1H), 7.44 (d, 1H), 7.36 (dd, 1H), 7.18 (d, 1H), 5.06 (s, 2H), 3.85 (s, 3H).

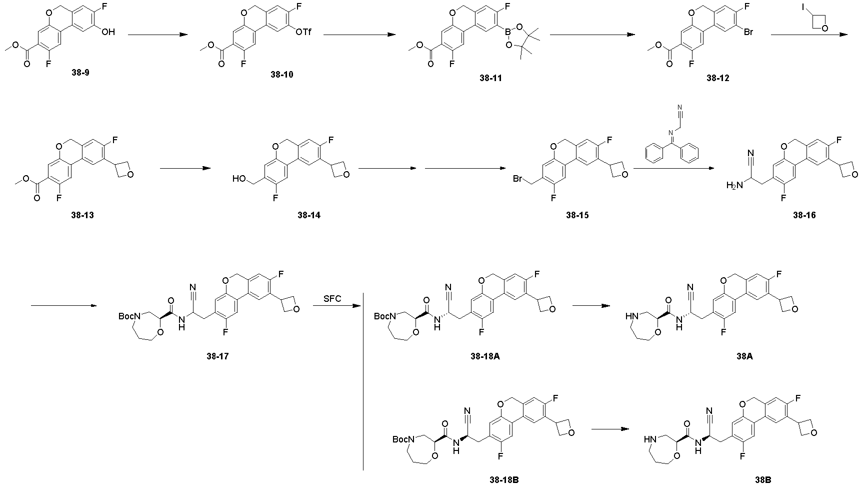

### Synthesis of compound 38-10

At room temperature, methyl 2,8-difluoro-9-hydroxy-6*H*-benzo[c]chromene-3-carboxylate (compound **38-9)** (5.30 g, 18.10 mmol) and pyridine (5.73 g, 72.50 mmol) were dissolved in dichloromethane (30 mL). At 0°C, trifluoromethanesulfonic anhydride (7.67 g, 27.18 mmol) was added dropwise, and the reaction mixture was stirred at 0°C for 2 hours. After the reaction was complete, the reaction mixture was diluted with water (80 mL), and extracted with ethyl acetate (80 mL×3). The organic phases were combined, dried over anhydrous sodium sulfate and filtered. The filtrate was concentrated under reduced pressure, and the resulting residue was purified by column chromatography (SiO₂, ethyl acetate/petroleum ether=1/1) to obtain compound **38-10.** MS-ESI: *m*/*z* 425.0 [M+1]⁺. ¹H NMR (400 MHz, CDCl₃) δ 7.58 (t, 2H), 7.37 (d, 1H), 7.14 (d, 1H), 5.12 (s, 2H), 3.95 (s, 3H).

### Synthesis of compound 38-11

At room temperature and in a nitrogen atmosphere, compound **38-10** (3.40 g, 8.01 mmol), bis(pinacolato)diboron (4.07 g, 16.03 mmol), potassium acetate (2.36 g, 24.04 mmol) and 1,1-bis(diphenylphosphino)ferrocene palladium**(II)** chloride (586 mg, 0.80 mmol) were dissolved in dioxane (20 mL). The reaction mixture was heated to 110°C and stirred for 16 hours. After the reaction was complete, the reaction mixture was cooled to room temperature, diluted with water (50 mL), and extracted with ethyl acetate (100 mL×3). The organic phases were combined, washed with water (80 mL) and saturated aqueous sodium chloride solution (50 mL) successively, dried over anhydrous sodium sulfate and filtered. The filtrate was concentrated under reduced pressure, and the resulting residue was purified by column chromatography (SiO₂, ethyl acetate/petroleum ether=1/1) to obtain the crude product, which was then triturated in methyl *tert-*butyl ether (10 mL) to obtain compound **38-11.** MS-ESI: *m*/*z* 403.2 [M+1]⁺.

### Synthesis of compound 38-12

At room temperature, compound **38-11** (5.70 g, 14.10 mmol) was dissolved in methanol (170 mL), followed by the addition of aqueous copper bromide solution (170 mL, 42.50 mmol, 0.25M). The reaction mixture was heated to 80°C and stirred for 16 hours. The reaction mixture was cooled to room temperature and concentrated under reduced pressure, and the resulting residue was extracted with (80 mL×3). The organic phases were combined, washed with water (50 mL) and saturated aqueous sodium chloride solution (30 mL) successively, dried over anhydrous sodium sulfate and filtered. The filtrate was concentrated under reduced pressure, and the resulting residue was purified by column chromatography (SiO₂, ethyl acetate/petroleum ether=2/1) to obtain compound **38-12.** MS-ESI: *m*/*z* 355.0 [M+1]⁺. ¹H NMR (400 MHz, DMSO-*d*₆) δ 8.35 (d, 1H), 8.00 (d, 1H), 7.39 (m, 2H), 5.15 (s, 2H), 3.85 (s, 3H).

### Synthesis of compound 38-13

At room temperature and in a nitrogen atmosphere, compound **38-12** (2.00 g, 5.18 mmol), 3-iodooxetane (1.24 g, 6.73 mmol), [4,4'-bis(1,1-dimethylethyl)-2,2'-bipyridine-*N*₁,*N*_{1'}]bis[3,5-difluoro-2-[5-(trifluoromethyl)-2-pyridinyl-*N*]phenyl-*C*]iridium(III) hexafluorophosphate (58.3 mg, 0.05 mmol), sodium carbonate (1.09 g, 10.28 mmol), [4,4'-bis(1,1-dimethylethyl)-2,2'-bipyridine]nickel(II) dichloride (10.2 mg, 0.03 mmol) and tris(trimethylsilyl)silane (1.29 g, 5.18 mmol) were dissolved in ethylene glycol dimethyl ether (80 mL). The reaction mixture was stirred at room temperature for 14 hours under the irradiation of a 34 W blue LED lamp. The reaction mixture was diluted with water (50 mL×3), and extracted with ethyl acetate (100 mL×3). The organic phases were combined, washed with water (50 mL) and saturated aqueous sodium chloride solution (50 mL) successively, dried over anhydrous sodium sulfate and filtered. The filtrate was concentrated under reduced pressure, and the resulting residue was purified by column chromatography (SiO₂, ethyl acetate/petroleum ether=1/1) to obtain compound **38-13.** MS-ESI: *m*/*z* 333.1 [M+1]⁺. ¹H NMR (400 MHz, CDCl₃) δ 7.75 (d, 1H), 7.55 (d, 1H), 7.48 (d, 1H), 6.90 (d, 1H), 5.12 (dd, 2H), 5.09 (s, 2H), 4.87 (t, 2H), 4.61-4.51 (m, 1H), 3.95 (s, 3H).

### Synthesis of compound 38-14

At room temperature and in a nitrogen atmosphere, compound **38-13** (1.80 g, 4.71 mmol) was dissolved in tetrahydrofuran (15 mL). At 0°C, a solution of lithium aluminum hydride in tetrahydrofuran (5.2 mL, 5.20 mmol, 1 mol/L) was slowly added dropwise, and the reaction mixture was stirred at room temperature for 20 minutes. After the reaction was complete, the reaction mixture was quenched with saturated aqueous potassium sodium tartrate solution (10 mL) in an ice bath, and extracted with ethyl acetate (100 mL×3). The organic phases were combined, washed with water (80 mL) and saturated aqueous sodium chloride solution (50 mL) successively, dried over anhydrous sodium sulfate and filtered. The filtrate was concentrated under reduced pressure, and the resulting residue was purified by column chromatography (SiO₂, ethyl acetate/petroleum ether=1/1) to obtain compound **38-14.** MS-ESI: *m*/*z* 287.1 [M-18+1]⁺. ¹H NMR (400 MHz, CDCl₃) δ 7.69 (d, 1H), 7.41 (d, 1H), 7.08 (d, 1H), 6.88 (d, 1H), 5.12 (dd, 2H), 5.06 (s, 2H), 4.88 (t, 2H), 4.76 (s, 2H), 4.61-4.52 (m, 1H).

### Synthesis of compound 38-15

At room temperature, compound **38-14** (1.10 g, 3.61 mmol) and diisopropylethylamine (1.87 g, 14.47 mmol) were dissolved in dichloromethane (20 mL), followed by slowly addition of methanesulfonic anhydride (1.26 g, 7.23 mmol) at 0°C. The reaction mixture was stirred at 0°C for 1 hour. After the reaction was complete, water (20 mL) was added, and the reaction mixture was extracted with ethyl acetate (20 mL×3). The organic phases were combined, dried over anhydrous sodium sulfate and filtered. The filtrate was concentrated under reduced pressure to obtain the crude intermediate, which was directly used in the next step.

At room temperature, the above crude intermediate (1.30 g, 3.40 mmol) and sodium bromide (3.50 g, 34.02 mmol) were dissolved in acetone (10 mL), and the reaction mixture was heated to 60°C and stirred for 1 hour. After the reaction was complete, the reaction mixture was cooled to room temperature and filtered. The filtrate was concentrated under reduced pressure, and the resulting residue was purified by column chromatography (SiO₂, ethyl acetate/petroleum ether=5/1) to obtain compound **38-15.** ¹H NMR (400 MHz, CDCl₃) δ 7.70 (d, 1H), 7.42 (d, 1H), 7.02 (d, 1H), 6.88 (d, 1H), 5.12 (dd, 2H), 5.07 (s, 2H), 4.88 (t, 2H), 4.62-4.51 (m, 1H), 4.50 (s, 2H).

### Synthesis of compound 38-16

At room temperature, *N*-(diphenylmethylene)aminoacetonitrile (2.64 g, 12.56 mmol) was dissolved in tetrahydrofuran (50 mL). At 0°C, sodium hydride (263 mg, 6.59 mmol, 60%) was added, the reaction mixture was stirred at 0°C for 30 minutes. Compound **38-15** (1.10 g, 2.99 mmol) was added, and the reaction mixture was stirred at room temperature for 2 hours. After the reaction was complete, 1M hydrochloric acid (20 mL) was slowly added at 0°C to adjust the pH to 2-3. The reaction mixture was diluted with 1 M aqueous hydrochloric acid solution (30 mL), and washed with ethyl acetate (50 mL×3). The aqueous phase was adjusted to pH=8 with solid sodium bicarbonate, and extracted with ethyl acetate (80 mL×3). The organic phases were combined, washed with water (50 mL) and saturated aqueous sodium chloride solution (50 mL) successively, dried over anhydrous sodium sulfate and filtered. The filtrate was concentrated under reduced pressure to obtain the crude compound **38-16,** which was directly used in the next step. MS-ESI: *m*/*z* 343.2 [M+1]⁺. ¹H NMR (400 MHz, DMSO-*d₆*) δ 7.96 (d, 1H), 7.91 (d, 1H), 7.17 (d, 1H), 7.01 (d, 1H), 5.09 (s, 2H), 4.96-4.89 (m, 2H), 4.88-4.82 (m, 2H), 4.59-4.47 (m, 1H), 4.02-3.94 (m, 1H), 2.94 (dd, 2H).

### Synthesis of compound 38-17

At room temperature, the crude compound **38-16** (700 mg, 2.04 mmol), compound **2-6** (551 mg, 2.24 mmol) and *N,N*-diisopropylethylamine (792 mg, 6.13 mmol) were dissolved in *N,N*-dimethylformamide (20 mL), followed by the addition of O-benzotriazole-*N,N,N,N*-tetramethyluronium hexafluorophosphate (930 mg, 2.45 mmol). The reaction mixture was stirred at room temperature for 1 hour. After the reaction was complete, the reaction mixture was diluted with water (20 mL), and extracted with ethyl acetate (50 mL×3). The organic phases were combined, washed with water (20 mL) and saturated aqueous sodium chloride solution (20 mL) successively, dried over anhydrous sodium sulfate and filtered. The filtrate was concentrated under reduced pressure, and the resulting residue was purified by column chromatography (SiO₂, ethyl acetate/petroleum ether=1/2) to obtain compound **38-17.**

### Synthesis of compound 38-18A and compound 38-18B

Compound **38-17** (730 mg, 1.19 mmol) was resolved by preparative SFC (column: DAICEL CHIRALCEL OJ, 250*30 mm, 10 µm; mobile phase: supercritical carbon dioxide, methanol (0.1% ammonia monohydrate); gradient: carbon dioxide phase 65%; flow rate: 65 mL/min; column temperature: room temperature) to obtain compounds **38-18A** and **38-18B** (two diastereomer peaks in total, compound **38-18A** being the second elution peak, compound **38-18B** being the first elution peak).

### Synthesis of compound 38A

It was prepared with reference to the synthesis of compound **14** with similar conditions except that the temperature was changed to 50°C. MS-ESI: *m*/*z* 468.2 [M-1]⁻. ¹H NMR (400 MHz, DMSO-*d*₆) δ 8.69 (d, 1H), 7.98-7.87 (m, 2H), 7.17 (d, 1H), 6.97 (d, 1H), 5.13-4.98 (m, 3H), 4.94-4.88 (m, 2H), 4.84 (t, 2H), 4.57-4.48 (m, 1H), 3.99 (dd, 1H), 3.90-3.81 (m, 1H), 3.77-3.67 (m, 1H), 3.26-3.20 (m, 1H), 3.18-3.12 (m, 1H), 3.02 (dd, 1H), 2.82-2.73 (m, 1H), 2.63-2.53 (m, 2H), 1.81-1.62 (m, 2H).

### Synthesis of compound 38B

It was prepared with reference to the synthesis of compound **14** with similar conditions except that the temperature was changed to 50°C. MS-ESI: *m*/*z* 470.1 [M+1]⁺. ¹H NMR (400 MHz, DMSO-*d*₆) δ 8.65 (d, 1H), 8.01-7.89 (m, 2H), 7.17 (d, 1H), 6.98 (d, 1H), 5.09 (s, 2H), 4.99-4.94 (m, 1H), 4.92 (dd, 2H), 4.84 (t, 2H), 4.57-4.47 (m, 1H), 3.94-3.86 (m, 2H), 3.72-3.68 (m, 1H), 3.26-3.18 (m, 2H), 3.16-3.08 (m, 1H), 2.83-2.77 (m, 1H), 2.73-2.66 (m, 2H), 1.81-1.66 (m, 2H).

### Example 39

### 4-Amino-N-(1-cyano-2-(3-fluoro-6,7-dihydrodibenzo[b,d]oxepin-9-yl)ethyl)tetrahydro-2H-pyran-4-carboxamide

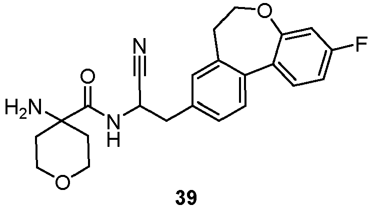

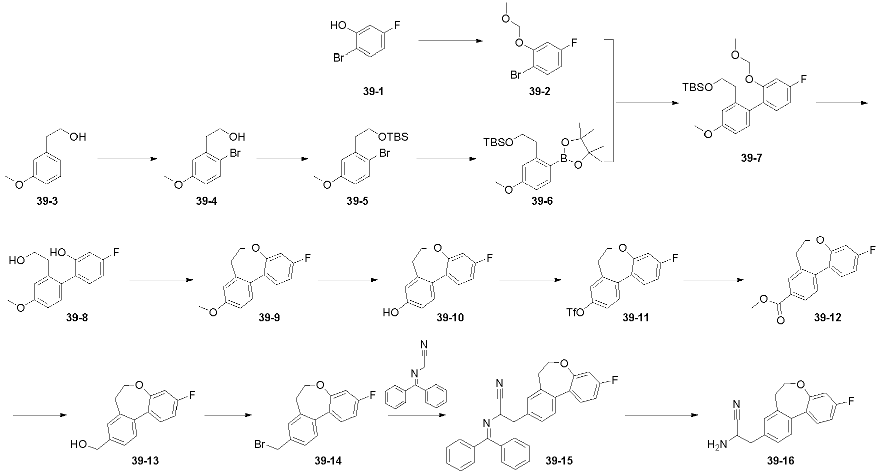

### Synthesis of compound 39-2

At room temperature and in a nitrogen atmosphere, 2-bromo-5-fluorophenol (compound **39-1)** (10.00 g, 52.65 mmol) was dissolved in tetrahydrofuran (50 mL). At 0°C, sodium hydride (3.10 g, 77.50 mmol, 60%) was added, and the reaction mixture was stirred at 0°C for 1 hour. Bromomethyl methyl ether (9.50 g, 76.02 mmol) was added, and the reaction mixture was stirred at room temperature for 2 hours. After the reaction was complete, the reaction mixture was poured into ice water (50 mL), and extracted with ethyl acetate (50 mL×3). The organic phase was washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **39-2.** ¹H NMR (400 MHz, CDCl₃) δ 7.50-7.44 (m, 1H), 6.93 (dd, 1H), 6.68-6.60 (m, 1H), 5.23 (s, 2H), 3.51 (s, 3H).

### Synthesis of compound 39-4

At room temperature, 2-(3-methoxyphenyl)ethan-1-ol (compound **39-3)** (10.00 g, 65.75 mmol) was dissolved in acetonitrile (80 mL), followed by the addition of *N-*bromosuccinimide (11.70 g, 65.74 mmol). The reaction mixture was stirred at room temperature for 2 hours. After the reaction was complete, the reaction mixture was poured into water (100 mL), and extracted with ethyl acetate (50 mL×3). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **39-4.** ¹H NMR (400 MHz, CDCl₃) δ 7.41 (d, 1H), 6.82 (d, 1H), 6.68-6.61 (m, 1H), 3.88-3.78 (m, 2H), 3.76 (s, 3H), 3.00-2.92 (m, 2H), 2.05-1.96 (m, 1H).

### Synthesis of compound 39-5

At room temperature, compound **39-4** (12.50 g, 54.10 mmol) was dissolved in dichloromethane (150 mL), followed by the addition of *tert*-butyldimethylsilyl chloride (10.00 g, 66.40 mmol) and imidazole (6.00 g, 88.10 mmol). The reaction mixture was stirred at room temperature for 2 hours. After the reaction was complete, the reaction solution was poured into water (100 mL), and extracted with dichloromethane (50 mL×3). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=19/1) to obtain compound **39-5.** ¹H NMR (400 MHz, CDCl₃) δ 7.40 (d, 1H), 6.83 (d, 1H), 6.68-6.62 (m, 1H), 3.87-3.80 (m, 2H), 3.78 (s, 3H), 2.98-2.90 (m, 2H), 0.88 (s, 9H), 0.01 (s, 6H).

### Synthesis of compound 39-6

At room temperature and in a nitrogen atmosphere, compound **39-5** (15.00 g, 43.40 mmol) and bis(pinacolato)diboron (15.00 g, 59.07 mmol) were dissolved in 1,4-dioxane (200 mL), followed by the addition of potassium acetate (13.00 g, 132.46 mmol) and [1,1'-bis(diphenylphosphino)ferrocene]palladium(II) dichloride (2.00 g, 2.73 mmol). The reaction mixture was heated to 95°C and stirred for 3 hours. After the reaction was complete, the reaction mixture was cooled to room temperature, poured into water (100 mL), and extracted with ethyl acetate (80 mL×3). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=19/1) to obtain compound **39-6.** ¹H NMR (400 MHz, CDCl₃) δ 7.80-7.73 (m, 1H), 6.84-6.72 (m, 2H), 3.86-3.76 (m, 5H), 3.14 (t, 2H), 1.34 (s, 12H), 0.89 (s, 9H), 0.01 (s, 6H).

### Synthesis of compound 39-7

It was prepared with reference to the synthesis of compound **36-6** with similar conditions except that the temperature was changed from 80°C to 95°C. ¹H NMR (300 MHz, CDCl₃) δ 7.19-7.8 (m, 2H), 7.06-6.99 (m, 1H), 6.97-6.92 (m, 1H), 6.90-6.77 (m, 2H), 5.16-5.08 (m, 2H), 3.90 (s, 3H), 3.70-3.61 (m, 2H), 3.42 (s, 3H), 2.80-2.70 (m, 2H), 0.88 (s, 9H), 0.00 (s, 6H).

### Synthesis of compound 39-8

At room temperature, compound **39-7** (5.80 g, 13.80 mmol) was dissolved in tetrahydrofuran (30 mL), followed by the addition of concentrated hydrochloric acid (5 mL). The reaction mixture was stirred at 40°C for 3 hours. After the reaction was complete, the reaction mixture was cooled to room temperature, poured into water (50 mL) and extracted with ethyl acetate (50 mL×3). The organic phases were combined, washed with saturated brine (80 mL), dried over anhydrous sodium sulfate and filtered. The filtrate was concentrated under reduced pressure, and the resulting residue was purified by column chromatography (SiO₂, petroleum ether/ethyl acetate=2/1) to obtain compound **39-8.** ¹H NMR (400 MHz, CDCl₃) δ 7.13-7.08 (m, 1H), 7.03-6.97 (m, 1H), 6.93-6.88 (m, 1H), 6.87-6.83 (m, 1H), 6.70-6.61 (m, 2H), 3.83 (s, 3H), 3.76-3.71 (m, 2H), 2.76-2.60 (m, 2H).

### Synthesis of compound 39-9

It was prepared with reference to the synthesis of compound **36-9.** ¹H NMR (400 MHz, CDCl₃) δ 7.35-7.28 (m, 2H), 6.97-6.89 (m, 2H), 6.88-6.83 (m, 2H), 4.56 (t, 2H), 3.85 (s, 3H), 2.78 (t, 2H).

### Synthesis of compound 39-10

At room temperature and in a nitrogen atmosphere, compound **39-9** (2.00 g, 8.19 mmol) was dissolved in dichloromethane (20 mL). Boron tribromide (12.0 mL, 12.00 mmol, 1.0 mol/L in dichloromethane) was added dropwise, and the reaction mixture was stirred at room temperature for 2 hours. After the reaction was complete, the reaction mixture was poured into water (30 mL), and extracted with dichloromethane (30 mL×3). The organic phase was washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **39-10.** ¹H NMR (400 MHz, CDCl₃) δ 7.34-7.27 (m, 1H), 7.27-7.24 (m, 1H), 6.96-6.89 (m, 1H), 6.88-6.81 (m, 2H), 6.79-6.75 (m, 1H), 4.97 (s, 1H), 4.56 (t, 2H), 2.76 (t, 2H).

### Synthesis of compound 39-11

At room temperature, compound **39-10** (500 mg, 2.17 mmol) and triethylamine (0.7 mL, 4.94 mmol) were dissolved in dichloromethane (10 mL), followed by the addition of N-phenylbis(trifluoromethanesulfonimide) (900 mg, 2.52 mmol). The reaction mixture was stirred at room temperature for 1 hour. After the reaction was complete, the reaction mixture was poured into water (30 mL), and extracted with dichloromethane (30 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **39-11.** ¹H NMR (400 MHz, CDCl₃) δ 7.47-7.41 (m, 1H), 7.39-7.27 (m, 2H), 7.24-7.18 (m, 1H), 7.02-6.95 (m, 1H), 6.93-6.87 (m, 1H), 4.58 (t, 2H), 2.84 (t, 2H).

### Synthesis of compound 39-12

At room temperature, compound **39-11** (450 mg, 1.24 mmol) and [1,1'-bis(diphenylphosphino)ferrocene]palladium(II) dichloride (200 mg, 0.27 mmol) were dissolved in methanol (10 mL), followed by the addition of triethylamine (0.52 mL, 3.76 mmol). The reaction mixture was heated to 100°C in a carbon monoxide atmosphere (5 MPa) and stirred for 18 hours. The reaction mixture was cooled to room temperature, poured into water (30 mL) and extracted with dichloromethane (30 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **39-12** (220 mg). ¹H NMR (400 MHz, CDCl₃) δ 8.07-8.02 (m, 1H), 7.98-7.94 (m, 1H), 7.49-7.43 (m, 1H), 7.42-7.36 (m, 1H), 7.01-6.94 (m, 1H), 6.92-6.85 (m, 1H), 4.59 (t, 2H), 3.95 (s, 3H), 2.87 (t, 2H).

### Synthesis of compound 39-13

It was prepared with reference to the synthesis of compound **12-4** with similar conditions except that the temperature was changed from 0°C to room temperature. MS-ESI: *m*/*z* 227.1 [M-18+1]⁺.

### Synthesis of compound 39-14

It was prepared with reference to the synthesis of compound **14-A4.** ¹H NMR (400 MHz, CDCl₃) δ 7.43-7.30 (m, 4H), 6.99-6.92 (m, 1H), 6.90-6.84 (m, 1H), 4.57 (t, 2H), 4.54 (s, 2H), 2.81 (t, 2H).

### Synthesis of compound 39-15

It was prepared with reference to the synthesis of compound **14-A5** with similar conditions except that benzyltrimethylammonium chloride was absent. MS-ESI: *m*/*z* 447.3 [M+1]⁺.

### Synthesis of compound 39-16

It was prepared with reference to the synthesis of compound **14-A6.** MS-ESI: *m*/*z* 266.1 [M-17+1]⁺.

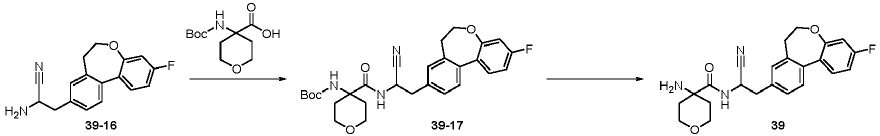

### Synthesis of compound 39-17

At room temperature, 2-amino-3-(3-fluoro-6,7-dihydrodibenzo[*b,d*]oxepin-9-yl)propanenitrile (compound **39-16)** (40 mg, 0.14 mmol) and *4-*((*tert-*butoxycarbonyl)amino)tetrahydro-2*H*-pyran-4-carboxylic acid (40 mg, 0.16 mmol) were dissolved in *N,N*-dimethylformamide (2 mL), followed by the addition of *N,N,N' ,N'-*tetramethyl-O-(7-azabenzotriazol-1-yl)uronium hexafluorophosphate (55 mg, 0.15 mmol) and triethylamine (50 mg, 0.49 mmol). The reaction mixture was heated to 40°C and stirred for 3 hours. After the reaction was complete, the reaction mixture was cooled to room temperature, poured into water (10 mL), and extracted with ethyl acetate (10 mL×3). The organic phases were combined, washed with saturated brine (30 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **39-17.** MS-ESI: *m*/*z* 410.3 [M-100+1]⁺.

### Synthesis of compound 39

It was prepared with reference to the synthesis of compound **14** with similar conditions except that the temperature was changed from 40°C to 50°C. MS-ESI: *m*/*z* 410.2 [M+1]⁺. ¹H NMR (400 MHz, DMSO-*d*₆) δ 7.50-7.42 (m, 1H), 7.39-7.35 (m, 1H), 7.33-7.29 (m, 1H), 7.26 (s, 1H), 7.15-7.08 (m, 1H), 7.03-6.97 (m, 1H), 5.03-4.97 (m, 1H), 4.52-4.45 (m, 2H), 3.68-3.54 (m, 3H), 3.52-3.44 (m, 1H), 3.23-3.12 (m, 2H), 2.78-2.70 (m, 2H), 1.93-1.85 (m, 1H), 1.80-1.70 (m, 1H), 1.24-1.10 (m, 2H).

### Example 40

### (S)-4-Amino-N-(1-cyano-2-(9-fluoro-6-methyl-5-oxo-6,7-dihydro-5H-dibenzo[c,e]azepin-3-yl)ethyl)tetrahydro-2H-pyran-4-carboxamide

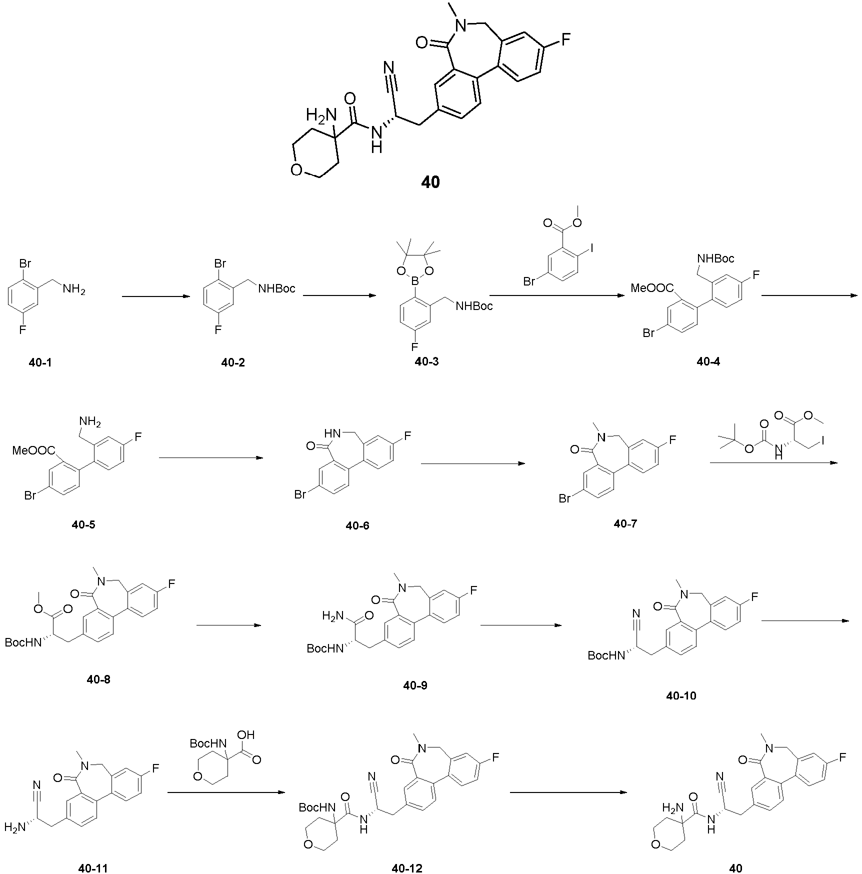

### Synthesis of compound 40-2

At room temperature, (2-bromo-5-fluorophenyl)methanamine (compound **40-1)** (10.00 g, 49.01 mmol) and triethylamine (6.8 mL, 49.01 mmol) were dissolved in dichloromethane (100 mL), followed by the addition of di-*tert*-butyl dicarbonate (16.04 g, 73.52 mmol). The reaction mixture was stirred at room temperature for 12 hours. The reaction mixture was concentrated under reduced pressure, and the resulting residue was purified by column chromatography (SiO₂, petroleum ether/ethyl acetate=1/1) to obtain compound **40-2.** MS-ESI: *m*/*z* 248.1 [M-56+1]⁺.

### Synthesis of compound 40-3

At room temperature and in a nitrogen atmosphere, compound **40-2** (11.00 g, 36.17 mmol), bis(pinacolato)diboron (18.37 g, 72.33 mmol) and potassium acetate (7.10 g, 72.33 mmol) were dissolved in dioxane (50 mL), followed by the addition of [1,1'-bis(diphenylphosphino)ferrocene]palladium(II) dichloride (2.95 g, 3.62 mmol). The reaction mixture was heated to 80°C and stirred for 3 hours. After the reaction was complete, the reaction mixture was cooled to room temperature, poured into saturated aqueous sodium bicarbonate solution (200 mL), and extracted with dichloromethane (200 mL×3). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **40-3.** MS-ESI: *m*/*z* 252.3 [M-100+1]⁺. ¹H NMR (400 MHz, CDCl₃) δ 7.85-7.78 (m, 1H), 7.12-7.07 (m, 1H), 6.98-6.90 (m, 1H), 5.39 (s, 1H), 4.44 (s, 2H), 1.43 (s, 9H), 1.53 (s, 12H).

### Synthesis of compound 40-4

At room temperature and in a nitrogen atmosphere, compound **40-3** (8.00 g, 22.78 mmol), methyl 5-bromo-2-iodobenzoate (8.54 g, 25.06 mmol), sodium carbonate (2.41 g, 22.78 mmol) were dissolved in a mixed solvent of dioxane (80 mL) and water (20 mL), followed by the addition of [1,1'-bis(diphenylphosphino)ferrocene]palladium(II) dichloride (0.93 g, 1.14 mmol). The reaction mixture was heated to 60°C and stirred for 4 hours. After the reaction was complete, the reaction mixture was cooled to room temperature, poured into saturated aqueous sodium bicarbonate solution (200 mL), and extracted with dichloromethane (200 mL×3). The organic phases were combined, washed with saturated brine (200 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **40-4.** MS-ESI: *m*/*z* 340.1 [M-100+1]⁺.

### Synthesis of compound 40-5

At 0°C, compound **40-4** (1.32 g, 3.01 mmol) was dissolved in dichloromethane (20 mL), followed by the addition of trifluoroacetic acid (4 mL). The reaction mixture was stirred at room temperature for 2 hours. After the reaction was complete, the reaction mixture was concentrated under reduced pressure. A saturated aqueous sodium bicarbonate solution (10 mL) was added to the resulting residue, and the mixture was extracted with dichloromethane (100 mL×3). The organic phases were combined, washed with saturated brine (50 mL), dried over anhydrous sodium sulfate and filtered. The filtrate was concentrated under reduced pressure to obtain the crude compound **40-5,** which was directly used in the next step. MS-ESI: *m*/*z* 340.1 [M+1]⁺.

### Synthesis of compound 40-6

At room temperature, the crude compound **40-5** (1.00 g, 2.96 mmol) was dissolved in toluene (20 mL), and the reaction mixture was heated to 120°C and stirred for 18 hours. The reaction mixture was cooled to room temperature and concentrated under reduced pressure. The resulting residue was purified by column chromatography (SiO₂, petroleum ether/ethyl acetate=5/1) to obtain compound **40-6.** MS-ESI: *m*/*z* 306.1 [M+1]⁺.

### Synthesis of compound 40-7

At room temperature, compound **40-6** (800 mg, 2.60 mmol) was dissolved in *N,N-*dimethylformamide (200 mL). At 0°C, sodium hydride (156 mg, 3.89 mmol, 60%) was added in batches, and the reaction mixture was stirred at 0°C for 1 hour. Iodomethane (1.11 g, 7.79 mmol) was added, and the reaction mixture was stirred at 0°C for 2 hours. After the reaction was complete, water (150 mL) was added, and the reaction mixture was extracted with ethyl acetate (50 mL×2). The organic phases were combined, washed with saturated brine (50 mL), dried over anhydrous sodium sulfate and filtered. The filtrate was concentrated under reduced pressure, and the resulting residue was purified by column chromatography (SiO₂, petroleum ether/ethyl acetate=5/1) to obtain compound **40-7.** MS-ESI: *m*/*z* 320.1 [M+1]⁺.

### Synthesis of compound 40-8

It was prepared with reference to the synthesis of compound **1-3** with similar conditions except that the temperature was changed from 60°C to 70°C. MS-ESI: *m*/*z* 443.4 [M+1]⁺.

### Synthesis of compound 40-9

It was prepared with reference to the synthesis of compound **1-4.** MS-ESI: *m*/*z* 428.6 [M+1]⁺.

### Synthesis of compound 40-10

It was prepared with reference to the synthesis of compound **1-5.** MS-ESI: *m*/*z* 410.1 [M+1]⁺.

### Synthesis of compound 40-11

It was prepared with reference to the synthesis of compound **1-6.** MS-ESI: *m*/*z* 310.1 [M+1]⁺.

### Synthesis of compound 40-12

It was prepared with reference to the synthesis of compound **1-7** with similar conditions except that *N*,*N*-dimethylformamide was replaced with dichloromethane. MS-ESI: *m*/*z* 537.2 [M+1]⁺.

### Synthesis of compound 40

It was prepared with reference to the synthesis of compound **1.** MS-ESI: *m*/*z* 437.3 [M+1]⁺. ¹H NMR (400 MHz, DMSO-*d6*) δ 7.73-7.66 (m, 2H), 7.57-7.52 (m, 2H), 7.50-7.43 (m, 1H), 7.38-7.328 (m, 1H), 5.09-4.99 (m, 1H), 4.24-4.07 (m, 2H), 3.67-3.46 (m, 3H), 3.42-3.31 (m, 1H), 3.30-3.24 (m, 2H), 3.06 (s, 3H), 1.92-1.82 (m, 1H), 1.72-1.67 (m, 1H), 1.24-0.98 (m, 2H).

### Example 41

### (S)-N-((S)-1-Cyano-2-(9-cyano-2-fluoro-6-methyl-5-oxo-6,7-dihydro-5H-dibenzo[c,e]azepin-3-yl)ethyl)-1,4-oxazepane-2-carboxamide

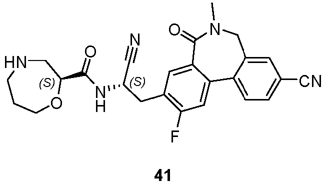

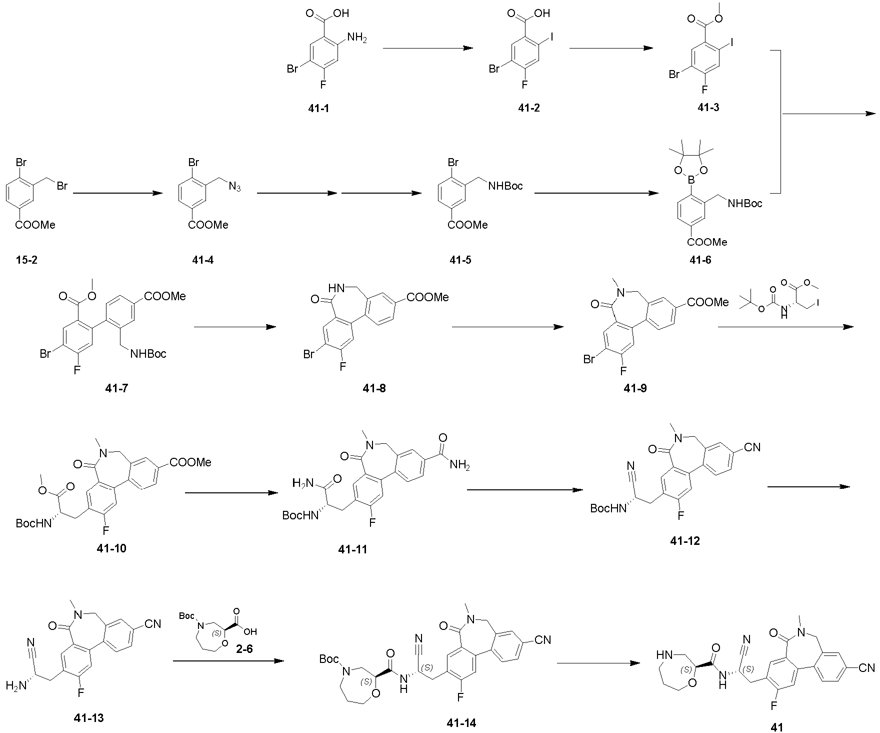

### Synthesis of compound 41-2

At room temperature, 2-amino-5-bromo-4-fluorobenzoic acid (compound **41-1)** (5.00 g, 21.40 mmol) was added to a solution of concentrated hydrochloric acid (17.8 mL, 214.00 mmol) in water (10 mL). At 0°C, a solution of sodium nitrite (1.77 g, 25.65 mmol) in water (10 mL) was added slowly, and the reaction mixture was stirred at 0°C for 30 minutes. A solution of potassium iodide (5.32 g, 32.00 mmol) in water (10 mL) was added slowly, and the reaction mixture was stirred at 0°C for two hours. After the reaction was complete, the reaction mixture was diluted with water (100 mL), and extracted with methyl *tert-*butyl ether (50 mL×2). The organic phases were combined, washed with saturated brine (50 mL), dried over anhydrous sodium sulfate and filtered. The filtrate was concentrated under reduced pressure to obtain the crude compound **41-2,** which was directly used in the next step. ¹H NMR (400 MHz, DMSO-*d*₆) δ 13.61 (br s, 1H), 8.09-8.01 (m, 2H).

### Synthesis of compound 41-3

At room temperature, compound **41-2** (6.50 g, 18.80 mmol) was dissolved in methanol (100 mL), followed by slowly addition of concentrated sulfuric acid (3.77 g, 37.70 mmol). The reaction mixture was heated to 65°C and stirred for 15 hours. After the reaction was complete, the reaction mixture was cooled to room temperature, slowly poured into saturated aqueous sodium bicarbonate solution (300 mL) (pH of the system = 9), and extracted with methyl *tert*-butyl ether (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=3/1) to obtain compound **41-3.** ¹H NMR (400 MHz, CDCl₃) δ 8.09 (d, 1H), 7.76 (d, 1H), 3.94 (s, 3H).

### Synthesis of compound 41-4

At room temperature, methyl 4-bromo-3-(bromomethyl)benzoate (compound **15-2)** (25.00 g, 69.00 mmol) was dissolved in dimethyl sulfoxide (150 mL), followed by the addition of sodium azide (5.39 g, 82.91 mmol) in batches. The reaction mixture was stirred at room temperature for 4 hours. After the reaction was complete, the reaction mixture was diluted with ice water (300 mL) and filtered. The filter cake was washed with ice water (100 mL) to obtain the crude compound **41-4,** which was directly used in the next step. ¹H NMR (400 MHz, CDCl₃) δ 8.00 (d, 1H), 7.79 (dd, 1H), 7.62 (d, 1H), 4.47 (s, 2H), 3.87 (s, 3H).

### Synthesis of compound 41-5

At room temperature, the crude compound **41-4** (18.60 g, 68.90 mmol) was dissolved in a mixed solvent of water (15 mL) and tetrahydrofuran (200 mL), followed by the addition of triphenylphosphine (21.70 g, 82.73 mmol) in batches. The reaction mixture was stirred at room temperature for 16 hours. After the reaction was complete, the reaction mixture was diluted with ethyl acetate (200 ml), and washed with saturated brine (100 mL). The organic phases were combined and extracted with 1 M hydrochloric acid (100 mL×2). The aqueous phases were combined, and di-*tert*-butyl dicarbonate (22.1 mL, 96.20 mmol), water (15 mL), tetrahydrofuran (200 mL) and sodium bicarbonate (28.90 g, 344.01 mmol) were added successively, and the reaction mixture was stirred at room temperature for 16 hours. The reaction mixture was diluted with saturated aqueous sodium chloride solution (150 mL), and extracted with ethyl acetate (300 mL×2). The organic phases were combined, dried over anhydrous sodium sulfate and filtered. The filtrate was concentrated under reduced pressure, and the resulting residue was purified by column chromatography (SiO₂, petroleum ether/ethyl acetate=5/1) to obtain compound **41-5.** MS-ESI: *m*/*z* 288.0 [M-56+1]⁺. ¹H NMR (400 MHz, CDCl₃) δ 7.94 (s, 1H), 7.72 (dd, 1H), 7.55 (d, 1H), 5.09-4.92 (m, 1H), 4.35 (d, 2H), 3.84 (s, 3H), 1.40 (s, 9H).

### Synthesis of compound 41-6

At room temperature and in a nitrogen atmosphere, compound **41-5** (5.50 g, 14.70 mmol), bis(pinacolato)diboron (5.71 mL, 22.10 mmol) and potassium acetate (2.89 g, 29.40 mmol) were dissolved in dioxane (150 mL), followed by the addition of [1,1'-bis(diphenylphosphino)ferrocene]palladium(II) dichloride (538 mg, 0.74 mmol). The reaction mixture was heated to 100°C and stirred for 12 hours. After the reaction was complete, the reaction mixture was cooled to room temperature and concentrated under reduced pressure. The resulting residue was purified by column chromatography (SiO₂, petroleum ether/ethyl acetate=6/1) to obtain compound **41-6.** MS-ESI: *m*/*z* 292.0 [M-100+1]⁺. ¹H NMR (400 MHz, CDCl₃) δ 8.02 (s, 1H), 7.97-7.87 (m, 2H), 5.44-5.32 (m, 1H), 4.50 (d, 2H), 3.92 (s, 3H), 1.44 (s, 9H), 1.38 (s, 12H).

### Synthesis of compound 41-7

It was prepared with reference to the synthesis of compound **40-4** with similar conditions except that the temperature was changed from 60°C to 80°C. MS-ESI: *m*/*z* 398.0 [M-100+1]⁺. ¹H NMR (400 MHz, CDCl₃) δ 8.28 (d, 1H), 8.08 (s, 1H), 7.97 (dd, 1H), 7.12 (d, 1H), 7.01 (d, 1H), 4.85-4.76 (m, 1H), 4.23-4.02 (m, 2H), 3.94 (s, 3H), 3.68 (s, 3H), 1.40 (s, 9H).

### Synthesis of compound 41-8

At room temperature, compound **41-7** (3.00 g, 6.04 mmol) was dissolved in dichloromethane (20 mL). Trifluoroacetic acid (4 mL) was added dropwise, and the reaction mixture was stirred at room temperature for 5 hours. After the reaction was complete, the reaction mixture was concentrated under reduced pressure. Toluene (30 mL) and N,N-diisopropylethylamine (5.00 mL, 30.20 mmol) were added, and the reaction mixture was heated to 100°C and stirred for 16 hours. The reaction mixture was cooled to room temperature, and concentrated under reduced pressure. The resulting residue was triturated in a mixed solvent of petroleum ether and ethyl acetate (5/1, 100 mL) to obtain compound **41-8.** MS-ESI: *m*/*z* 363.7 [M-100+1]⁺. ¹H NMR (400 MHz, DMSO-*d*₆) δ 8.76 (t, 1H), 8.09 (d, 1H), 8.06-7.97 (m, 2H), 7.85 (d, 1H), 7.74 (d, 1H), 4.19-4.00 (m, 2H), 3.90 (s, 3H).

### Synthesis of compound 41-9

At room temperature, compound **41-8** (900 mg, 2.47 mmol) was dissolved in *N,N-*dimethylformamide (20 mL). At 0°C, sodium hydride (148 mg, 3.71 mmol, 60%) was slowly added, and the reaction mixture was stirred at 0°C for 20 minutes. Iodomethane (0.3 mL, 4.94 mmol) was added, and the reaction mixture was stirred at 0°C for 30 minutes. After the reaction was complete, the reaction mixture was quenched with water (30 mL) and filtered. The filter cake was collected and concentrated under reduced pressure to obtain the crude compound **41-9,** which was directly used in the next step. MS-ESI: *m*/*z* 378.1 [M-100+1]⁺. ¹H NMR (400 MHz, DMSO-*d₆*) δ 8.18 (d, 1H), 8.08 (d, 1H), 8.05 (dd, 1H), 7.88 (d, 1H), 7.74 (d, 1H), 4.47-4.30 (m, 2H), 3.90 (s, 3H), 3.06 (s, 3H).

### Synthesis of compound 41-10

It was prepared with reference to the synthesis of compound **1-3** with similar conditions except that the temperature was changed from 60°C to 80°C. MS-ESI: *m*/*z* 501.1 [M+1]⁺. ¹H NMR (400 MHz, CDCl₃) δ 8.12 (d, 1H), 8.01 (s, 1H), 7.83 (dd, 1H), 7.64 (d, 1H), 7.24 (dd, 1H), 5.17 (t, 1H), 4.73-4.58 (m, 1H), 4.52-4.43 (m, 1H), 4.02-3.93 (m, 4H), 3.78 (d, 3H), 3.42-3.27 (m, 1H), 3.18 (s, 3H), 3.16-2.99 (m, 1H).

### Synthesis of compound 41-11

At room temperature, compound **41-10** (1.00 g, 2.00 mmol) was dissolved in methanol (10 mL), followed by the addition of a 7 M solution of ammonia in methanol (30 mL). The reaction mixture was sealed, heated to 70°C and stirred for 16 hours. The reaction mixture was cooled to room temperature and concentrated under reduced pressure. The resulting residue was purified by column chromatography (SiO₂, ethyl acetate) to obtain compound **41-11.** MS-ESI: *m*/*z* 471.1 [M+1]⁺.

### Synthesis of compound 41-12

It was prepared with reference to the synthesis of compound **1-5.** MS-ESI: *m*/*z* 435.1 [M+1]⁺.

### Synthesis of compound 41-13

It was prepared with reference to the synthesis of compound **14.** MS-ESI: *m*/*z* 335.1 [M+1]⁺.

### Synthesis of compound 41-14

It was prepared with reference to the synthesis of compound **1-7** with similar conditions except that 4-((*tert*-butoxycarbonyl)amino)tetrahydro-2*H*-pyran-4-carboxylic acid was replaced with (*S*)-4-(*tert*-butoxycarbonyl)-1,4-oxazepane-2-carboxylic acid. MS-ESI: *m*/*z* 462.0 [M-100+1]⁺.

### Synthesis of compound 41

It was prepared with reference to the synthesis of compound **14.** MS-ESI: *m*/*z* 462.3 [M+1]⁺. ¹H NMR (400 MHz, DMSO-*d₆*) δ 8.59-8.45 (m, 1H), 8.05 (s, 1H), 7.96-7.86 (m, 3H), 7.52 (d, 1H), 5.18-5.04 (m, 1H), 4.28 (s, 2H), 3.99 (dd, 1H), 3.94-3.86 (m, 1H), 3.77-3.70 (m, 1H), 3.47-3.28 (m, 2H), 3.09 (s, 3H), 3.04-2.97 (m, 1H), 2.90-2.74 (m, 1H), 2.70-2.57 (m, 2H), 2.34-2.16 (m, 2H).

### Example 42

### (S)-4-Amino-N-(1-cyano-2-(7-fluorodibenzo[b,d]furan-3-yl)ethyl)tetrahydro-2H-pyran-4-carboxamide

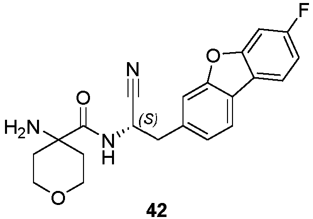

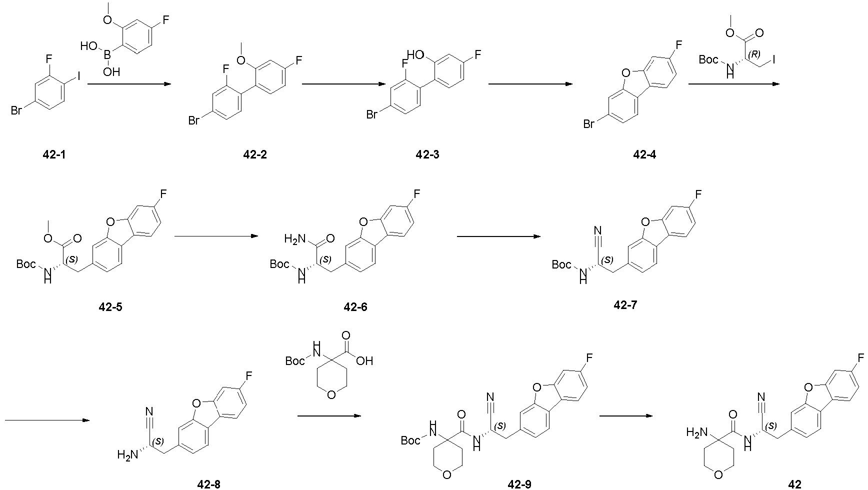

### Synthesis of compound 42-2

At room temperature and in a nitrogen atmosphere, 4-bromo-2-fluoro-1-iodobenzene (compound **42-1)** (6.00 g, 19.94 mmol) and compound 4-fluoro-2-methoxyphenylboronic acid (3.56 g, 20.94 mmol) were dissolved in toluene (60 mL), followed by the addition of a solution sodium carbonate (4.23 g, 39.88 mmol) in water (30 mL) and tetrakis(triphenylphosphine)palladium (0.69 g, 0.60 mmol). The reaction mixture was heated to 100°C and stirred for 15 hours. The reaction mixture was cooled to room temperature, poured into water (20 mL), and extracted with ethyl acetate (25 mL×3). The organic phases were combined, washed with saturated brine (10 mL), dried over anhydrous sodium sulfate and filtered. The filtrate was concentrated under reduced pressure, and the resulting residue was purified by column chromatography (SiO₂, petroleum ether/ethyl acetate=20/1) to obtain compound **42-2.** ¹H NMR (300 MHz, CDCl₃) δ 7.35-7.24 (m, 2H), 7.23-7.14 (m, 2H), 6.77-6.67 (m, 2H), 3.79 (s, 3H).

### Synthesis of compound 42-3

At room temperature, compound **42-2** (3.80 g, 12.70 mmol) was dissolved in dichloromethane (50 mL). At 0°C, a solution of boron tribromide in dichloromethane (15.25 mL, 15.25 mmol, 1.0 mol/L) was added dropwise, and the reaction mixture was stirred at 0°C for 15 hours. The reaction mixture was concentrated under reduced pressure. Water (30 mL) was added to the resulting residue, and the mixture was extracted with ethyl acetate (25 mL×3). The organic phases were combined, washed with saturated brine (10 mL), dried over anhydrous sodium sulfate and filtered. The filtrate was concentrated under reduced pressure to obtain the crude compound **42-3,** which was directly used in the next step. ¹H NMR (300 MHz, CDCl₃) δ 7.44-7.35 (m, 2H), 7.26-7.22 (m, 1H), 7.22-7.13 (m, 1H), 6.78-6.68 (m, 2H), 4.98 (s, 1H).

### Synthesis of compound 42-4

At room temperature, the crude compound **42-3** (3.50 g, 12.28 mmol) and potassium carbonate (3.39 g, 24.55 mmol) were dissolved in *N*-methylpyrrolidone (30 mL), and the reaction mixture was heated to 170°C and stirred for 2 hours. After the reaction was complete, the reaction mixture was cooled to room temperature, followed by the addition of water (100 mL), and extracted with ethyl acetate (25 mL×3). The organic phases were combined, washed with water (10 mL×2) and saturated brine (10 mL) successively, dried over anhydrous sodium sulfate and filtered. The filtrate was concentrated under reduced pressure, and the resulting residue was purified by column chromatography (SiO₂, petroleum ether/ethyl acetate=10/1) to obtain compound **42-4.** ¹H NMR (300 MHz, CDCl₃) δ 7.89-7.81 (m, 1H), 7.79-7.71 (m, 2H), 7.51-7.44 (m, 1H), 7.32-7.26 (m, 1H), 7.16-7.05 (m, 1H).

### Synthesis of compound 42-5

It was prepared with reference to the synthesis of compound **14-B7** with similar conditions except that the temperature was changed from 70°C to room temperature. ¹H NMR (300 MHz, CDCl₃) δ 7.87-7.78 (m, 2H), 7.35-7.27 (m, 1H), 7.27-7.24 (m, 1H), 7.15-7.03 (m, 2H), 5.05-5.00 (m, 1H), 4.68-4.63 (m, 1H), 3.74 (s, 3H), 3.30-3.20 (m, 2H), 1.47-1.40 (m, 9H).

### Synthesis of compound 42-6

At room temperature, compound **42-5** was dissolved in a 5 M solution of ammonia in methanol (15 mL), and the reaction mixture was sealed, heated to 100°C and stirred for 5 hours. After the reaction was complete, the reaction mixture was cooled to room temperature, and concentrated under reduced pressure. The resulting residue was triturated in a mixed solvent of petroleum ether and ethyl acetate (3/1, 5 mL) to obtain compound **42-6.** MS-ESI: *m*/*z* 272.9 [M-100+1]⁺.

### Synthesis of compound 42-7

At room temperature, compound **42-6** (600 mg, 1.61 mmol) and *N,N-*diisopropylethylamine (416.46 mg, 3.22 mmol) were dissolved in dichloromethane (5 mL). At 0°C, trifluoroacetic anhydride (508 mg, 2.42 mmol) was added dropwise, and the reaction mixture was stirred at 0°C for 20 minutes. After the reaction was complete, the reaction mixture was concentrated under reduced pressure. Water (5 mL) was added to the resulting residue, and the mixture was extracted with ethyl acetate (10 mL×3). The organic phases were combined, washed with saturated brine (5 mL), dried over anhydrous sodium sulfate and filtered. The filtrate was concentrated under reduced pressure, and the resulting residue was triturated in a mixed solvent of petroleum ether and ethyl acetate (3/1, 5 mL) to obtain compound **42-7.** MS-ESI: *m*/*z* 355.2 [M+1]⁺. ¹H NMR (400 MHz, CDCl₃) δ 7.93-7.83 (m, 2H), 7.49 (s, 1H), 7.32-7.25 (m, 2H), 7.15-7.06 (m, 1H), 4.91-4.80 (m, 2H), 3.32-3.16 (m, 2H), 1.44 (s, 9H).

### Synthesis of compound 42-8

It was prepared with reference to the synthesis of compound **14** with similar conditions except that the temperature was changed from 40°C to 25°C. MS-ESI: *m*/*z* 238.0 [M-17+1]⁺.

### Synthesis of compound 42-9

It was prepared with reference to the synthesis of compound **14-A7** with similar conditions except that (S)-4-(*tert-*butoxycarbonyl)-1,4-oxazepane-2-carboxylic acid was replaced with 4-((*tert*-butoxycarbonyl)amino)tetrahydro-2*H*-pyran-4-carboxylic acid. MS-ESI: *m*/*z* 382.3 [M-100+1]⁺.

### Synthesis of compound 42

It was prepared with reference to the synthesis of compound **14** with similar conditions except that the temperature was changed from 40°C to room temperature. MS-ESI: *m*/*z* 382.1 [M-100+1]⁺. ¹H NMR (400 MHz, DMSO-*d6*) δ 8.17-8.12 (m, 1H), 8.08-8.04 (m, 1H), 7.68-7.63 (m, 2H), 7.37-7.31 (m, 1H), 7.31-7.23 (m, 1H), 5.08 (t, 1H), 3.65-3.50 (m, 3H), 3.45-3.36 (m, 1H), 3.36-3.30 (m, 2H), 1.92-1.82 (m, 1H), 1.74-1.64 (m, 1H), 1.21-1.15 (m, 1H), 1.14-1.06 (m, 1H).

### Example 43

### (2S)-N-(1-Cyano-2-(2-fluoro-9-(methylsulfonyl)-6H-benzo[c]chromen-3-yl)ethyl)-1,4-oxazepane-2-carboxamide

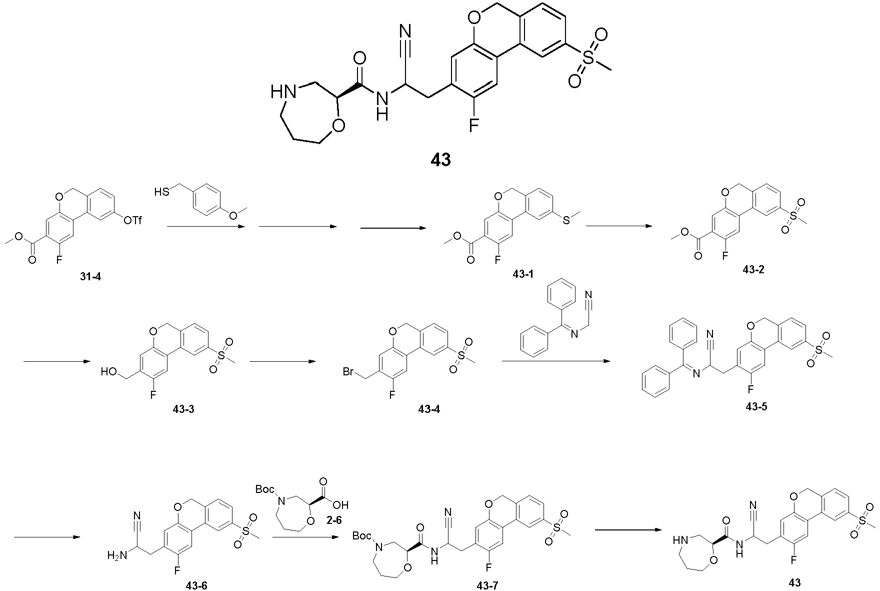

### Synthesis of compound 43-1

It was prepared with reference to the synthesis of compounds **29-8, 29-9.** MS-ESI: *m*/*z* 305.0 [M+1]⁺.

### Synthesis of compound 43-2

It was prepared with reference to the synthesis of compound **29-10.** MS-ESI: *m*/*z* 337.1 [M+1]⁺.

### Synthesis of compound 43-3

It was prepared with reference to the synthesis of compound **12-4** with similar conditions except that the reaction temperature was changed from 0°C to room temperature. MS-ESI: *m*/*z* 326.1 [M+18]⁺.

### Synthesis of compound 43-4

It was prepared with reference to the synthesis of compound **14-A4.** MS-ESI: *m*/*z* 388.0 [M+18]⁺.

### Synthesis of compound 43-5

It was prepared with reference to the synthesis of compound **14-A5** with similar conditions except that benzyltrimethylammonium chloride was not added. MS-ESI: *m*/*z* 511.0 [M+1]⁺.

### Synthesis of compound 43-6

It was prepared with reference to the synthesis of compound **14-A6.** MS-ESI: *m*/*z* 347.0 [M+1]⁺.

### Synthesis of compound 43-7

It was prepared with reference to the synthesis of compound **14-A7** with similar conditions except that *N,N*-diisopropylethylamine was replaced with triethylamine. MS-ESI: *m*/*z* 518.1 [M-56+1]⁺.

### Synthesis of compound 43

It was prepared with reference to the synthesis of compound **14.** MS-ESI: *m*/*z 474.2* [M+1]⁺. ¹H NMR (400 MHz, DMSO-*d*₆) δ 8.74-8.65 (m, 1H), 8.37 (s, 1H), 7.99-7.93 (m, 1H), 7.88 (d, 1H), 7.56 (d, 1H), 7.07-7.01 (m, 1H), 5.28-5.18 (m, 2H), 5.10-4.95 (m, 1H), 4.03-3.82 (m, 2H), 3.77-3.66 (m, 1H), 3.31 (s, 3H), 3.26-2.98 (m, 3H), 2.84-2.55 (m, 3H), 1.82-1.64 (m, 2H).

### Example 44

### (S)-4-Amino-N-(1-cyano-2-4-(4-fluorophenyl)-5-oxo-2,3,4,5-tetrahydrobenzo[f][1,4]oxazepin-8-yl)ethyl)tetrahydro-2H-pyran-4-carboxamide

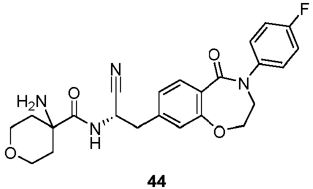

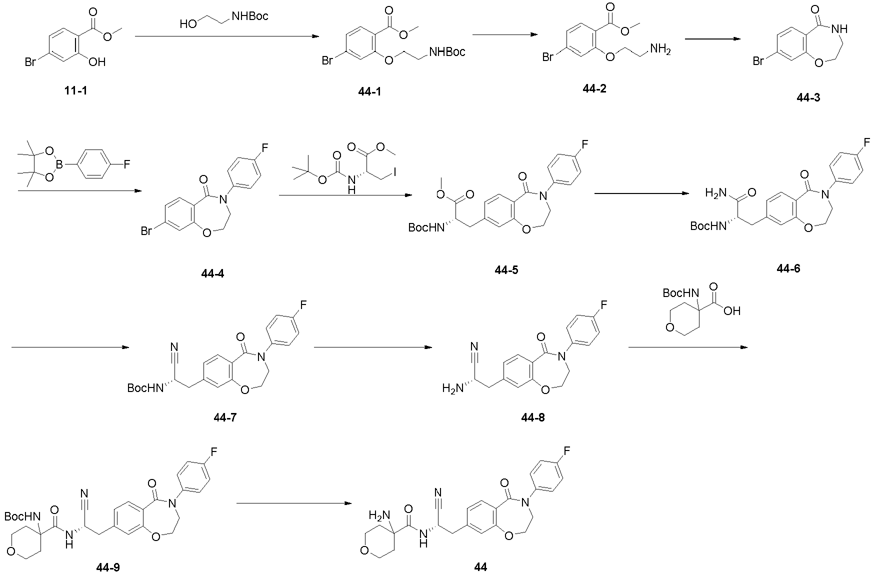

### Synthesis of compound 44-1

At room temperature, compound **11-1** (10 g, 43.48 mmol), *tert*-butyl (2-hydroxyethyl)carbamate (7 g, 43.44 mmol), triphenylphosphine (17 g, 64.81 mmol) and di-*tert*-butyl azodicarboxylate (15 g, 65.14 mmol) were dissolved in toluene (100 mL). The reaction mixture was heated to 60°C and stirred for 4 hours. After the reaction was complete, the reaction mixture was cooled to room temperature and filtered. The filtrate was diluted with water (200 mL), and extracted with dichloromethane (200 mL×3). The organic phases were combined, washed with saturated brine (100 mL), dried over anhydrous sodium sulfate and filtered. The filtrate was concentrated under reduced pressure, and the resulting residue was purified by column chromatography (SiO₂, dichloromethane/methanol=19/1) to obtain compound **44-1.** MS-ESI: *m*/*z* 273.9 [M-100+1]⁺.

### Synthesis of compound 44-2

At 0°C, compound **44-1** (15 g, 40.21 mmol) was dissolved in dichloromethane (200 mL), followed by the addition of trifluoroacetic acid (50 mL). The reaction mixture was stirred at room temperature for 2 hours. After the reaction was complete, the reaction mixture was concentrated under reduced pressure. Saturated aqueous sodium bicarbonate solution (10 mL) was added to the residue, and the mixture was extracted with dichloromethane (100 mL×3). The organic phases were combined, washed with saturated brine (50 mL), dried over anhydrous sodium sulfate and filtered. The filtrate was concentrated under reduced pressure to obtain compound **44-2.** MS-ESI: *m*/*z* 275.9 [M+1]⁺.

### Synthesis of compound 44-3

At room temperature, compound **44-2** (10.80 g, 39.56 mmol) was dissolved in toluene (150 mL), and the reaction mixture was heated to 120°C and stirred for 18 hours. The reaction mixture was cooled to room temperature, and concentrated under reduced pressure. Ethyl acetate (10 mL) was added, and the mixture was triturated in petroleum ether (150 mL) to obtain compound **44-3.** MS-ESI: *m*/*z* 243.9 [M+1]⁺.

### Synthesis of compound 44-4

At room temperature, compound **44-3** (4 g, 16.35 mmol), 2-(4-fluorophenyl)-4,4,5,5-tetramethyl-1,3-dioxaborolane (7.34 g, 33.06 mmol) and cuprous oxide (11.99 g, 83.79 mmol) were dissolved in dimethyl sulfoxide (20 mL). The reaction mixture was heated to 120°C and stirred for 18 hours. The reaction mixture was cooled to room temperature, filtered, poured into water (100 mL) and extracted with dichloromethane (100 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, dichloromethane/methanol=19/1) to obtain compound **44-4.** MS-ESI: *m*/*z* 337.9 [M+1]⁺.

### Synthesis of compound 44-5

It was prepared with reference to the synthesis of compound **1-3.** MS-ESI: *m*/*z* 402.9 [M-56+1]⁺.

### Synthesis of compound 44-6

It was prepared with reference to the synthesis of compound **1-4.** MS-ESI: *m*/*z* 344.0 [M-100+1]⁺.

### Synthesis of compound 44-7

It was prepared with reference to the synthesis of compound **1-5.** MS-ESI: *m*/*z* 370.0 [M-56+1]⁺.

### Synthesis of compound 44-8

It was prepared with reference to the synthesis of compound **1-6.** MS-ESI: *m*/*z* 326.0 [M-56+1]⁺.

### Synthesis of compound 44-9

It was prepared with reference to the synthesis of compound **1-7** with similar conditions except that the solvent *N,N*-dimethylformamide was replaced with dichloromethane. MS-ESI: *m*/*z* 453.1 [M-100+1]⁺.

### Synthesis of compound 44

It was prepared with reference to the synthesis of compound **1.** MS-ESI: *m*/*z* 453.4 [M-100+1]⁺. ¹H NMR (400 MHz, CDCl₃) δ 8.27 (d, 1H), 7.86 (d, 1H), 7.35-7.31 (m, 2H), 7.15-7.08 (m, 3H), 6.99 (d, 1H), 5.13-5.08 (m, 1H), 4.49 (t, 2H), 3.94-3.87 (m, 4H), 3.65-3.58 (m, 2H), 3.13 (d, 2H), 2.34-2.18 (m, 2H), 1.44 (br s, 2H), 1.30-1.20 (m, 2H).

### Example 45

### (S)-4-Amino-N-(1-cyano-2-(2-(4-cyanophenyl)-1-oxoisoindolin-5-yl)ethyl)tetrahydro-2H-pyran-4-carboxamide

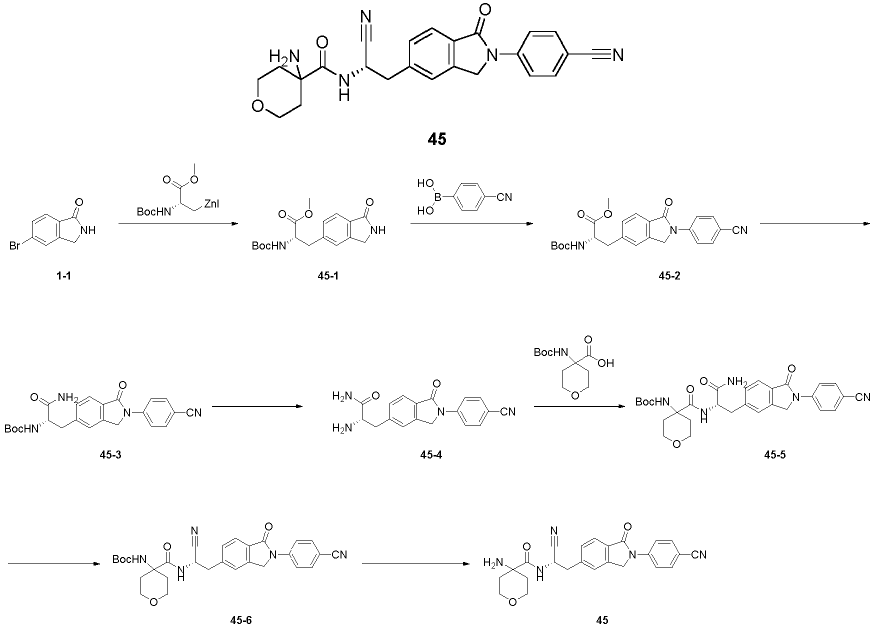

### Synthesis of compound 45-1

It was prepared with reference to the synthesis of compound **1-3.** MS-ESI: *m*/*z* 335.1 [M+1]⁺.

### Synthesis of compound 45-2

It was prepared with reference to the synthesis of compound **1-2.** MS-ESI: *m*/*z* 380.1 [M-56+1]⁺.

### Synthesis of compound 45-3

It was prepared with reference to the synthesis of compound **1-4.** MS-ESI: *m*/*z* 365.1 [M-56+1]⁺.

### Synthesis of compound 45-4

It was prepared with reference to the synthesis of compound **1-6.** MS-ESI: *m*/*z* 321.2 [M+1]⁺.

### Synthesis of compound 45-5

It was prepared with reference to the synthesis of compound **1-7** with similar conditions except that the solvent *N,N*-dimethylformamide was replaced with dichloromethane. MS-ESI: *m*/*z* 448.2 [M-100+1]⁺.

### Synthesis of compound 45-6

It was prepared with reference to the synthesis of compound **1-5.** MS-ESI: *m*/*z* 474.2 [M-56+1]⁺.

### Synthesis of compound 45

It was prepared with reference to the synthesis of compound 1. MS-ESI: *m*/*z* 430.1 [M+1]⁺. ¹H NMR (400 MHz, DMSO-*d6*) δ 8.14 (d, 2H), 7.92 (d, 2H), 7.77 (d, 1H), 7.59 (s, 1H), 7.48 (d, 1H), 5.08-5.04 (m, 3H), 3.64-3.55 (m, 3H), 3.53-3.20 (m, 3H), 1.91-1.84 (m, 1H), 1.76-1.68 (m, 1H), 1.21-1.17 (m, 1H), 1.12-1.09 (m, 1H).

### Example 46

### (S)-4-Amino-N-(1-cyano-2-(2-(4-cyanobenzoyl)isoindolin-5-yl)ethyl)tetrahydro-2H-pyran-4-carboxamide

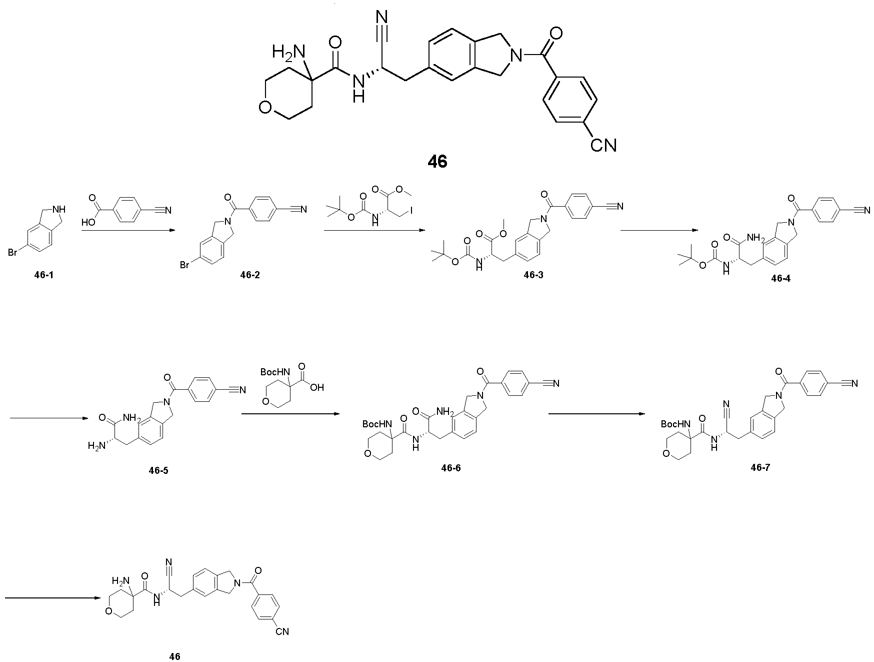

### Synthesis of compound 46-2

At room temperature, 5-bromoisoindoline (compound **46-1)** (2.00 g, 10.15 mmol) and 4-cyanobenzoic acid (2.01 g, 12.18 mmol) were dissolved in a mixed solvent of *N,N-*dimethylformamide (5 mL) and dichloromethane (100 mL), followed by the addition of O-benzotriazole-*N,N,N',N*'-tetramethyluronium hexafluorophosphate (5.78 g, 15.23 mmol) and *N,N*-diisopropylethylamine (2.62 g, 20.30 mmol). The reaction mixture was stirred at room temperature for 16 hours. After the reaction was complete, saturated aqueous sodium bicarbonate solution (50 mL) was added, and the reaction mixture was extracted with dichloromethane (200 mL×3). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=7/3) to obtain compound **46-2.** MS-ESI: *m*/*z* 326.9 [M+1]⁺.

### Synthesis of compound 46-3

It was prepared with reference to the synthesis of compound **1-3.** MS-ESI: *m*/*z* 350.0 [M-100+1]⁺.

### Synthesis of compound 46-4

It was prepared with reference to the synthesis of compound **1-4.** MS-ESI: *m*/*z* 335.0 [M-100+1]⁺.

### Synthesis of compound 46-5

It was prepared with reference to the synthesis of compound **9-6.** MS-ESI: *m*/*z* 335.0 [M+1]⁺.

### Synthesis of compound 46-6

It was prepared with reference to the synthesis of compound **1-7** with similar conditions except that the solvent *N,N*-dimethylformamide was replaced with a mixed solvent of *N*,*N*-dimethylformamide and dichloromethane (1/5). MS-ESI: *m*/*z* 462.0 [M-100+1]⁺.

### Synthesis of compound 46-7

It was prepared with reference to the synthesis of compound **1-5.** MS-ESI: *m*/*z* 444.1 [M-100+1]⁺.

### Synthesis of compound 46

It was prepared with reference to the synthesis of compound **1.** MS-ESI: *m*/*z* 444.1 [M+1]⁺. ¹H NMR (400 MHz, DMSO-*d6*) δ 7.97 (dd, 2H), 7.79 (d, 2H), 7.36-7.31 (m, 1H), 7.25-7.17 (m, 2H), 4.95-4.88 (m, 1H), 4.84 (s, 2H), 4.69 (s, 2H), 3.62-3.51 (m, 4H), 3.16-3.11 (m, 2H), 1.90-1.87 (m, 1H), 1.78-1.73 (m, 1H), 1.22-1.11 (m, 2H).

### Example 47

### (S)-4-Amino-N-(1-cyano-2-(2-(3-cyanophenyl)-1-oxoisoindolin-5-yl)ethyl)tetrahydro-2H-pyran-4-carboxamide formate

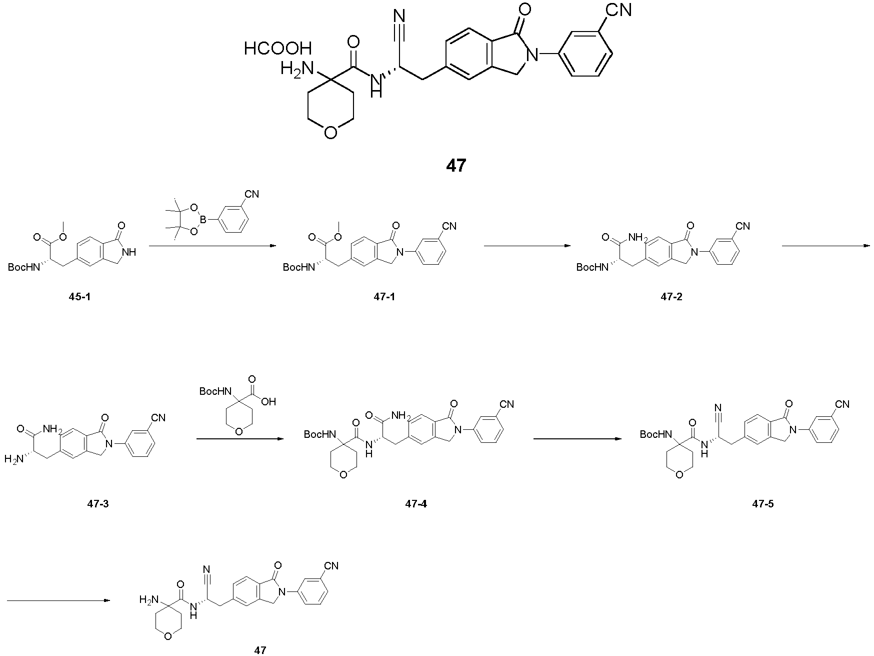

### Synthesis of compound 47-1

It was prepared with reference to the synthesis of compound **44-4** with similar conditions except that the temperature was changed from 120°C to 110°C. MS-ESI: *m*/*z* 380.1 [M-56+1]⁺.

### Synthesis of compound 47-2

It was prepared with reference to the synthesis of compound **1-4.** MS-ESI: *m*/*z* 365.1 [M-55+1]⁺.

### Synthesis of compound 47-3

It was prepared with reference to the synthesis of compound **1-6.** MS-ESI: *m*/*z* 321.2 [M+1]⁺.

### Synthesis of compound 47-5

It was prepared with reference to the synthesis of compound **1-7** with similar conditions except that the solvent *N,N*-dimethylformamide was replaced with dichloromethane. MS-ESI: *m*/*z* 448.2 [M-100+1]⁺.

### Synthesis of compound 47-6

It was prepared with reference to the synthesis of compound **1-5.** MS-ESI: *m*/*z* 471.2 [M-56+1]⁺.

### Synthesis of compound 47

It was prepared with reference to the synthesis of compound **1.** MS-ESI: *m*/*z* 430.0 [M+1]⁺. ¹H NMR (400 MHz, CDCl₃) δ 8.34 (d, 1H), 8.22 (s, 1H), 8.01 (s, 1H), 8.17 (d, 1H), 7.92 (d, 1H), 7.57-7.53 (m, 2H), 7.47 (d, 1H), 7.41 (d, 1H), 5.19-5.13 (m, 1H), 4.89 (s, 2H), 3.92-3.88 (m, 2H), 3.65-3.59 (m, 2H), 3.25 (d, 2H), 2.33-2.17 (m, 2H), 1.31-1.19 (m, 2H).

### Example 48

### (S)-4-Amino-N-(1-cyano-2-(2-(3-methyl-2-oxo-2,3-dihydrobenzo[d]oxazol-5-yl)-1-oxoisoindolin-5-yl)ethyl)tetrahydro-2H-pyran-4-carboxamide

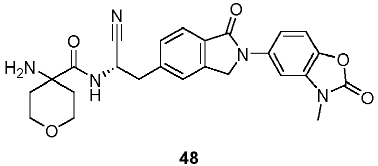

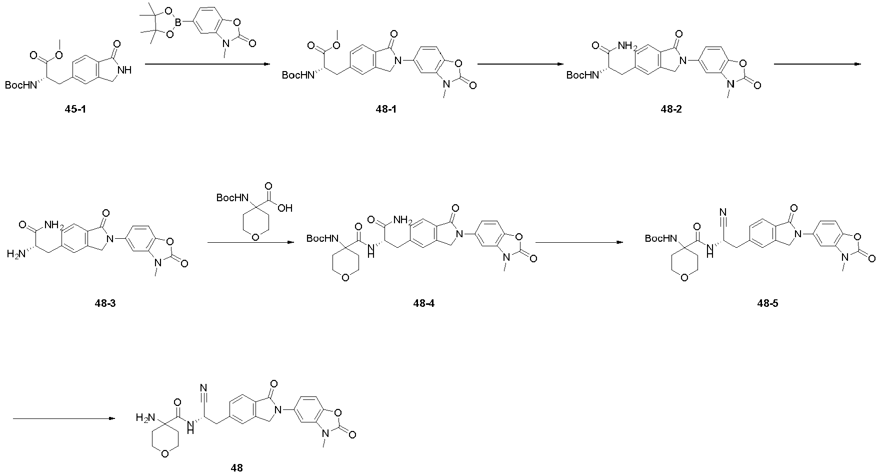

### Synthesis of compound 48-1

It was prepared with reference to the synthesis of compound **44-4** with similar conditions except that the reaction temperature was changed from 120°C to 110°C. MS-ESI: *m*/*z* 482.2 [M+1]⁺.

### Synthesis of compound 48-2

It was prepared with reference to the synthesis of compound **1-4.** MS-ESI: *m*/*z* 467.2 [M+1]⁺.

### Synthesis of compound 48-3

It was prepared with reference to the synthesis of compound **1-6.** MS-ESI: *m*/*z* 367.2 [M+1]⁺.

### Synthesis of compound 48-4

It was prepared with reference to the synthesis of compound **1-7** with similar conditions except that the solvent *N,N*-dimethylformamide was replaced with dichloromethane. MS-ESI: *m*/*z* 494.2 [M-100+1]⁺.

### Synthesis of compound 48-5

It was prepared with reference to the synthesis of compound **1-5.** MS-ESI: *m*/*z* 520.2 [M-56+1]⁺.

### Synthesis of compound 48

It was prepared with reference to the synthesis of compound **1.** MS-ESI: *m*/*z* 476.2 [M+1]⁺. ¹H NMR (400 MHz, CDCl₃) δ 8.32 (d, 1H), 8.04 (d, 1H), 7.91 (d, 1H), 7.52 (s, 1H), 7.40 (d, 1H), 7.24 (d, 1H), 7.18-7.15 (m, 1H), 5.18-5.13 (m, 1H), 4.89 (s, 2H), 3.98-3.82 (m, 2H), 3.64-3.56 (m, 2H), 3.45 (s, 3H), 3.24 (d, 2H), 2.34-2.26 (m, 1H), 2.25-2.17 (m, 1H), 1.31-1.18 (m, 2H).

### Example 49

### (S)-4-Amino-N-(1-cyano-2-(1-oxo-2-phenyl-1,2,3,4-tetrahydroisoquinolin-6-yl)ethyl)tetrahydro-2H-pyran-4-carboxamide

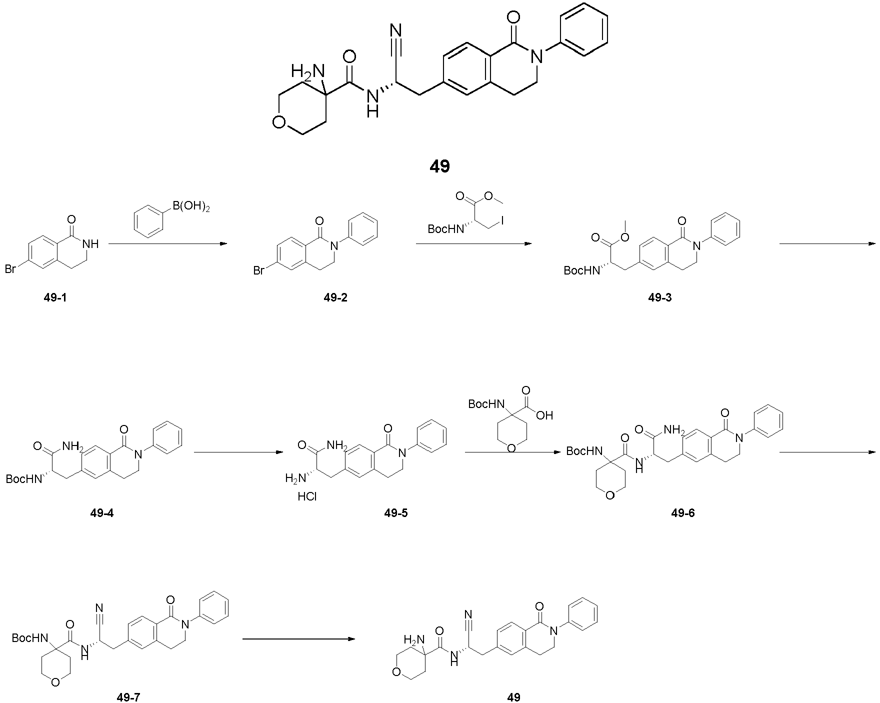

### Synthesis of compound 49-2

It was prepared with reference to the synthesis of compound **1-2.** MS-ESI: *m*/*z* 302.0 [M+1]⁺.

### Synthesis of compound 49-3

It was prepared with reference to the synthesis of compound **1-3.** MS-ESI: *m*/*z* 425.1 [M+1]⁺.

### Synthesis of compound 49-4

It was prepared with reference to the synthesis of compound **1-4.** MS-ESI: *m*/*z* 410.2 [M+1]⁺.

### Synthesis of compound 49-5

It was prepared with reference to the synthesis of compound **9-6.** MS-ESI: *m*/*z* 310.2 [M+1]⁺.

### Synthesis of compound 49-6

It was prepared with reference to the synthesis of compound **1-7.** MS-ESI: *m*/*z* 437.2 [M-100+1]⁺.

### Synthesis of compound 49-7

It was prepared with reference to the synthesis of compound **1-5** with similar conditions except that dichloromethane was replaced with *N*,*N*-dimethylformamide. MS-ESI: *m*/*z* 519.3 [M+1]⁺.

### Synthesis of compound 49

It was prepared with reference to the synthesis of compound **1.** MS-ESI: *m*/*z* 419.2 [M+1]⁺. ¹H NMR (400 MHz, DMSO-*d6*) δ 7.87 (d, 1H), 7.47-7.35 (m, 4H), 7.35-7.21 (m, 3H), 5.06-5.02 (m, 1H), 3.93 (t, 2H), 3.66-3.57 (m, 3H), 3.24-3.22 (m, 2H), 3.13-3.05 (m, 3H), 1.96-1.74 (m, 2H), 1.25-1.12 (m, 2H).

### Example 50

### (S)-4-Amino-N-(1-cyano-2-(3-oxo-2-phenylisoindolin-5-yl)ethyl)tetrahydro-2H-pyran-4-carboxamide

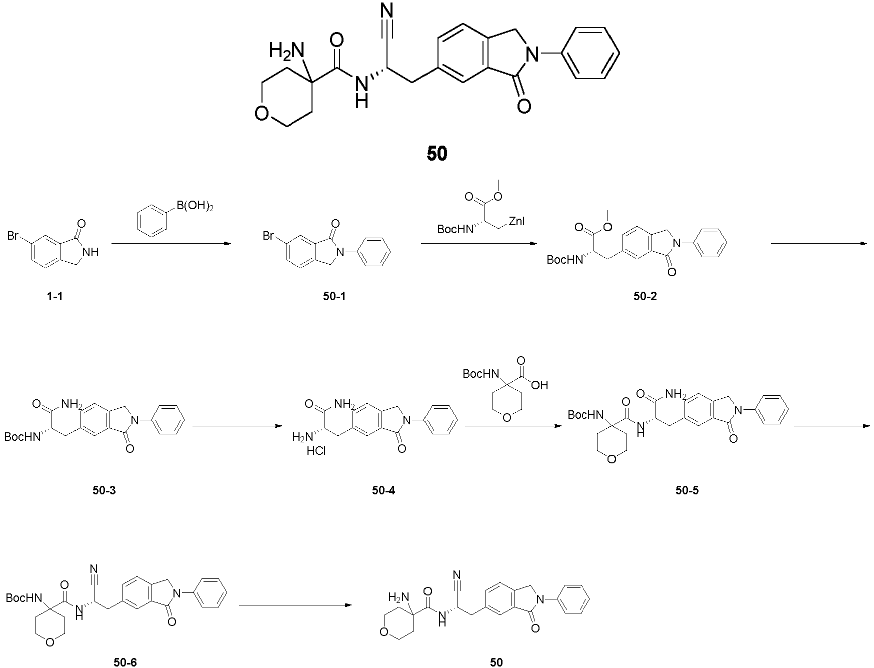

### Synthesis of compound 50-1

It was prepared with reference to the synthesis of compound **1-2.** MS-ESI: *m*/*z* 288.0 [M+1]⁺.

### Synthesis of compound 50-2

It was prepared with reference to the synthesis of compound **1-3.** ¹H NMR (400 MHz, DMSO-*d6*) δ 7.91 (d, 2H), 7.68 (s, 1H), 7.60-7.53 (m, 2H), 7.46-7.42 (m, 2H), 7.38 (d, 1H), 7.18 (t, 1H), 4.99 (s, 2H), 4.25-4.20 (m, 1H), 3.64 (s, 3H), 3.17-3.12 (m, 1H), 3.00-2.94 (m, 1H), 1.31 (s, 9H).

### Synthesis of compound 50-3

It was prepared with reference to the synthesis of compound **1-4.** MS-ESI: *m*/*z* 340.1 [M-56+1]⁺.

### Synthesis of compound 50-4

It was prepared with reference to the synthesis of compound **9-6.** MS-ESI: *m*/*z* 296.5 [M+1]⁺.

### Synthesis of compound 50-5

It was prepared with reference to the synthesis of compound **1-7** with similar conditions except that the solvent *N,N*-dimethylformamide was replaced with a mixed solvent of *N,N*-dimethylformamide and dichloromethane (1/5). MS-ESI: *m*/*z* 523.3 [M+1]⁺.

### Synthesis of compound 50-6

It was prepared with reference to the synthesis of compound **1-5.** MS-ESI: *m*/*z* 505.3 [M+1]⁺.

### Synthesis of compound 50

It was prepared with reference to the synthesis of compound **1.** MS-ESI: *m*/*z* 405.2 [M+1]⁺. ¹H NMR (400 MHz, DMSO-*d6*) δ 8.15 (s, 1H), 7.90 (d, 2H), 7.74 (s, 1H), 7.62-7.58 (m, 2H), 7.49-7.40 (m, 2H), 7.18 (t, 1H), 5.07-4.99 (m, 3H), 3.64-3.56 (m, 3H), 3.47-3.42 (m, 1H), 3.29-3.26 (m, 2H), 1.92-1.85 (m, 1H), 1.75-1.68 (m, 1H), 1.22-1.13 (m, 2H).

### Example 51

### (S)-4-Amino-N-(1-cyano-2-(2-methyl-1,2,3,4-tetrahydroisoquinolin-6-yl)ethyl)tetrahydro-2H-pyran-4-carboxamide

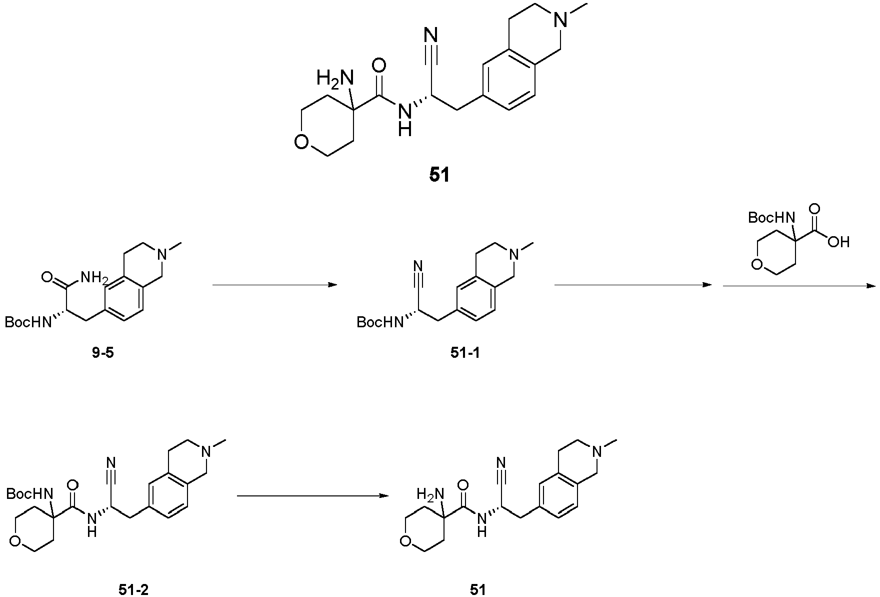

### Synthesis of compound 51-1

It was prepared with reference to the synthesis of compound **1-5.** MS-ESI: *m*/*z* 316.5 [M+1]⁺.

### Synthesis of compound 51-2

At room temperature, compound **51-1** (180 mg, 0.57 mmol) was dissolved in acetonitrile (5 mL), followed by the addition of trimethylchlorosilane (186 mg, 1.71 mmol) and sodium iodide (256.6 mg, 1.71 mmol). The reaction mixture was stirred at room temperature for 1 hour. After the reaction was complete, methanol (5 mL) was added, and the reaction mixture was concentrated under reduced pressure. Saturated aqueous sodium bicarbonate solution (50 mL) was added to the residue, and the mixture was extracted with dichloromethane (50 mL×3). The organic phases were combined, washed with saturated brine (50 mL), dried over anhydrous sodium sulfate and filtered. The filtrate was concentrated under reduced pressure to obtain a residue, which was dissolved in *N,N*-dimethylformamide (5 mL) at room temperature. 4-((*Tert-*butoxycarbonyl)amino)tetrahydro-2*H*-pyran-4-carboxylic acid (176 mg, 0.72 mmol), O-benzotriazole-*N,N,N',N*'-tetramethyluronium hexafluorophosphate (370 mg, 0.98 mmol) and *N,N*-diisopropylethylamine (168 mg, 1.30 mmol) were added, and the reaction mixture was stirred at room temperature for 16 hours. Saturated aqueous sodium bicarbonate solution (20 mL) was added, and the mixture was extracted with dichloromethane (20 mL×3). The organic phases were combined, washed with saturated brine (20 mL), dried over anhydrous sodium sulfate and filtered. The filtrate was concentrated under reduced pressure, and the resulting residue was purified by column chromatography (SiO₂, dichloromethane/methanol=9/1) to obtain compound **51-2.** MS-ESI: *m*/*z* 443.2 [M+1]⁺.

### Synthesis of compound 51

It was prepared with reference to the synthesis of compound **1.** MS-ESI: *m*/*z* 343.1 [M+1]⁺. ¹H NMR (400 MHz, DMSO-*d₆*) δ 7.03-6.97 (m, 3H), 4.92-4.88 (m, 1H), 3.65-3.56 (m, 3H), 3.51-3.47 (m, 1H), 3.43 (s, 2H), 3.10-2.99 (m, 2H), 2.77 (t, 2H), 2.56 (t, 2H), 2.32 (s, 3H), 1.93-1.85 (m, 1H), 1.79-1.72 (m, 1H), 1.22-1.12 (m, 2H).

### Example 52

### (S)-4-Amino-N-(1-cyano-2-(isochroman-7-yl)ethyl)tetrahydro-2H-pyran-4-carboxamide trifluoroacetate

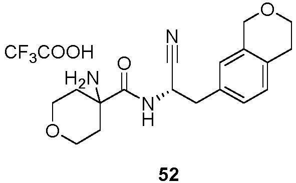

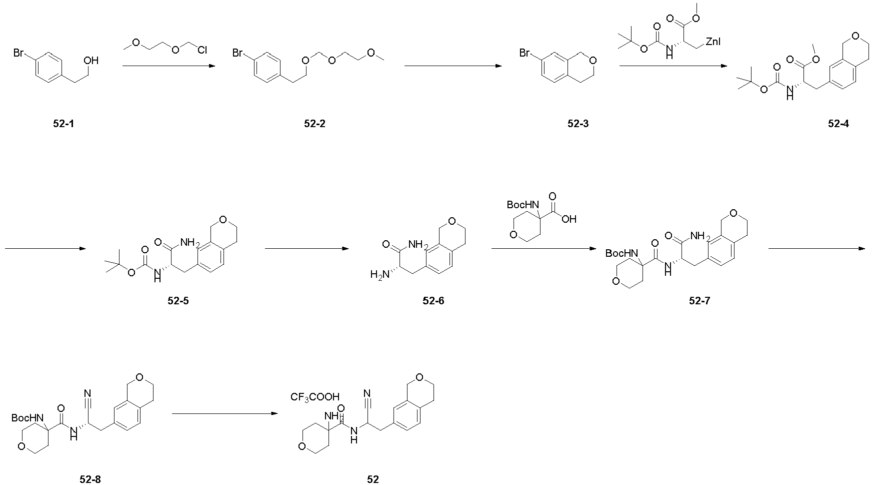

### Synthesis of compound 52-2

At room temperature, compound **52-1** (10.00 g, 50.25 mmol) was dissolved in dichloromethane (200 mL), followed by the addition of *N*,*N*-diisopropylethylamine (9.63 g, 74.64 mmol) and 2-methoxyethoxymethyl chloride (9.30 g, 74.98 mmol). The reaction mixture was stirred at room temperature for 12 hours. The reaction mixture was washed successively with 1 M hydrochloric acid (50 mL) and saturated brine (50 mL), dried over anhydrous sodium sulfate and filtered. The filtrate was concentrated under reduced pressure to obtain the crude compound **52-2,** which was directly used in the next step. MS-ESI: *m*/*z* 289.3 [M+1]⁺.

### Synthesis of compound 52-3

At 0°C, 2-(4-bromophenyl)ethan-1-ol (compound **52-1)** (14.69 g, 51.00 mmol) was dissolved in acetonitrile (200 mL), followed by the addition of trimethylsilyl trifluoromethanesulfonate (3.40 g, 15.29 mmol). The reaction mixture was stirred at room temperature for 12 hours. The reaction mixture was concentrated under reduced pressure. The resulting residue was dissolved in ethyl acetate (200 mL), washed with saturated aqueous sodium bicarbonate solution (200 mL), dried over anhydrous sodium sulfate and filtered. The filtrate was concentrated under reduced pressure, and the resulting residue was purified by column chromatography (SiO₂, petroleum ether/ethyl acetate=5/1) to obtain compound **52-3.** ¹H NMR (400 MHz, CDCl₃) δ 7.30-7.24 (m, 1H), 7.13 (d, 1H), 6.98 (d, 1H), 4.72 (s, 2H), 3.95 (t, 2H), 2.79 (t, 2H).

### Synthesis of compound 52-4

It was prepared with reference to the synthesis of compound **1-3.** MS-ESI: *m*/*z* 236.4 [M-100+1]⁺.

### Synthesis of compound 52-5

It was prepared with reference to the synthesis of compound **1-4.** MS-ESI: *m*/*z* 321.2 [M+1]⁺.

### Synthesis of compound 52-6

It was prepared with reference to the synthesis of compound **9-6.** MS-ESI: *m*/*z* 221.2 [M+1]⁺.

### Synthesis of compound 52-7

It was prepared with reference to the synthesis of compound **1-7.** MS-ESI: *m*/*z* 448.3 [M+1]⁺.

### Synthesis of compound 52-8

It was prepared with reference to the synthesis of compound **1-5** with similar conditions except that dichloromethane was replaced with *N*,*N*-dimethylformamide. MS-ESI: *m*/*z* 330.2 [M-100+1]⁺.

### Synthesis of compound 52

It was prepared with reference to the synthesis of compound **1.** MS-ESI: *m*/*z* 330.2 [M+1]⁺. ¹H NMR (400 MHz, DMSO-*d6*) δ 9.18 (br s, 1H), 8.43 (br s, 3H), 7.16-7.05 (m, 2H), 6.94 (s, 1H), 5.04-4.99 (m, 1H), 4.67-4.59 (m, 2H), 3.91-3.79 (m, 2H), 3.68-3.60 (m, 4H), 3.18-3.05 (m, 2H), 2.74 (t, 2H), 2.20-2.13 (m, 1H), 2.07-1.99 (m, 1H), 1.67 (d, 1H), 1.49 (d, 1H).

### Example 53

### (S)-4-Amino-N-(1-cyano-2-(4-(4-fluorophenyl)-2,3,4,5-tetrahydrobenzo[f][1,4]oxazepin-8-yl)ethyl)tetrahydro-2H-pyran-4-carboxamide

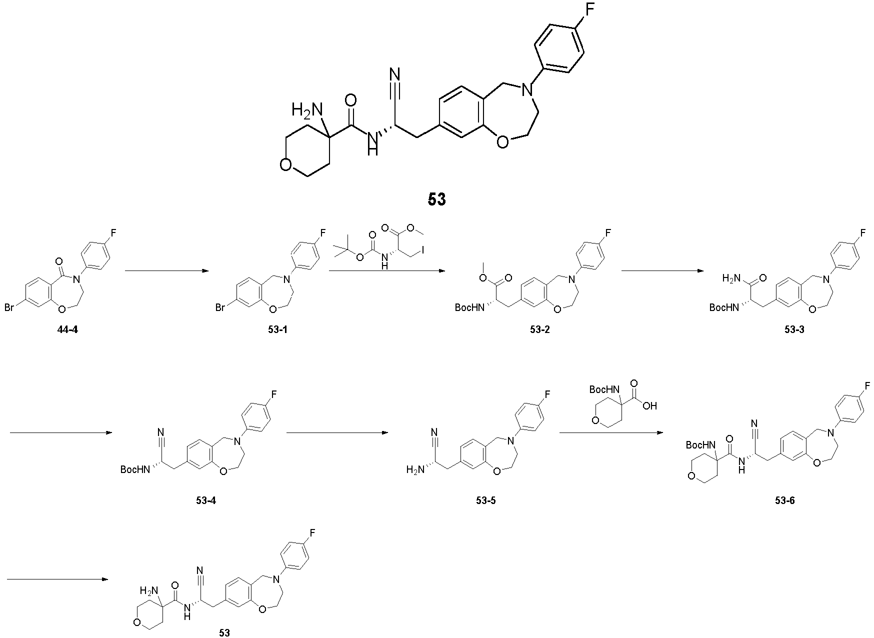

### Synthesis of compound 53-1

At room temperature, 8-bromo-4-(4-fluorophenyl)-3,4-dihydrobenzo[*f*][1,4]oxazepin-5(2H)-one (compound **44-4**) (1.40 g, 4.17 mmol) was dissolved in tetrahydrofuran (10 mL), followed by the addition of a 1 M solution of borane tetrahydrofuran complex in tetrahydrofuran (10.4 mL, 10.40 mmol). The reaction mixture was heated to 60°C and stirred for 12 hours. The reaction mixture was cooled to room temperature, filtered, poured into water (100 mL) and extracted with dichloromethane (100 mL×3). The organic phases were combined, washed with saturated brine (50 mL), dried over anhydrous sodium sulfate and filtered. The filtrate was concentrated under reduced pressure, and the resulting residue was purified by column chromatography (SiO₂, dichloromethane/methanol=19/1) to obtain compound **53-1.** MS-ESI: *m*/*z* 321.9 [M+1]⁺.

### Synthesis of compound 53-2

It was prepared with reference to the synthesis of compound **1-3.** MS-ESI: *m*/*z* 445.1 [M+1]⁺.

### Synthesis of compound 53-3

It was prepared with reference to the synthesis of compound **1-4.** MS-ESI: *m*/*z* 430.0 [M+1]⁺.

### Synthesis of compound 53-4

It was prepared with reference to the synthesis of compound **1-5.** MS-ESI: *m*/*z* 412.1 [M+1]⁺.

### Synthesis of compound 53-5

It was prepared with reference to the synthesis of compound **1-6.** MS-ESI: *m*/*z* 312.1 [M+1]⁺.

### Synthesis of compound 53-6

It was prepared with reference to the synthesis of compound **1-7** with similar conditions except that *N,N*-dimethylformamide was replaced with dichloromethane. MS-ESI: *m*/*z* 439.4 [M-100+1]⁺.

### Synthesis of compound 53

It was prepared with reference to the synthesis of compound **1.** MS-ESI: *m*/*z* 439.4 [M+1]⁺. ¹H NMR (400 MHz, CDCl₃) δ 8.11 (d, 1H), 7.25 (d, 1H), 6.93-6.85 (m, 4H), 6.75-6.71 (m, 2H), 5.06-5.00 (m, 1H), 4.53 (s, 2H), 4.14 (d, 2H), 3.91-3.76 (m, 4H), 3.61-3.50 (m, 2H), 3.04-3.01 (m, 2H), 2.28-2.20 (m, 1H), 2.13-2.08 (m, 1H), 1.33 (br s, 2H), 1.26-1.22 (m, 1H), 1.09-1.06 (m, 1H).

### Example 54

### (S)-4-Amino-N-(1-cyano-2-(2-(4-fluorophenyl)isoindolin-5-yl)ethyl)tetrahydro-2H-pyran-4-carboxamide

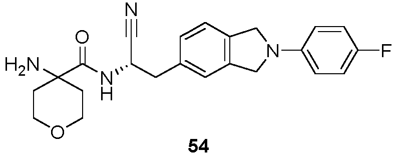

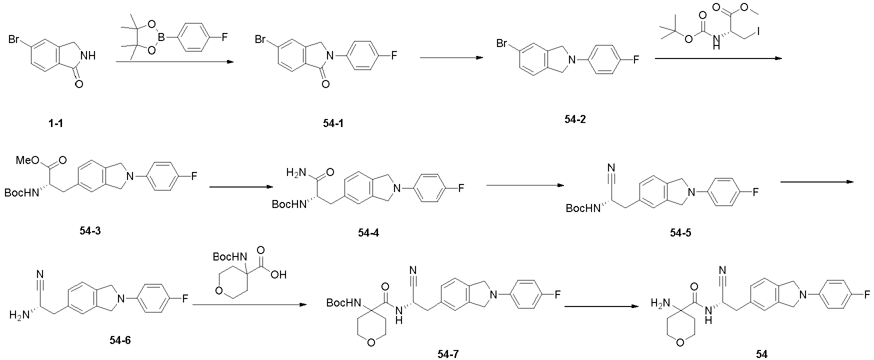

### Synthesis of compound 54-1

It was prepared with reference to the synthesis of compound **44-4.** MS-ESI: *m*/*z* 305.9 [M+1]⁺.

### Synthesis of compound 54-2

It was prepared with reference to the synthesis of compound **53-1** with similar conditions except that the reaction temperature was changed from 60°C to 70°C. MS-ESI: *m*/*z* 291.9 [M+1]⁺.

### Synthesis of compound 54-3

It was prepared with reference to the synthesis of compound **1-3.** MS-ESI: *m*/*z* 415.1 [M+1]⁺.

### Synthesis of compound 54-4

It was prepared with reference to the synthesis of compound **1-4.** MS-ESI: *m*/*z* 400.1 [M+1]⁺.

### Synthesis of compound 54-5

It was prepared with reference to the synthesis of compound **1-5.** MS-ESI: *m*/*z* 382.1 [M+1]⁺.

### Synthesis of compound 54-6

It was prepared with reference to the synthesis of compound **1-6.** MS-ESI: *m*/*z* 282.1 [M+1]⁺.

### Synthesis of compound 54-7

It was prepared with reference to the synthesis of compound **1-7** with similar conditions except that the solvent *N,N*-dimethylformamide was replaced with dichloromethane. MS-ESI: *m*/*z* 509.1 [M+1]⁺.

### Synthesis of compound 54

It was prepared with reference to the synthesis of compound **1.** MS-ESI: *m*/*z* 409.2 [M+1]⁺. ¹H NMR (400 MHz, DMSO-d6) δ 7.35-7.31 (m, 2H), 7.23 (d, 1H), 7.09 (t, 2H), 6.67-6.63 (m, 2H), 4.96-4.94 (m, 1H), 4.56-4.51 (m, 4H), 3.63-3.58 (m, 3H), 3.52-3.47 (m, 1H), 3.20-3.14 (m, 2H), 1.91-1.85 (m, 1H), 1.78-1.72 (m, 1H), 1.45-1.22 (m, 2H).

### Example 55

### (S)-4-Amino-N-(1-cyano-2-(2-(4-fluorobenzoyl)isoindolin-5-yl)ethyl)tetrahydro-2H-pyran-4-carboxamide

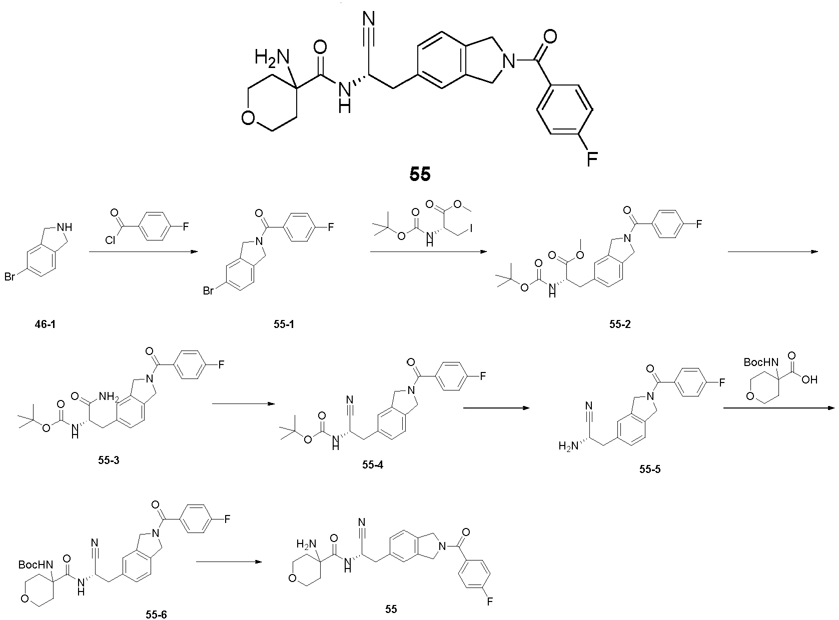

### Synthesis of compound 55-1

At room temperature, compound **46-1** (1.40 g, 7.1 mmol) was dissolved in dichloromethane (5 mL), followed by the addition of 4-fluorobenzoyl chloride (1.42 g, 8.99 mmol) and triethylamine (0.09 mL, 0.62 mmol). The reaction mixture was stirred at room temperature for 12 hours. Saturated aqueous sodium bicarbonate solution (20 mL) was added, and the reaction mixture was extracted with dichloromethane (20 mL×3). The organic phases were combined, washed with saturated brine (20 mL), dried over anhydrous sodium sulfate and filtered. The filtrate was concentrated under reduced pressure, and the residue was purified by column chromatography (SiO₂, dichloromethane/methanol=9/1) to obtain compound **55-1.** MS-ESI: *m*/*z* 319.9 [M+1]⁺.

### Synthesis of compound 55-2

It was prepared with reference to the synthesis of compound **1-3.** MS-ESI: *m*/*z* 343.0 [M-100+1]⁺.

### Synthesis of compound 55-3

It was prepared with reference to the synthesis of compound **1-4.** MS-ESI: *m*/*z* 428.1 [M+1]⁺.

### Synthesis of compound 55-4

It was prepared with reference to the synthesis of compound **1-5.** MS-ESI: *m*/*z* 410.3 [M+1]⁺.

### Synthesis of compound 55-5

It was prepared with reference to the synthesis of compound **1-6.** MS-ESI: *m*/*z* 310.2 [M+1]⁺.

### Synthesis of compound 55-6

It was prepared with reference to the synthesis of compound **1-7.** MS-ESI: *m*/*z* 437.4 [M-100+1]⁺.

### Synthesis of compound 55

It was prepared with reference to the synthesis of compound **1.** MS-ESI: *m*/*z* 437.4 [M+1]⁺. ¹H NMR (400 MHz, DMSO-*d6*) δ 7.71-7.68 (m, 2H), 7.36-7.25 (m, 3H), 7.21-7.18 (m, 2H), 4.93-4.90 (m, 1H), 4.82 (s, 2H), 4.75 (s, 2H), 3.69-3.60 (m, 3H), 3.53-3.48 (m, 1H), 3.18-3.12 (m, 2H), 1.88-1.81 (m, 1H), 1.80-1.73 (m, 1H), 1.23-1.12 (m, 2H).

### Example 56

### (S)-4-Amino-N-(1-cyano-2-(2-(4-fluorobenzoyl)-1,2,3,4-tetrahydroisoquinolin-6-yl)ethyl)tetrahydro-2H-pyran-4-carboxamide

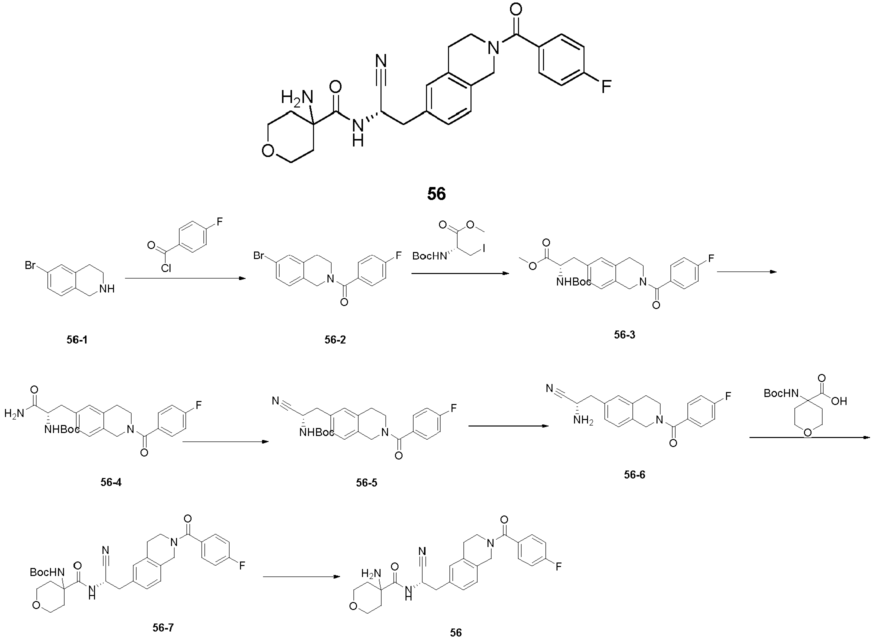

### Synthesis of compound 56-2

At room temperature, 6-bromo-1,2,3,4-tetrahydroisoquinoline (compound **56-1)** (2.50 g, 11.85 mmol) and 4-fluorobenzoyl chloride (1.90 g, 11.85 mmol) were dissolved in dichloromethane (50 mL), followed by the addition of N,N-diisopropylethylamine (3.05 g, 23.70 mmol). The reaction mixture was stirred at room temperature for 16 hours. Saturated aqueous sodium bicarbonate solution (50 mL) was added, and the reaction mixture was extracted with dichloromethane (200 mL×3). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **56-2.** MS-ESI: *m*/*z* 335.9 [M+1]⁺.

### Synthesis of compound 56-3

It was prepared with reference to the synthesis of compound **1-3.** MS-ESI: *m*/*z* 401.1 [M+1]⁺.

### Synthesis of compound 56-4

It was prepared with reference to the synthesis of compound **1-4.** MS-ESI: *m*/*z* 342.0 [M-100+1]⁺.

### Synthesis of compound 56-5

It was prepared with reference to the synthesis of compound **1-5.** MS-ESI: *m*/*z* 424.1 [M-100+1]⁺.

### Synthesis of compound 56-6

It was prepared with reference to the synthesis of compound **1-6.** MS-ESI: *m*/*z* 324.2 [M+1]⁺.

### Synthesis of compound 56-7

It was prepared with reference to the synthesis of compound **1-7** with similar conditions except that *N,N*-dimethylformamide was replaced with dichloromethane. MS-ESI: *m*/*z* 451.1 [M-100+1]⁺.

### Synthesis of compound 56

It was prepared with reference to the synthesis of compound **1.** MS-ESI: *m*/*z* 451.4 [M+1]⁺. ¹H NMR (400 MHz, DMSO-*d₆*) δ 7.52-7.50 (m, 2H), 7.32-7.27 (t, 2H), 7.20-7.09 (m, 3H), 6.05 (s, 2H), 4.94-4.90 (m, 1H), 4.72-4.55 (m, 2H), 3.83-3.78 (m, 1H), 3.65-3.51 (m, 5H), 3.20-3.09 (m, 2H), 2.82-2.80 (m, 2H), 1.92-1.85 (m, 1H), 1.77-1.72 (m, 1H), 1.24-1.14 (m, 2H).

### Example 57

### (S)-4-Amino-N-(1-cyano-2-(2-(4-fluorophenyl)-1,2,3,4-tetrahydroisoquinolin-6-yl)ethyl)tetrahydro-2H-pyran-4-carboxamide

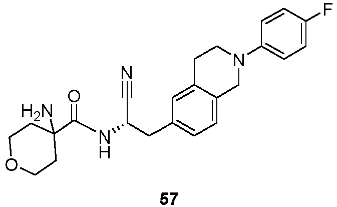

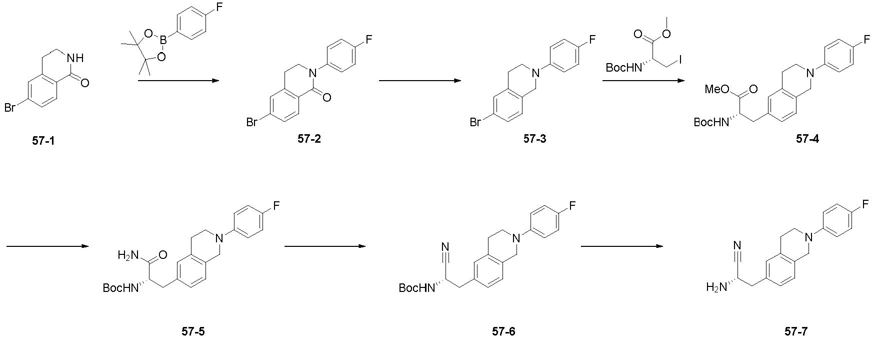

### Synthesis of compound 57-2

It was prepared with reference to the synthesis of compound **44-4** with similar conditions except that the reaction temperature was changed from 120°C to 110°C. MS-ESI: *m*/*z* 321.9 [M+1]⁺.

### Synthesis of compound 57-3

It was prepared with reference to the synthesis of compound **53-1** with similar conditions except that the reaction temperature was changed from 60°C to 70°C.

### Synthesis of compound 57-4

It was prepared with reference to the synthesis of compound **1-3.** MS-ESI: *m*/*z* 373.3 [M-56+1]⁺.

### Synthesis of compound 57-5

It was prepared with reference to the synthesis of compound **1-4.** MS-ESI: *m*/*z* 414.1 [M+1]⁺.

### Synthesis of compound 57-6

It was prepared with reference to the synthesis of compound **1-5.** MS-ESI: *m*/*z* 396.1 [M+1]⁺.

### Synthesis of compound 57-7

It was prepared with reference to the synthesis of compound **1-6.** MS-ESI: *m*/*z* 296.3 [M+1]⁺.

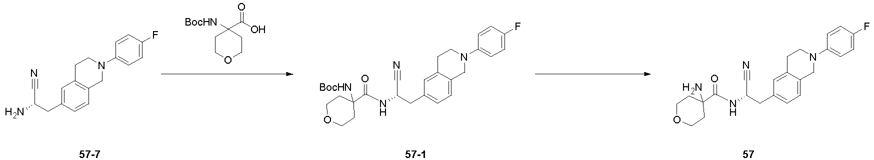

### Synthesis of compound 57-1

It was prepared with reference to the synthesis of compound **1-7** with similar conditions except that *N,N*-dimethylformamide was replaced with dichloromethane. MS-ESI: *m*/*z* 523.5 [M+1]⁺.

### Synthesis of compound 57

It was prepared with reference to the synthesis of compound **1.** MS-ESI: *m*/*z* 423.4 [M+1]⁺. ¹H NMR (400 MHz, CD₃OD) δ 7.17-7.07 (m, 3H), 7.06-6.94 (m, 4H), 5.01 (t, 1H), 4.28 (s, 2H), 3.72-3.66 (m, 3H), 3.65-3.55 (m, 1H), 3.50-3.40 (m, 2H), 3.22-3.06 (m, 2H), 2.95 (t, 2H), 2.08-2.00 (m, 1H), 1.98-1.88 (m, 1H), 1.33-1.20 (m, 2H).

### Example 58

### (S)-1-Amino-N-(1-cyano-2-(2-(4-fluorophenyl)-3-oxo-1,2,3,4-tetrahydroisoquinolin-6-yl)ethyl)cyclohexane-1-carboxamide

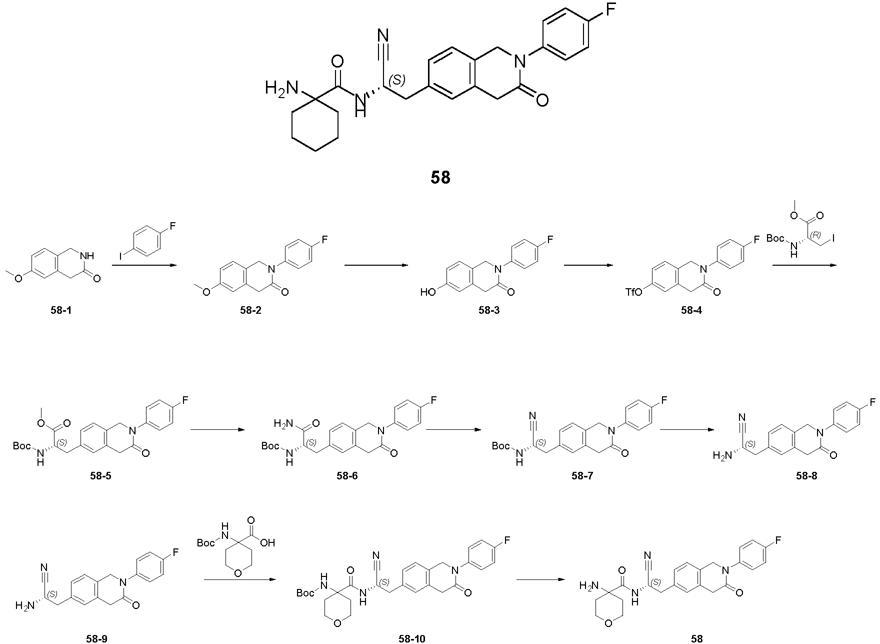

### Synthesis of compound 58-2

At room temperature, 6-methoxy-1,4-dihydroisoquinolin-3(2*H*)-one (compound **58-1)** (5 g, 28.22 mmol), *p*-fluoroiodobenzene (6.90 g, 31.04 mmol), cuprous iodide (536 mg, 2.82 mmol), *trans-N,N'*-dimethyl-1,2-cyclohexanediamine (0.32 g, 2.82 mmol) and potassium phosphate (16.24 g, 70.54 mmol) were dissolved in *N,N*-dimethylformamide (80 mL). The reaction mixture was heated to 100°C and stirred for 3 hours. After the reaction was complete, the reaction mixture was cooled to room temperature, followed by the addition of water (200 mL) and extracted with ethyl acetate (100 mL×2). The organic phases were combined, washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=9/1) to obtain compound **58-2.** MS-ESI: *m*/*z* 272.0 [M+1]⁺.

### Synthesis of compound 58-3

At room temperature, compound **58-2** (5 g, 17.69 mmol) was dissolved in dichloromethane (50 mL). At 0°C, a solution of boron tribromide in dichloromethane (26.54 mL, 26.54 mmol, 1 mol/L) was added dropwise, and the reaction mixture was stirred at 0°C for 12 hours. The reaction mixture was poured into water (100 mL), and extracted with dichloromethane (100 mL×2). The organic phase was washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=1/1) to obtain compound **58-3.** MS-ESI: *m*/*z* 257.9 [M+1]⁺.

### Synthesis of compound 58-4

At room temperature, compound **58-3** (3.70 g, 11.22 mmol) and triethylamine (3.41 g, 33.66 mmol) were dissolved in *N,N*-dimethylformamide (30 mL), followed by the addition of *N*-phenylbis(trifluoromethanesulfonimide) (4.81 g, 13.46 mmol) in batches at 0°C. The reaction mixture was stirred at 0°C for 20 minutes. After the reaction was complete, the reaction mixture was poured into water (100 mL), and extracted with dichloromethane (100 mL×2). The organic phase was washed with saturated brine (100 mL), dried over anhydrous sodium sulfate and filtered. The filtrate was concentrated under reduced pressure, and the resulting residue was purified by column chromatography (SiO₂, petroleum ether/ethyl acetate=4/1) to obtain compound **58-4.** MS-ESI: *m*/*z* 389.9 [M+1]⁺.

### Synthesis of compound 58-5

It was prepared with reference to the synthesis of compound **14-B7.** MS-ESI: *m*/*z* 443.1 [M+1]⁺.

### Synthesis of compound 58-6

At room temperature, compound **58-5** (4.4 g, 9.55 mmol) was dissolved in a 8 M solution of ammonia in methanol (50 mL), and the reaction mixture was heated to 100°C and stirred for 12 hours. The reaction mixture was cooled to room temperature and concentrated under reduced pressure. The resulting residue was triturated in dichloromethane (30 mL) to obtain compound **58-6.** MS-ESI: *m*/*z* 428.1 [M+1]⁺.

### Synthesis of compound 58-7

It was prepared with reference to the synthesis of compound **14-B9** with similar conditions except that tetrahydrofuran was replaced with dichloromethane. MS-ESI: *m*/*z* 410.1 [M+1]⁺.

### Synthesis of compound 58-8

It was prepared with reference to the synthesis of compound **14.** MS-ESI: *m*/*z* 310.1 [M+1]⁺.

### Synthesis of compound 58-9

It was prepared with reference to the synthesis of compound **14-A7.** MS-ESI: *m*/*z* 481.1 [M-56+1]⁺.

### Synthesis of compound 58

It was prepared with reference to the synthesis of compound **14** with similar conditions except that the reaction temperature was changed from 40°C to 30°C. MS-ESI: *m*/*z* 437.3 [M+1]⁺. ¹H NMR (400 MHz, CDCl₃) δ 8.26 (d, 1H), 8.12 (d, 1H), 7.38-7.30 (m, 2H), 7.24-7.21 (m, 2H), 7.13-7.08 (m, 2H), 5.17-5.08 (m, 1H), 4.00-3.85 (m, 4H), 3.67-3.56 (m, 2H), 3.19-3.12 (m, 4H), 2.34-2.15 (m, 2H), 1.45 (br, 2H), 1.31-1.18 (m, 2H).

### Biological Assay

The present disclosure is further described and explained by the following test examples, but these test examples are not intended to limit the scope of the present disclosure.

### Test Example 1 In vitro enzyme activity experiment of rhCatC

### 1. Experimental materials

| **Name** | **Brand** | **Item No./Model** |
|---|---|---|
| rhCatC | Sinobio | 10484-H08H-20 |
| rhCatL | R&D | 952-CY-010 |
| H-Gly-Arg-AMC | Lai' ang | Leonbio01 |
| AZD7986 (alias: INS 1007) | MedChemExpress | HY-101056 |
| OptiPlate^{™}-384 F black-bottom analysis plate | PerkinElmer | 6007279 |
| 384-well echo plate | Labcyte | PP-0200 |
| Activation buffer | 25 mM MES, 5 mM DTT, pH 6.0 | |
| Reaction buffer | 25 mM MES, 50 mM NaCl, 5 mM DTT, pH 6.0 | |

### Control compound AZD7986

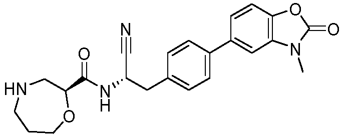

The control compound can be obtained with reference to the preparation method in CN105980367.

### 2. Experimental procedures

The *in vitro* inhibitory effect of compounds and positive control on rhCatC enzyme activity was evaluated by AMC fluorescence assay. rhCatC can catalyze the reaction of substrate peptide H-Gly-Arg-AMC, and release AMC to generate fluorescence. The assay was carried out in a black-bottom 384-well plate, the initial maximum concentration was usually 30 µM, and the enzyme activity was tested at eight concentrations by semilogarithmic dilution.
1) Preparation of test compound plate: The test compounds and positive control AZD7986 were dissolved in 100% DMSO to obtain compound stock solutions with a final concentration of 10 mM. The compound was diluted with DMSO to a 384-well echo plate at a concentration of 100X of the highest concentration at the beginning of the experiment. 0.2 µL of the diluted compound was pipetted to a black-bottomed 384-well reaction plate for later use. The negative control well was DMSO.
2) Activation of rhCatC: RhCatC and rhCatL were added to the activation buffer to obtain a final concentration of rhCatC of 100 µg/mL and a final concentration of rhCatL of 20 µg/mL, and incubated at room temperature for 1 hour.
3) Enzyme activity reaction: Activated rhCatC was diluted to 0.4 µg/mL with the reaction buffer. The solution was added to the black-bottom 384-well reaction plate (10 µL per well). 10 µL of the buffer was added for the vehicle control group. The plate containing compounds in its wells was incubated at room temperature for 30 minutes. H-Gly-Arg-AMC was diluted to 97 µM with reaction buffer, and 10 µL of the solution was added to each well.
4) Fluorescence detection: The plate was read with Envision, and the fluorescence intensity was measured at Ex 355 nM and Em 460 nM.
5) Calculation of inhibition rate and IC₅₀: Inhibition rate: formula (1): inhibition rate % = (maximum value - signal value)/(maximum value - minimum value) × 100, wherein the maximum value refers to the reading value of the DMSO control, and the minimum value refers to the reading value of the control without enzyme activity; IC₅₀ value: formula (2): Y = bottom platform value + (top platform value - bottom platform value)/(1 + (IC₅₀/X) × HillSlope), wherein Y is % inhibition rate, and X is compound concentration.

The RLU data was read, the inhibition rate was calculated, and the IC₅₀ value was calculated according to the fitting curve of concentration and inhibition rate.

The *in vitro* inhibitory activity of the compounds of the present disclosure on CatC enzyme activity was determined through the above test, and the IC₅₀ values obtained are shown in Table **1.**

**Table 1**

| Compound | Biological activity IC₅₀ (nM) |
|---|---|
| Compound **1** | 42 |
| Compound **2** | 710 |
| Compound **3** | 4254 |
| Compound **4** | 54 |
| Compound **5** | 3586 |
| Compound **6** | 56 |
| Compound **7** | 6.8 |
| Compound **8** | 60 |
| Compound **9** | 1744 |
| Compound **10** | 74 |
| Compound **11** | 74 |
| Compound **12** | 16 |
| Compound **13** | 37 |
| Compound **14** | 5.31 |
| Compound **15** | 304 |
| Compound **16** | 91 |
| Compound **17** | 5.7 |
| Compound **18** | 49 |
| Compound **19** | 32 |
| Compound **20** | 11 |
| Compound **22** | 3.2 |
| Compound **23** | 14.5 |
| Compound **24** | 2.7 |
| Compound **25** | 3.1 |
| Compound **26** | 8.9 |
| Compound **27** | 23 |
| Compound **29** | 17.1 |
| Compound **30** | 10 |
| Compound **32** | 13 |
| Compound **33** | 85 |
| Compound **34** | 6.2 |
| Compound **35** | 15 |
| Compound **36** | 7.1 |
| Compound **37** | 14 |
| Compound **38A** | 1.3 |
| Compound **38B** | 76 |
| Compound **39** | 46 |
| Compound **40** | 3.7 |
| Compound **41** | 30.5 |
| Compound **42** | 5.7 |
| Compound **43** | 26 |
| Compound **44** | 39 |
| Compound **45** | 72 |
| Compound **46** | 54 |
| Compound **47** | 29 |
| Compound **48** | 29 |
| Compound **49** | 57 |
| Compound **50** | 19 |
| Compound **51** | 39 |
| Compound **52** | 138 |
| Compound **53** | 196 |
| Compound **54** | 28 |
| Compound **55** | 174 |
| Compound **56** | 37 |
| Compound **57** | 48 |
| Compound **58** | 17 |
| AZD7986 | 7.69 (n=35) |

### Test Example 2 In vitro CatC cell activity experiment

### 1. Experimental materials

| **Name** | **Brand** | **Item No./Model** |
|---|---|---|
| U937 | ATCC | CRL-1593.2 |
| H-Gly-Phe-AMC | Lai' ang | P201221-K3 |
| AZD7986 (alias: INS 1007) | MedChemExpress | HY-101056 |
| OptiPlate^{™}-384 F black-bottom analysis plate | PerkinElmer | 6007279 |
| RPMI1640 culture medium + 10% FBS | Gbico | |

### 2. Experimental procedures

Complete culture medium RPMI 1640+10% FBS was formulated and mixed well. The U937 cell line was resuscitated and passed for about two generations, and the cell line with good growth status was selected. The cell suspension was transferred into a centrifuge tube, and centrifuged at 800 to 1000 rpm for 3 to 5 minutes. The supernatant was discarded. An appropriate volume of culture medium was added to the centrifuge tube, which was gently pipetted to resuspend the cells evenly. The cells were counted using a Vi-Cell XR cell counter. The cell suspension was adjusted to an appropriate concentration. The cell suspension was added into a 384-well plate (25000 µL/well). The compound was formulated into a 10 mM solution with DMSO. The compound was diluted with DMSO to obtain a 1 mM solution, and a semi-logarithmi dilution was carried out by HPD300 with DMSO to obtain 10 concentrations. Gly-Phe-AFC was formulated into a 35 mM solution with DMSO and divided. Gly-Phe-AFC was formulated to 1.75 mM with serum-free culture medium. After adding the compounds and incubating for 1 hour in an incubator, substrate-AFC was added (12.5 µL was added to the plate). The plate was incubated for 30 minutes and read. The plate was read by the EnSpire, and the fluorescence values were measured at Ex 400 nm and Em 505 nm. The inhibition rate was calculated according to the following formula: inhibition rate (%) = (1 - (RFU compound - RFU blank)/(RFU DMSO - RFU blank)) × 100%. XLFit was used to draw the drug efficacy inhibition rate curve and calculate the IC₅₀ value. 4-parameter model [fit = (A + ((B - A)/ (1 + ((C/x) ^D))))] was used.

The *in vitro* inhibitory activity of the compounds of the present disclosure on CatC cell activity was determined through the above test, and the IC₅₀ values obtained are shown in Table **2.**

**Table 2**

| Compound | Biological activity IC₅₀ (nM) |
|---|---|
| Compound **7** | 10.2 |
| Compound **8** | 220 |
| Compound **12** | 22.99 |
| Compound **13** | 87.6 |
| Compound **14** | 6.1 |
| Compound **16** | 186.4 |
| Compound **17** | 6.2 |
| Compound **18** | 29.5 |
| Compound **20** | 7.8 |
| Compound **21** | 24.6 |
| Compound **22** | 5.5 |
| Compound **23** | 8.3 |
| Compound **24** | 4.3 |
| Compound **25** | 5.52 |
| Compound **26** | 6.7 |
| Compound **28** | 50.1 |
| Compound **29** | 36.8 |
| Compound **30** | 9.6 |
| Compound **31** | 38.4 |
| Compound **32** | 7.0 |
| Compound **35** | 37.9 |
| Compound **36** | 23.7 |
| Compound **37** | 8.6 |
| Compound **40** | 10.3 |
| Compound **41** | 117.3 |
| Compound **58** | 99.35 |
| AZD7986 | 12.21 (n=15) |

### Test Example 3 Pharmacokinetic experiment of the compounds in BALB/c mice

### 1. Experimental materials:

BALB/c mice (male, 20 to 30 g, fasting)

### 2. Experimental procedures:

Pharmacokinetic characteristics of the compounds in rodent after intravenous or oral administration were determined by standard protocols. In the experiment, the compound was formulated into a clear solution, and administered to mice by single intravenous injection or oral administration. The intravenous injection vehicle was a mixed vehicle of 5% dimethyl sulfoxide and 95% (30% aqueous sulfobutylether cyclodextrin solution). The oral vehicle is a solution of 0.5% hydroxypropylmethylcellulose and 0.1% polysorbate 80 in water. Whole blood samples within 24 hours were collected, and the supernatants were separated to obtain plasma samples. Quantitative analysis of plasma concentration was carried out by LC-MS/MS analysis method, and the pharmacokinetic parameters, such as peak concentration (Cₘₐₓ), clearance (Cl), half-life (T_{1/2}), volume of distribution (Vdₛₛ) and area under the drug-time curve (AUC), were calculated.

The parameters determined in the pharmacokinetic experiment of the compounds of the present disclosure in BALB/c mice are shown in Table **3.**

**Table 3**

| Administration method | PK parameters | Compound **14*** | **AZD7986*** |
|---|---|---|---|
| IV | AUC₀₋ₗₐₛₜ (ng.h/mL) | 3769 | 2121 |
| | Cl (mL/min/kg) | 8.49 | 15.1 |
| | Vdₛₛ (L/kg) | 1.75 | 2.34 |
| | T _{1/2} (h) | 3.08 | 2.74 |
| PO | AUC₀₋ₗₐₛₜ (ng.h/mL) | 6613 | 2132 |
| | Cₘₐₓ | 1336 | 525 |
| | T _{1/2} (h) | 2.69 | 3.18 |
| | Tₘₐₓ (h) | 0.5 | 1.50 |
| | F% | 65.3 | 40.2 |

| | | | |
|---|---|---|---|
| * In the PK assay of the two compounds, the test dose was 2 mg/kg iv, 5 mg/kg po; three animals per group. | | | |

It can be seen from the PK data that compared with AZD7986, the oral exposure of compound **14** increased by 3.1 times, the intravenous exposure increased by 1.8 times, the clearance rate decreased by about 1.8 times, and the oral bioavailability increased. In summary, compound **14** has better PK properties than AZD7986 in BALB/c mice.

### Test Example 4 Pharmacokinetic experiment of the compounds in beagle dogs

### 1. Experimental materials:

Beagle dogs (male, 9 to 11 kg)

### 2. Experimental procedures:

Pharmacokinetic characteristics of the compounds in beagle dogs after intravenous or oral administration were determined by standard protocols. In the experiment, the compound was formulated into a clear solution, and administered to beagle dogs by single intravenous injection or oral administration. The intravenous injection vehicle was a mixed vehicle of 5% dimethyl sulfoxide and 95% (28% aqueous hydroxypropyl beta cyclodextrin solution). The oral vehicle is a solution of 0.5% hydroxypropylmethylcellulose and 0.1% polysorbate 80 in water. Whole blood samples within 24 hours were collected, and the supernatants were separated to obtain plasma samples. The sample was centrifuged to obtain the supernatant, to which an equal volume of water was added. The resulting solution was centrifuged to obtain the supernatant, which was injected into the analysis machine. Quantitative analysis of plasma concentration was carried out by LC-MS/MS analysis method, and the pharmacokinetic parameters, such as peak concentration (Cₘₐₓ), clearance (Cl), half-life (T_{1/2}), volume of distribution (Vdₛₛ) and area under the drug-time curve (AUC), were calculated.

The parameters determined in the pharmacokinetic experiment of the compounds of the present disclosure in beagle dogs are shown in Table **4.**

**Table 4**

| Administration method | PK parameters | Compound **14*** | **AZD7986*** |
|---|---|---|---|
| IV | AUC₀₋ₗₐₛₜ (ng.h/mL) | 4792 | 1408 |
| | Cl (L/hr/kg) | 0.172 | 0.521 |
| | Vdₛₛ (L/kg) | 2.29 | 9.26 |
| | T _{1/2} (h) | 10.2 | 13.5 |
| PO | AUC₀₋ₗₐₛₜ (ng.h/mL) | 10864 | 3669 |
| | Cₘₐₓ | 577 | 197 |
| | T _{1/2} (h) | 12.3 | 15.3 |
| | Tₘₐₓ (h) | 3.33 | 1.67 |
| | F% | 100 | 104 |

| | | | |
|---|---|---|---|
| * In the PK assay of the two compounds, the test dose was 1 mg/kg iv, 2 mg/kg po; three animals per group. | | | |

It can be seen from the PK data that compared with AZD7986, the oral exposure of compound **14** increased by 3.0 times, the intravenous exposure increased by 3.4 times, the clearance rate decreased by about 3 times. In summary, compound **14** has better PK properties than AZD7986 in beagle dogs.

### Test Example 5 Target binding model in C57BL/6 mice

1. Experimental protocol: In order to investigate the inhibitory effect of the compounds on the activity of neutrophil elastase (hereinafter referred to as NE) downstream of CatC in neutrophil, female C57BL/6 mice were administered according to the following table:

| Group | Number of mouse | Compound | Administration dose | Administration route | Administration time |
|---|---|---|---|---|---|
| 1 | 5 | Vehicle control | - | PO | On Day 1 to 7, BID, on Day 8, QD |
| 2 | 5 | AZD7986 | 3 mp/kg | PO | On Day 1 to 7, BID, on Day 8, QD |
| 3 | 5 | Compound | 3 mp/kg | PO | On Day 1 to 7, BID, on Day 8, QD |

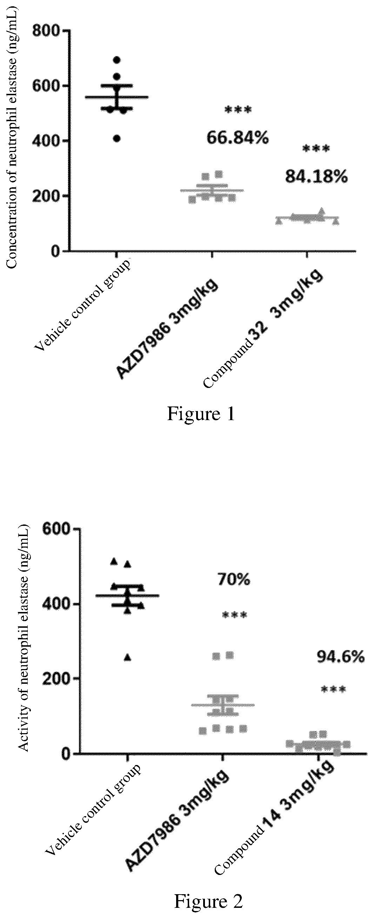

Compound vehicle: 0.5% HPMC, 0.1% Tween-80, pH 3.0, 0.1 M citrate buffer.
2. Experimental procedures: The compound or vehicle control was administered continuously for 8 days. On Day 1 to 7, the compound or vehicle control was administered twice a day with an administration interval of 6 hours. On Day 8, the test material was collected one hour after the first administration. In order to determine the NE protease activity in bone marrow, femurs and tibias of mice were collected, and immediately rinsed with pre-cooled RPMI1640 culture medium to obtain bone marrow cells. The sample was centrifuged at 400 g for 10 minutes, and washed once with pre-cooled Dulbecco's phosphate buffer. Erythrocyte lysis was carried out for the bone marrow cell sample. For each sample, 5 mL of erythrocyte lysate (BD #555899) was used, and the sample was incubated at room temperature for 15 minutes. The sample was centrifuged at 200 g for 5 minutes, and the supernatant was discarded. The sample was washed once with Dulbecco's phosphate buffer, centrifuged at 400 g for 5 minutes and counted. Cell lysate (50 mM Tris hydrochloride buffer, 500 mM sodium chloride solution, 1% Triton X-100 pH 7.5) was added at a cell density of 5e7 cells/mL, and the sample was incubated on ice for 15 minutes. The sample was centrifuged at 10,000 g at 4°C for 5 minutes to obtain the supernatant. The supernatant was stored at -80°C until protease activity was determined. The protease activity in the cell lysates of each group was determined using the NE protease activity detection kit (Sigma-Aldrich MAK246). 7.5 µL of 3x diluted bone marrow cell lysate or 7.5 µL of double-diluted NE standard and 7.5 µL of 25x diluted substrate were added to a 384-well plate, and the NE protease activity was immediately monitored in real-time at Ex 380 nm and Em 500 nm for 30 minutes. The RFU within the linear reaction time was determined, and the NE protease activity (ng/ml) in the sample was calibrated by the standard curve. The percentage inhibition rate was calculated as (vehicle control group - compound group)/vehicle control group × 100%.
3. Experimental conclusions: The percentage inhibition rate was shown in Figure 1 and Figure 2.

At a dose of 3 mg/kg, compound **14** and compound **32** both showed a higher inhibitory rate of NE protease activity than the reference compound AZD7986 in the mouse pharmacodynamic model.

## Claims

1. A compound of formula VI-a or a pharmaceutically acceptable salt thereof, 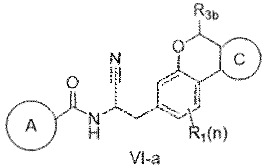
ring C is phenyl, and the phenyl is optionally substituted by one to three R_{3b};
ring A is selected from the group consisting of 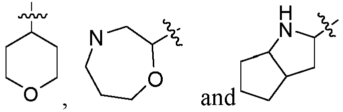 optionally substituted by one or more substituent(s) selected from the group consisting of deuterium, halogen, hydroxy, cyano, amino, nitro, oxo, C₁₋₆ alkyl and C₁₋₆ alkoxy; the C₁₋₆ alkyl and C₁₋₆ alkoxy are each independently optionally substituted by one to three R₃ₐ;
preferably, ring A is 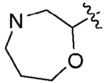
optionally substituted by one or more substituent(s) selected from the group consisting of deuterium, halogen, hydroxy, cyano, amino, nitro, oxo, C₁₋₆ alkyl and C₁₋₆ alkoxy; the C₁₋₆ alkyl and C₁₋₆ alkoxy are each independently optionally substituted by one to three R₃ₐ;
each R₃ₐ is independently selected from the group consisting of hydrogen, halogen, deuterium, hydroxy, oxo, nitro, cyano, amino, acyl, amido, C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl, and the C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl are each independently optionally substituted by one or more substituent(s) selected from the group consisting of halogen, deuterium, hydroxy, oxo, nitro and cyano;
each R_{3b} is independently selected from the group consisting of hydrogen, halogen, deuterium, hydroxy, oxo, nitro, cyano, amino, acyl, amido, C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl, 3- to 6-membered heteroaryl, methanesulfonyl and 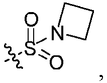 and the C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl are each independently optionally substituted by one or more substituent(s) selected from the group consisting of halogen, deuterium, hydroxy, oxo, nitro and cyano;
each R₁ is independently selected from the group consisting of halogen, hydroxy, oxo, nitro, cyano, alkyl, cycloalkyl, amino, amido, acyl, alkoxy, alkenyloxy, alkynyloxy and cycloalkoxy; and
n is an integer selected from the group consisting of 0 to 3; preferably, n is an integer selected from the group consisting of 1 to 3 or n is an integer selected from the group consisting of 0 to 2; and more preferably, n is an integer selected from the group consisting of 1 to 2;
the heteroatom is selected from the group consisting of nitrogen atom, oxygen atom and sulfur atom, and preferably nitrogen atom or oxygen atom.

2. The compound or the pharmaceutically acceptable salt thereof according to claim 1, wherein ring A is 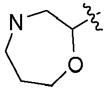 optionally substituted by one or more substituent(s) selected from the group consisting of deuterium, halogen, hydroxy, cyano, amino and nitro;
preferably, ring A is 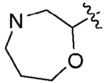 optionally substituted by one or more substituent(s) selected from the group consisting of deuterium and halogen;
and more preferably, ring A is 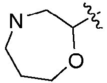 optionally substituted by one or more halogen(s);
and most preferably, ring A is

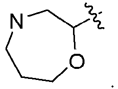

3. The compound or the pharmaceutically acceptable salt thereof according to claim 1, wherein the compound of formula VI-a is selected from the group consisting of 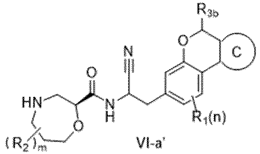 wherein,
each R₂ is independently selected from the group consisting of halogen, nitro, cyano, amino, oxo and hydroxy;
m is an integer selected from the group consisting of 0 to 3, and preferably, m is 0; and
R₁, n, ring C and R_{3b} are as defined in claim 1;
in particular,
the compound of formula VI-a is selected from the group consisting of 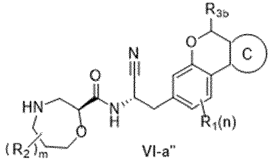
wherein,
each R₂ is independently selected from the group consisting of halogen, nitro, cyano, amino, oxo and hydroxy;
m is an integer selected from the group consisting of 0 to 3, and preferably, m is 0; and
R₁, n, ring C and R_{3b} are as defined in claim 1.

4. The compound or the pharmaceutically acceptable salt thereof according to any one of claims 1 to 3, wherein R₁ is selected from the group consisting of halogen, hydroxy, cyano, amino, nitro, acyl, amido, C₁₋₆ alkyl, C₃₋₆ cycloalkyl and C₁₋₆ alkoxy; preferably, R₁ is selected from the group consisting of halogen, hydroxy, cyano, amino, nitro, C₁₋₆ alkyl, C₃₋₆ cycloalkyl and C₁₋₆ alkoxy;
more preferably, R₁ is selected from the group consisting of halogen, hydroxy, C₁₋₆ alkyl, C₃₋₆ cycloalkyl, nitro and cyano;
even more preferably, R₁ is halogen;
and still even more preferably, R₁ is fluorine.

5. The compound or the pharmaceutically acceptable salt thereof according to any one of claims 1, 3 to 4, wherein each R₃ₐ is independently selected from the group consisting of hydrogen, halogen, deuterium, hydroxy, oxo, nitro, cyano and amino;
or
each R₃ₐ is independently selected from the group consisting of hydrogen, fluorine, chlorine, deuterium, oxo (=O), hydroxy, amino, methoxy, cyclopropoxy, cyclopropyl, cyclopentyl, pyridinyl, piperidinyl and phenyl, and preferably, R₃ₐ is hydrogen or amino.

6. The compound or the pharmaceutically acceptable salt thereof according to any one of claims 1 to 5, wherein,
each R_{3b} is independently selected from the group consisting of hydrogen, halogen, deuterium, hydroxy, oxo, nitro, cyano, amino, acyl, amido, C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl, and the C₁₋₆ alkyl, C₁₋₆ alkoxy, C₂₋₆ alkenyloxy, C₂₋₆ alkynyloxy, C₃₋₆ cycloalkyl, 3- to 6-membered heterocycloalkyl, C₃₋₆ cycloalkoxy, 3- to 6-membered heterocycloalkoxy, C₃₋₈ cycloalkenyloxy, 5- to 6-membered aryl and 3- to 6-membered heteroaryl are each independently optionally substituted by one or more substituent(s) selected from the group consisting of halogen, deuterium, hydroxy, oxo, nitro and cyano; and preferably, each R_{3b} is independently selected from the group consisting of hydrogen, halogen, deuterium, hydroxy, oxo, nitro, cyano and amino;
or
each R_{3b} is independently selected from the group consisting of hydrogen, halogen, deuterium, hydroxy, oxo, nitro, cyano, amino, acyl and amido; and preferably, each R_{3b} is independently halogen or cyano;
or
each R_{3b} is independently a 3- to 6-membered heterocycloalkyl, and the 3- to 6-membered heterocycloalkyl is optionally substituted by one to three substituent(s) selected from the group consisting of halogen, cyano and hydroxy;
or
each R_{3b} is independently selected from the group consisting of hydrogen, halogen, deuterium, cyano, 3- to 6-membered heterocycloalkyl, methanesulfonyl and 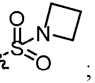 and preferably, the 3- to 6-membered heterocycloalkyl is selected from the group consisting of 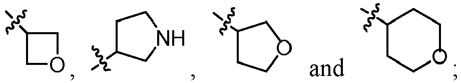 or
each R_{3b} is independently selected from the group consisting of hydrogen, halogen, deuterium, hydroxy, oxo, nitro, cyano, amino, amido, acetyl, methanesulfonyl, 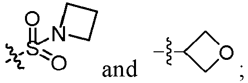 or
each R_{3b} is independently selected from the group consisting of cyano, methanesulfonyl and 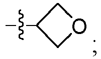 and preferably, R_{3b} is cyano;
or
each R_{3b} is independently a C₁₋₆ alkyl or C₁₋₆ alkoxy, and the C₁₋₆ alkyl or C₁₋₆ alkoxy is optionally substituted by one or more substituent(s) selected from the group consisting of halogen, deuterium, hydroxy, oxo, nitro and cyano;
or
each R_{3b} is independently selected from the group consisting of C₁₋₆ alkoxy, C₃₋₆ cycloalkoxy, 3- to 6-membered heterocycloalkoxy, phenyl and 5- to 6-membered heteroaryl, and the C₁₋₆ alkoxy, C₃₋₆ cycloalkoxy, 3- to 6-membered heterocycloalkoxy, phenyl and 5- to 6-membered heteroaryl are each independently optionally substituted by one to three substituent(s) selected from the group consisting of fluorine, chlorine, deuterium, hydroxy, oxo, nitro and cyano;
or
each R_{3b} is independently selected from the group consisting of hydrogen, fluorine, chlorine, deuterium, oxo (=O), hydroxy, amino, methoxy, cyclopropoxy, cyclopropyl, cyclopentyl, pyridinyl, piperidinyl and phenyl, and preferably, R_{3b} is selected from the group consisting of hydrogen, methyl, oxo, fluorine and chlorine.

7. The compound or the pharmaceutically acceptable salt thereof according to claim 1, wherein the compound of formula VI-a is selected from the group consisting of 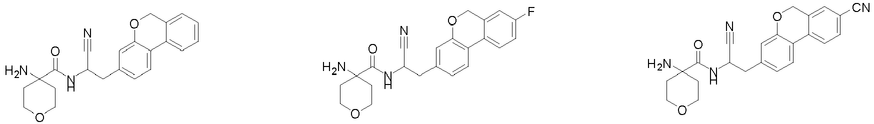 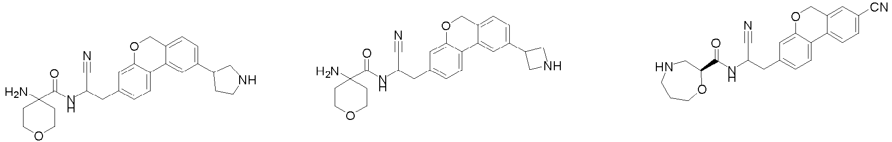 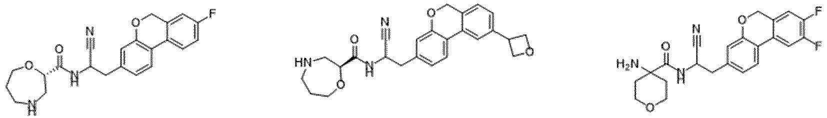 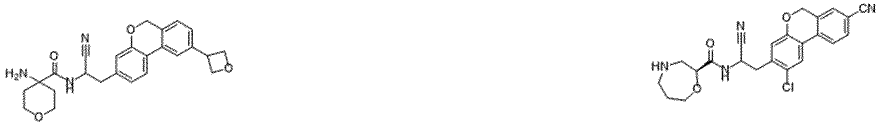 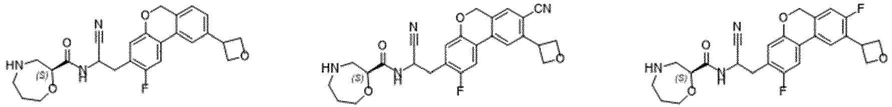 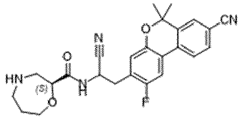 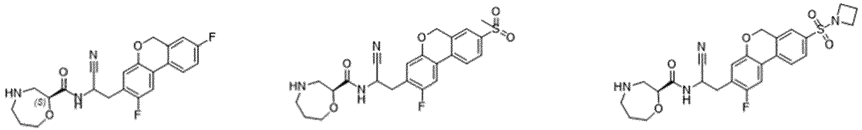 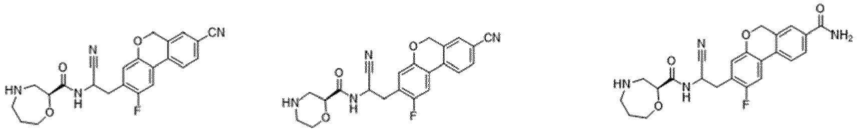 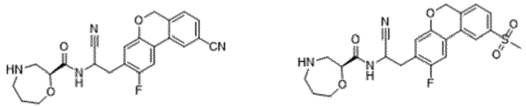 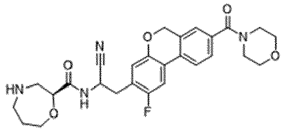 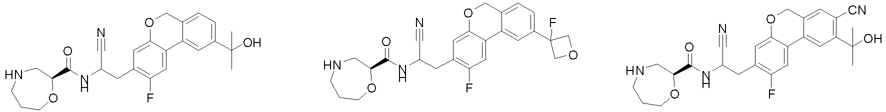 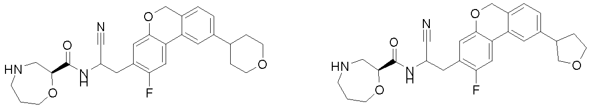 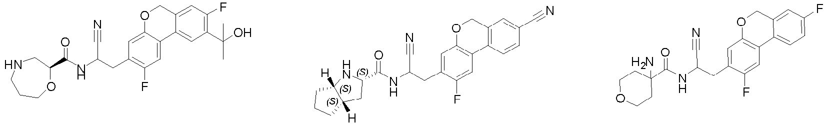 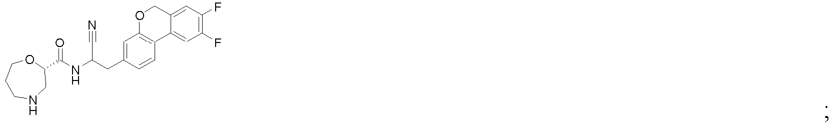 Preferably

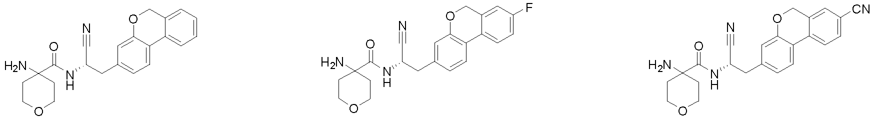

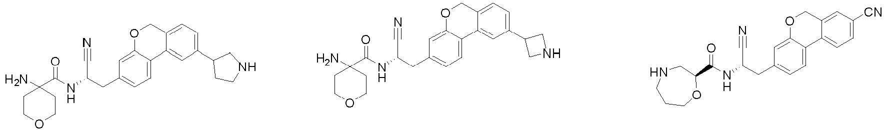

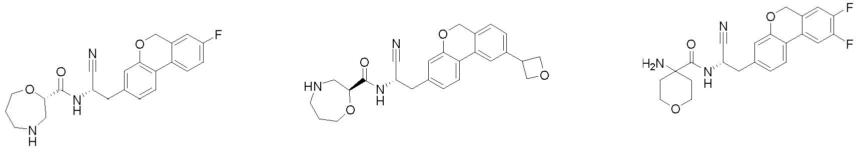

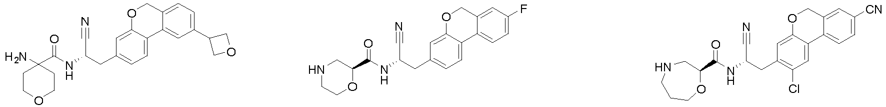

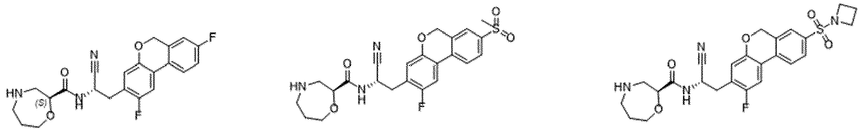

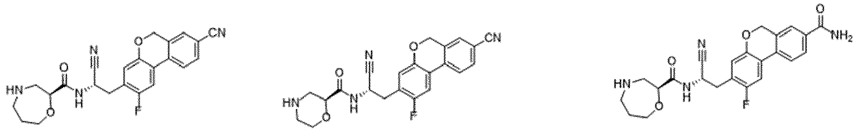

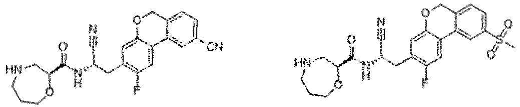

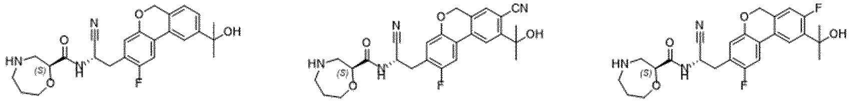

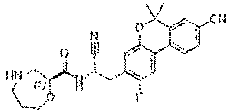

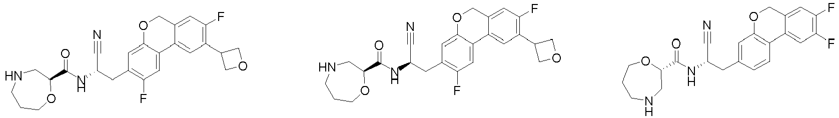

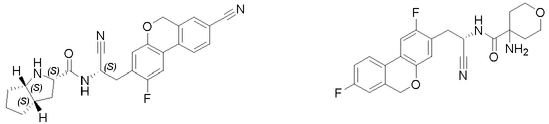

8. An isotope substitute of the compound or the pharmaceutically acceptable salt thereof according to any one of claims 1 to 7, and preferably, the isotope substitute is a deuterium atom substitute.

9. A pharmaceutical composition, comprising a therapeutically effective amount of at least one compound or pharmaceutically acceptable salt thereof according to any one of claims 1 to 7 or the isotope substitute according to claim 8, and a pharmaceutically acceptable excipient.

10. The compound or the pharmaceutically acceptable salt thereof according to any one of claims 1 to 7, or the isotope substitute according to claim 8, or the pharmaceutical composition according to claim 9 for use in preventing and/or treating cathepsin C-related disorder, preferably for use in preventing and/or treating asthma, obstructive pulmonary disease, bronchiectasis, ANCA-associated vasculitis, psoriasis, alpha 1-antitrypsin deficiency, lupus nephritis, diabetes, inflammatory bowel disease or rheumatoid arthritis.

11. A compound of the following formula 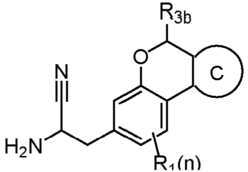 wherein, R₁, n, ring C and R_{3b} are as defined in claim 1.

12. A compound according to claim 11, wherein the compound is of the following formula 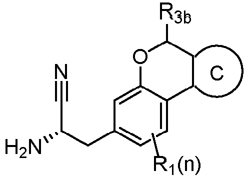 wherein, R₁, n, C and R_{3b} are as defined in claim 1.

## Patentansprüche

1. Verbindung der Formel VI-a oder pharmazeutisch annehmbares Salz davon, 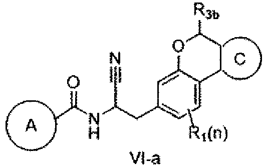
wobei Ring C Phenyl ist und das Phenyl gegebenenfalls mit einem bis drei R_{3b} substituiert ist;
Ring A ausgewählt ist aus der Gruppe bestehend aus 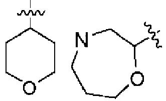 und 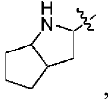 gegebenenfalls substituiert mit einem oder mehreren Substituenten ausgewählt aus der Gruppe bestehend aus Deuterium, Halogen, Hydroxy, Cyano, Amino, Nitro, Oxo, C₁₋₆-Alkyl und C₁₋₆-Alkoxy; wobei das C₁₋₆-Alkyl und C₁₋₆-Alkoxy jeweils unabhängig gegebenenfalls substituiert sind einem bis drei R₃ₐ;
vorzugsweise Ring A 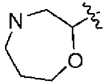 ist, gegebenenfalls substituiert mit einem oder mehreren Substituenten ausgewählt aus der Gruppe bestehend aus Deuterium, Halogen, Hydroxy, Cyano, Amino, Nitro, Oxo, C₁₋₆-Alkyl und C₁₋₆-Alkoxy; wobei das C₁₋₆-Alkyl und C₁₋₆-Alkoxy jeweils unabhängig gegebenenfalls substituiert sind mit einem bis drei R₃ₐ;
jedes R₃ₐ unabhängig ausgewählt ist aus der Gruppe bestehend aus Wasserstoff, Halogen, Deuterium, Hydroxy, Oxo, Nitro, Cyano, Amino, Acyl, Amido, C₁₋₆-Alkyl, C₁₋₆-Alkoxy, C₂₋₆-Alkenyloxy, C₂₋₆-Alkinyloxy, C₃₋₆-Cycloalkyl, 3- bis 6-gliedrigem Heterocycloalkyl, C₃₋₆-Cycloalkoxy, 3- bis 6-gliedrigem Heterocycloalkoxy, C₃₋₈-Cycloalkenyloxy, 5- bis 6-gliedrigem Aryl und 3- bis 6-gliedrigem Heteroaryl, und das C₁₋₆-Alkyl, C₁₋₆-Alkoxy, C₂₋₆-Alkenyloxy, C₂₋₆-Alkinyloxy, C₃₋₆-Cycloalkyl, 3- bis 6-gliedrige Heterocycloalkyl, C₃₋₆-Cycloalkoxy, 3- bis 6-gliedrige Heterocycloalkoxy, C₃₋₈-Cycloalkenyloxy, 5- bis 6-gliedrige Aryl und 3- bis 6-gliedrige Heteroaryl jeweils unabhängig gegebenenfalls substituiert sind mit einem oder mehreren Substituenten ausgewählt aus der Gruppe bestehend aus Halogen, Deuterium, Hydroxy, Oxo, Nitro und Cyano;
jedes R_{3b} unabhängig ausgewählt ist aus der Gruppe bestehend aus Wasserstoff, Halogen, Deuterium, Hydroxy, Oxo, Nitro, Cyano, Amino, Acyl, Amido, C₁₋₆-Alkyl, C₁₋₆-Alkoxy, C₂₋₆-Alkenyloxy, C₂₋₆-Alkinyloxy, C₃₋₆-Cycloalkyl, 3- bis 6-gliedrigem Heterocycloalkyl, C₃₋₆-Cycloalkoxy, 3- bis 6-gliedrigem Heterocycloalkoxy, C₃₋₈-Cycloalkenyloxy, 5- bis 6-gliedrigem Aryl, 3-bis 6-gliedrigem Heteroaryl, Methansulfonyl und 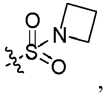 und das C₁₋₆-Alkyl, C₁₋₆-Alkoxy, C₂₋₆-Alkenyloxy, C₂₋₆-Alkinyloxy, C₃₋₆-Cycloalkyl, 3- bis 6-gliedrige Heterocycloalkyl, C₃₋₆-Cycloalkoxy, 3- bis 6-gliedrige Heterocycloalkoxy, C₃₋₈-Cycloalkenyloxy, 5- bis 6-gliedrige Aryl und 3- bis 6-gliedrige Heteroaryl jeweils unabhängig gegebenenfalls substituiert sind mit einem oder mehreren Substituenten ausgewählt aus der Gruppe bestehend aus Halogen, Deuterium, Hydroxy, Oxo, Nitro und Cyano;
jedes R₁ unabhängig ausgewählt ist aus der Gruppe bestehend aus Halogen, Hydroxy, Oxo, Nitro, Cyano, Alkyl, Cycloalkyl, Amino, Amido, Acyl, Alkoxy, Alkenyloxy, Alkinyloxy und Cycloalkoxy; und
n eine ganze Zahl ausgewählt aus der Gruppe bestehend aus 0 bis 3 ist; vorzugsweise n eine ganze Zahl ausgewählt aus der Gruppe bestehend aus 1 bis 3 ist, oder n eine ganze Zahl ausgewählt aus der Gruppe bestehend aus 0 bis 2 ist; und bevorzugter n eine ganze Zahl ausgewählt aus der Gruppe bestehend aus 1 bis 2 ist;
das Heteroatom ausgewählt ist aus der Gruppe bestehend aus Stickstoffatom, Sauerstoffatom und Schwefelatom, vorzugsweise Stickstoffatom oder Sauerstoffatom.

2. Verbindung oder pharmazeutisch annehmbares Salz davon nach Anspruch 1, wobei Ring A 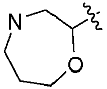 ist, gegebenenfalls substituiert mit einem oder mehreren Substituenten ausgewählt aus der Gruppe bestehend aus Deuterium, Halogen, Hydroxy, Cyano, Amino und Nitro;
vorzugsweise Ring A 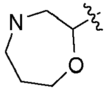 ist, gegebenenfalls substituiert mit einem oder mehreren Substituenten ausgewählt aus der Gruppe bestehend aus Deuterium und Halogen;
und bevorzugter Ring A 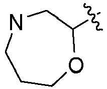 ist, gegebenenfalls substituiert mit einem oder mehreren Halogenen;
und höchst bevorzugt Ring A 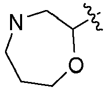 ist.

3. Verbindung oder pharmazeutisch annehmbares Salz davon nach Anspruch 1, wobei die Verbindung der Formel VI-a ausgewählt ist aus der Gruppe bestehend aus 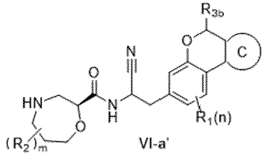 wobei:
jedes R₂ unabhängig ausgewählt ist aus der Gruppe bestehend aus Halogen, Nitro, Cyano, Amino, Oxo und Hydroxy;
m eine ganze Zahl ausgewählt aus der Gruppe bestehend aus 0 bis 3 ist und vorzugsweise m 0 ist; und
R₁, n, Ring C und R_{3b} wie in Anspruch 1 definiert sind;
insbesondere
die Verbindung der Formel VI-a ausgewählt ist aus der Gruppe bestehend aus 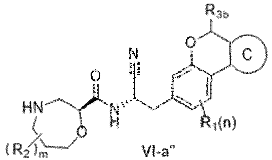
wobei:
jedes R₂ unabhängig ausgewählt ist aus der Gruppe bestehend aus Halogen, Nitro, Cyano, Amino, Oxo und Hydroxy;
m eine ganze Zahl ausgewählt aus der Gruppe bestehend aus 0 bis 3 ist und vorzugsweise m 0 ist; und
R₁, n, Ring C und R_{3b} wie in Anspruch 1 definiert sind.

4. Verbindung oder pharmazeutisch annehmbares Salz davon nach einem der Ansprüche 1 bis 3, wobei R₁ ausgewählt ist aus der Gruppe bestehend aus Halogen, Hydroxy, Cyano, Amino, Nitro, Acyl, Amido, C₁₋₆-Alkyl, C₃₋₆-Cycloalkyl und C₁₋₆-Alkoxy; vorzugsweise R₁ ausgewählt ist aus der Gruppe bestehend aus Halogen, Hydroxy, Cyano, Amino, Nitro, C₁₋₆-Alkyl, C₃₋₆-Cycloalkyl und C₁₋₆-Alkoxy;
bevorzugter R₁ ausgewählt ist aus der Gruppe bestehend aus Halogen, Hydroxy, C₁₋₆ -Alkyl, C₃₋₆-Cycloalkyl, Nitro und Cyano;
noch bevorzugter R₁ Halogen ist;
und sogar noch bevorzugter R₁ Fluor ist.

5. Verbindung oder pharmazeutisch annehmbares Salz davon nach einem der Ansprüche 1, 3 bis 4, wobei jedes R₃ₐ unabhängig ausgewählt ist aus der Gruppe bestehend aus Wasserstoff, Halogen, Deuterium, Hydroxy, Oxo, Nitro, Cyano und Amino;
oder
jedes R₃ₐ unabhängig ausgewählt ist aus der Gruppe bestehend aus Wasserstoff, Fluor, Chlor, Deuterium, Oxo (=O), Hydroxy, Amino, Methoxy, Cyclopropoxy, Cyclopropyl, Cyclopentyl, Pyridinyl, Piperidinyl und Phenyl, und vorzugsweise R₃ₐ Wasserstoff oder Amino ist.

6. Verbindung oder pharmazeutisch annehmbares Salz davon nach einem der Ansprüche 1 bis 5, wobei
jedes R_{3b} unabhängig ausgewählt ist aus der Gruppe bestehend aus Wasserstoff, Halogen, Deuterium, Hydroxy, Oxo, Nitro, Cyano, Amino, Acyl, Amido, C₁₋₆-Alkyl, C₁₋₆-Alkoxy, C₂₋₆-Alkenyloxy, C₂₋₆-Alkinyloxy, C₃₋₆-Cycloalkyl, 3- bis 6-gliedrigem Heterocycloalkyl, C₃₋₆-Cycloalkoxy, 3- bis 6-gliedrigem Heterocycloalkoxy, C₃₋₈-Cycloalkenyloxy, 5- bis 6-gliedrigem Aryl und 3- bis 6-gliedrigem Heteroaryl, und das C₁₋₆-Alkyl, C₁₋₆-Alkoxy, C₂₋₆-Alkenyloxy, C₂₋₆-Alkinyloxy, C₃₋₆-Cycloalkyl, 3- bis 6-gliedrige Heterocycloalkyl, C₃₋₆-Cycloalkoxy, 3- bis 6-gliedrige Heterocycloalkoxy, C₃₋₈-Cycloalkenyloxy, 5- bis 6-gliedrige Aryl und 3- bis 6-gliedrige Heteroaryl jeweils unabhängig gegebenenfalls substituiert sind mit einem oder mehreren Substituenten ausgewählt aus der Gruppe bestehend aus Halogen, Deuterium, Hydroxy, Oxo, Nitro und Cyano; und vorzugsweise jedes R_{3b} unabhängig ausgewählt ist aus der Gruppe bestehend aus Wasserstoff, Halogen, Deuterium, Hydroxy, Oxo, Nitro, Cyano und Amino;
oder
jedes R_{3b} unabhängig ausgewählt ist aus der Gruppe bestehend aus Wasserstoff, Halogen, Deuterium, Hydroxy, Oxo, Nitro, Cyano, Amino, Acyl und Amido; und vorzugsweise, jedes R_{3b} unabhängig Halogen oder Cyano ist;
oder
jedes R_{3b} unabhängig ein 3- bis 6-gliedriges Heterocycloalkyl ist und das 3-bis 6-gliedrige Heterocycloalkyl gegebenenfalls substituiert ist mit einem bis drei Substituenten ausgewählt aus der Gruppe bestehend aus Halogen, Cyano und Hydroxy;
oder
jedes R_{3b} unabhängig ausgewählt ist aus der Gruppe bestehend aus Wasserstoff, Halogen, Deuterium, Cyano, 3- bis 6-gliedrigem Heterocycloalkyl, Methansulfonyl und 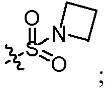 und vorzugsweise das 3- bis 6-gliedrige Heterocycloalkyl ausgewählt ist aus der Gruppe bestehend aus 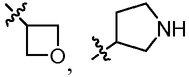 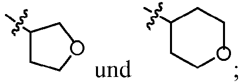 oder
jedes R_{3b} unabhängig ausgewählt ist aus der Gruppe bestehend aus Wasserstoff, Halogen, Deuterium, Hydroxy, Oxo, Nitro, Cyano, Amino, Amido, Acetyl, Methansulfonyl, 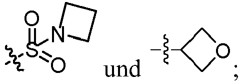 oder
jedes R_{3b} unabhängig ausgewählt ist aus der Gruppe bestehend aus Cyano, Methansulfonyl und 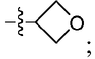 und vorzugsweise R_{3b} Cyano ist;
oder
jedes R_{3b} unabhängig ein C₁₋₆-Alkyl oder C₁₋₆-Alkoxy ist und das C₁₋₆-Alkyl oder C₁₋₆-Alkoxy gegebenenfalls substituiert ist mit einem oder mehreren Substituenten ausgewählt aus der Gruppe bestehend aus Halogen, Deuterium, Hydroxy, Oxo, Nitro und Cyano;
oder
jedes R_{3b} unabhängig ausgewählt ist aus der Gruppe bestehend aus C₁₋₆-Alkoxy, C₃₋₆-Cycloalkoxy, 3- bis 6-gliedrigem Heterocycloalkoxy, Phenyl und 5-bis 6-gliedrigem Heteroaryl, und das C₁₋₆-Alkoxy, C₃₋₆-Cycloalkoxy, 3- bis 6-gliedrige Heterocycloalkoxy, Phenyl und 5- bis 6-gliedrige Heteroaryl jeweils unabhängig gegebenenfalls substituiert sind mit einem bis drei Substituenten ausgewählt aus der Gruppe bestehend aus Fluor, Hydroxy, Deuterium, Nitro und Cyano;
oder
jedes R_{3b} unabhängig ausgewählt ist aus der Gruppe bestehend aus Wasserstoff, Fluor, Chlor, Deuterium, Oxo (=O), Hydroxy, Amino, Methoxy, Cyclopropoxy, Cyclopropyl, Cyclopentyl, Pyridinyl, Piperidinyl und Phenyl, und vorzugsweise R_{3b} ausgewählt ist aus der Gruppe bestehend aus Wasserstoff, Methyl, Oxo, Fluor und Chlor.

7. Verbindung oder pharmazeutisch annehmbares Salz davon nach Anspruch 1, wobei die Verbindung der Formel VI-a ausgewählt ist aus der Gruppe bestehend aus 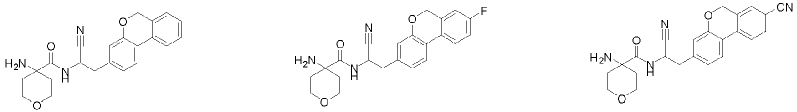 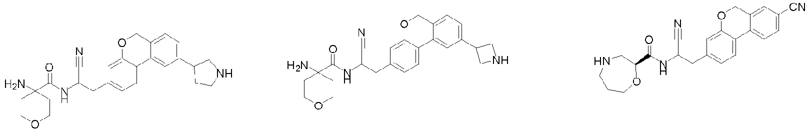 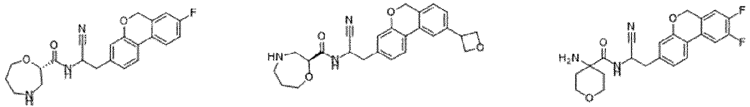 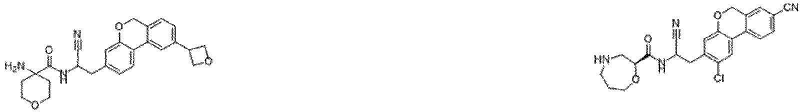 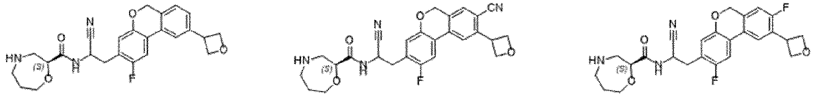 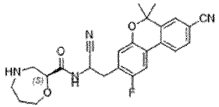 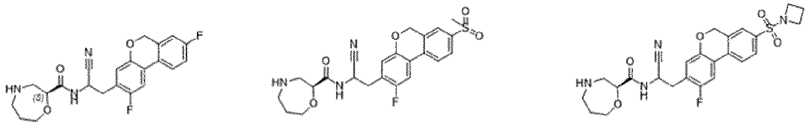 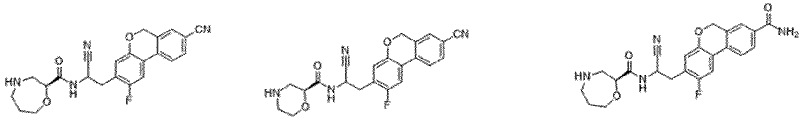 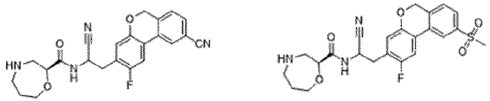 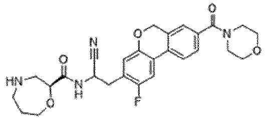 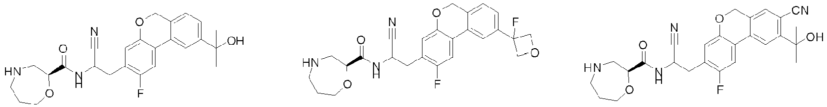 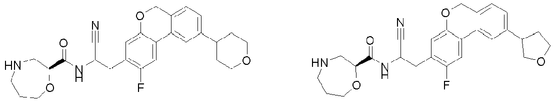 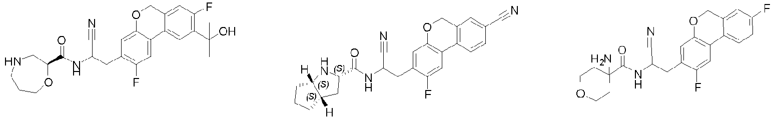 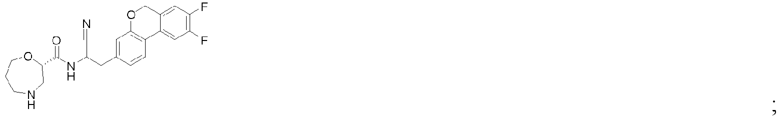 vorzugsweise

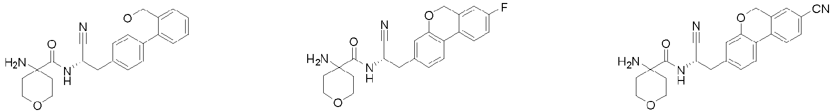

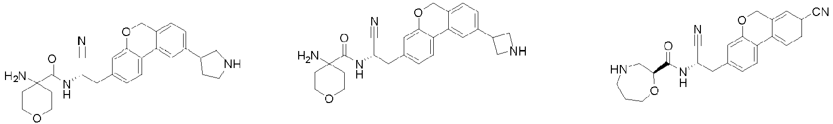

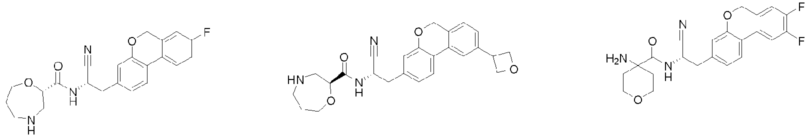

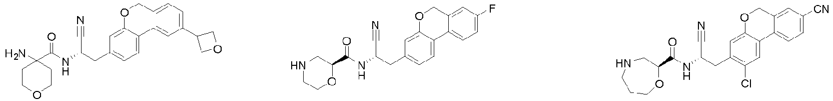

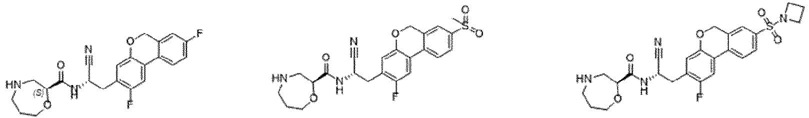

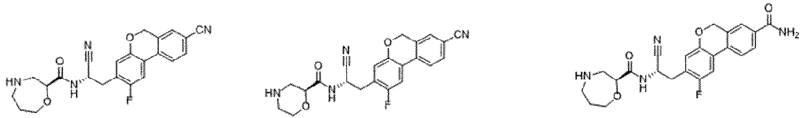

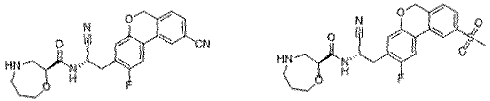

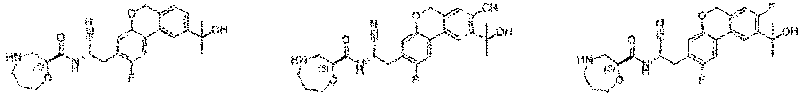

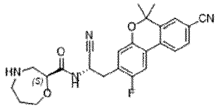

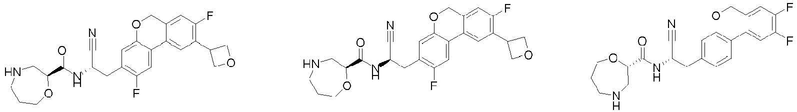

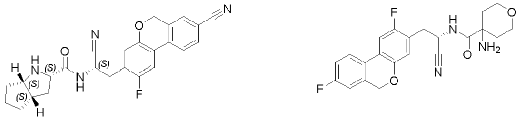

8. Isotopensubstitut der Verbindung oder des pharmazeutisch annehmbaren Salzes davon nach einem der Ansprüche 1 bis 7, wobei das Isotopensubstitut ein Deuteriumatom-Substitut ist.

9. Pharmazeutische Zusammensetzung, umfassend eine therapeutisch wirksame Menge mindestens einer Verbindung oder eines pharmazeutisch verträglichen Salzes davon nach einem der Ansprüche 1 bis 7 oder des Isotopensubstitut nach Anspruch 8 und einen pharmazeutisch annehmbaren Hilfsstoff.

10. Verbindung oder pharmazeutisch annehmbares Salz davon nach einem der Ansprüche 1 bis 7 oder Isotopensubstitut nach Anspruch 8 oder pharmazeutische Zusammensetzung nach Anspruch 9 zur Verwendung bei der Prävention und/oder Behandlung von Cathepsin-C-assoziierter Störung, vorzugsweise zur Verwendung bei der Prävention und/oder Behandlung von Asthma, obstruktiver Lungenerkrankung, Bronchiektasie, ANCA-assoziierter Vaskulitis, Psoriasis, alpha-1-Antitrypsinmangel, Lupus Nephritis, Diabetes, entzündlicher Darmerkrankung oder rheumatoider Arthritis.

11. Verbindung der folgenden Formel 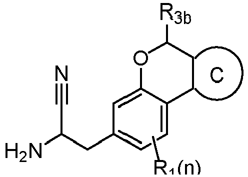 wobei R₁, n, Ring C und R_{3b} wie in Anspruch 1 definiert sind.

12. Verbindung nach Anspruch 11, wobei die Verbindung die folgende Formel aufweist 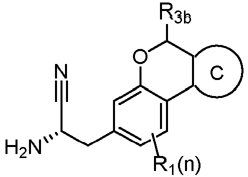 wobei R₁, n, C und R_{3b} wie in Anspruch 1 definiert sind.

## Revendications

1. Composé de formule VI-a ou sel pharmaceutiquement acceptable correspondant, 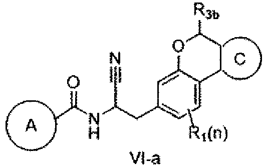
le cycle C étant phényle, et le phényle étant éventuellement substitué par un à trois R_{3b} ;
le cycle A étant choisi dans le groupe constitué par 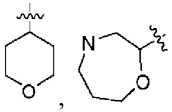 et 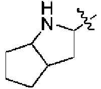 éventuellement substitué par un ou plusieurs substituants choisis dans le groupe constitué par deutérium, halogène, hydroxy, cyano, amino, nitro, oxo, C₁₋₆ alkyle et C₁₋₆ alcoxy ; les C₁₋₆ alkyle et C₁₋₆ alcoxy étant chacun indépendamment éventuellement substitués par un à trois R₃ₐ ;
préférablement, le cycle A étant 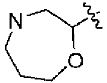
éventuellement substitué par un ou plusieurs substituants choisis dans le groupe constitué par deutérium, halogène, hydroxy, cyano, amino, nitro, oxo, C₁₋₆ alkyle et C₁₋₆ alcoxy ; les C₁₋₆ alkyle et C₁₋₆ alcoxy étant chacun indépendamment éventuellement substitués par un à trois R₃ₐ ;
chaque R₃ₐ étant indépendamment choisi dans le groupe constitué par hydrogène, halogène, deutérium, hydroxy, oxo, nitro, cyano, amino, acyle, amido, C₁₋₆ alkyle, C₁₋₆ alcoxy, C₂₋₆ alcényloxy, C₂₋₆ alcynyloxy, C₃₋₆ cycloalkyle, hétérocycloalkyle à 3 à 6 chaînons, C₃₋₆ cycloalcoxy, hétérocycloalcoxy à 3 à 6 chaînons, C₃₋₈ cycloalcényloxy, aryle à 5 à 6 chaînons et hétéroaryle à 3 à 6 chaînons, et les C₁₋₆ alkyle, C₁₋₆ alcoxy, C₂₋₆ alcényloxy, C₂₋₆ alcynyloxy, C₃₋₆ cycloalkyle, hétérocycloalkyle à 3 à 6 chaînons, C₃₋₆ cycloalcoxy, hétérocycloalcoxy à 3 à 6 chaînons, C₃₋₈ cycloalcényloxy, aryle à 5 à 6 chaînons et hétéroaryle à 3 à 6 chaînons étant chacun indépendamment éventuellement substitués par un ou plusieurs substituants choisis dans le groupe constitué par halogène, deutérium, hydroxy, oxo, nitro et cyano ;
chaque R_{3b} étant indépendamment choisi dans le groupe constitué par hydrogène, halogène, deutérium, hydroxy, oxo, nitro, cyano, amino, acyle, amido, C₁₋₆ alkyle, C₁₋₆ alcoxy, C₂₋₆ alcényloxy, C₂₋₆ alcynyloxy, C₃₋₆ cycloalkyle, hétérocycloalkyle à 3 à 6 chaînons, C₃₋₆ cycloalcoxy, hétérocycloalcoxy à 3 à 6 chaînons, C₃₋₈ cycloalcényloxy, aryle à 5 à 6 chaînons, hétéroaryle à 3 à 6 chaînons, méthanesulfonyle et 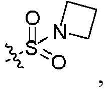 et les C₁₋₆ alkyle, C₁₋₆ alcoxy, C₂₋₆ alcényloxy, C₂₋₆ alcynyloxy, C₃₋₆ cycloalkyle, hétérocycloalkyle à 3 à 6 chaînons, C₃₋₆ cycloalcoxy, hétérocycloalcoxy à 3 à 6 chaînons, C₃₋₈ cycloalcényloxy, aryle à 5 à 6 chaînons et hétéroaryle à 3 à 6 chaînons étant chacun indépendamment éventuellement substitués par un ou plusieurs substituants choisis dans le groupe constitué par halogène, deutérium, hydroxy, oxo, nitro et cyano ;
chaque R₁ étant indépendamment choisi dans le groupe constitué par halogène, hydroxy, oxo, nitro, cyano, alkyle, cycloalkyle, amino, amido, acyle, alcoxy, alcényloxy, alcynyloxy et cycloalcoxy ; et
n étant un entier choisi dans le groupe constitué par 0 à 3 ; préférablement, n étant un entier choisi dans le groupe constitué par 1 à 3 ou n étant un entier choisi dans le groupe constitué par 0 à 2 ; et plus préférablement, n étant un entier choisi dans le groupe constitué par 1 à 2 ;
l'hétéroatome étant choisi dans le groupe constitué par un atome d'azote, un atome d'oxygène et un atome de soufre, et préférablement un atome d'azote ou un atome d'oxygène.

2. Composé ou sel pharmaceutiquement acceptable correspondant selon la revendication 1, le cycle A étant 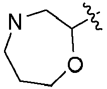 éventuellement substitué par un ou plusieurs substituants choisis dans le groupe constitué par deutérium, halogène, hydroxy, cyano, amino et nitro ;
préférablement, le cycle A étant 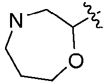 éventuellement substitué par un ou plusieurs substituants choisis dans le groupe constitué par deutérium et halogène ;
et plus préférablement, le cycle A étant 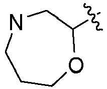 éventuellement substitué par un ou plusieurs halogènes ;
et le plus préférablement, le cycle A étant

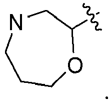

3. Composé ou sel pharmaceutiquement acceptable correspondant selon la revendication 1, le composé de formule VI-a étant choisi dans le groupe constitué par 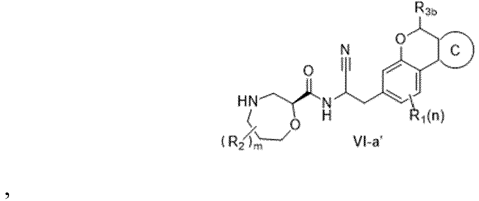
chaque R₂ étant indépendamment choisi dans le groupe constitué par halogène, nitro, cyano, amino, oxo et hydroxy ;
m étant un entier choisi dans le groupe constitué par 0 à 3, et préférablement, m étant 0 ; et
R₁, n, le cycle C et R_{3b} étant tels que définis dans la revendication 1 ;
en particulier,
le composé de formule VI-a étant choisi dans le groupe constitué par 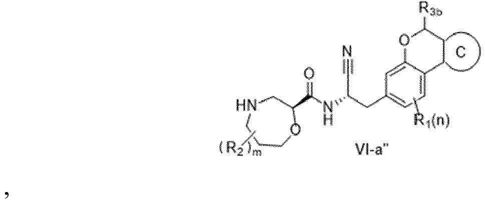
chaque R₂ étant indépendamment choisi dans le groupe constitué par halogène, nitro, cyano, amino, oxo et hydroxy ;
m étant un entier choisi dans le groupe constitué par 0 à 3, et préférablement, m étant 0 ; et
R₁, n, le cycle C et R_{3b} étant tels que définis dans la revendication 1.

4. Composé ou sel pharmaceutiquement acceptable correspondant selon l'une quelconque des revendications 1 à 3, R₁ étant choisi dans le groupe constitué par halogène, hydroxy, cyano, amino, nitro, acyle, amido, C₁₋₆ alkyle, C₃₋₆ cycloalkyle et C₁₋₆ alcoxy ; préférablement, R₁ étant choisi dans le groupe constitué par halogène, hydroxy, cyano, amino, nitro, C₁₋₆ alkyle, C₃₋₆ cycloalkyle et C₁₋₆ alcoxy ;
plus préférablement, R₁ étant choisi dans le groupe constitué par halogène, hydroxy, C₁₋₆ alkyle, C₃₋₆ cycloalkyle, nitro et cyano ;
encore plus préférablement, R₁ étant halogène ;
et même encore plus préférablement, R₁ étant fluor.

5. Composé ou sel pharmaceutiquement acceptable correspondant selon l'une quelconque des revendications 1, 3 à 4, chaque R₃ₐ étant indépendamment choisi dans le groupe constitué par hydrogène, halogène, deutérium, hydroxy, oxo, nitro, cyano et amino ;
ou
chaque R₃ₐ étant indépendamment choisi dans le groupe constitué par hydrogène, fluor, chlore, deutérium, oxo (=O), hydroxy, amino, méthoxy, cyclopropoxy, cyclopropyle, cyclopentyle, pyridinyle, pipéridinyle et phényle, et préférablement, R₃ₐ étant hydrogène ou amino.

6. Composé ou sel pharmaceutiquement acceptable correspondant selon l'une quelconque des revendications 1 à 5,
chaque R_{3b} étant indépendamment choisi dans le groupe constitué par hydrogène, halogène, deutérium, hydroxy, oxo, nitro, cyano, amino, acyle, amido, C₁₋₆ alkyle, C₁₋₆ alcoxy, C₂₋₆ alcényloxy, C₂₋₆ alcynyloxy, C₃₋₆ cycloalkyle, hétérocycloalkyle à 3 à 6 chaînons, C₃₋₆ cycloalcoxy, hétérocycloalcoxy à 3 à 6 chaînons, C₃₋₈ cycloalcényloxy, aryle à 5 à 6 chaînons et hétéroaryle à 3 à 6 chaînons, et les C₁₋₆ alkyle, C₁₋₆ alcoxy, C₂₋₆ alcényloxy, C₂₋₆ alcynyloxy, C₃₋₆ cycloalkyle, hétérocycloalkyle à 3 à 6 chaînons, C₃₋₆ cycloalcoxy, hétérocycloalcoxy à 3 à 6 chaînons, C₃₋₈ cycloalcényloxy, aryle à 5 à 6 chaînons et hétéroaryle à 3 à 6 chaînons étant chacun indépendamment éventuellement substitués par un ou plusieurs substituants choisis dans le groupe constitué par halogène, deutérium, hydroxy, oxo, nitro et cyano ; et préférablement, chaque R_{3b} étant indépendamment choisi dans le groupe constitué par hydrogène, halogène, deutérium, hydroxy, oxo, nitro, cyano et amino ;
ou
chaque R_{3b} étant indépendamment choisi dans le groupe constitué par hydrogène, halogène, deutérium, hydroxy, oxo, nitro, cyano, amino, acyle et amido ; et préférablement, chaque R_{3b} étant indépendamment halogène ou cyano ;
ou
chaque R_{3b} étant indépendamment un hétérocycloalkyle à 3 à 6 chaînons, et l'hétérocycloalkyle à 3 à 6 chaînons étant éventuellement substitué par un à trois substituants choisis dans le groupe constitué par halogène, cyano et hydroxy ;
ou
chaque R_{3b} étant indépendamment choisi dans le groupe constitué par hydrogène, halogène, deutérium, cyano, hétérocycloalkyle à 3 à 6 chaînons, méthanesulfonyle et 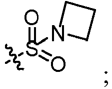 et préférablement, l'hétérocycloalkyle à 3 à 6 chaînons étant choisi dans le groupe constitué par 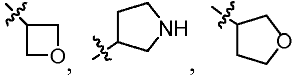 et 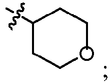 ou
chaque R_{3b} étant indépendamment choisi dans le groupe constitué par hydrogène, halogène, deutérium, hydroxy, oxo, nitro, cyano, amino, amido, acétyle, méthanesulfonyle, 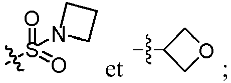 ou
chaque R_{3b} étant indépendamment choisi dans le groupe constitué par cyano, méthanesulfonyle et 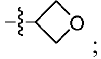 et préférablement, R_{3b} étant cyano ;
ou
chaque R_{3b} étant indépendamment a C₁₋₆ alkyle ou C₁₋₆ alcoxy, et le C₁₋₆ alkyle ou C₁₋₆ alcoxy étant éventuellement substitué par un ou plusieurs substituants choisis dans le groupe constitué par halogène, deutérium, hydroxy, oxo, nitro et cyano ;
ou
chaque R_{3b} étant indépendamment choisi dans le groupe constitué par C₁₋₆ alcoxy, C₃₋₆ cycloalcoxy, hétérocycloalcoxy à 3 à 6 chaînons, phényle et hétéroaryle à 5 à 6 chaînons, et les C₁₋₆ alcoxy, C₃₋₆ cycloalcoxy, hétérocycloalcoxy à 3 à 6 chaînons, phényle et hétéroaryle à 5 à 6 chaînons étant chacun indépendamment éventuellement substitués par un à trois substituants choisis dans le groupe constitué par fluor, chlore, deutérium, hydroxy, oxo, nitro et cyano ;
ou
chaque R_{3b} étant indépendamment choisi dans le groupe constitué par hydrogène, fluor, chlore, deutérium, oxo (=O), hydroxy, amino, méthoxy, cyclopropoxy, cyclopropyle, cyclopentyle, pyridinyle, pipéridinyle et phényle, et préférablement, R_{3b} étant choisi dans le groupe constitué par hydrogène, méthyle, oxo, fluor et chlore.

7. Composé ou sel pharmaceutiquement acceptable correspondant selon la revendication 1, le composé de formule VI-a étant choisi dans le groupe constitué par 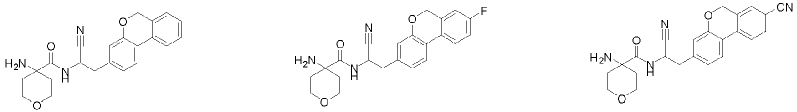 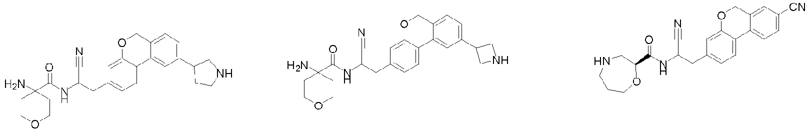 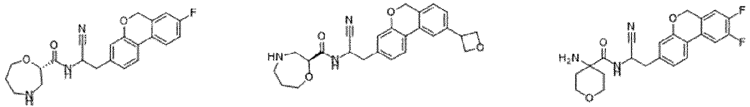 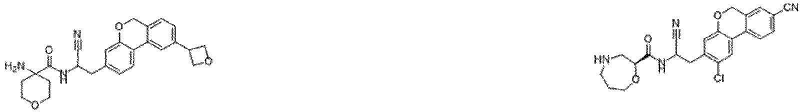 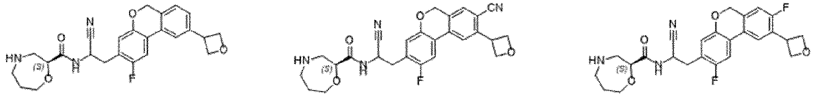 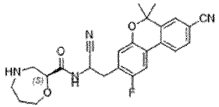 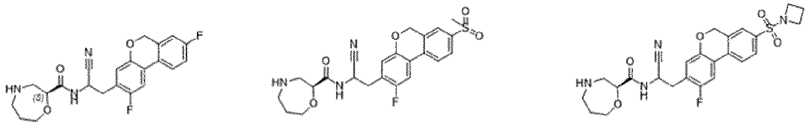 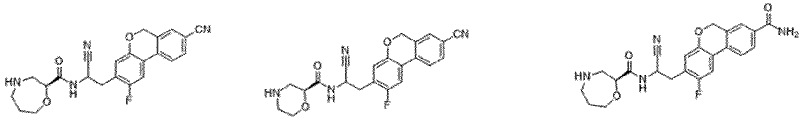 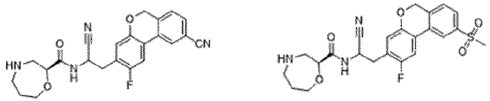 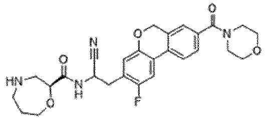 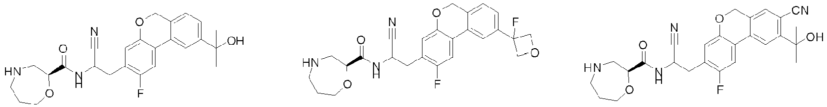 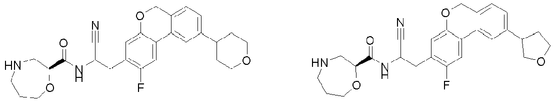 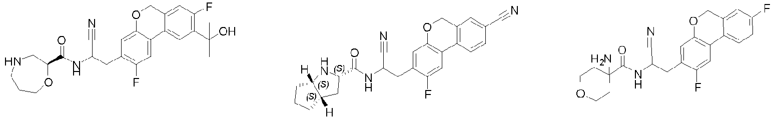 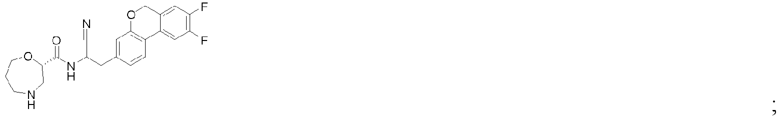 préférablement

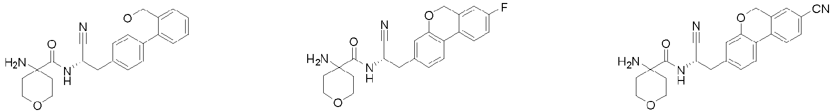

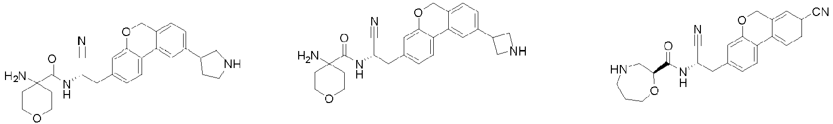

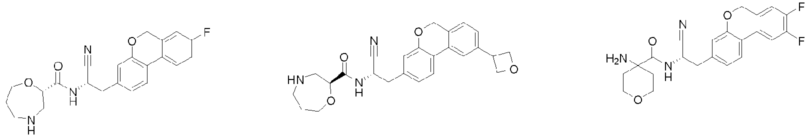

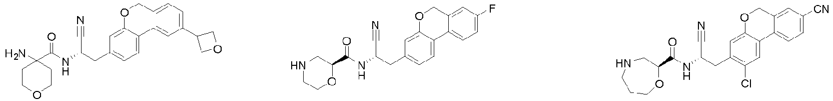

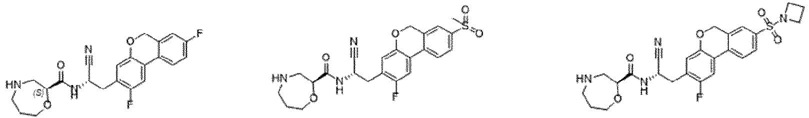

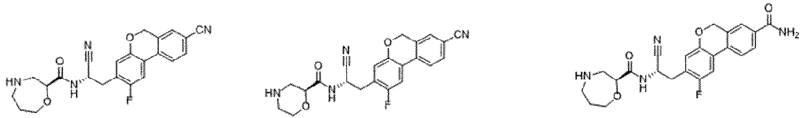

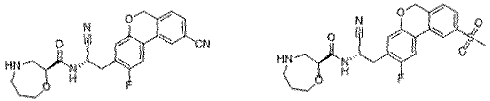

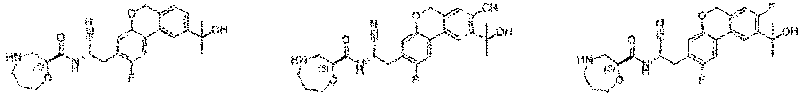

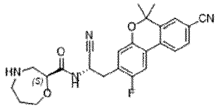

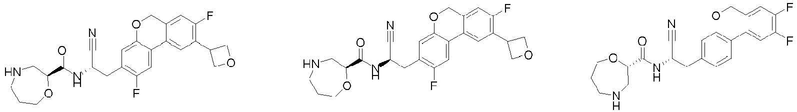

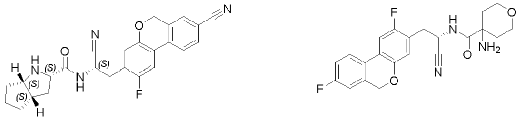

8. Substitut isotopique du composé ou de son sel pharmaceutiquement acceptable selon l'une quelconque des revendications 1 à 7, et de préférence, le substitut isotopique étant un substitut par un atome de deutérium.

9. Composition pharmaceutique, comprenant une quantité thérapeutiquement efficace d'au moins un composé ou d'un sel pharmaceutiquement acceptable de celui-ci selon l'une quelconque des revendications 1 à 7 ou du substitut isotopique selon la revendication 8, et un excipient pharmaceutiquement acceptable.

10. Composé ou sel pharmaceutiquement acceptable de celui-ci selon l'une quelconque des revendications 1 à 7, ou substitut isotopique selon la revendication 8, ou composition pharmaceutique selon la revendication 9 pour une utilisation dans la prévention et/ou le traitement d'un trouble lié à la cathepsine C, de préférence pour une utilisation dans la prévention et/ou le traitement de l'asthme, la maladie pulmonaire obstructive, la bronchectasie, la vascularite associée aux ANCA, le psoriasis, le déficit en alpha 1-antitrypsine, la néphrite lupique, le diabète, la maladie inflammatoire de l'intestin ou la polyarthrite rhumatoïde.

11. Composé de la formule suivante 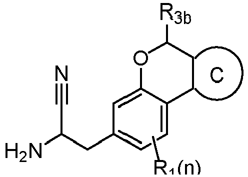 R₁, n, le cycle C et R_{3b} étant tels que définis dans la revendication 1.

12. Composé selon la revendication 11, le composé étant de la formule suivante 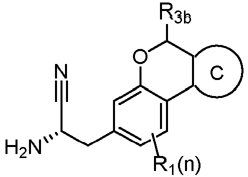 R₁, n, C et R_{3b} étant tels que définis dans la revendication 1.
